# EUROPEAN PATENT SPECIFICATION

(11) **EP 3 909 958 B1**
(45) Date of publication and mention of the grant of the patent: **27.12.2023**
(21) Application number: 20891826.8
(22) Date of filing: 27.11.2020
(51) Int. Cl.: H10K 85/30, H10K 85/60, H10K 99/00, H10K 101/00, C07F 5/02, C07F 7/08, C09K 11/06

(54) **COMPOUND AND ORGANIC LIGHT-EMITTING DEVICE COMPRISING SAME**
VERBINDUNG UND ORGANISCHE LICHTEMITTIERENDE VORRICHTUNG DAMIT
COMPOSÉ ET DISPOSITIF ÉLECTROLUMINESCENT ORGANIQUE LE COMPRENANT

(30) Priority: 29.11.2019 KR 20190156840; 20.05.2020 KR 20200060597
(43) Date of publication of application: 17.11.2021
(73) Proprietor: LG Chem, Ltd., Seoul 07336, (KR)
(72) Inventor: KIM, Seonwoo, Daejeon 34122 (KR); HONG, Wanpyo, Daejeon 34122 (KR); GEUM, Sujeong, Daejeon 34122 (KR); KIM, Moung Gon, Daejeon 34122 (KR); KIM, Kyunghee, Daejeon 34122 (KR); CHO, Hye Min, Daejeon 34122 (KR)
(74) Representative: Hoffmann Eitle
(86) International application number: PCT/KR2020/017069
(87) International publication number: WO 2021/107678

(56) References cited:
- EP-A1- 3 565 018
- WO-A1-2017/188111
- WO-A1-2018/186374
- KR-A- 20170 130 434
- KR-A- 20190 127 529
- KR-A- 20200 125 583

## Description

### [Technical Field]

This application claims priority to and the benefits of Korean Patent Application No. 10-2019-0156840, filed with the Korean Intellectual Property Office on November 29, 2019, and Korean Patent Application No. 10-2020-0060597, filed with the Korean Intellectual Property Office on May 20, 2020.

The present specification relates to a compound, and an organic light emitting device including the same.

### [Background Art]

An organic light emission phenomenon generally refers to a phenomenon converting electrical energy to light energy using an organic material. An organic light emitting device using an organic light emission phenomenon normally has a structure including an anode, a cathode, and an organic material layer therebetween. Herein, the organic material layer is often formed in a multilayer structure formed with different materials in order to increase efficiency and stability of the organic light emitting device, and for example, may be formed with a hole injection layer, a hole transfer layer, a light emitting layer, an electron transfer layer, an electron injection layer and the like. When a voltage is applied between the two electrodes in such an organic light emitting device structure, holes and electrons are injected to the organic material layer from the anode and the cathode, respectively, and when the injected holes and electrons meet, excitons are formed, and light emits when these excitons fall back to the ground state.

Development of new materials for such an organic light emitting device has been continuously required.

EP 3 565 018 A1 describes organic electroluminescent devices comprising a light-emitting layer B comprising two host materials, a n-type (electron-transporting) and a p-type (hole-transporting) host material, a thermally activated delayed fluorescence (TADF) material and an emitter material.

### [Disclosure]

### [Technical Problem]

The present specification is directed to providing a compound, and an organic light emitting device including the same.

### [Technical Solution]

One embodiment of the present specification provides a compound represented by the following Chemical Formula 1. 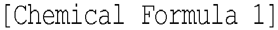 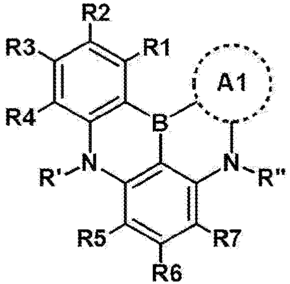 in Chemical Formula 1,
A1 is a monocyclic or polycyclic heteroring substituted or unsubstituted, and including a 5-membered ring including O or S,
R1 to R7, R' and R" are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring, and
wherein "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

Another embodiment of the present specification provides an organic light emitting device including a first electrode; a second electrode provided opposite to the first electrode; and one or more organic material layers provided between the first electrode and the second electrode, wherein one or more layers of the organic material layers include the compound.

### [Advantageous Effects]

A compound described in the present specification can be used as a material of an organic material layer of an organic light emitting device. A compound according to another embodiment is capable of enhancing efficiency, lowering a driving voltage and/or enhancing lifetime properties in an organic light emitting device. Particularly, a compound described in the present specification can be used as a hole injection, hole transfer, hole injection and hole transfer, electron blocking, light emitting, hole blocking, electron transfer or electron injection material. In addition, an organic light emitting device according to one embodiment of the present specification is effective in obtaining low driving voltage, high efficiency or long lifetime.

### [Description of Drawings]

FIG. 1 to FIG. 3 each illustrate an example of an organic light emitting device according to one embodiment of the present specification.
FIG. 4 is a diagram showing a photoluminescence analysis graph according to Experimental Example 1 of the present specification.
FIG. 5 is a diagram showing a thermos gravimetric analysis graph of Compound 19 according to Experimental Example 3 of the present specification.
FIG. 6 is a diagram showing a thermos gravimetric analysis graph of Compound BD-A according to Experimental Example 3 of the present specification.
FIG. 7 is a diagram showing a thermos gravimetric analysis graph of Compound BD-C according to Experimental Example 3 of the present specification.

### [Reference Numeral]

1: Substrate
2: First Electrode
3: Light Emitting Layer
4: Second Electrode
5: Hole Injection Layer
6: Hole Blocking Layer
7: Electron Injection and Transfer Layer
8: Hole Transfer Layer
9: Electron Blocking Layer
10: First Electron Transfer Layer
11: Second Electron Transfer Layer
12: Electron Injection Layer

### [Best Mode]

Hereinafter, the present specification will be described in more detail.

Existing boron compounds have a full width at half maximum of approximately 23 nm to 30 nm and the basic core structure has a wavelength of approximately 453 nm, but have a limit of a reduced lifetime since material stability is relatively inferior compared to amine compounds. Accordingly, methods of securing a long lifetime by, through controlling substituents of a boron compound, increasing material stability while maintaining excellent optical properties have been required.

A compound of Chemical Formula 1 according to one embodiment of the present specification has a structure in which A1 includes a monocyclic or polycyclic heteroring substituted or unsubstituted and including a 5-membered ring including O or S, and structural stability and excellent electrochemical properties of the compound may be secured using sufficient electrons.

Throughout the specification of the present application, a term "combination thereof" included in a Markush-type expression means a mixture or a combination of one or more selected from the group consisting of constituents described in the Markush-type expression, and means including one or more selected from the group consisting of the constituents.

Examples of substituents in the present specification are described below, however, the substituents are not limited thereto.

In the present specification, 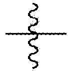 means a linking site.

The term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, the term "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

In the present specification, linking two or more substituents refers to linking hydrogen of any one substituent to another substituent. For example, linking two substituents may include a phenyl group and a naphthyl group being linked to become a substituent of 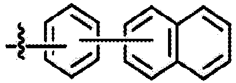 or 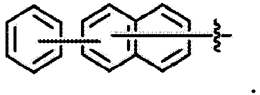 In addition, linking three substituents includes not only continuously linking (substituent 1)-(substituent 2)-(substituent 3), but also linking (substituent 2) and (substituent 3) to (substituent 1). For example, a phenyl group, a naphthyl group and an isopropyl group may be linked to become a substituent of 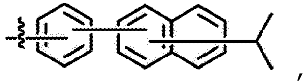 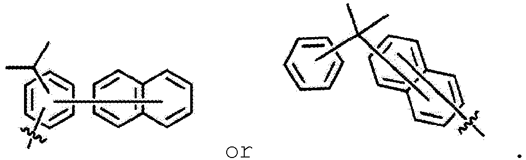 The same rule described above applies to cases of linking four or more substituents.

In the present specification, examples of the halogen group may include fluorine, chlorine, bromine or iodine.

In the present specification, the alkyl group may be linear or branched, and although not particularly limited thereto, the number of carbon atoms is preferably from 1 to 30. Specific examples thereof may include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl and the like, but are not limited thereto.

In the present specification, the cycloalkyl group is not particularly limited, but preferably has 3 to 30 carbon atoms. Specific examples thereof may include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, an adamantyl group, a bicyclo[2.2.1]octyl group, a norbornyl group and the like, but are not limited thereto.

In the present specification, the alkoxy group may be linear, branched or cyclic. The number of carbon atoms of the alkoxy group is not particularly limited, but is preferably from 1 to 30. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the alkenyl group may be linear or branched, and although not particularly limited thereto, the number of carbon atoms is preferably from 2 to 30. Specific examples thereof may include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group and the like, but are not limited thereto.

In the present specification, the haloalkyl group means, in the definition of the alkyl group, hydrogen of the alkyl group being substituted with at least one halogen group.

In the present specification, the haloalkoxy group means, in the definition of the alkoxy group, hydrogen of the alkoxy group being substituted with at least one halogen group.

In the present specification, the aryl group is not particularly limited, but preferably has 6 to 30 carbon atoms, and the aryl group may be monocyclic or polycyclic.

When the aryl group is a monocyclic aryl group, the number of carbon atoms is not particularly limited, but is preferably from 6 to 30. Specific examples of the monocyclic aryl group may include a phenyl group, a biphenyl group, a terphenyl group and the like, but are not limited thereto.

When the aryl group is a polycyclic aryl group, the number of carbon atoms is not particularly limited, but is preferably from 10 to 30. Specific examples of the polycyclic aryl group may include a naphthyl group, an anthracene group, a phenanthrene group, a triphenylene group, a pyrene group, a phenalene group, a perylene group, a chrysene group, a fluorene group and the like, but are not limited thereto.

In the present specification, the fluorene group may be substituted, and adjacent groups may bond to each other to form a ring.

When the fluorene group is substituted, 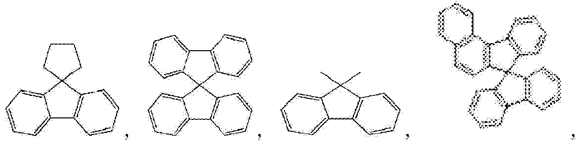 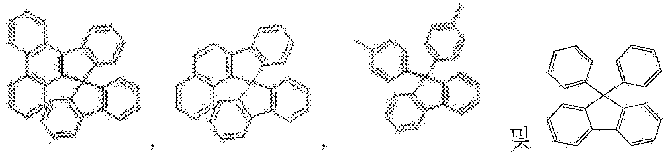 and the like may be included, however, the structure is not limited thereto.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In the present specification, the arylalkyl group means the alkyl group being substituted with an aryl group, and the examples of the aryl group and the alkyl group described above may be applied to the aryl group and the alkyl group of the arylalkyl group.

In the present specification, the aryloxy group means, in the definition of the alkoxy group, the alkyl group of the alkoxy group being substituted with an aryl group. Examples of the aryloxy group may include a phenoxy group, a p-tolyloxy group, an m-tolyloxy group, a 3,5-dimethyl-phenoxy group, a 2,4,6-trimethylphenoxy group, a p-tert-butylphenoxy group, a 3-biphenyloxy group, a 4-biphenyloxy group, a 1-naphthyloxy group, a 2-naphthyloxy group, a 4-methyl-1-naphthyloxy group, a 5-methyl-2-naphthyloxy group, a 1-anthryloxy group, a 2-anthryloxy group, a 9-anthryloxy group, a 1-phenanthryloxy group, a 3-phenanthryloxy group, a 9-phenanthryloxy group and the like, but are not limited thereto.

In the present specification, the alkyl group of the alkylthioxy group is the same as the examples of the alkyl group described above. Specific examples of the alkylthioxy group may include a methylthioxy group, an ethylthioxy group, a tert-butylthioxy group, a hexylthioxy group, an octylthioxy group and the like, but are not limited thereto.

In the present specification, the aryl group in the arylthioxy group is the same as the examples of the aryl group described above. Specific examples of the arylthioxy group may include a phenylthioxy group, a 2-methylphenylthioxy group, a 4-tert-butylphenylthioxy group and the like, but are not limited thereto.

In the present specification, the heterocyclic group is a group including one or more atoms that are not carbon, that is, heteroatoms, and specifically, the heteroatom may include one or more atoms selected from the group consisting of O, N, Se, S and the like. The number of carbon atoms is not particularly limited, but is preferably from 2 to 30, and the heterocyclic group may be monocyclic or polycyclic. Examples of the heterocyclic group may include a thiophene group, a furan group, a pyrrole group, an imidazole group, a thiazole group, an oxazole group, an oxadiazole group, a pyridine group, a bipyridine group, a pyrimidine group, a triazine group, a triazole group, an acridine group, a pyridazine group, a pyrazine group, a quinoline group, a quinazoline group, a quinoxaline group, a phthalazine group, a pyridopyrimidine group, a pyridopyrazine group, a pyrazinopyrazine group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthridine group, a phenanthroline group, an isoxazole group, a thiadiazole group, a dibenzofuran group, dibenzosilole group, a phenoxanthine group, a phenoxazine group, a phenothiazine group, a dihydroindenocarbazole group, a spirofluorenexanthene group, a spirofluorenethioxanthene group, a tetrahydronaphthothiophene group, a tetrahydronaphthofuran group, a tetrahydrobenzothiophene group, a tetrahydrobenzofuran group and the like, but are not limited thereto.

In the present specification, the silyl group may be an alkylsilyl group, an arylsilyl group, an alkylarylsilyl group, a heteroarylsilyl group or the like. As the alkyl group in the alkylsilyl group, the examples of the alkyl group described above may be applied, and as the aryl group in the arylsilyl group, the examples of the aryl group described above may be applied. As the alkyl group and the aryl group in the alkylarylsilyl group, the examples of the alkyl group and the aryl group may be applied, and as the heteroaryl group in the heteroarylsilyl group, the examples of the heterocyclic group may be applied.

In the present specification, the boron group may be - BR₁₀₀R₁₀₁. R₁₀₀ and R₁₀₁ are the same as or different from each other, and may be each independently selected from the group consisting of hydrogen; deuterium; halogen; a nitrile group; a substituted or unsubstituted monocyclic or polycyclic cycloalkyl group having 3 to 30 carbon atoms; a substituted or unsubstituted linear or branched alkyl group having 1 to 30 carbon atoms; a substituted or unsubstituted monocyclic or polycyclic aryl group having 6 to 30 carbon atoms; and a substituted or unsubstituted monocyclic or polycyclic heterocyclic group having 2 to 30 carbon atoms. Specific examples of the boron group may include a trimethylboron group, a triethylboron group, a t-butyldimethylboron group, a triphenylboron group, a phenylboron group and the like, but are not limited thereto.

In the present specification, the amine group may be selected from the group consisting of -NH₂, an alkylamine group, an N-alkylarylamine group, an arylamine group, an N-arylheteroarylamine group, an N-alkylheteroarylamine group and a heteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, an anthracenylamine group, a 9-methyl-anthracenylamine group, a diphenylamine group, a ditolylamine group, an N-phenyltolylamine group, a triphenylamine group, an N-phenylbiphenylamine group, an N-phenylnaphthylamine group, an N-biphenylnaphthylamine group, an N-naphthylfluorenylamine group, an N-phenylphenanthrenylamine group, an N-biphenylphenanthrenylamine group, an N-phenylfluorenylamine group, an N-phenylterphenylamine group, an N-phenanthrenylfluorenylamine group, an N-biphenylfluorenylamine group and the like, but are not limited thereto.

In the present specification, the N-alkylarylamine group means an amine group in which N of the amine group is substituted with an alkyl group and an aryl group. The alkyl group and the aryl group in the N-alkylarylamine group are the same as the examples of the alkyl group and the aryl group described above.

In the present specification, the N-arylheteroarylamine group means an amine group in which N of the amine group is substituted with an aryl group and a heteroaryl group. The aryl group and the heteroaryl group in the N-arylheteroarylamine group are the same as the examples of the aryl group and the heterocyclic group described above.

In the present specification, the N-alkylheteroarylamine group means an amine group in which N of the amine group is substituted with an alkyl group and a heteroaryl group. The alkyl group and the heteroaryl group in the N-alkylheteroarylamine group are the same as the examples of the alkyl group and the heterocyclic group described above.

In the present specification, examples of the alkylamine group include a substituted or unsubstituted monoalkylamine group, or a substituted or unsubstituted dialkylamine group. The alkyl group in the alkylamine group may be a linear or branched alkyl group. The alkylamine group including two or more alkyl groups may include linear alkyl groups, branched alkyl groups, or both linear alkyl groups and branched alkyl groups. For example, the alkyl group in the alkylamine group may be selected from among the examples of the alkyl group described above.

In the present specification, examples of the heteroarylamine group include a substituted or unsubstituted monoheteroarylamine group, or a substituted or unsubstituted diheteroarylamine group. The heteroarylamine group including two or more heteroaryl groups may include monocyclic heteroaryl groups, polycyclic heteroaryl groups, or both monocyclic heteroaryl groups and polycyclic heteroaryl groups. For example, the heteroaryl group in the heteroarylamine group may be selected from among the examples of the heterocyclic group described above.

In the present specification, the meaning of "adjacent two of the substituents bonding to each other to form a ring" is adjacent groups bonding to each other to form a substituted or unsubstituted hydrocarbon ring; or a substituted or unsubstituted heteroring.

In the present specification, the "ring" in the substituted or unsubstituted ring formed by bonding to each other means a substituted or unsubstituted hydrocarbon ring; or a substituted or unsubstituted heteroring.

In the present specification, the hydrocarbon ring group may be an aromatic hydrocarbon ring, an aliphatic hydrocarbon ring, or a fused ring group of aromatic hydrocarbon and aliphatic hydrocarbon, and may be selected from among the examples of the cycloalkyl group, the aryl group, and a combination thereof. Examples of the hydrocarbon ring group may include a phenyl group, a cyclohexyl group, an adamantyl group, a tetrahydronaphthalene group, a tetrahydroanthracene group and the like, but are not limited thereto.

In the present specification, the hydrocarbon ring may be an aromatic hydrocarbon ring, an aliphatic hydrocarbon ring, or a fused ring group of aromatic hydrocarbon and aliphatic hydrocarbon, and may be selected from among the examples of the cycloalkyl group and the aryl group except for those that are not monovalent. Examples of the hydrocarbon ring may include benzene, cyclohexane, adamantane, tetrahydronaphthalene, tetrahydroanthracene and the like, but are not limited thereto.

In the present specification, the heteroring includes one or more atoms that are not carbon, that is, heteroatoms, and specifically, the heteroatom may include one or more atoms selected from the group consisting of O, N, Se, S and the like. The heteroring may be monocyclic or polycyclic, may be aromatic, aliphatic, or a fused ring of aromatic and aliphatic, and the aromatic heteroring may be selected from among the examples of the heterocyclic group except for those that are not monovalent.

In the present specification, the aliphatic heteroring means an aliphatic ring including one or more of heteroatoms. Examples of the aliphatic heteroring may include oxirane, tetrahydrofuran, 1,4-dioxane, pyrrolidine, piperidine, morpholine, oxepane, azokane, thiokane, tetrahydronaphthothiophene, tetrahydronaphthofuran, tetrahydrobenzothiophene, tetrahydrobenzofuran and the like, but are not limited thereto.

In the present specification, the arylene group means the aryl group having two bonding sites, that is, a divalent group. The descriptions on the aryl group provided above may be applied thereto except for those that are each a divalent group.

In the present specification, the heteroarylene group means the heteroaryl group having two bonding sites, that is, a divalent group. The descriptions on the heterocyclic group provided above may be applied thereto except for those that are each a divalent group.

According to one embodiment of the present specification, Chemical Formula 1 includes at least one fused aliphatic hydrocarbon ring unsubstituted or substituted with an alkyl group.

According to one embodiment of the present specification, A1 is a 5-membered monocyclic heteroring substituted or unsubstituted, and including O or S; or a polycyclic heteroring substituted or unsubstituted, and including a 5-membered heteroring including O or S.

According to one embodiment of the present specification, A1 is a 5-membered aromatic or aliphatic monocyclic heteroring substituted or unsubstituted, and including O or S; or an aromatic, aliphatic, or fused aromatic and aliphatic polycyclic heteroring substituted or unsubstituted, and including a 5-membered heteroring including O or S.

According to one embodiment of the present specification, Chemical Formula A1 is represented by the following Chemical Formula A1-1 or A1-2.

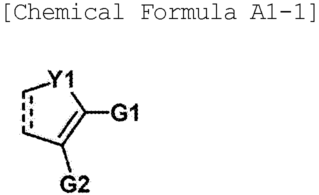

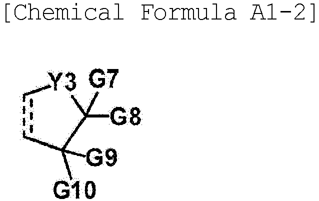

In Chemical Formulae A1-1 and A1-2,
Y1 and Y3 are the same as or different from each other, and each independently O; or S,
G1, G2 and G7 to G10 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring, and 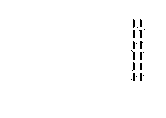 means a site bonding to Chemical Formula 1.

According to one embodiment of the present specification, A1 is a structure represented by any one of the following Chemical Formulae A1-3 to Al-5.

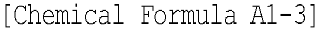

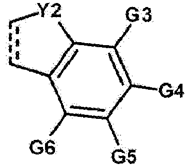

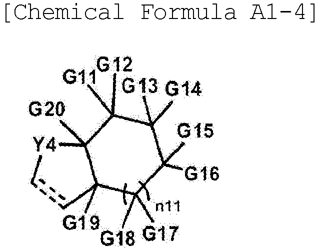

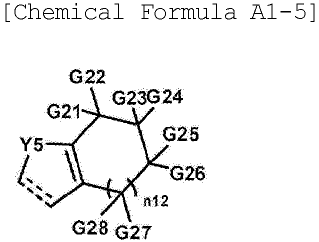

In Chemical Formulae A1-3 to A1-5,
Y2, Y4 and Y5 are the same as or different from each other, and each independently O; or S,
G3 to G6 and G11 to G28 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring,
n11 and n12 are each an integer of 0 to 2,
when n11 and n12 are 2, structures in the two parentheses are the same as or different from each other, and 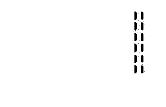 means a site bonding to Chemical Formula 1.

According to one embodiment of the present specification, Chemical Formula A1-1 is represented by the following Chemical Formula A1-1-1 or A1-1-2.

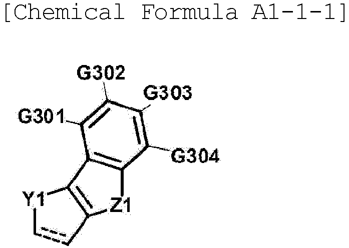

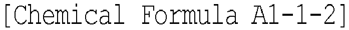

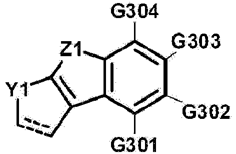

In Chemical Formulae A1-1-1 and A1-1-2,
Y1 is O; or S,
21 is O; S; CR201R202; or NR203,
G301 to G304 and R201 to R203 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring, and 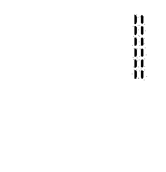 means a site bonding to Chemical Formula 1.

According to one embodiment of the present specification, Chemical Formula 1 is represented by any one of the following Chemical Formulae 1-1 to 1-5.

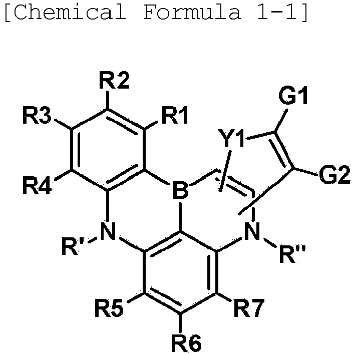

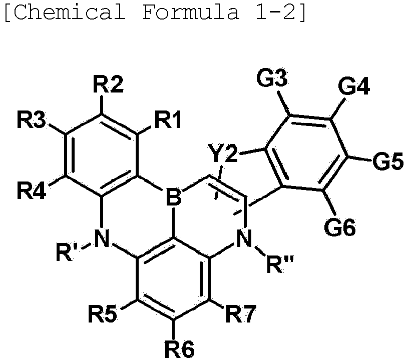

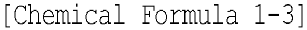

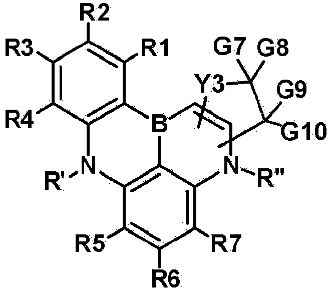

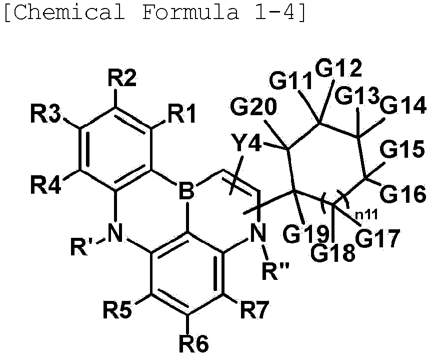

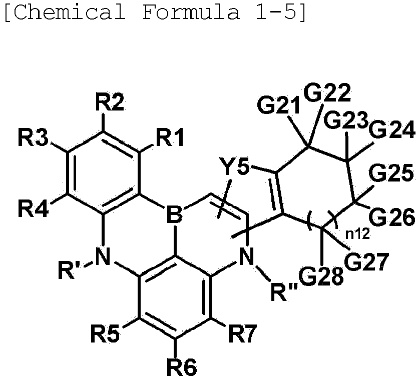

In Chemical Formulae 1-1 to 1-5,
R', R" and R1 to R7 have the same definitions as in Chemical Formula 1,
Y1 to Y5 are the same as or different from each other, and each independently O; or S,
G1 to G28 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring,
n11 and n12 are each an integer of 0 to 2, and
when n11 and n12 are 2, structures in the two parentheses are the same as or different from each other.

According to one embodiment of the present specification, Chemical Formula 1 is represented by any one of the following Chemical Formulae 1-1-1, 1-1-2, 1-1-3, 1-1-4, 1-1-5, 1-1-6, 1-2-1, 1-2-2, 1-3-1, 1-3-2, 1-4-1, 1-4-2, 1-5-1 and 1-5-2.

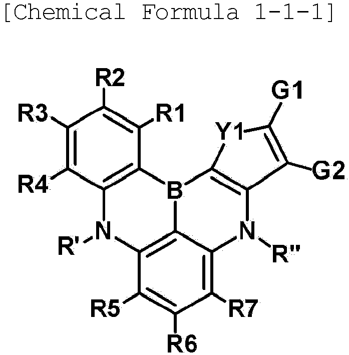

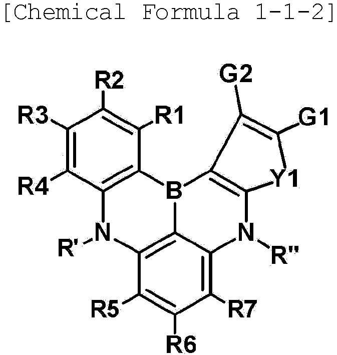

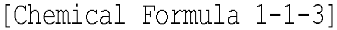

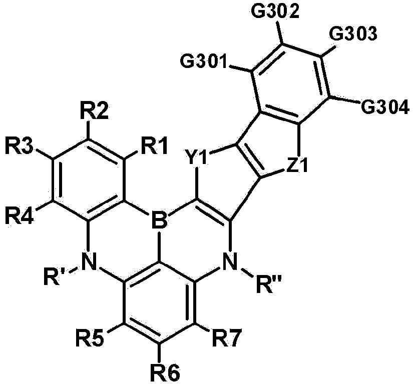

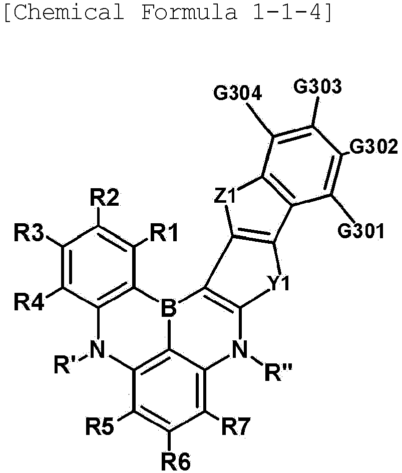

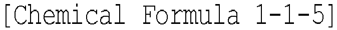

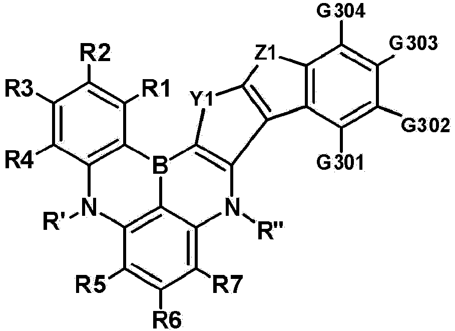

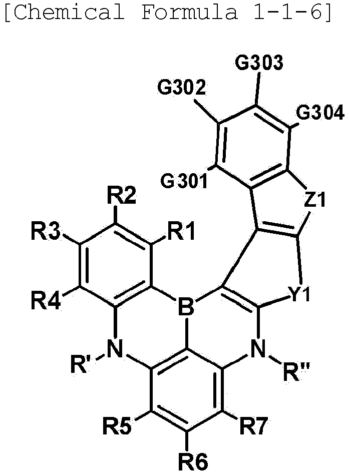

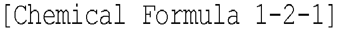

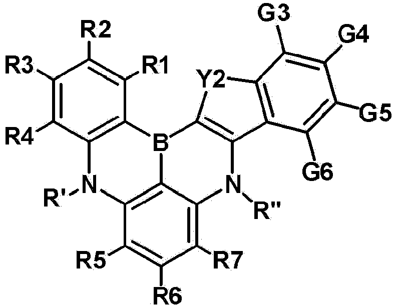

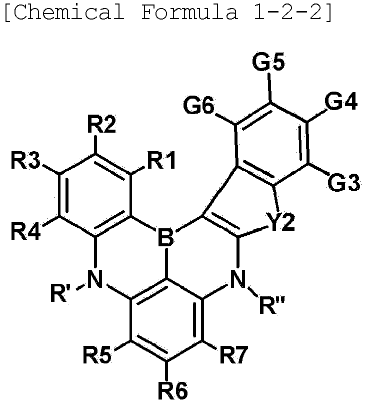

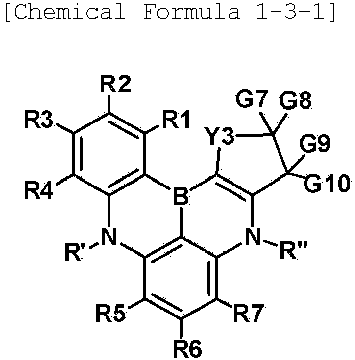

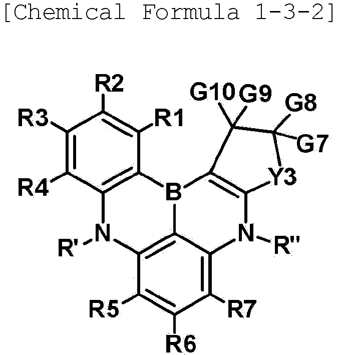

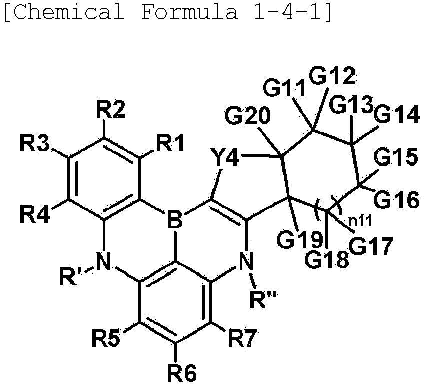

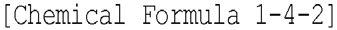

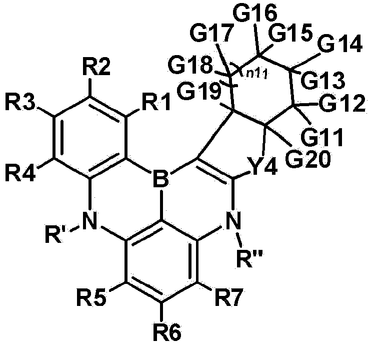

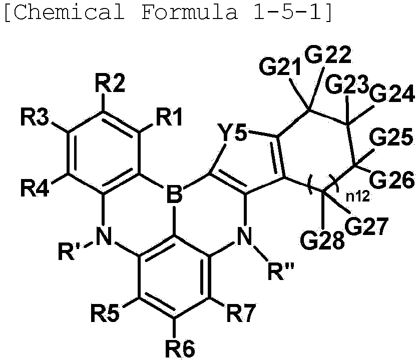

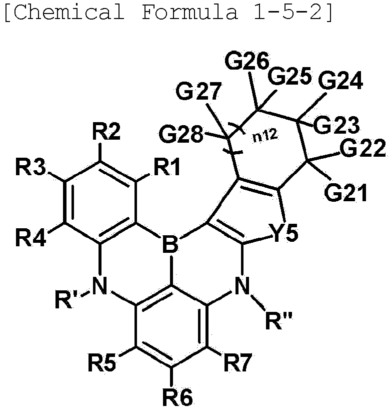

In Chemical Formulae 1-1-1, 1-1-2, 1-1-3, 1-1-4, 1-1-5, 1-1-6, 1-2-1, 1-2-2, 1-3-1, 1-3-2, 1-4-1, 1-4-2, 1-5-1 and 1-5-2,
R', R" and R1 to R7 have the same definitions as in Chemical Formula 1,
Y1 to Y5 are the same as or different from each other, and each independently O; or S,
21 is O; S; CR201R202; or NR203,
G1 to G28, G301 to G304 and R201 to R203 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring,
n11 and n12 are each an integer of 0 to 2, and
when n11 and n12 are 2, structures in the two parentheses are the same as or different from each other.

According to one embodiment of the present specification, adjacent groups among R1 to R4 bond to each other to form a substituted or unsubstituted 5-membered monocyclic heteroring; or a polycyclic heteroring substituted or unsubstituted, and including a 5-membered heteroring.

According to one embodiment of the present specification, adjacent groups among R1 to R4 bond to each other to form a 5-membered monocyclic heteroring substituted or unsubstituted, and including N, O or S; or a polycyclic heteroring substituted or unsubstituted, and including a 5-membered heteroring including N, O or S.

According to one embodiment of the present specification, adjacent groups among R1 to R4 bond to each other to form a 5-membered aromatic or aliphatic monocyclic heteroring substituted or unsubstituted, and including N, O or S; or an aromatic, aliphatic, or fused aromatic and aliphatic polycyclic heteroring substituted or unsubstituted, and including a 5-membered heteroring including N, O or S.

According to one embodiment of the present specification, adjacent groups among R1 to R4 bond to each other to form a ring represented by the following Chemical Formula B1-1 or B1-2.

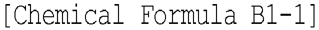

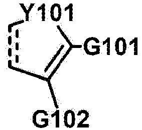

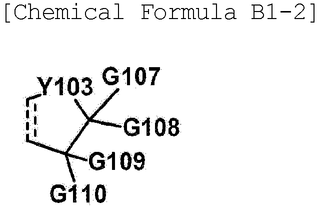

In Chemical Formulae B1-1 and B1-2,
Y101 and Y103 are the same as or different from each other, and each independently O; S; or NR"',
R"', G101, G102 and G107 to G110 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring, and 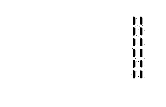 means a site bonding to Chemical Formula 1.

According to one embodiment of the present specification, adjacent groups among R1 to R4 bond to each other to form a ring represented by any one of the following Chemical Formulae B1-3 to Bl-5.

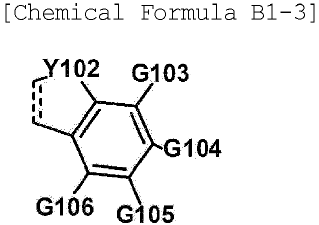

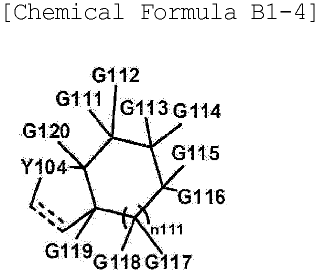

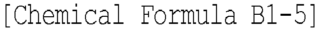

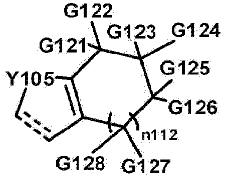

In Chemical Formulae B1-3 to B1-5,
Y102, Y104 and Y105 are the same as or different from each other, and each independently O; S; or NR"',
R"', G103 to G106 and G111 to G128 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring,
n111 and n112 are each an integer of 0 to 2,
when n111 and n112 are 2, structures in the two parentheses are the same as or different from each other, and 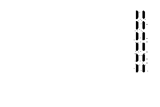 means a site bonding to Chemical Formula 1.

According to one embodiment of the present specification, Chemical Formula B1-1 is represented by the following Chemical Formula B1-1-1 or B1-1-2.

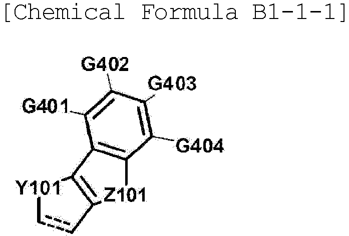

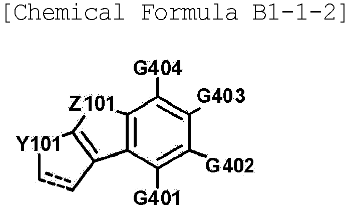

In Chemical Formulae B1-1-1 and B1-1-2,
Y101 is O; S; or NR304,
Z101 is O; S; CR301R302; or NR303,
G401 to G404 and R301 to R304 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring, and 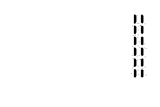 means a site bonding to Chemical Formula 1.

According to one embodiment of the present specification, Chemical Formula 1 is represented by any one of the following Chemical Formulae 1-6 to 1-30.

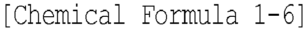

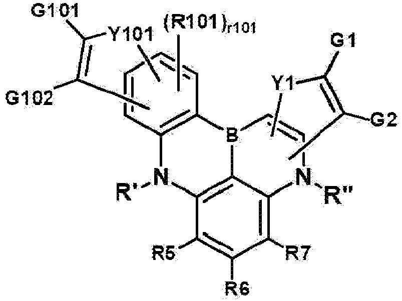

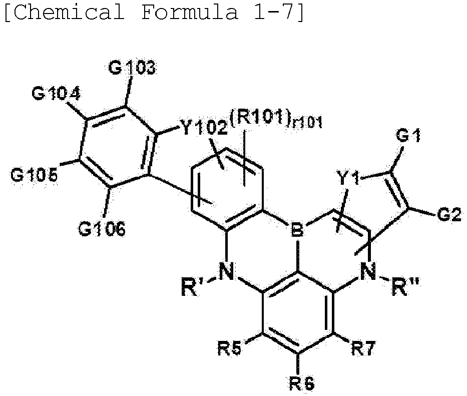

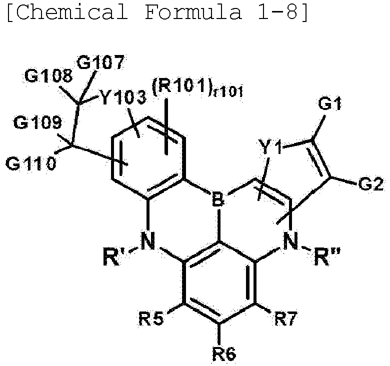

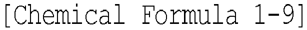

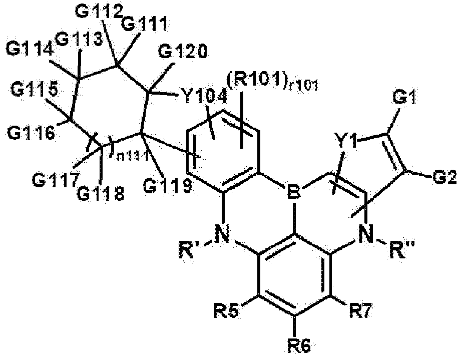

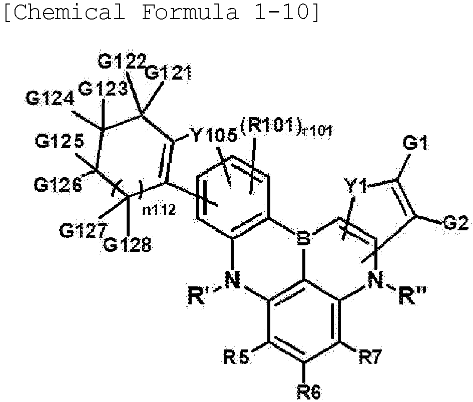

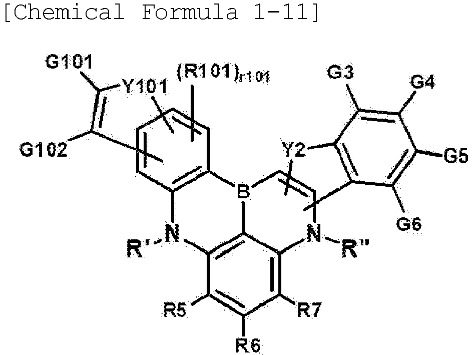

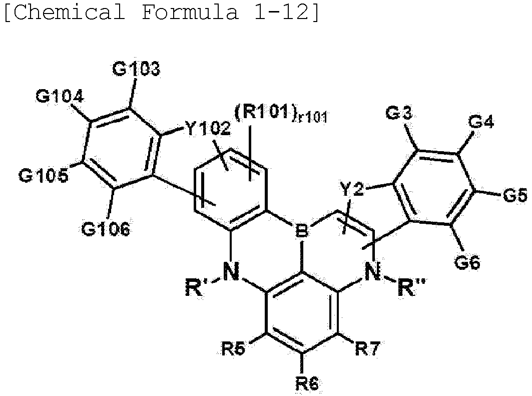

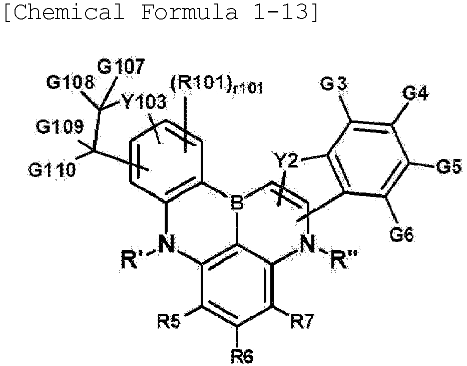

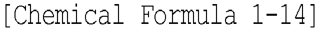

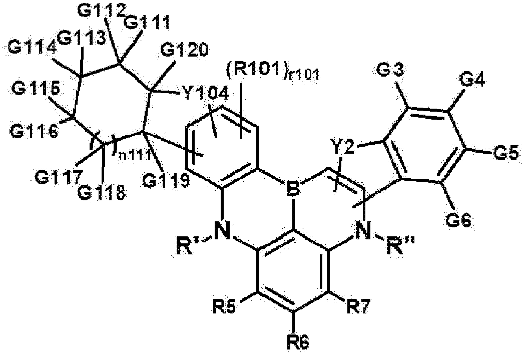

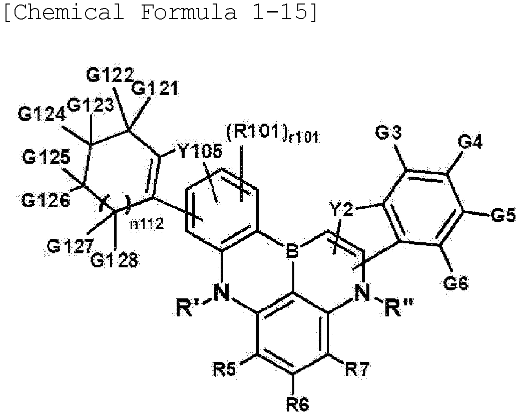

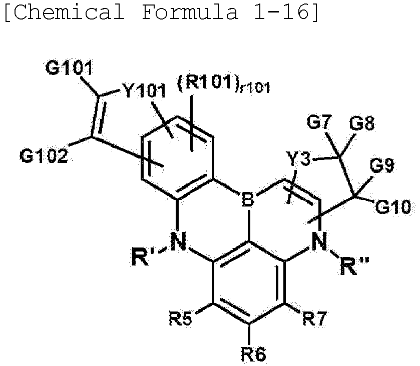

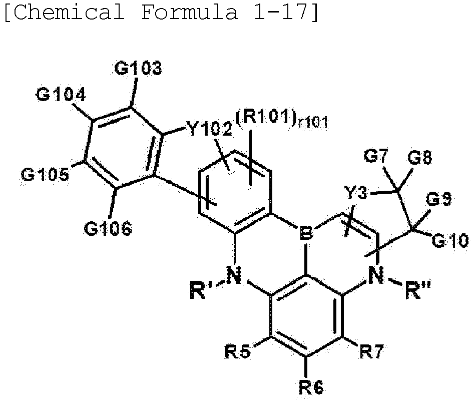

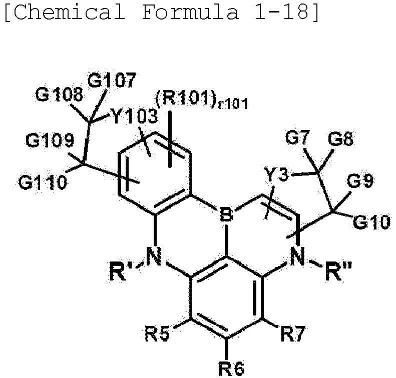

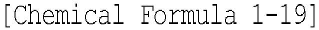

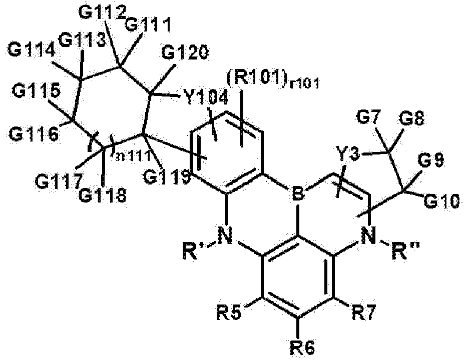

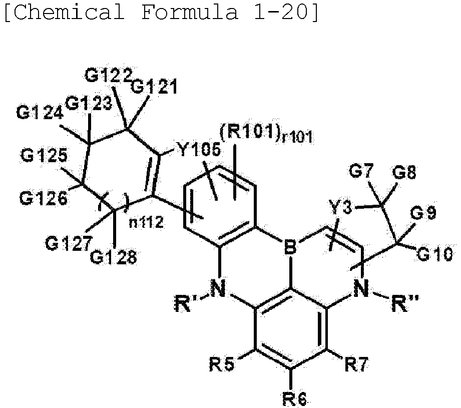

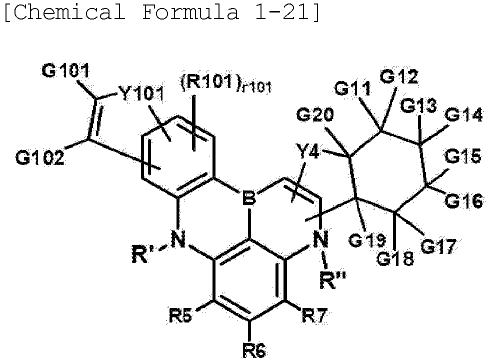

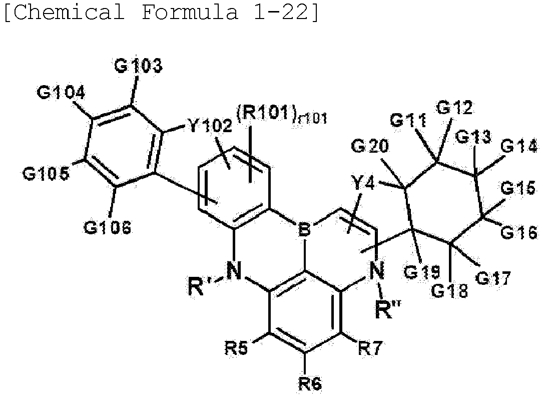

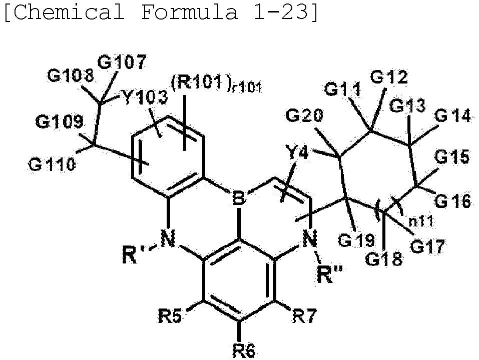

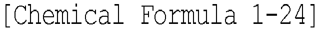

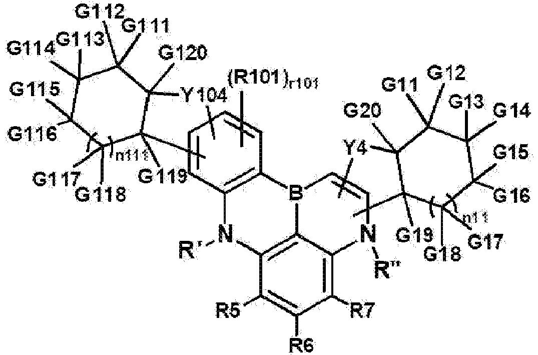

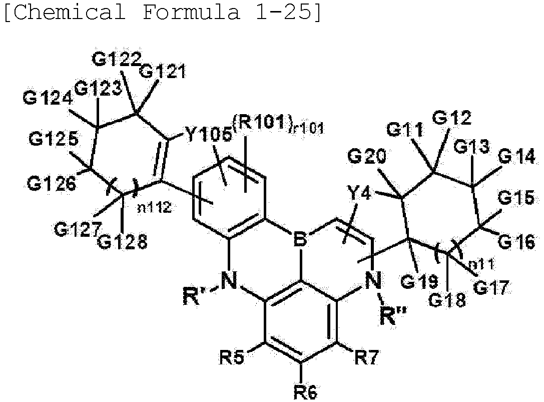

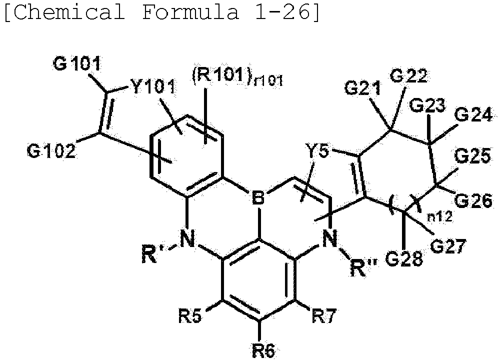

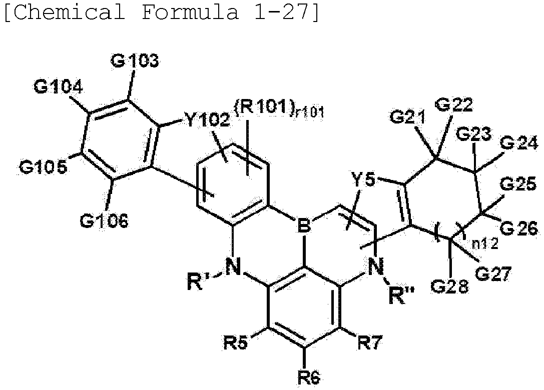

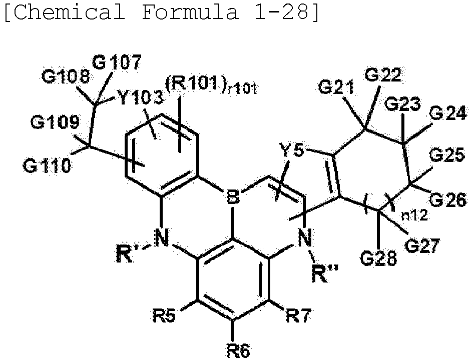

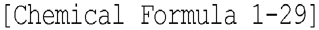

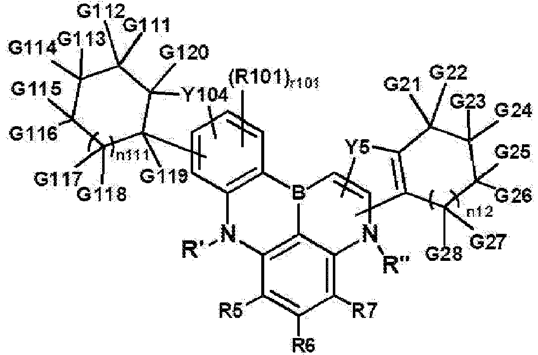

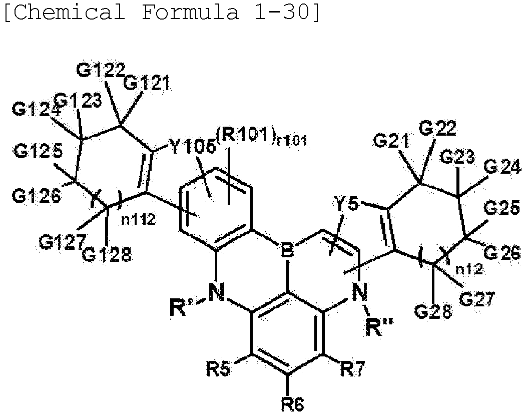

In Chemical Formulae 1-6 to 1-30,
R', R" and R1 to R7 have the same definitions as in Chemical Formula 1,
Y1 to Y5 are the same as or different from each other, and each independently O; or S,
Y101 to Y105 are the same as or different from each other, and each independently O; S; or NR"',
R"', R101, G1 to G28 and G101 to G128 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring,
n11, n12, n111 and n112 are each an integer of 0 to 2,
r101 is 1 or 2, and
when r101, n11, n12, n111 and n112 are 2, structures in the two parentheses are the same as or different from each other.

According to one embodiment of the present specification, Y2 is O or S.

According to one embodiment of the present specification, a group that adjacent groups among R1 to R4 do not form a substituted or unsubstituted ring is hydrogen.

According to one embodiment of the present specification, R1 to R4 are hydrogen.

According to one embodiment of the present specification, R101 is hydrogen.

According to one embodiment of the present specification, R1 to R7 are the same as or different from each other, and each independently hydrogen; deuterium; a cyano group; a substituted or unsubstituted linear or branched alkyl group having 1 to 30 carbon atoms; a substituted or unsubstituted monocyclic or polycyclic cycloalkyl group having 3 to 30 carbon atoms; a substituted or unsubstituted linear or branched alkylsilyl group having 1 to 30 carbon atoms; a substituted or unsubstituted linear or branched haloalkyl group having 1 to 30 carbon atoms; a substituted or unsubstituted linear or branched haloalkoxy group having 1 to 30 carbon atoms; a substituted or unsubstituted arylalkyl group having 6 to 30 carbon atoms; a substituted or unsubstituted monocyclic or polycyclic diarylamine group having 6 to 30 carbon atoms; a substituted or unsubstituted monocyclic or polycyclic aryl group having 6 to 30 carbon atoms; or a substituted or unsubstituted monocyclic or polycyclic heterocyclic group having 2 to 30 carbon atoms.

According to one embodiment of the present specification, R1 to R7 are the same as or different from each other, and each independently hydrogen; deuterium; a cyano group; a linear or branched alkyl group having 1 to 30 carbon atoms; a monocyclic or polycyclic cycloalkyl group having 3 to 30 carbon atoms; a linear or branched alkylsilyl group having 1 to 30 carbon atoms; a linear or branched haloalkyl group having 1 to 30 carbon atoms; a linear or branched haloalkoxy group having 1 to 30 carbon atoms; an arylalkyl group having 6 to 30 carbon atoms; a monocyclic or polycyclic diarylamine group having 6 to 30 carbon atoms; a monocyclic or polycyclic aryl group having 6 to 30 carbon atoms; or a monocyclic or polycyclic heterocyclic group having 2 to 30 carbon atoms, and the substituent is unsubstituted or substituted with one or more selected from the group consisting of deuterium, a halogen group, a cyano group, a linear or branched alkyl group having 1 to 30 carbon atoms, a linear or branched alkylsilyl group having 1 to 30 carbon atoms, a monocyclic or polycyclic aryl group having 6 to 30 carbon atoms, and combinations thereof.

According to one embodiment of the present specification, R1 to R7 are the same as or different from each other, and each independently hydrogen; deuterium; a cyano group; a methyl group; a tert-butyl group; -CD₃; -CF₃; -OCF₃; a cumyl group; a trimethylsilyl group; a diphenylamine group; a cyclohexyl group; a phenyl group; an adamantyl group; or a carbazole group, and the substituent is unsubstituted or substituted with one or more selected from the group consisting of deuterium, - F, a cyano group, a methyl group, a trimethylsilyl group, a tert-butyl group, and combinations thereof.

According to one embodiment of the present specification, R6 is deuterium; a cyano group; a methyl group; a tert-butyl group; -CD₃; -CF₃; -OCF₃; a cumyl group; a trimethylsilyl group; a diphenylamine group; a cyclohexyl group; a phenyl group; an adamantyl group; or a carbazole group, and the substituent is unsubstituted or substituted with one or more selected from the group consisting of deuterium, -F, a cyano group, a methyl group, a trimethylsilyl group, a tert-butyl group, and combinations thereof.

According to one embodiment of the present specification, when R6, a para position of the boron core that is the core of Chemical Formula 1, is substituted with functional groups, the functional groups may donate electrons to the boron, a heteroatom with insufficient electrons in the compound, to increase stability of the compound, and optical properties of the compound may be readily controlled by properly adjusting HOMO and LUMO energy levels effectively.

According to one embodiment of the present specification, R1 to R5 and R7 are the same as or different from each other, and each independently hydrogen; deuterium; a cyano group; a methyl group; a tert-butyl group; -CD₃; -CF₃; -OCF₃; a cumyl group; a trimethylsilyl group; a diphenylamine group; a cyclohexyl group; a phenyl group; an adamantyl group; or a carbazole group, and the substituent is unsubstituted or substituted with one or more selected from the group consisting of deuterium, -F, a cyano group, a methyl group, a trimethylsilyl group, a tert-butyl group, and combinations thereof.

The cumyl group means

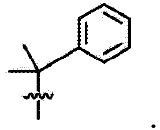

According to one embodiment of the present specification, R1 to R7 are the same as or different from each other, and each independently a group represented by any one of structures of the following Group C.

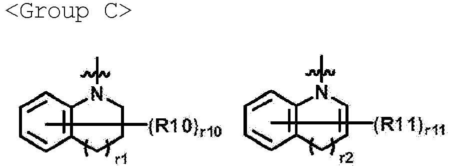

In the structures,
R10 and R11 are the same as or different from each other, and each independently hydrogen; deuterium; a halogen group; a cyano group; a substituted or unsubstituted alkyl group; a substituted or unsubstituted alkoxy group; a substituted or unsubstituted haloalkoxy group; a substituted or unsubstituted alkenyl group; a substituted or unsubstituted silyl group; a substituted or unsubstituted arylalkyl group; a substituted or unsubstituted boron group; a substituted or unsubstituted aryl group; or a substituted or unsubstituted heterocyclic group, or bond to adjacent groups to form a substituted or unsubstituted ring,
r10 is an integer of 1 to 10,
r11 is an integer of 1 to 8,
when r10 and r11 are each 2 or greater, structures in the two or more parentheses are the same as or different from each other,
r1 is 0 or 1,
r2 is 0 or 1, and 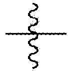
is a site bonding to Chemical Formula 1.

According to one embodiment of the present specification, R1 to R7 are the same as or different from each other, and each independently a group represented by any one of structures of the following Group D.

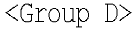

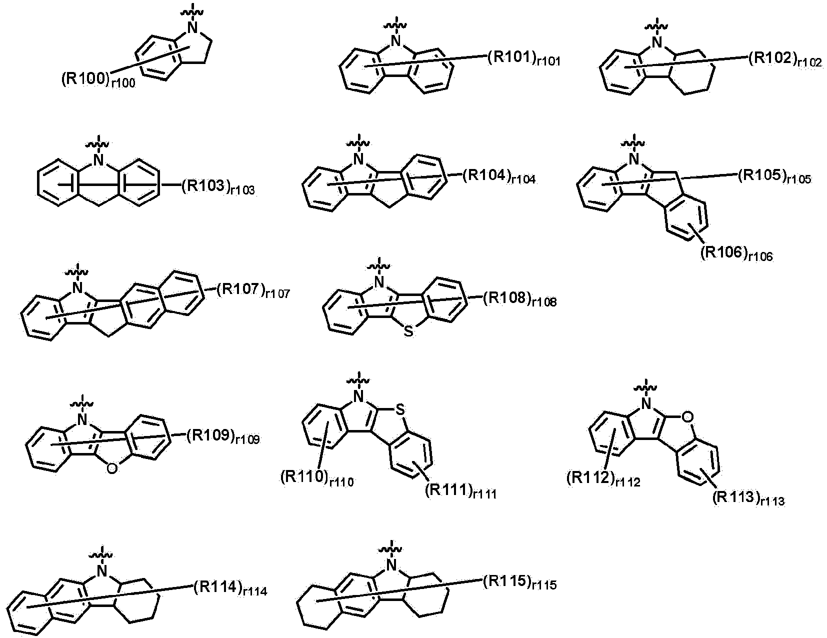

In the structures,
R100 to R115 are the same as or different from each other, and each independently hydrogen; deuterium; a halogen group; a cyano group; a substituted or unsubstituted alkyl group; a substituted or unsubstituted alkoxy group; a substituted or unsubstituted haloalkoxy group; a substituted or unsubstituted alkenyl group; a substituted or unsubstituted silyl group; a substituted or unsubstituted arylalkyl group; a substituted or unsubstituted boron group; a substituted or unsubstituted aryl group; or a substituted or unsubstituted heterocyclic group,
r100, r101, r108 and r109 are each an integer of 1 to 8,
r102 and r107 are each an integer of 1 to 12,
r103 and r104 are each an integer of 1 to 10,
r105 is an integer of 1 to 6,
r106 and r110 to r113 are each an integer of 1 to 4,
r114 is an integer of 1 to 14,
r115 is an integer of 1 to 18,
when r100 to r115 are each 2 or greater, structures in the two or more parentheses are the same as or different from each other, and 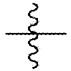
is a site bonding to Chemical Formula 1.

According to one embodiment of the present specification, R100 to R115 in Group D are the same as or different from each other, and each independently hydrogen; deuterium; a substituted or unsubstituted alkyl group; a substituted or unsubstituted cycloalkyl group; a substituted or unsubstituted silyl group; or a substituted or unsubstituted aryl group.

According to one embodiment of the present specification, R100 to R115 in Group D are the same as or different from each other, and each independently hydrogen; deuterium; a substituted or unsubstituted linear or branched alkyl group having 1 to 30 carbon atoms; a substituted or unsubstituted monocyclic or polycyclic cycloalkyl group having 3 to 30 carbon atoms; a substituted or unsubstituted linear or branched alkylsilyl group having 1 to 30 carbon atoms; or a substituted or unsubstituted monocyclic or polycyclic aryl group having 6 to 30 carbon atoms.

According to one embodiment of the present specification, R100 to R115 in Group D are the same as or different from each other, and each independently hydrogen; deuterium; a substituted or unsubstituted linear or branched alkyl group having 1 to 20 carbon atoms; a substituted or unsubstituted monocyclic or polycyclic cycloalkyl group having 3 to 20 carbon atoms; a substituted or unsubstituted linear or branched alkylsilyl group having 1 to 20 carbon atoms; or a substituted or unsubstituted monocyclic or polycyclic aryl group having 6 to 20 carbon atoms.

According to one embodiment of the present specification, R100 to R115 in Group D are the same as or different from each other, and each independently hydrogen; deuterium; a linear or branched alkyl group having 1 to 30 carbon atoms unsubstituted or substituted with deuterium; a monocyclic or polycyclic cycloalkyl group having 3 to 30 carbon atoms unsubstituted or substituted with deuterium; a linear or branched alkylsilyl group having 1 to 30 carbon atoms unsubstituted or substituted with deuterium; or a monocyclic or polycyclic aryl group having 6 to 30 carbon atoms unsubstituted or substituted with deuterium.

According to one embodiment of the present specification, R100 to R115 in Group D are the same as or different from each other, and each independently hydrogen; deuterium; a linear or branched alkyl group having 1 to 20 carbon atoms unsubstituted or substituted with deuterium; a monocyclic or polycyclic cycloalkyl group having 3 to 20 carbon atoms unsubstituted or substituted with deuterium; a linear or branched alkylsilyl group having 1 to 20 carbon atoms unsubstituted or substituted with deuterium; or a monocyclic or polycyclic aryl group having 6 to 20 carbon atoms unsubstituted or substituted with deuterium.

According to one embodiment of the present specification, R100 to R115 in Group D are the same as or different from each other, and each independently hydrogen; deuterium; a methyl group; -CD₃; a tert-butyl group; a cyclohexyl group; a trimethylsilyl group; or a phenyl group unsubstituted or substituted with deuterium.

According to one embodiment of the present specification, adjacent groups among R1 to R4 bond to each other to form a substituted or unsubstituted hydrocarbon ring; or a substituted or unsubstituted heteroring.

According to one embodiment of the present specification, adjacent groups among R1 to R4 bond to each other to form a substituted or unsubstituted monocyclic or polycyclic hydrocarbon ring having 3 to 30 carbon atoms; or a substituted or unsubstituted monocyclic or polycyclic heteroring having 2 to 30 carbon atoms.

According to one embodiment of the present specification, adjacent groups among R1 to R4 bond to each other to form a substituted or unsubstituted monocyclic or polycyclic aliphatic, aromatic, or fused aliphatic and aromatic hydrocarbon ring having 3 to 30 carbon atoms; or a substituted or unsubstituted monocyclic or polycyclic aliphatic heteroring having 2 to 30 carbon atoms.

According to one embodiment of the present specification, adjacent groups among R1 to R4 bond to each other to form a monocyclic or polycyclic aliphatic, aromatic, or fused aliphatic and aromatic hydrocarbon ring having 3 to 30 carbon atoms; or a monocyclic or polycyclic aliphatic heteroring having 2 to 30 carbon atoms, and the ring is unsubstituted or substituted with one or more selected from the group consisting of a linear or branched alkyl group having 1 to 30 carbon atoms, and a linear or branched haloalkyl group having 1 to 30 carbon atoms.

According to one embodiment of the present specification, adjacent groups among R1 to R4 bond to each other to form a cyclopentane ring; a cyclohexane ring; a cycloheptane ring; a bicyclo[2.2.1]octane ring; a norbornane ring; an adamantane ring; an indene ring; a phenanthrene ring; a tetrahydrofuran ring; a tetrahydrothiophene ring; a pyrrolidine ring; a tetrahydrobenzofuran ring; a tetrahydrobenzothiophene ring; an octahydrobenzofuran ring; an octahydrobenzothiophene ring; an octahydrocycloheptaindole ring; an octahydroheptapyrrole ring; an octahydroindene ring; a benzene ring; a phenanthrene ring; a benzofuran ring; a fluorene ring; a dihydroacridine ring; a tetrahydroquinoline ring; a dihydroanthracene ring; a chroman ring; a carbazole ring; or an indolocarbazole ring, and the ring is unsubstituted or substituted with one or more selected from the group consisting of a methyl group, a tert-butyl group and -CF₃.

According to one embodiment of the present specification, R' is a substituted or unsubstituted linear or branched alkyl group having 1 to 30 carbon atoms; a substituted or unsubstituted arylalkyl group having 6 to 30 carbon atoms; a substituted or unsubstituted monocyclic or polycyclic hydrocarbon ring having 3 to 30 carbon atoms group; or a substituted or unsubstituted monocyclic or polycyclic heterocyclic group having 2 to 30 carbon atoms.

According to one embodiment of the present specification, R' is a linear or branched alkyl group having 1 to 30 carbon atoms; an arylalkyl group having 6 to 30 carbon atoms; a monocyclic or polycyclic hydrocarbon ring having 3 to 30 carbon atoms group; or a monocyclic or polycyclic heterocyclic group having 2 to 30 carbon atoms, and the substituent is unsubstituted or substituted with one or more selected from the group consisting of deuterium, a halogen group, a cyano group, a linear or branched alkyl group having 1 to 30 carbon atoms, a monocyclic or polycyclic cycloalkyl group having 3 to 30 carbon atoms, a linear or branched alkylsilyl group having 1 to 30 carbon atoms, an arylsilyl group having 3 to 30 carbon atoms, a linear or branched alkoxy group having 1 to 30 carbon atoms, a linear or branched haloalkyl group having 1 to 30 carbon atoms, a linear or branched haloalkoxy group having 1 to 30 carbon atoms, an aryloxy group having 6 to 30 carbon atoms, a monocyclic or polycyclic arylalkyl group having 6 to 30 carbon atoms, a monocyclic or polycyclic diarylamine group having 6 to 30 carbon atoms, a monocyclic or polycyclic aryl group having 6 to 30 carbon atoms, a monocyclic or polycyclic heterocyclic group having 2 to 30 carbon atoms, a fused ring group of monocyclic or polycyclic aromatic hydrocarbon having 6 to 30 carbon atoms and monocyclic or polycyclic aliphatic hydrocarbon having 3 to 30 carbon atoms, and combinations thereof.

According to one embodiment of the present specification, R' is a methyl group; a tert-butyl group; a phenyl group; a biphenyl group; a terphenyl group; a naphthyl group; a fluorene group; a triphenylene group; a dibenzofuran group; a cumyl group; an adamantyl group; a tetrahydronaphthyl group; or a hexahydrodibenzofuran group, and the substituent is unsubstituted or substituted with one or more selected from the group consisting of deuterium, F, a cyano group, a methyl group, a tert-butyl group, -CD₃, -CF₃, -OCF₃, a cyclohexyl group, a phenyl group, a diphenylamine group, a pyridine group, a pyrimidine group, a methoxy group, an i-propyl group, a phenoxy group, a triphenylsilyl group, a trimethylsilyl group, a tetrahydronaphthyl group, and combinations thereof.

According to one embodiment of the present specification, R" is a substituted or unsubstituted linear or branched alkyl group having 1 to 30 carbon atoms; a substituted or unsubstituted arylalkyl group having 6 to 30 carbon atoms; a substituted or unsubstituted monocyclic or polycyclic hydrocarbon ring having 3 to 30 carbon atoms group; or a substituted or unsubstituted monocyclic or polycyclic heterocyclic group having 2 to 30 carbon atoms.

According to one embodiment of the present specification, R" is a linear or branched alkyl group having 1 to 30 carbon atoms; an arylalkyl group having 6 to 30 carbon atoms; a monocyclic or polycyclic hydrocarbon ring having 3 to 30 carbon atoms group; or a monocyclic or polycyclic heterocyclic group having 2 to 30 carbon atoms, and the substituent is unsubstituted or substituted with one or more selected from the group consisting of deuterium, a halogen group, a cyano group, a linear or branched alkyl group having 1 to 30 carbon atoms, a monocyclic or polycyclic cycloalkyl group having 3 to 30 carbon atoms, a linear or branched alkylsilyl group having 1 to 30 carbon atoms, an arylsilyl group having 3 to 30 carbon atoms, a linear or branched alkoxy group having 1 to 30 carbon atoms, a linear or branched haloalkyl group having 1 to 30 carbon atoms, a linear or branched haloalkoxy group having 1 to 30 carbon atoms, an aryloxy group having 6 to 30 carbon atoms, a monocyclic or polycyclic arylalkyl group having 6 to 30 carbon atoms, a monocyclic or polycyclic diarylamine group having 6 to 30 carbon atoms, a monocyclic or polycyclic aryl group having 6 to 30 carbon atoms, a monocyclic or polycyclic heterocyclic group having 2 to 30 carbon atoms, a fused ring group of monocyclic or polycyclic aliphatic hydrocarbon having 3 to 30 carbon atoms and monocyclic or polycyclic aromatic hydrocarbon having 6 to 30 carbon atoms, and combinations thereof.

According to one embodiment of the present specification, R" is a methyl group; a tert-butyl group; a phenyl group; a biphenyl group; a terphenyl group; a naphthyl group; a fluorene group; a triphenylene group; a dibenzofuran group; a cumyl group; an adamantyl group; a tetrahydronaphthyl group; or a hexahydrodibenzofuran group, and the substituent is unsubstituted or substituted with one or more selected from the group consisting of deuterium, F, a cyano group, a methyl group, a tert-butyl group, -CD₃, -CF₃, -OCF₃, a cyclohexyl group, a phenyl group, a diphenylamine group, a pyridine group, a pyrimidine group, a methoxy group, an i-propyl group, a phenoxy group, a triphenylsilyl group, a trimethylsilyl group, a tetrahydronaphthyl group, and combinations thereof.

According to one embodiment of the present specification, Y1 is O.

According to one embodiment of the present specification, Y1 is S.

According to one embodiment of the present specification, Y2 is O.

According to one embodiment of the present specification, Y2 is S.

According to one embodiment of the present specification, Y3 is O.

According to one embodiment of the present specification, Y3 is S.

According to one embodiment of the present specification, Y4 is O.

According to one embodiment of the present specification, Y4 is S.

According to one embodiment of the present specification, Y5 is O.

According to one embodiment of the present specification, Y5 is S.

According to one embodiment of the present specification, Y101 is O.

According to one embodiment of the present specification, Y101 is S.

According to one embodiment of the present specification, Y101 is NR"'.

According to one embodiment of the present specification, Y102 is O.

According to one embodiment of the present specification, Y102 is S.

According to one embodiment of the present specification, Y102 is NR"'.

According to one embodiment of the present specification, Y103 is O.

According to one embodiment of the present specification, Y103 is S.

According to one embodiment of the present specification, Y103 is NR"'.

According to one embodiment of the present specification, Y104 is O.

According to one embodiment of the present specification, Y104 is S.

According to one embodiment of the present specification, Y104 is NR"'.

According to one embodiment of the present specification, Y105 is O.

According to one embodiment of the present specification, Y105 is S.

According to one embodiment of the present specification, Y105 is NR"'.

According to one embodiment of the present specification, R1 and G2 bond to each other to form a substituted or unsubstituted heteroring.

According to one embodiment of the present specification, R1 and G2 bond to each other to form a substituted or unsubstituted heteroring including B and N.

According to one embodiment of the present specification, R1 and G2 bond to each other to form a substituted or unsubstituted 6-membered heteroring including B and N.

According to one embodiment of the present specification, R1 and G2 bond to each other to form a heteroring substituted with an aryl group.

According to one embodiment of the present specification, R1 and G2 bond to each other to form a heteroring including B and N and substituted with an aryl group.

According to one embodiment of the present specification, R1 and G2 bond to each other to form a 6-membered heteroring including B and Si and substituted with a phenyl group.

According to one embodiment of the present specification, R4 and R' bond to each other to form any one ring of structures represented by the following Group A.

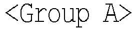

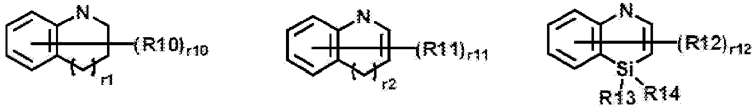

In the structures,
R10 to R14 are the same as or different from each other, and each independently hydrogen; deuterium; a halogen group; a cyano group; a substituted or unsubstituted alkyl group; a substituted or unsubstituted alkoxy group; a substituted or unsubstituted haloalkoxy group; a substituted or unsubstituted alkenyl group; a substituted or unsubstituted silyl group; a substituted or unsubstituted arylalkyl group; a substituted or unsubstituted boron group; a substituted or unsubstituted aryl group; or a substituted or unsubstituted heterocyclic group, or bond to adjacent groups to form a substituted or unsubstituted ring,
r10 is an integer of 1 to 10,
r11 is an integer of 1 to 8,
r12 is an integer of 1 to 6,
when r10 to r12 are each 2 or greater, structures in the two or more parentheses are the same as or different from each other,
r1 is 0 or 1, and
r2 is 0 or 1.

According to one embodiment of the present specification, R4 and R' bond to each other to form any one ring of structures represented by the following Group B.

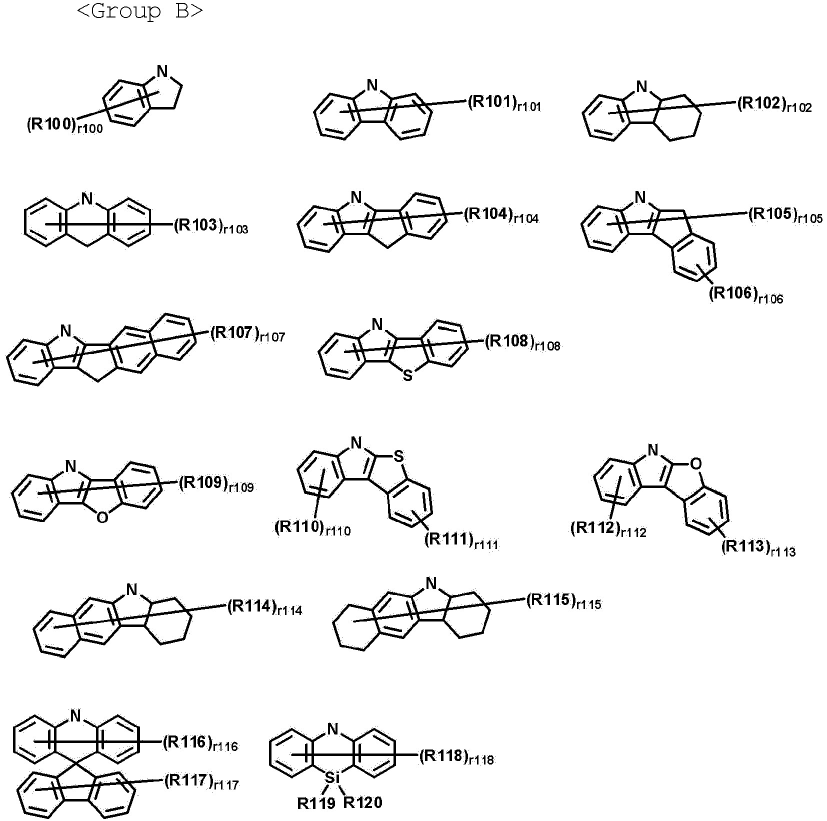

In the structures,
R100 to R120 are the same as or different from each other, and each independently hydrogen; deuterium; a halogen group; a cyano group; a substituted or unsubstituted alkyl group; a substituted or unsubstituted alkoxy group; a substituted or unsubstituted haloalkoxy group; a substituted or unsubstituted alkenyl group; a substituted or unsubstituted silyl group; a substituted or unsubstituted arylalkyl group; a substituted or unsubstituted boron group; a substituted or unsubstituted aryl group; or a substituted or unsubstituted heterocyclic group,
r100, r101, r108, r109 and r116 to r118 are each an integer of 1 to 8,
r102 and r107 are each an integer of 1 to 12,
r103 and r104 are each an integer of 1 to 10,
r105 is an integer of 1 to 6,
r106 and r110 to r113 are each an integer of 1 to 4,
r114 is an integer of 1 to 14,
r115 is an integer of 1 to 18, and
when r100 to r118 are each 2 or greater, structures in the two or more parentheses are the same as or different from each other.

According to one embodiment of the present specification, R100 to R120 in Group B are the same as or different from each other, and each independently hydrogen; deuterium; a substituted or unsubstituted alkyl group; a substituted or unsubstituted cycloalkyl group; a substituted or unsubstituted silyl group; or a substituted or unsubstituted aryl group.

According to one embodiment of the present specification, R100 to R120 in Group B are the same as or different from each other, and each independently hydrogen; deuterium; a substituted or unsubstituted linear or branched alkyl group having 1 to 30 carbon atoms; a substituted or unsubstituted monocyclic or polycyclic cycloalkyl group having 3 to 30 carbon atoms; a substituted or unsubstituted linear or branched alkylsilyl group having 1 to 30 carbon atoms; or a substituted or unsubstituted monocyclic or polycyclic aryl group having 6 to 30 carbon atoms.

According to one embodiment of the present specification, R100 to R120 in Group B are the same as or different from each other, and each independently hydrogen; deuterium; a substituted or unsubstituted linear or branched alkyl group having 1 to 20 carbon atoms; a substituted or unsubstituted monocyclic or polycyclic cycloalkyl group having 3 to 20 carbon atoms; a substituted or unsubstituted linear or branched alkylsilyl group having 1 to 20 carbon atoms; or a substituted or unsubstituted monocyclic or polycyclic aryl group having 6 to 20 carbon atoms.

According to one embodiment of the present specification, R100 to R120 in Group B are the same as or different from each other, and each independently hydrogen; deuterium; a linear or branched alkyl group having 1 to 30 carbon atoms unsubstituted or substituted with deuterium; a monocyclic or polycyclic cycloalkyl group having 3 to 30 carbon atoms unsubstituted or substituted with deuterium; a linear or branched alkylsilyl group having 1 to 30 carbon atoms unsubstituted or substituted with deuterium; or a monocyclic or polycyclic aryl group having 6 to 30 carbon atoms unsubstituted or substituted with deuterium.

According to one embodiment of the present specification, R100 to R120 in Group B are the same as or different from each other, and each independently hydrogen; deuterium; a linear or branched alkyl group having 1 to 20 carbon atoms unsubstituted or substituted with deuterium; a monocyclic or polycyclic cycloalkyl group having 3 to 20 carbon atoms unsubstituted or substituted with deuterium; a linear or branched alkylsilyl group having 1 to 20 carbon atoms unsubstituted or substituted with deuterium; or a monocyclic or polycyclic aryl group having 6 to 20 carbon atoms unsubstituted or substituted with deuterium.

According to one embodiment of the present specification, R100 to R120 in Group B are the same as or different from each other, and each independently hydrogen; deuterium; a methyl group; -CD₃; a tert-butyl group; a cyclohexyl group; a trimethylsilyl group; or a phenyl group unsubstituted or substituted with deuterium.

According to one embodiment of the present specification, G1 to G28 and G101 to G128 are the same as or different from each other, and each independently hydrogen; deuterium; a cyano group; a substituted or unsubstituted linear or branched alkyl group having 1 to 30 carbon atoms; a substituted or unsubstituted linear or branched haloalkyl group having 1 to 30 carbon atoms; a substituted or unsubstituted monocyclic or polycyclic cycloalkyl group having 3 to 30 carbon atoms; a substituted or unsubstituted linear or branched alkylsilyl group having 1 to 30 carbon atoms; a substituted or unsubstituted monocyclic or polycyclic arylsilyl group having 6 to 30 carbon atoms; a substituted or unsubstituted arylalkyl group having 6 to 30 carbon atoms; a substituted or unsubstituted monocyclic or polycyclic diarylamine group having 6 to 30 carbon atoms; a substituted or unsubstituted monocyclic or polycyclic aryl group having 6 to 30 carbon atoms; or a substituted or unsubstituted monocyclic or polycyclic heterocyclic group having 2 to 30 carbon atoms.

According to one embodiment of the present specification, G1 to G28 and G101 to G128 are the same as or different from each other, and each independently hydrogen; deuterium; a cyano group; a linear or branched alkyl group having 1 to 30 carbon atoms; a linear or branched haloalkyl group having 1 to 30 carbon atoms; a monocyclic or polycyclic cycloalkyl group having 3 to 30 carbon atoms; a linear or branched alkylsilyl group having 1 to 30 carbon atoms; a monocyclic or polycyclic arylsilyl group having 6 to 30 carbon atoms; an arylalkyl group having 6 to 30 carbon atoms; a monocyclic or polycyclic diarylamine group having 6 to 30 carbon atoms; a monocyclic or polycyclic aryl group having 6 to 30 carbon atoms; or a monocyclic or polycyclic heterocyclic group having 2 to 30 carbon atoms, and the substituent is unsubstituted or substituted with one or more selected from the group consisting of deuterium, a halogen group, a linear or branched alkyl group having 1 to 30 carbon atoms, and combinations thereof.

According to one embodiment of the present specification, G1 to G28 and G101 to G128 are the same as or different from each other, and each independently hydrogen; deuterium; a cyano group; a methyl group; an i-propyl group; a tert-butyl group; - CF₃; an adamantyl group; a trimethylsilyl group; a triphenylsilyl group; a diphenylamine group; a phenyl group; a naphthyl group; a cumyl group; a pyridyl group; or a carbazole group, and the substituent is unsubstituted or substituted with one or more selected from the group consisting of deuterium, F, a methyl group, a tert-butyl group, and combinations thereof.

According to one embodiment of the present specification, R"' is a substituted or unsubstituted linear or branched alkyl group having 1 to 30 carbon atoms; a substituted or unsubstituted monocyclic or polycyclic aliphatic, aromatic, or fused aliphatic and aromatic hydrocarbon ring group having 6 to 30 carbon atoms; or a substituted or unsubstituted monocyclic or polycyclic heterocyclic group having 2 to 30 carbon atoms.

According to one embodiment of the present specification, R"' is a linear or branched alkyl group having 1 to 30 carbon atoms; a monocyclic or polycyclic aliphatic, aromatic, or fused aliphatic and aromatic hydrocarbon ring group having 6 to 30 carbon atoms; or a monocyclic or polycyclic heterocyclic group having 2 to 30 carbon atoms, and the substituent is unsubstituted or substituted with one or more selected from the group consisting of deuterium, a halogen group, a linear or branched alkyl group having 1 to 30 carbon atoms, a linear or branched haloalkyl group having 1 to 30 carbon atoms, and combinations thereof.

According to one embodiment of the present specification, R"' is a tert-butyl group; a phenyl group; a biphenyl group; a tetrahydrobenzofuran group; a pyridine group; or a triazine group, and the substituent is unsubstituted or substituted with one or more selected from the group consisting of deuterium, F, -CF₃, a methyl group, a tert-butyl group, and combinations thereof.

According to one embodiment of the present specification, G1 to G28 and G101 to G128 are the same as or different from each other, and each independently a group represented by any one of structures of the following Group C.

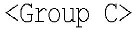

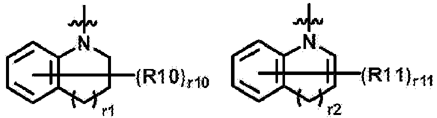

In the structures,
R10 and R11 are the same as or different from each other, and each independently hydrogen; deuterium; a halogen group; a cyano group; a substituted or unsubstituted alkyl group; a substituted or unsubstituted alkoxy group; a substituted or unsubstituted haloalkoxy group; a substituted or unsubstituted alkenyl group; a substituted or unsubstituted silyl group; a substituted or unsubstituted arylalkyl group; a substituted or unsubstituted boron group; a substituted or unsubstituted aryl group; or a substituted or unsubstituted heterocyclic group, or bond to adjacent groups to form a substituted or unsubstituted ring,
r10 is an integer of 1 to 10,
r11 is an integer of 1 to 8,
when r10 and r11 are each 2 or greater, structures in the two or more parentheses are the same as or different from each other,
r1 is 0 or 1,
r2 is 0 or 1, and 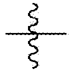
is a site bonding to Chemical Formula 1.

According to one embodiment of the present specification, G1 to G28 and G101 to G128 are the same as or different from each other, and each independently a group represented by any one of structures of the following Group D.

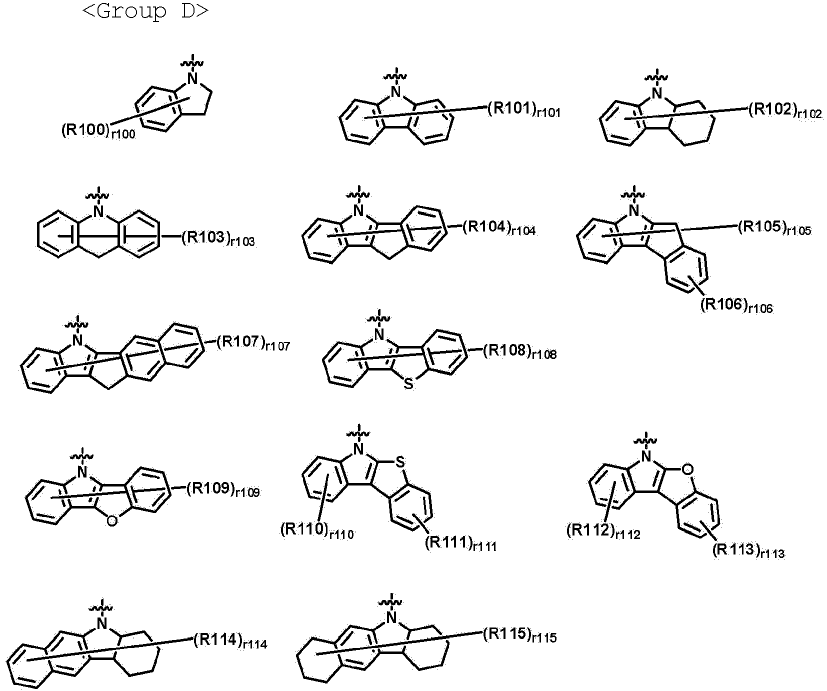

In the structures,
R100 to R115 are the same as or different from each other, and each independently hydrogen; deuterium; a halogen group; a cyano group; a substituted or unsubstituted alkyl group; a substituted or unsubstituted alkoxy group; a substituted or unsubstituted haloalkoxy group; a substituted or unsubstituted alkenyl group; a substituted or unsubstituted silyl group; a substituted or unsubstituted arylalkyl group; a substituted or unsubstituted boron group; a substituted or unsubstituted aryl group; or a substituted or unsubstituted heterocyclic group,
r100, r101, r108 and r109 are each an integer of 1 to 8,
r102 and r107 are each an integer of 1 to 12,
r103 and r104 are each an integer of 1 to 10,
r105 is an integer of 1 to 6,
r106 and r110 to r113 are each an integer of 1 to 4,
r114 is an integer of 1 to 14,
r115 is an integer of 1 to 18,
when r100 to r115 are each 2 or greater, structures in the two or more parentheses are the same as or different from each other, and 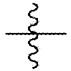
is a site bonding to Chemical Formula 1.

According to one embodiment of the present specification, R100 to R115 in Group D are the same as or different from each other, and each independently hydrogen; deuterium; a substituted or unsubstituted alkyl group; a substituted or unsubstituted cycloalkyl group; a substituted or unsubstituted silyl group; or a substituted or unsubstituted aryl group.

According to one embodiment of the present specification, R100 to R115 in Group D are the same as or different from each other, and each independently hydrogen; deuterium; a substituted or unsubstituted linear or branched alkyl group having 1 to 30 carbon atoms; a substituted or unsubstituted monocyclic or polycyclic cycloalkyl group having 3 to 30 carbon atoms; a substituted or unsubstituted linear or branched alkylsilyl group having 1 to 30 carbon atoms; or a substituted or unsubstituted monocyclic or polycyclic aryl group having 6 to 30 carbon atoms.

According to one embodiment of the present specification, R100 to R115 in Group D are the same as or different from each other, and each independently hydrogen; deuterium; a substituted or unsubstituted linear or branched alkyl group having 1 to 20 carbon atoms; a substituted or unsubstituted monocyclic or polycyclic cycloalkyl group having 3 to 20 carbon atoms; a substituted or unsubstituted linear or branched alkylsilyl group having 1 to 20 carbon atoms; or a substituted or unsubstituted monocyclic or polycyclic aryl group having 6 to 20 carbon atoms.

According to one embodiment of the present specification, R100 to R115 in Group D are the same as or different from each other, and each independently hydrogen; deuterium; a linear or branched alkyl group having 1 to 30 carbon atoms unsubstituted or substituted with deuterium; a monocyclic or polycyclic cycloalkyl group having 3 to 30 carbon atoms unsubstituted or substituted with deuterium; a linear or branched alkylsilyl group having 1 to 30 carbon atoms unsubstituted or substituted with deuterium; or a monocyclic or polycyclic aryl group having 6 to 30 carbon atoms unsubstituted or substituted with deuterium.

According to one embodiment of the present specification, R100 to R115 in Group D are the same as or different from each other, and each independently hydrogen; deuterium; a linear or branched alkyl group having 1 to 20 carbon atoms unsubstituted or substituted with deuterium; a monocyclic or polycyclic cycloalkyl group having 3 to 20 carbon atoms unsubstituted or substituted with deuterium; a linear or branched alkylsilyl group having 1 to 20 carbon atoms unsubstituted or substituted with deuterium; or a monocyclic or polycyclic aryl group having 6 to 20 carbon atoms unsubstituted or substituted with deuterium.

According to one embodiment of the present specification, R100 to R115 in Group D are the same as or different from each other, and each independently hydrogen; deuterium; a methyl group; -CD₃; a tert-butyl group; a cyclohexyl group; a trimethylsilyl group; or a phenyl group unsubstituted or substituted with deuterium.

According to one embodiment of the present specification, adjacent groups among G1 to G28 and G101 to G128 bond to each other to form a substituted or unsubstituted hydrocarbon ring; or a substituted or unsubstituted heteroring.

According to one embodiment of the present specification, adjacent groups among G1 to G28 and G101 to G128 bond to each other to form a substituted or unsubstituted monocyclic or polycyclic hydrocarbon ring having 3 to 30 carbon atoms; or a substituted or unsubstituted monocyclic or polycyclic heteroring having 2 to 30 carbon atoms.

According to one embodiment of the present specification, adjacent groups among G1 to G28 and G101 to G128 bond to each other to form a substituted or unsubstituted monocyclic or polycyclic aliphatic, aromatic, or fused aliphatic and aromatic hydrocarbon ring having 3 to 30 carbon atoms; or a substituted or unsubstituted monocyclic or polycyclic aliphatic heteroring having 2 to 30 carbon atoms.

According to one embodiment of the present specification, adjacent groups among G1 to G28 and G101 to G128 bond to each other to form a monocyclic or polycyclic aliphatic, aromatic, or fused aliphatic and aromatic hydrocarbon ring having 3 to 30 carbon atoms; or a monocyclic or polycyclic aliphatic heteroring having 2 to 30 carbon atoms, and the ring is unsubstituted or substituted with one or more selected from the group consisting of a monocyclic or polycyclic aryl group having 6 to 30 carbon atoms unsubstituted or substituted with a linear or branched alkyl group having 1 to 30 carbon atoms, a linear or branched alkyl group having 1 to 30 carbon atoms, and a linear or branched haloalkyl group having 1 to 30 carbon atoms.

According to one embodiment of the present specification, adjacent groups among G1 to G28 and G101 to G128 bond to each other to form a cyclopentane ring; a cyclohexane ring; a cycloheptane ring; a bicyclo[2.2.1]octane ring; a norbornane ring; an adamantane ring; an indene ring; a phenanthrene ring; a tetrahydrofuran ring; a tetrahydrothiophene ring; a tetrahydronaphthofuran ring; a tetrahydronaphthothiophene ring; a pyrrolidine ring; an octahydrobenzofuran ring; an octahydrobenzothiophene ring; an octahydroindene ring; a benzene ring; a phenanthrene ring; a benzofuran ring; a fluorene ring; a dihydroanthracene ring; a chroman ring; a carbazole ring; benzimidazole; a tetrahydrobenzoindole ring; an indole ring; or an indolocarbazole ring, and the ring is unsubstituted or substituted with one or more selected from the group consisting of a phenyl group substituted with a tert-butyl group, a methyl group, a tert-butyl group and -CF₃.

According to one embodiment of the present specification, R'" and G102 bond to each other to form a substituted or unsubstituted heteroring.

According to one embodiment of the present specification, R"' and G102 bond to each other to form a substituted or unsubstituted heteroring including B and N.

According to one embodiment of the present specification, R"' and G102 bond to each other to form a substituted or unsubstituted 6-membered heteroring including B and N.

According to one embodiment of the present specification, R"' and G102 bond to each other to form a heteroring.

According to one embodiment of the present specification, R"' and G102 bond to each other to form a heteroring including B and N.

According to one embodiment of the present specification, R"' and G102 bond to each other to form a 6-membered heteroring including B and N.

According to one embodiment of the present specification, Chemical Formula 1 is any one selected from among the following compounds.

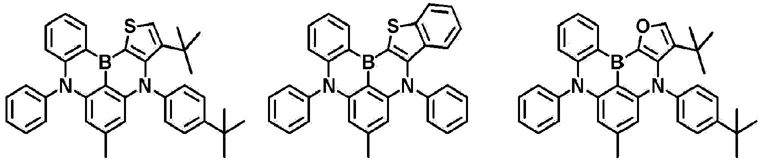

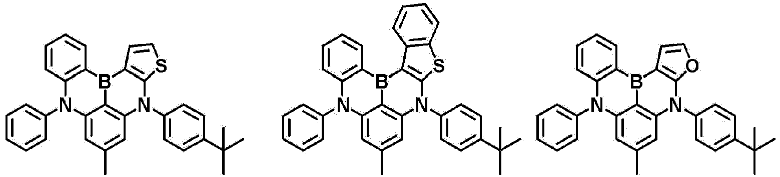

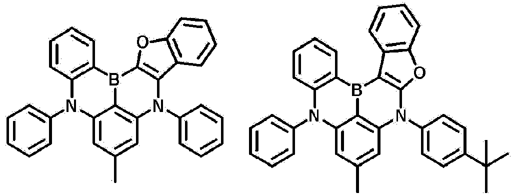

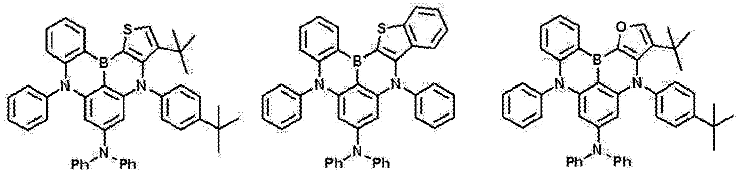

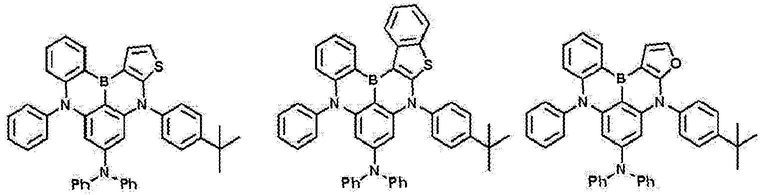

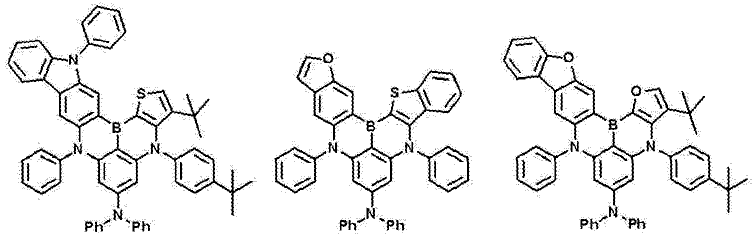

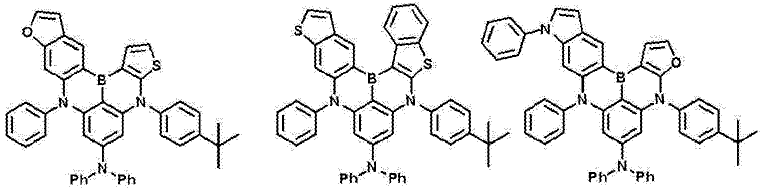

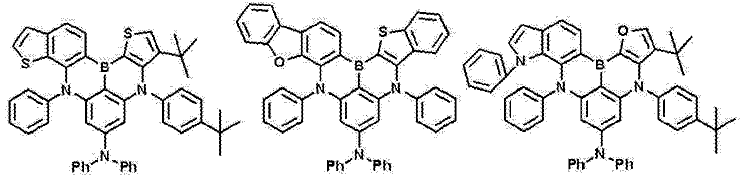

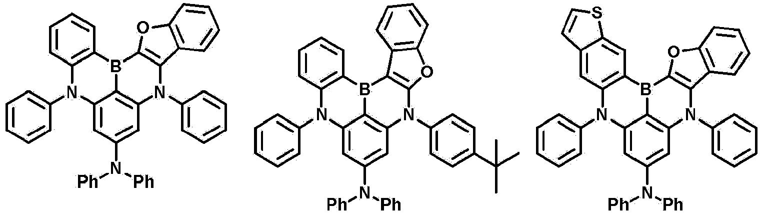

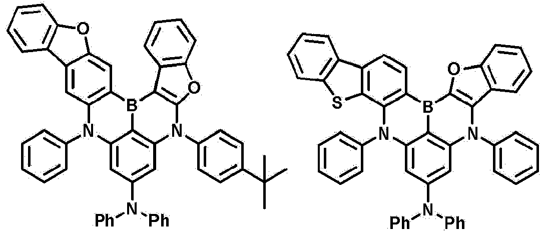

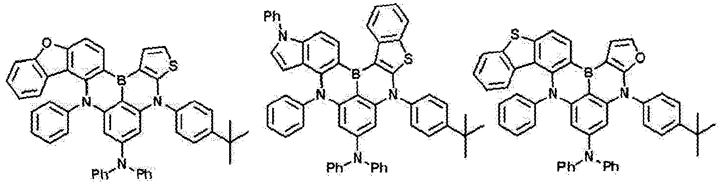

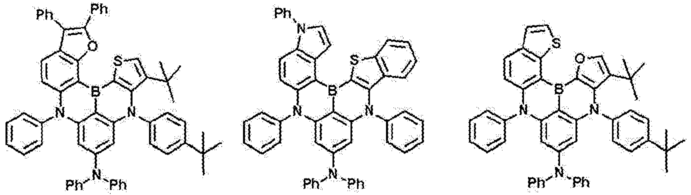

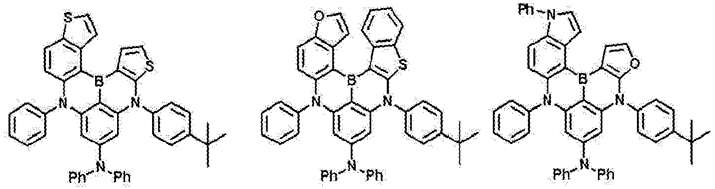

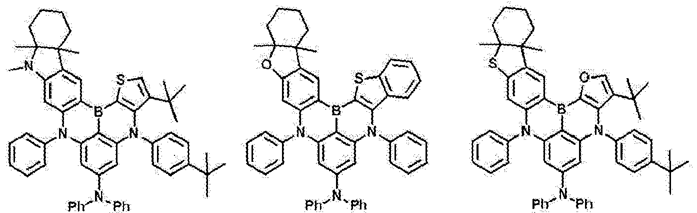

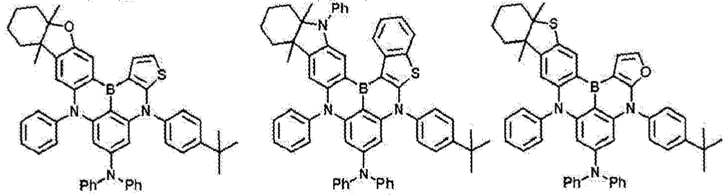

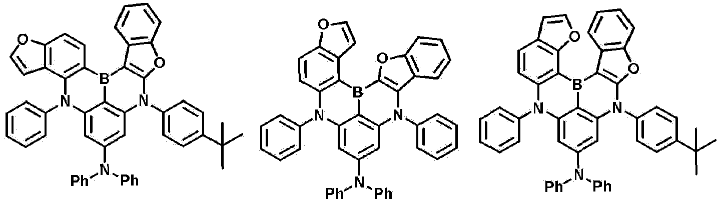

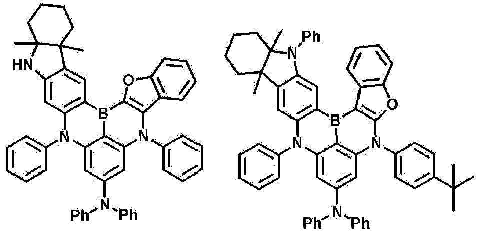

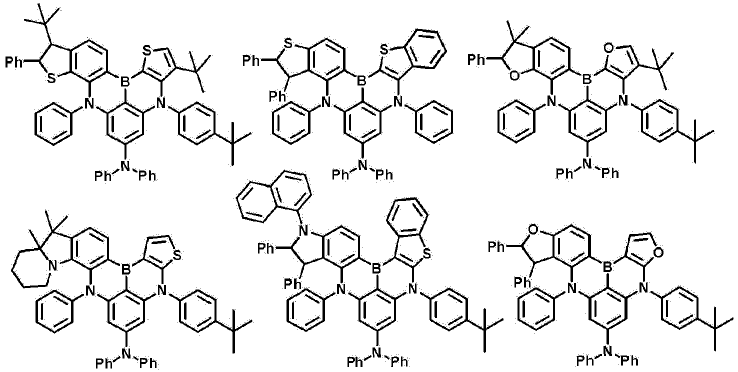

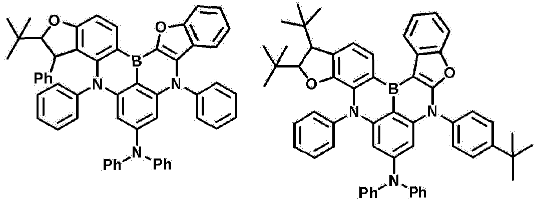

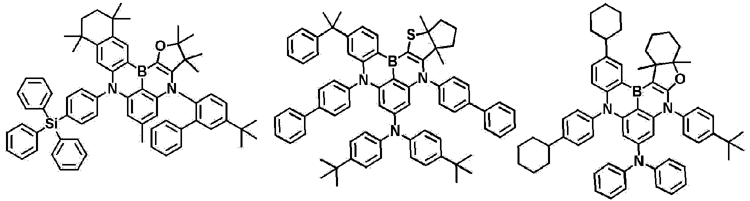

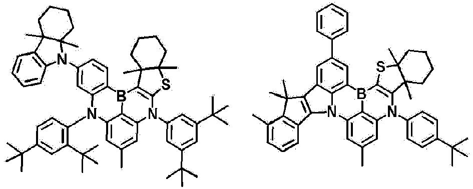

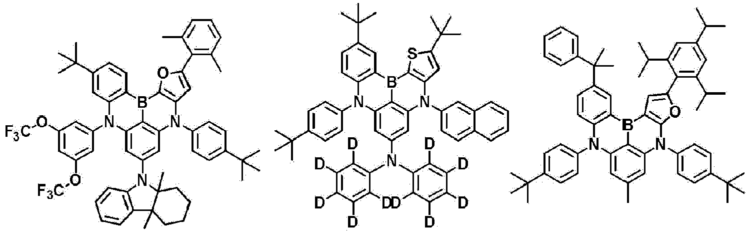

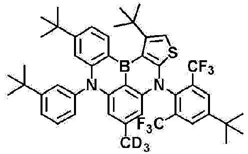

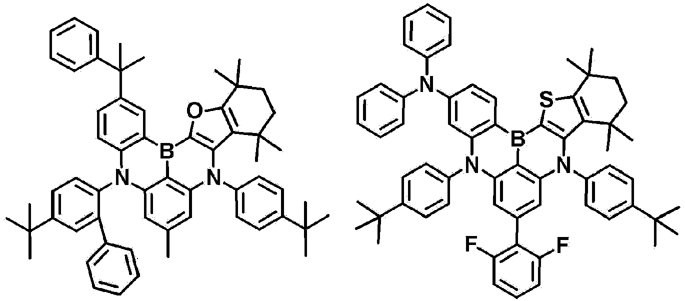

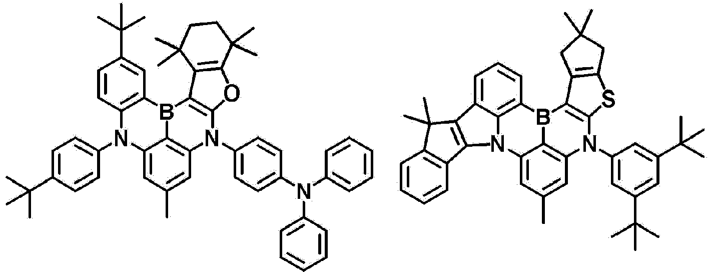

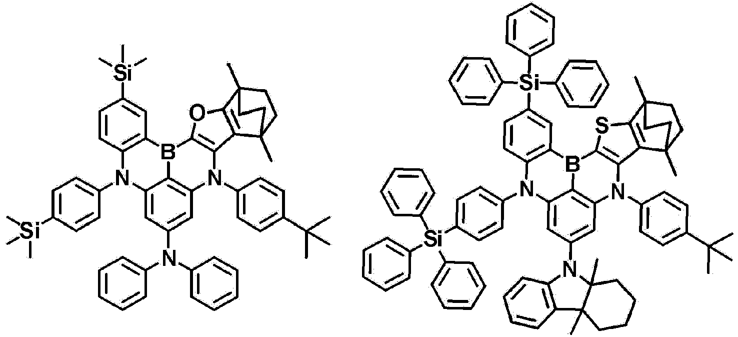

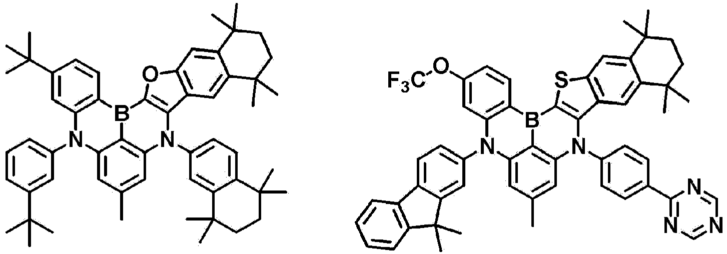

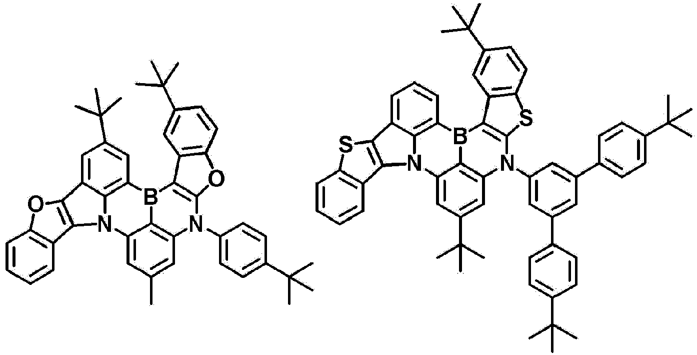

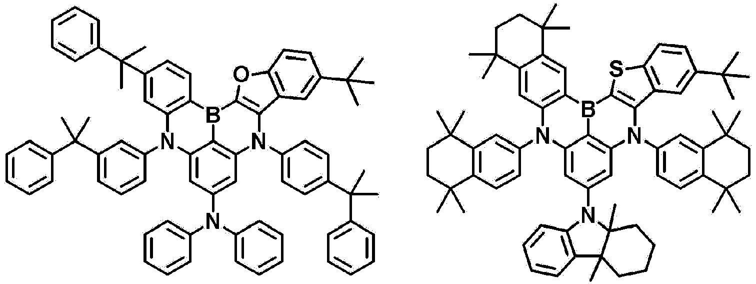

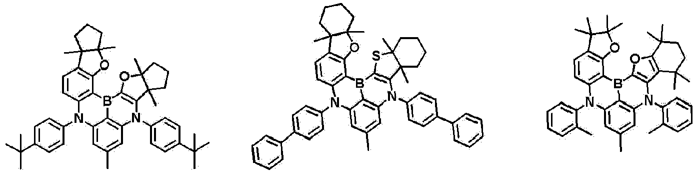

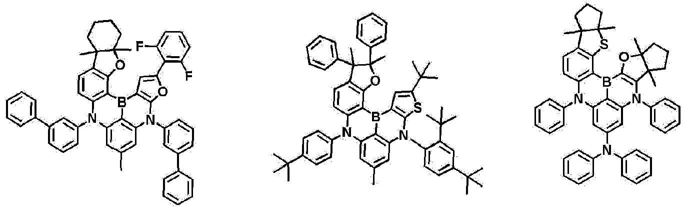

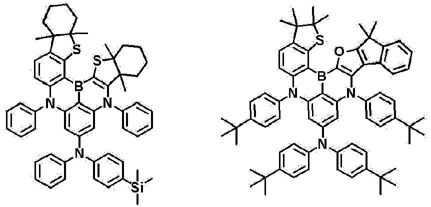

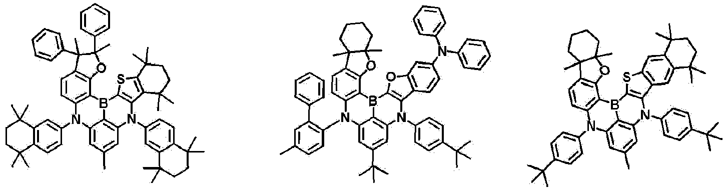

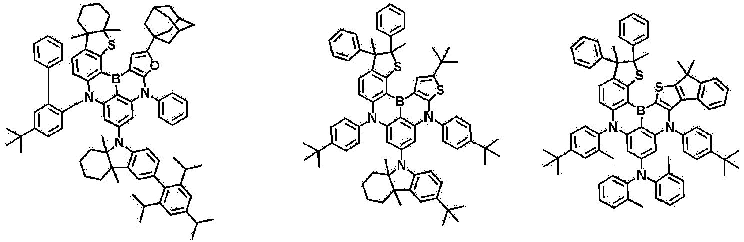

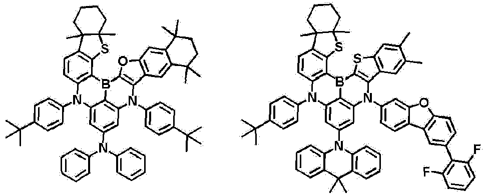

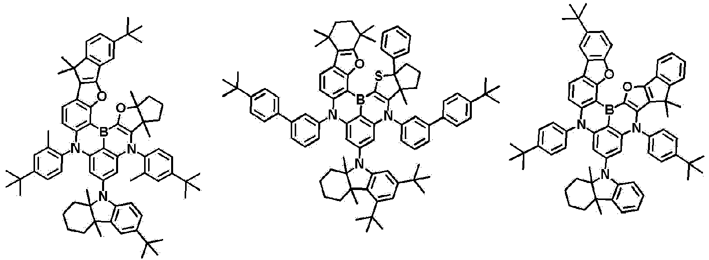

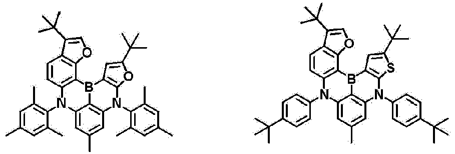

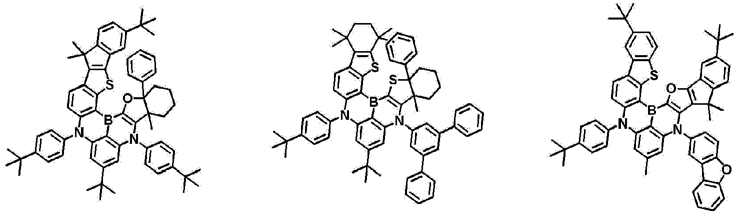

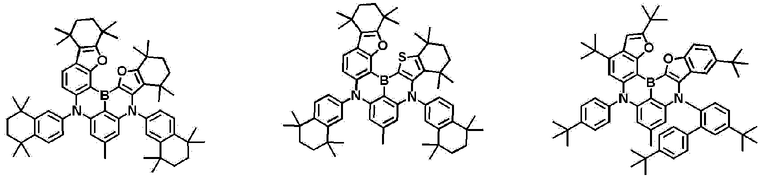

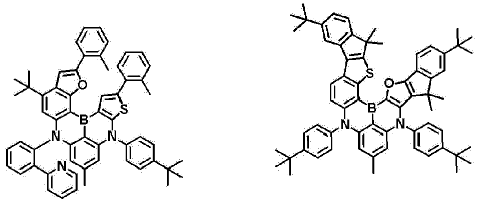

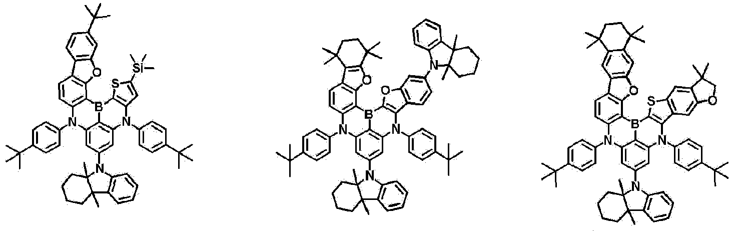

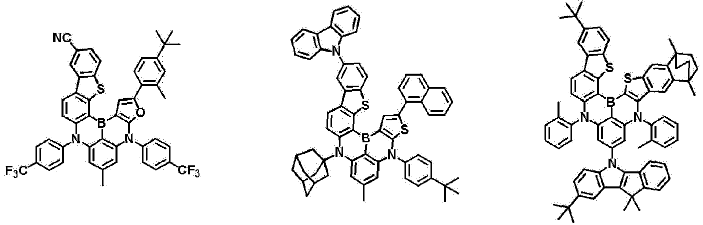

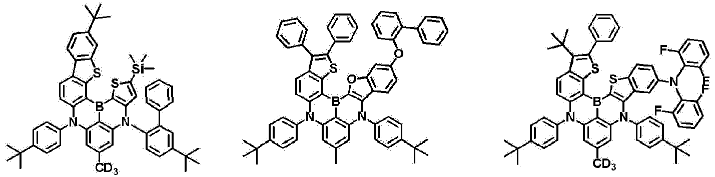

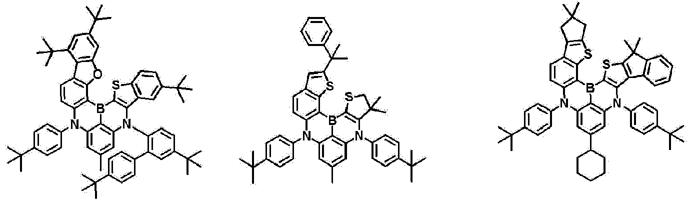

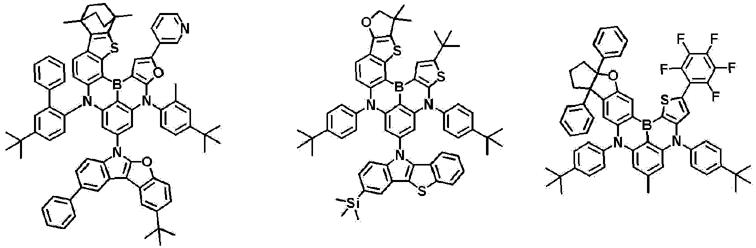

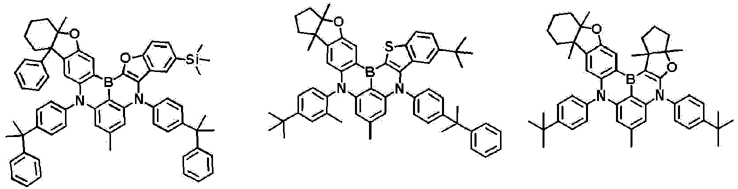

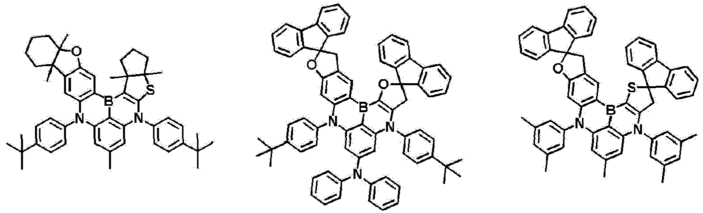

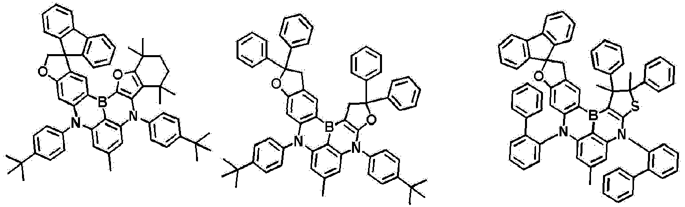

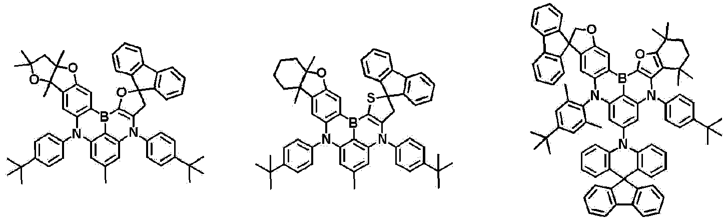

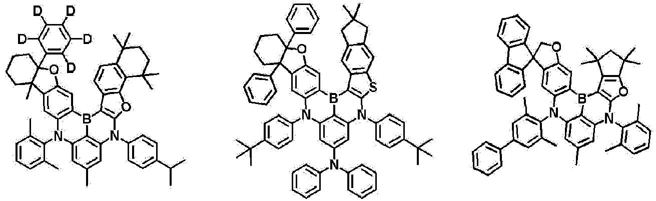

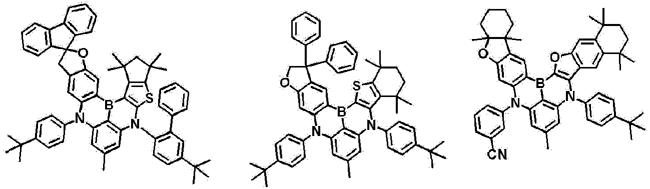

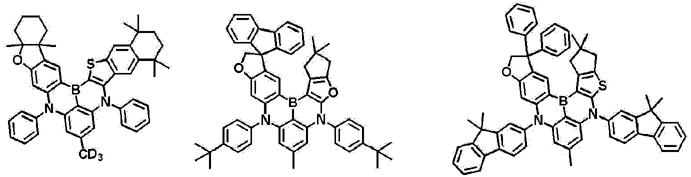

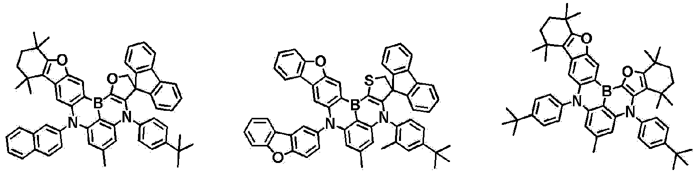

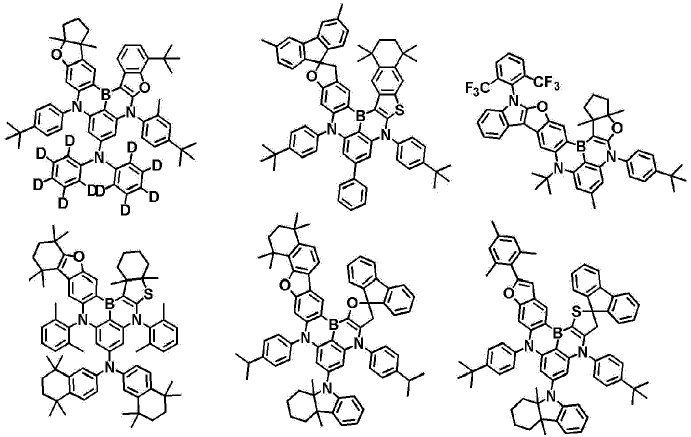

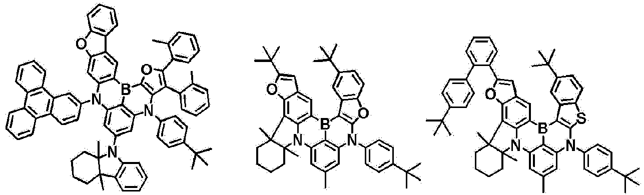

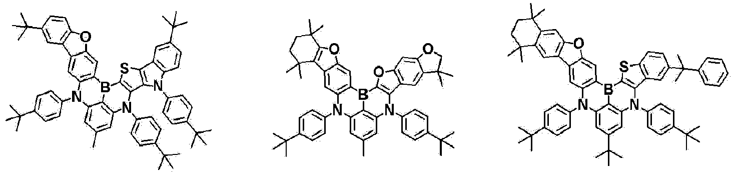

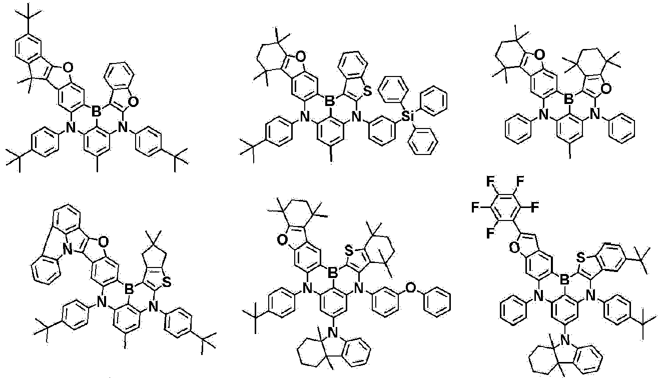

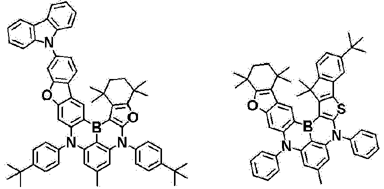

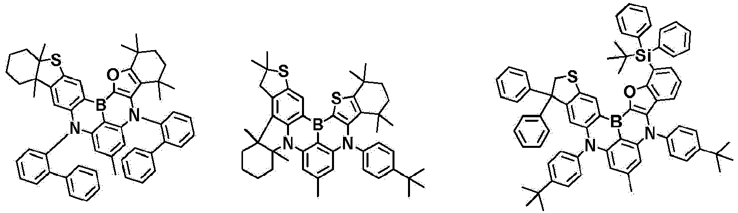

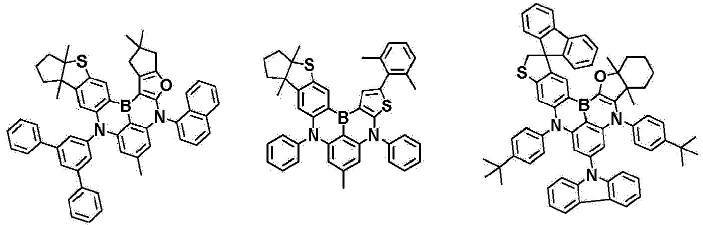

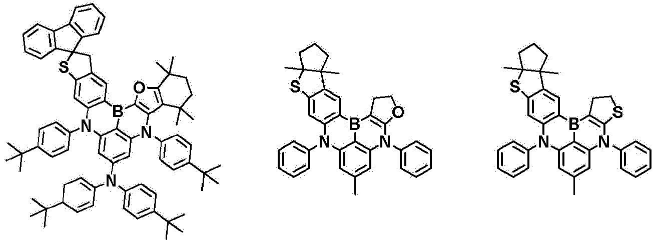

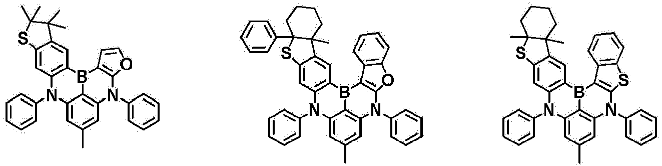

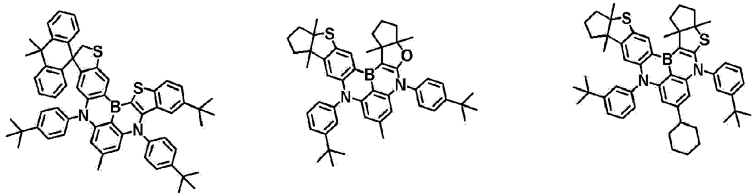

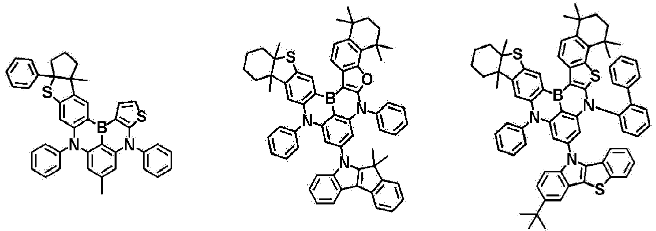

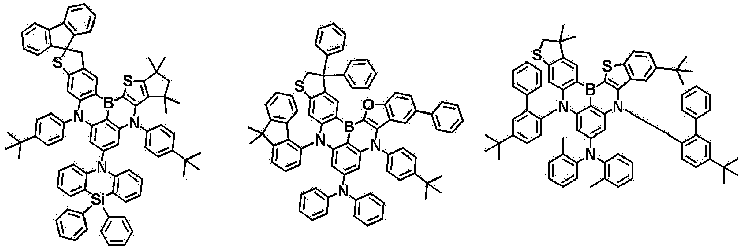

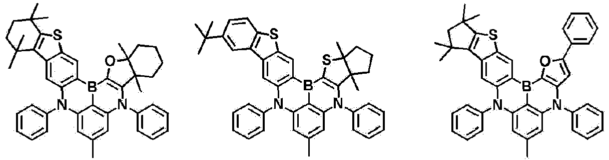

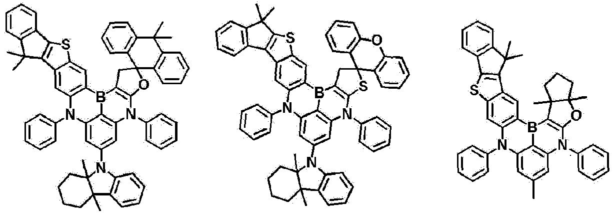

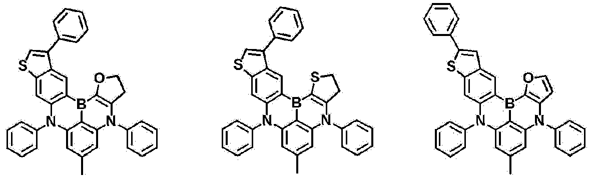

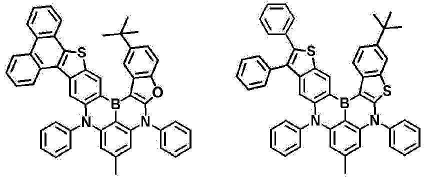

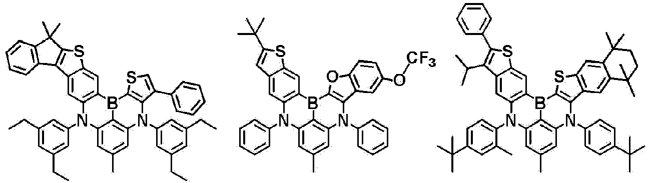

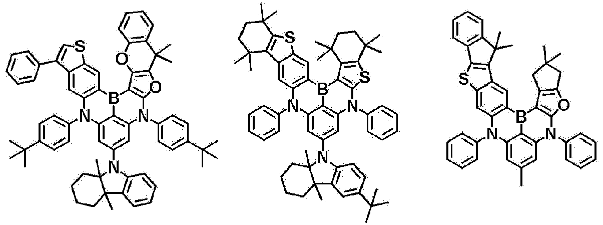

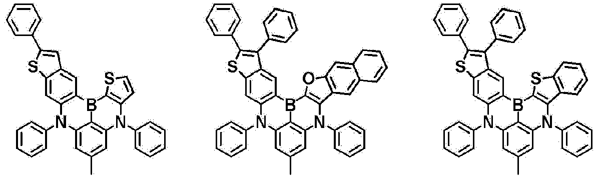

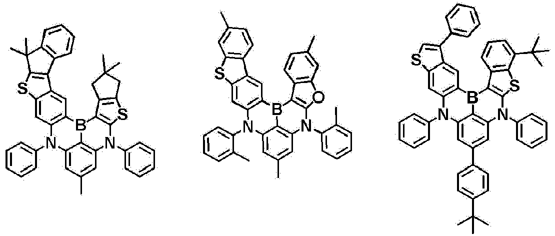

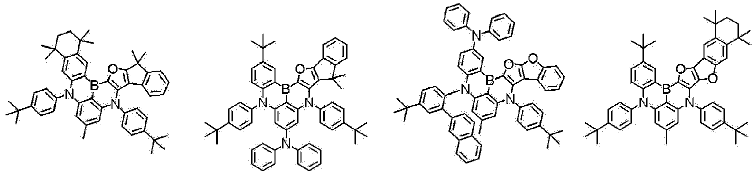

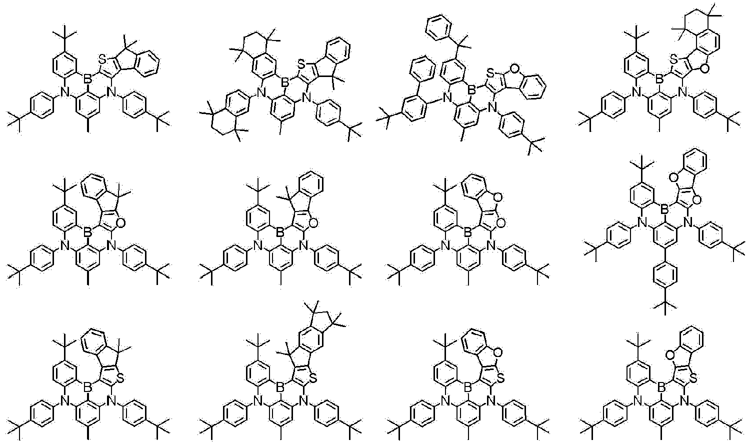

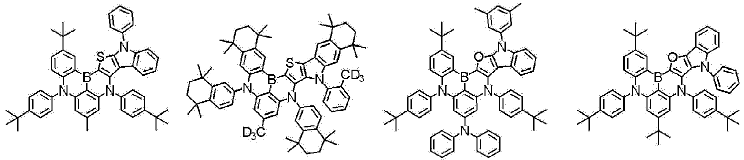

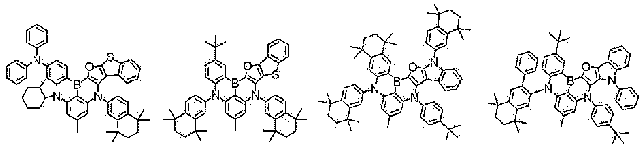

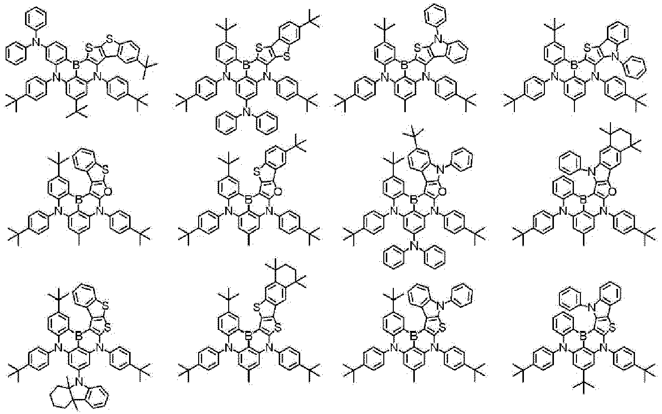

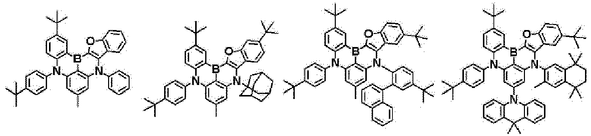

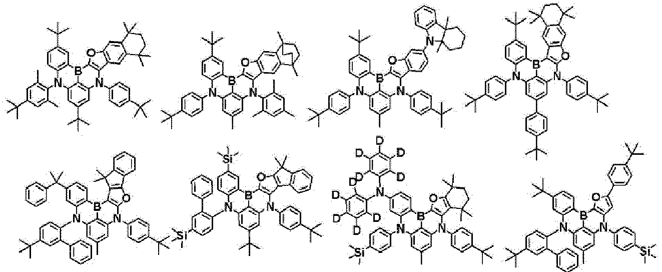

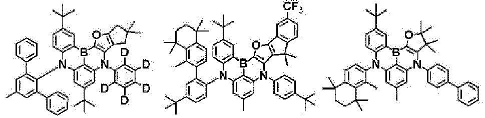

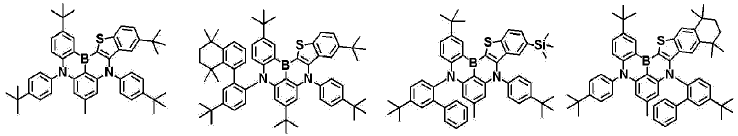

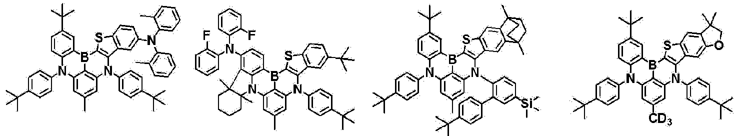

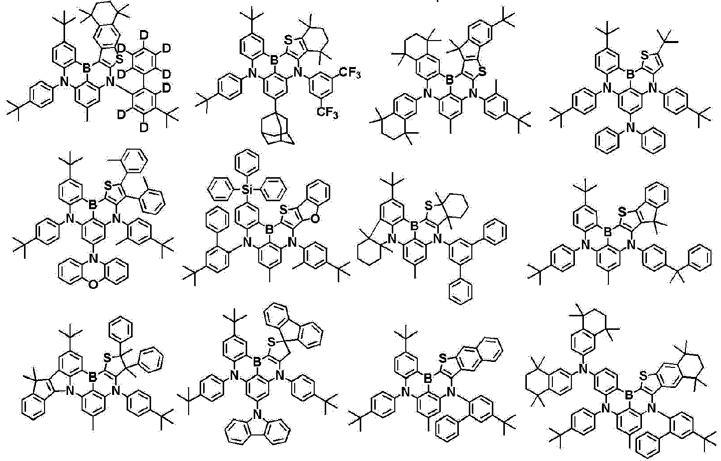

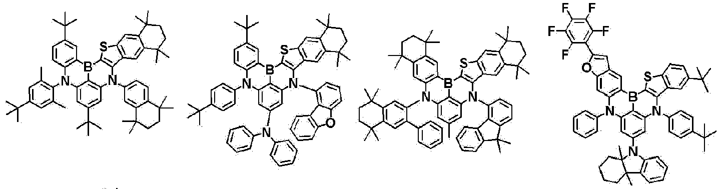

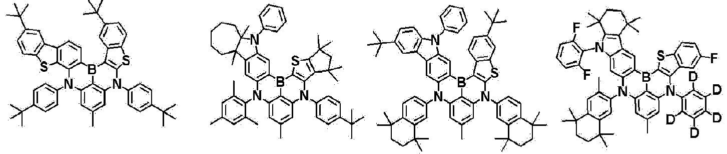

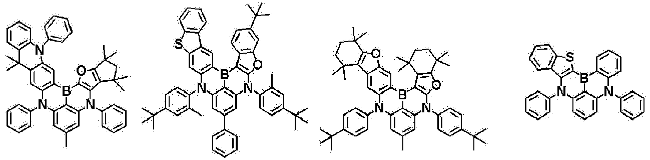

In the compounds, Ph means a phenyl group.

According to one embodiment of the present specification, when A1 is benzofuran substituted with a t-butyl group or benzothiophene substituted with a t-butyl group, R' and R" are each independently a phenyl group substituted with a t-butyl group or an isopropyl group, or a biphenyl group substituted with t-butyl group, and R2 or R3 is a t-butyl group or hydrogen in Chemical Formula 1, compounds in which R6 is a tert-butyl group, a phenyl group or an adamantyl group are excluded.

According to one embodiment of the present specification, when A1 is benzofuran or dibenzofuran in Chemical Formula 1, compounds in which R2 is a diphenylamine group unsubstituted or substituted with a t-butyl group are excluded.

According to one embodiment of the present specification, when A1 is benzofuran or dibenzofuran, R' and R" are each independently a phenyl group substituted with a t-butyl group, and R3 is a t-butyl group in Chemical Formula 1, compounds in which R6 is a methyl group or a cyclohexyl group are excluded.

According to one embodiment of the present specification, when A1 is benzofuran or dibenzofuran, R' and R" are each independently a biphenyl group substituted with a t-butyl group, and R3 is a phenyl group substituted with a t-butyl group in Chemical Formula 1, compounds in which R6 is a methyl group are excluded.

According to one embodiment of the present specification, when A1 is benzofuran or dibenzofuran, R' and R" are each independently a biphenyl group, or a phenyl group substituted with a diphenylamine group, and R1 to R4 are hydrogen in Chemical Formula 1, compounds in which R6 is a cyclohexyl group are excluded.

According to one embodiment of the present specification, when A1 of Chemical Formula 1 includes a 5-membered aliphatic or aromatic heteroring, and A1 of Chemical Formula 1 includes a 5-membered aromatic heteroring, Chemical Formula 1 includes at least one fused aliphatic hydrocarbon ring.

One embodiment of the present specification provides an organic light emitting device including the compound described above.

In the present specification, a description of a certain member being placed "on" another member includes not only a case of the one member being in contact with the another member but a case of still another member being present between the two members.

In the present specification, a description of a certain part "including" certain constituents means capable of further including other constituents, and does not exclude other constituents unless particularly stated on the contrary.

In the present specification, the "layer" has a meaning compatible with a `film' mainly used in the art, and means coating covering a target area. The size of the "layer" is not limited, and each "layer" may have the same or a different size. According to one embodiment, the size of the "layer" may be the same as the whole device, may correspond to the size of a specific functional area, or may be as small as a single subpixel.

In the present specification, a meaning of a specific A material being included in a B layer includes both i) one or more types of A materials being included in one B layer, and ii) a B layer being formed in one or more layers, and an A material being included in one or more of the B layers that is a multilayer.

In the present specification, a meaning of a specific A material being included in a C layer or a D layer includes both i) being included in one or more layers of one or more C layers, ii) being included in one or more layers of one or more D layers, or iii) being included in each of one or more C layers and one or more D layers.

One embodiment of the present specification provides an organic light emitting device including a first electrode; a second electrode provided opposite to the first electrode; and one or more organic material layers provided between the first electrode and the second electrode, wherein one or more layers of the organic material layers include the compound represented by Chemical Formula 1.

The organic material layer of the organic light emitting device of the present specification may be formed in a single layer structure, but may also be formed in a multilayer structure in which two or more organic material layers are laminated. For example, the organic light emitting device of the present specification may have a structure including a hole injection layer, a hole transfer layer, a light emitting layer, an electron transfer layer, an electron injection layer, an electron blocking layer, a hole blocking layer and the like. However, the structure of the organic light emitting device is not limited thereto, and may include a smaller number of organic layers.

In one embodiment of the present specification, the organic material layer includes a light emitting layer, and the light emitting layer includes the compound represented by Chemical Formula 1.

In one embodiment of the present specification, the organic material layer includes a light emitting layer, and the light emitting layer includes the compound represented by Chemical Formula 1 as a dopant of the light emitting layer.

In one embodiment of the present specification, the organic material layer includes a light emitting layer, and the light emitting layer includes the compound represented by Chemical Formula 1 as a blue fluorescent dopant of the light emitting layer.

In one embodiment of the present specification, the organic light emitting device further includes one, two or more layers selected from the group consisting of a hole injection layer, a hole transfer layer, a light emitting layer, an electron transfer layer, an electron injection layer, a hole blocking layer and an electron blocking layer.

In one embodiment of the present specification, the light emitting layer further includes a host compound.

In one embodiment of the present specification, the light emitting layer further includes a host compound, and in the host compound, at least one hydrogen at a substitutable position is substituted with deuterium.

In one embodiment of the present specification, when the host compound is substituted with deuterium, the host compound is substituted with deuterium by 30% or more. In another embodiment, the host compound is substituted with deuterium by 40% or more. In another embodiment, the host compound is substituted with deuterium by 60% or more. In another embodiment, the host compound is substituted with deuterium by 80% or more. In another embodiment, the host compound is substituted with deuterium by 100%.

In one embodiment of the present specification, the light emitting layer further includes a compound represented by the following Chemical Formula H.

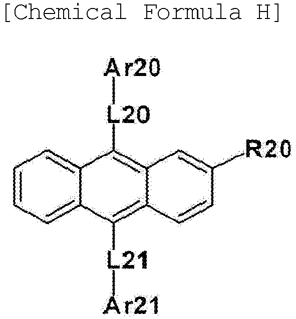

In Chemical Formula H,
L20 and L21 are the same as or different from each other, and each independently a direct bond; a substituted or unsubstituted arylene group; or a substituted or unsubstituted heteroarylene group,
Ar20 and Ar21 are the same as or different from each other, and each independently hydrogen; deuterium; a substituted or unsubstituted aryl group; or a substituted or unsubstituted heterocyclic group, and
R20 is hydrogen; deuterium; a halogen group; a substituted or unsubstituted alkyl group; a substituted or unsubstituted cycloalkyl group; a substituted or unsubstituted aryl group; or a substituted or unsubstituted heterocyclic group.

In one embodiment of the present specification, L20 and L21 are the same as or different from each other, and each independently a direct bond; a monocyclic or polycyclic arylene group having 6 to 30 carbon atoms; or a monocyclic or polycyclic heteroarylene group having 2 to 30 carbon atoms.

In one embodiment of the present specification, L20 and L21 are the same as or different from each other, and each independently a direct bond; a monocyclic or polycyclic arylene group having 6 to 20 carbon atoms; or a monocyclic or polycyclic heteroarylene group having 2 to 20 carbon atoms.

In one embodiment of the present specification, L20 and L21 are the same as or different from each other, and each independently a direct bond; a phenylene group; a biphenylylene group; a naphthylene group; a divalent dibenzofuran group; or a divalent dibenzothiophene group.

In one embodiment of the present specification, Ar20 and Ar21 are the same as or different from each other, and each independently a substituted or unsubstituted monocyclic or polycyclic aryl group having 6 to 30 carbon atoms; or a substituted or unsubstituted monocyclic or polycyclic heterocyclic group having 2 to 30 carbon atoms.

In one embodiment of the present specification, Ar20 and Ar21 are the same as or different from each other, and each independently a substituted or unsubstituted monocyclic or polycyclic aryl group having 6 to 20 carbon atoms; or a substituted or unsubstituted monocyclic or polycyclic heterocyclic group having 2 to 20 carbon atoms.

In one embodiment of the present specification, Ar20 and Ar21 are the same as or different from each other, and each independently a substituted or unsubstituted monocyclic to tetracyclic aryl group having 6 to 20 carbon atoms; or a substituted or unsubstituted monocyclic to tetracyclic heterocyclic group having 2 to 20 carbon atoms.

In one embodiment of the present specification, Ar20 and Ar21 are the same as or different from each other, and each independently a substituted or unsubstituted phenyl group; a substituted or unsubstituted biphenyl group; a substituted or unsubstituted terphenyl group; a substituted or unsubstituted naphthyl group; a substituted or unsubstituted anthracene group; a substituted or unsubstituted phenanthrene group; a substituted or unsubstituted phenalene group; a substituted or unsubstituted fluorene group; a substituted or unsubstituted benzofluorene group; a substituted or unsubstituted furan group; a substituted or unsubstituted thiophene group; a substituted or unsubstituted dibenzofuran group; a substituted or unsubstituted naphthobenzofuran group; a substituted or unsubstituted dibenzothiophene group; or a substituted or unsubstituted naphthobenzothiophene group.

In one embodiment of the present specification, Ar20 and Ar21 are the same as or different from each other, and each independently a phenyl group unsubstituted or substituted with deuterium, or a monocyclic or polycyclic aryl group having 6 to 20 carbon atoms; a biphenyl group unsubstituted or substituted with a monocyclic or polycyclic aryl group having 6 to 20 carbon atoms; a naphthyl group unsubstituted or substituted with deuterium, or a monocyclic or polycyclic aryl group having 6 to 20 carbon atoms; a dibenzofuran group unsubstituted or substituted with a monocyclic or polycyclic aryl group having 6 to 20 carbon atoms; a naphthobenzofuran group unsubstituted or substituted with a monocyclic or polycyclic aryl group having 6 to 20 carbon atoms; a dibenzothiophene group unsubstituted or substituted with a monocyclic or polycyclic aryl group having 6 to 20 carbon atoms; or a naphthobenzothiophene group unsubstituted or substituted with a monocyclic or polycyclic aryl group having 6 to 20 carbon atoms.

In one embodiment of the present specification, Ar20 and Ar21 are the same as or different from each other, and each independently a phenyl group unsubstituted or substituted with deuterium; a biphenyl group; a naphthyl group unsubstituted or substituted with deuterium; a dibenzofuran group; a naphthobenzofuran group; a dibenzothiophene group; or a naphthobenzothiophene group.

In one embodiment of the present specification, Ar20 is a substituted or unsubstituted heterocyclic group, and Ar21 is a substituted or unsubstituted aryl group.

In one embodiment of the present specification, R20 is hydrogen; deuterium; a halogen group; a substituted or unsubstituted linear or branched alkyl group having 1 to 30 carbon atoms; a substituted or unsubstituted monocyclic or polycyclic cycloalkyl group having 3 to 30 carbon atoms; a substituted or unsubstituted monocyclic or polycyclic aryl group having 6 to 30 carbon atoms; or a substituted or unsubstituted monocyclic or polycyclic heterocyclic group having 2 to 30 carbon atoms.

In one embodiment of the present specification, R20 is hydrogen; deuterium; fluorine; a substituted or unsubstituted linear or branched alkyl group having 1 to 10 carbon atoms; a substituted or unsubstituted monocyclic or polycyclic cycloalkyl group having 3 to 10 carbon atoms; a substituted or unsubstituted monocyclic or polycyclic aryl group having 6 to 30 carbon atoms; or a substituted or unsubstituted monocyclic or polycyclic heterocyclic group having 2 to 30 carbon atoms.

In one embodiment of the present specification, R20 is hydrogen; deuterium; a phenyl group unsubstituted or substituted with deuterium, a phenyl group or a naphthyl group; a naphthyl group unsubstituted or substituted with deuterium or a phenyl group; a biphenyl group; a dibenzofuran group; or a dibenzothiophene group.

In one embodiment of the present specification, when the compound represented by Chemical Formula H is substituted with deuterium, hydrogen at a substitutable position may be substituted with deuterium by 30% or more. In another embodiment, hydrogen at a substitutable position is substituted with deuterium by 40% or more in the structure of Chemical Formula H. In another embodiment, hydrogen at a substitutable position is substituted with deuterium by 60% or more in the structure of Chemical Formula H.

In another embodiment, hydrogen at a substitutable position is substituted with deuterium by 80% or more in the structure of Chemical Formula H. In another embodiment, hydrogen at a substitutable position is substituted with deuterium by 100% in the structure of Chemical Formula H.

In one embodiment of the present specification, the compound represented by Chemical Formula H is any one selected from among the following compounds.

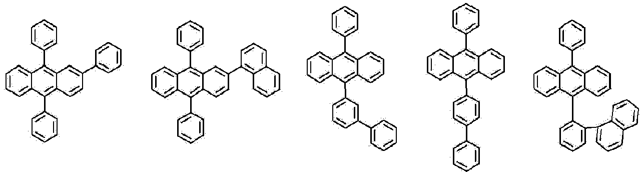

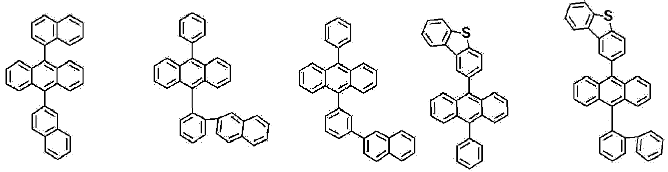

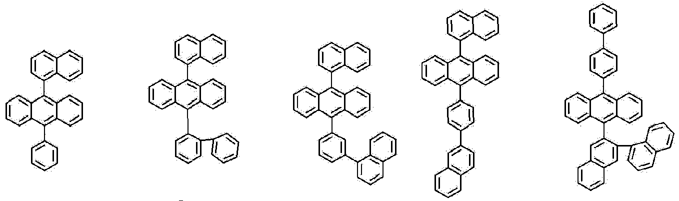

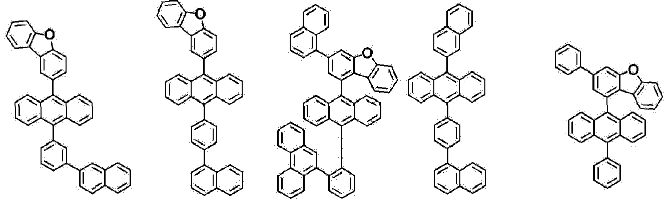

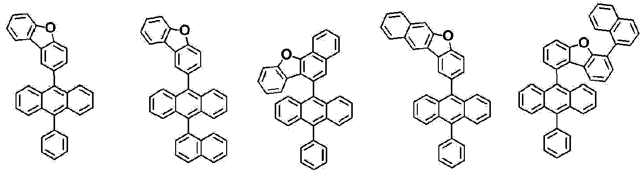

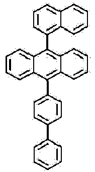

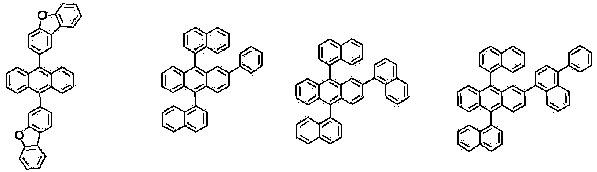

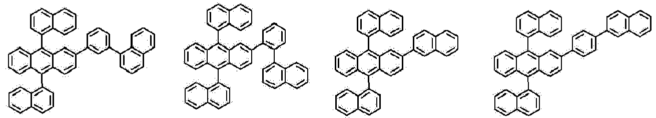

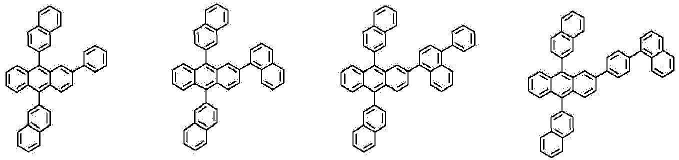

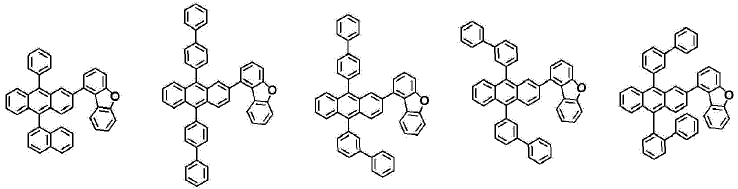

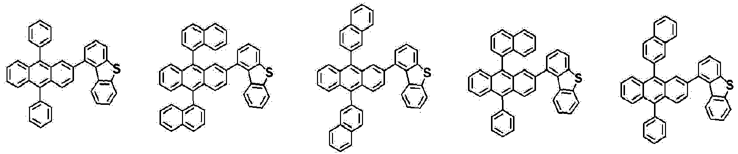

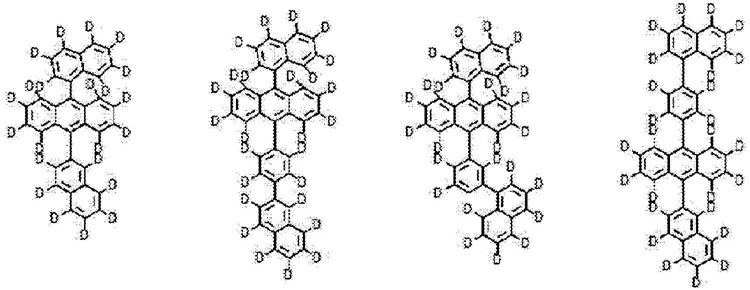

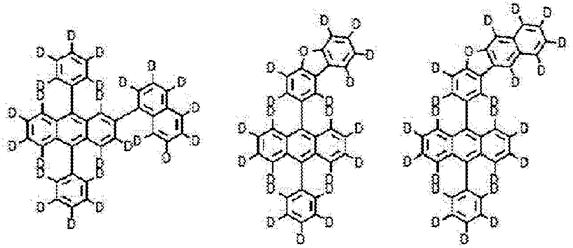

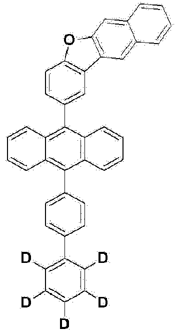

In one embodiment of the present specification, the compound represented by Chemical Formula 1 is used as a dopant, and the compound represented by Chemical Formula H is used as a host in the light emitting layer.

In one embodiment of the present specification, when the light emitting layer includes a host and a dopant, a content of the dopant may be selected in a range of 0.01 parts by weight to 10 parts by weight based on 100 parts by weight of the light emitting layer, however, the content is not limited thereto.

In one embodiment of the present specification, the light emitting layer includes a host and a dopant, and the host and the dopant are included in a weight ratio of 99:1 to 1:99, preferably in a weight ratio of 99: 1 to 70:30, and more preferably in a weight ratio of 99:1 to 90:10.

The light emitting layer may further include a host material, and the host includes fused aromatic ring derivatives, heteroring-containing compounds or the like. Specifically, anthracene derivatives, pyrene derivatives, naphthalene derivatives, pentacene derivatives, phenanthrene compounds, fluoranthene compounds or the like may be included as the fused aromatic ring derivative, and carbazole derivatives, dibenzofuran derivatives, ladder-type furan compounds, pyrimidine derivatives, triazine derivatives or the like may be included as the heteroring-containing compound, and mixtures of two or more types thereof may be included, however, the host material is not limited thereto.

According to one embodiment of the present specification, the organic material layer includes a light emitting layer, and the light emitting layer includes one or more types of dopants and a host.

According to one embodiment of the present specification, the organic material layer includes a light emitting layer, and the light emitting layer includes two or more types of mixed dopants and a host.

According to one embodiment of the present specification, one or more of the two or more types of mixed dopants include Chemical Formula 1, and the host includes the compound represented by Chemical Formula H. One or more of the two or more types of mixed dopants include Chemical Formula 1, and as the rest, dopant materials known in the art may be used, however, the dopant is not limited thereto.

According to one embodiment of the present specification, one or more of the two or more types of mixed dopants include Chemical Formula 1, and as the rest, one or more of boron-based compounds, pyrene-based compounds and delayed fluorescence-based compounds different from Chemical Formula 1 may be used, however, the dopant is not limited thereto.

According to one embodiment of the present specification, the organic material layer includes a light emitting layer, and the light emitting layer includes one or more types of hosts.

According to one embodiment of the present specification, the organic material layer includes a light emitting layer, and the light emitting layer includes two or more types of mixed hosts.

According to one embodiment of the present specification, one or more of the two or more types of mixed hosts are the compound represented by Chemical Formula H.

According to one embodiment of the present specification, the two or more types of mixed hosts are different from each other, and each independently the compound represented by Chemical Formula H.

According to one embodiment of the present specification, the organic material layer includes a light emitting layer, and the light emitting layer includes two types of mixed hosts.

According to one embodiment of the present specification, the organic material layer includes a light emitting layer, the light emitting layer includes two types of mixed hosts, the two types of mixed hosts are different from each other, and the two types of hosts are the compound represented by Chemical Formula H.

According to one embodiment of the present specification, the organic material layer includes a light emitting layer, the light emitting layer includes a first host represented by Chemical Formula H; and a second host represented by Chemical Formula H, and the first host and the second host are different from each other.

According to one embodiment of the present specification, the first host: the second host are included in a weight ratio of 95:5 to 5:95, and preferably in a weight ratio of 70:30 to 30:70.

According to one embodiment of the present specification, the organic material layer includes a light emitting layer, and the light emitting layer includes one or more types of hosts and a dopant.

According to one embodiment of the present specification, the organic material layer includes a light emitting layer, the light emitting layer includes one or more types of hosts and a dopant, the hosts include the compound represented by Chemical Formula H, and the dopant includes the compound represented by Chemical Formula 1.

According to one embodiment of the present specification, the organic material layer includes a light emitting layer, and the light emitting layer includes two or more types of mixed hosts and a dopant.

According to one embodiment of the present specification, one or more of the two or more types of mixed hosts include the compound represented by Chemical Formula H, and the dopant includes the compound represented by Chemical Formula 1.

In the present specification, the two or more types of mixed hosts are different from each other.

According to one embodiment of the present specification, the organic material layer includes a light emitting layer, and the light emitting layer includes two types of mixed hosts and a dopant.

According to one embodiment of the present specification, the two types of mixed hosts are different from each other, and each independently include the compound represented by Chemical Formula H, and the dopant includes the compound represented by Chemical Formula 1.

According to one embodiment of the present specification, the organic material layer includes a light emitting layer, the light emitting layer includes a first host represented by Chemical Formula H; a second host represented by Chemical Formula H; and a dopant represented by Chemical Formula 1, and the first host and the second host are different from each other.

According to one embodiment of the present specification, the organic material layer uses one or more types of hosts and one or more types of dopants, the one or more types of hosts include the compound represented by Chemical Formula H, and the one or more types of dopants include the compound represented by Chemical Formula 1.

According to one embodiment of the present specification, the organic material layer uses two or more types of mixed hosts and two or more types of mixed dopants, the two or more types of mixed hosts may use the same materials as described above, and the two or more types of mixed dopants may use the same materials as described above.

In one embodiment of the present specification, the organic light emitting device includes a first electrode; a second electrode; a light emitting layer provided between the first electrode and the second electrode; and two or more organic material layers provided between the light emitting layer and the first electrode, or between the light emitting layer and the second electrode, wherein at least one of the two or more organic material layers includes the compound represented by Chemical Formula 1.

In one embodiment of the present specification, as the two or more organic material layers, two or more may be selected from the group consisting of a light emitting layer, a hole transfer layer, a hole injection layer, a layer carrying hole transfer and hole injection at the same time, and an electron blocking layer.

In one embodiment of the present specification, the organic light emitting device may include two or more electron transfer layers, but is not limited thereto.

In one embodiment of the present specification, the organic material layer includes two or more electron transfer layers, and at least one of the two or more electron transfer layers includes the compound represented by Chemical Formula 1. Specifically, in one embodiment of the present specification, the compound represented by Chemical Formula 1 may be included in one of the two or more electron transfer layers, or may be included in each of the two or more electron transfer layers.

In addition, when the compound is included in each of the two or more electron transfer layers in one embodiment of the present specification, materials other than the compound represented by Chemical Formula 1 may be the same as or different from each other.

When the organic material layer including the compound represented by Chemical Formula 1 is an electron transfer layer, the electron transfer layer may further include an n-type dopant. As the n-type dopant, those known in the art may be used, and for example, metals or metal complexes may be used. For example, the electron transfer layer including the compound represented by Chemical Formula 1 may further include lithium quinolate (LiQ).

In one embodiment of the present specification, the organic material layer includes two or more hole transfer layers, and at least one of the two or more hole transfer layers includes the compound represented by Chemical Formula 1. Specifically, in one embodiment of the present specification, the compound represented by Chemical Formula 1 may be included in one of the two or more hole transfer layers, or may be included in each of the two or more hole transfer layers.

In addition, when the compound represented by Chemical Formula 1 is included in each of the two or more hole transfer layers in one embodiment of the present specification, materials other than the compound represented by Chemical Formula 1 may be the same as or different from each other.

In one embodiment of the present specification, the organic material layer may further include, in addition to the organic material layer including the compound represented by Chemical Formula 1, a hole injection layer or a hole transfer layer including a compound including an arylamine group, a carbazolyl group or a benzocarbazolyl group.

In one embodiment of the present specification, the first electrode is an anode or a cathode.

In one embodiment of the present specification, the second electrode is a cathode or an anode.

In one embodiment of the present specification, the organic light emitting device may be an organic light emitting device having a structure in which an anode, one or more organic material layers and a cathode are consecutively laminated on a substrate (normal type).

In one embodiment of the present specification, the organic light emitting device may be an organic light emitting device having a structure in a reverse direction in which a cathode, one or more organic material layers and an anode are consecutively laminated on a substrate (inverted type).

For example, structures of the organic light emitting device according to one embodiment of the present specification are illustrated in FIG. 1 to FIG. 3. FIG. 1 to FIG. 3 illustrate the organic light emitting device, and the organic light emitting device is not limited thereto.

FIG. 1 illustrates a structure of the organic light emitting device in which a substrate (1), a first electrode (2), a light emitting layer (3) and a second electrode (4) are consecutively laminated. In such a structure, the compound may be included in the light emitting layer (3).

FIG. 2 illustrates a structure of the organic light emitting device in which a substrate (1), a first electrode (2), a hole injection layer (5), a hole transfer layer (8), an electron blocking layer (9), a light emitting layer (3), a hole blocking layer (6), an electron injection and transfer layer (7) and a second electrode (4) are consecutively laminated. In such a structure, the compound may be included in one or more of the light emitting layer (3), the hole blocking layer (6), the electron injection and transfer layer (7) and the hole injection layer (8).

FIG. 3 illustrates a structure of the organic light emitting device in which a substrate (1), a first electrode (2), a hole injection layer (5), a hole transfer layer (8), an electron blocking layer (9), a light emitting layer (3), a first electron transfer layer (10), a second electron transfer layer (11), an electron injection layer (12) and a second electrode (4) are consecutively laminated. In such a structure, the compound may be included in the light emitting layer (3).

The organic light emitting device of the present specification may be manufactured using materials and methods known in the art, except that one or more layers of the organic material layers include the compound, that is, the compound represented by Chemical Formula 1.

When the organic light emitting device includes a plurality of organic material layers, the organic material layers may be formed with the same materials or different materials.

For example, the organic light emitting device of the present specification may be manufactured by consecutively laminating a first electrode, an organic material layer and a second electrode on a substrate. Herein, the organic light emitting device may be manufactured by forming an anode on a substrate by depositing a metal, a metal oxide having conductivity, or an alloy thereof using a physical vapor deposition (PVD) method such as sputtering or e-beam evaporation, and forming an organic material layer including a hole injection layer, a hole transfer layer, a light emitting layer and an electron transfer layer thereon, and then depositing a material usable as a cathode thereon. In addition to such a method, the organic light emitting device may also be manufactured by consecutively depositing a cathode material, an organic material layer and an anode material on a substrate.

In addition, the compound represented by Chemical Formula 1 may be formed into an organic material layer using a solution coating method as well as a vacuum deposition method when manufacturing the organic light emitting device. Herein, the solution coating method means spin coating, dip coating, doctor blading, inkjet printing, screen printing, a spray method, roll coating and the like, but is not limited thereto.

In addition to such a method, the organic light emitting device may also be manufactured by consecutively laminating a cathode material, an organic material layer and an anode material on a substrate (International Patent Application Laid Open Publication No. 2003/012890). However, the manufacturing method is not limited thereto.

As the first electrode material, materials having large work function are normally preferred so that hole injection to an organic material layer is smooth. Examples thereof include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, but are not limited thereto.

As the second electrode material, materials having small work function are normally preferred so that electron injection to an organic material layer is smooth. Examples thereof include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

The light emitting layer may include a host material and a dopant material. The host material includes fused aromatic ring derivatives, heteroring-containing compounds or the like. Specifically, the fused aromatic ring derivative includes anthracene derivatives, pyrene derivatives, naphthalene derivatives, pentacene derivatives, phenanthrene compounds, fluoranthene compounds and the like, and the heteroring-containing compound includes dibenzofuran derivatives, ladder-type furan compounds, pyrimidine derivatives and the like, however, the material is not limited thereto.

The dopant material includes, in addition to the compound represented by Chemical Formula 1, aromatic amine derivatives, styrylamine compounds, boron complexes, fluoranthene compounds, metal complexes and the like. Specifically, the aromatic amine derivative is a fused aromatic ring derivative having a substituted or unsubstituted arylamine group and includes arylamine group-including pyrene, anthracene, chrysene, peryflanthene and the like. In addition, the styrylamine compound is a compound in which substituted or unsubstituted arylamine is substituted with at least one arylvinyl group, and one, two or more substituents selected from the group consisting of an aryl group, a silyl group, an alkyl group, a cycloalkyl group and an arylamine group are substituted or unsubstituted. Specifically, styrylamine, styryldiamine, styryltriamine, styryltetramine or the like is included, however, the styrylamine compound is not limited thereto. In addition, the metal complex includes iridium complexes, platinum complexes or the like, but is not limited thereto.

In the present specification, when the compound represented by Chemical Formula 1 is included in an organic material layer other than the light emitting layer, or an additional light emitting layer is provided, a light emitting material of the light emitting layer is, as a material capable of emitting light in a visible region by receiving holes and electrons from a hole transfer layer and an electron transfer layer, respectively, and binding the holes and the electrons, preferably a material having favorable quantum efficiency for fluorescence or phosphorescence. Specific examples thereof include 8-hydroxy-quinoline aluminum complexes (Alq₃); carbazole-based compounds; dimerized styryl compounds; BAlq; 10-hydroxybenzoquinoline-metal compounds; benzoxazole-, benzothiazole- and benzimidazole-based compounds; poly(p-phenylenevinylene) (PPV)-based polymers; spiro compounds; polyfluorene; rubrene, and the like, but are not limited thereto.

The hole injection layer is a layer injecting holes from an electrode. The hole injection material preferably has, by having an ability to transfer holes, a hole injection effect in a first electrode and an excellent hole injection effect for a light emitting layer or a light emitting material. In addition, the hole injection material is preferably a material having an excellent ability to prevent excitons generated in the light emitting layer from moving to an electron injection layer or an electron injection material. In addition, a material having an excellent thin film forming ability is preferred. In addition, the highest occupied molecular orbital (HOMO) of the hole injection material is preferably in between the work function of a first electrode material and the HOMO of surrounding organic material layers. Specific examples of the hole injection material include metal porphyrins, oligothiophene, arylamine-based organic materials; carbazole-based organic materials; nitrile-based organic materials; hexanitrile hexaazatriphenylene-based organic materials; quinacridone-based organic materials; perylene-based organic materials; polythiophene-based conductive polymers such as anthraquinone or polyaniline, mixtures of two or more of the examples, and the like, but are not limited thereto.

The hole transfer layer is a layer receiving holes from a hole injection layer and transferring the holes to a light emitting layer. As the hole transfer material, materials having, as a material capable of receiving holes from a first electrode or a hole injection layer and moving the holes to a light emitting layer, high mobility for the holes are preferred. Specific examples thereof include arylamine-based organic materials, carbazole-based organic materials, conductive polymers, block copolymers having conjugated parts and non-conjugated parts together, and the like, but are not limited thereto.

The electron transfer layer is a layer receiving electrons from an electron injection layer and transferring the electrons to a light emitting layer. As the electron transfer material, materials capable of favorably receiving electrons from a second electrode, moving the electrons to a light emitting layer, and having high mobility for the electrons are preferred. Specific examples thereof include Al complexes of 8-hydroxyquinoline; complexes including Alq₃; organic radical compounds; hydroxyflavon-metal complexes; triazine derivatives; LiQ and the like, but are not limited thereto. The electron transfer layer may be used together with any desired first electrode material as used in the art. Particularly, the suitable first electrode material is a common material having low work function and having an aluminum layer or a silver layer following. Specifically, cesium, barium, calcium, ytterbium, samarium and the like are included, and in each case, an aluminum layer or a silver layer follows.

The electron injection layer is a layer injecting electrons from an electrode. As the electron injection material, materials having an excellent electron transferring ability, having an electron injection effect from a second electrode, and having an excellent electron injection effect for a light emitting layer or light emitting material are preferred. In addition, materials preventing excitons generated in the light emitting layer from moving to a hole injection layer, and having an excellent thin film forming ability are preferred. Specific examples thereof include fluorenone, anthraquinodimethane, diphenoquinone, thiopyran dioxide, oxazole, oxadiazole, triazole, triazine, midazole, perylene tetracarboxylic acid, fluorenylidene methane, anthrone or the like, and derivatives thereof, metal complex compounds, nitrogen-containing 5-membered ring derivatives, mixtures of two or more of the examples, and the like, but are not limited thereto.

The metal complex compound includes 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium and the like, but is not limited thereto.

The electron blocking layer is a layer capable of enhancing lifetime and efficiency of a device by preventing electrons injected from an electron injection layer from passing through a light emitting layer and entering a hole injection layer. Known material may be used without limit, and the electron blocking layer may be formed between the light emitting layer and the hole injection layer, or between the light emitting layer and a layer carrying out hole injection and hole transfer at the same time.

The hole blocking layer is a layer blocking holes from passing a light emitting layer and reaching a cathode, and may be generally formed under the same condition as the electron injection layer. Specific examples thereof may include oxadiazole derivatives, triazole derivatives, phenanthroline derivatives, aluminum complexes, pyridine, pyrimidine or triazine derivatives and the like, but are not limited thereto.

The organic light emitting device according to the present specification may be a top-emission type, a bottom-emission type or a dual-emission type depending on the materials used.

In one embodiment of the present specification, the compound represented by Chemical Formula 1 may be included in, in addition to the organic light emitting device, an organic solar cell or an organic transistor.

The compound according to the present specification may also be used in an organic light emitting device including an organic phosphorescent device, an organic solar cell, an organic photo conductor, an organic transistor and the like under a similar principle used in the organic light emitting device. For example, the organic solar cell may have a structure including a cathode, an anode, and a photoactive layer provided between the cathode and the anode, and the photoactive layer may include the compound.

The organic light emitting device of the present specification may be manufactured using common organic light emitting device manufacturing methods and materials except that one or more layers of the organic material layers are formed using the compound described above.

### [Mode for Invention]

Hereinafter, the present specification will be described in detail with reference to examples, comparative examples and the like. However, the examples and the comparative examples according to the present specification may be modified to various other forms, and the scope of the present specification is not to be construed as being limited to the examples and the comparative examples described below. Examples and comparative examples of the present specification are provided in order to more fully describe the present specification to those having average knowledge in the art.

### [Synthesis Example]

### Synthesis Example 1: Synthesis of Compound 1

### Step 1) Synthesis of Compound 1-a

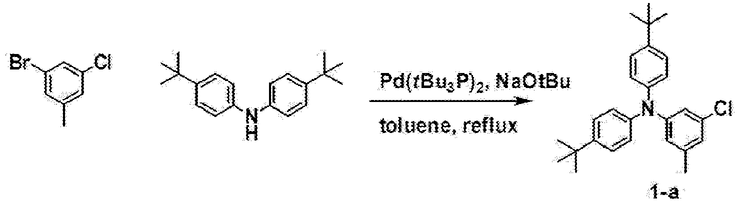

After dissolving 1-bromo-3-chloro-5-methylbenzene (146 mmol, 30 g) and bis(4-(tert-butyl)phenyl)amine (146 mmol, 41.1 g) in toluene (0.2 M, 730 ml) in a 3-neck flask, sodium tert-butoxide (219 mmol, 21 g) and bis(tri-tert-butylphosphine)palladium(0) (1.46 mmol, 0.75 g) were introduced thereto, and the result was stirred for 1 hour under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 1-a (49 g, yield 83%, MS[M+H]+=405).

### Step 2) Synthesis of Compound 1-b

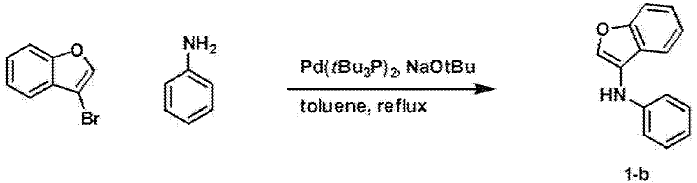

After dissolving 3-bromobenzofuran (101.5 mmol, 20 g) and aniline (101.5 mmol, 9.45 g) in toluene (0.2 M, 508 ml) in a 3-neck flask, sodium tert-butoxide (152 mmol, 14.6 g) and bis(tri-tert-butylphosphine)palladium(0) (1.0 mmol, 0.51 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 1-b (14.8 g, yield 70%, MS[M+H]+=209).

### Step 3) Synthesis of Compound 1-c

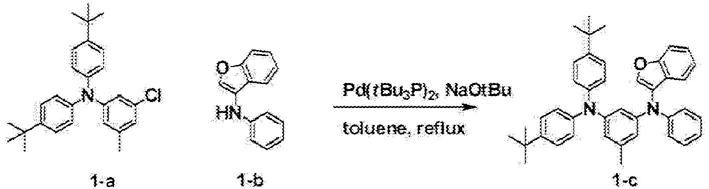

After dissolving Compound 1-a (49.3 mmol, 20 g) and Compound 1-b (49.3 mmol, 13.7 g) in toluene (0.2 M, 246 ml) in a 3-neck flask, sodium tert-butoxide (73.4 mmol, 7.1 g) and bis(tri-tert-butylphosphine)palladium(0) (0.49 mmol, 0.25 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 1-c (18.4 g, yield 65%, MS[M+H]+=579).

### Step 4) Synthesis of Compound 1

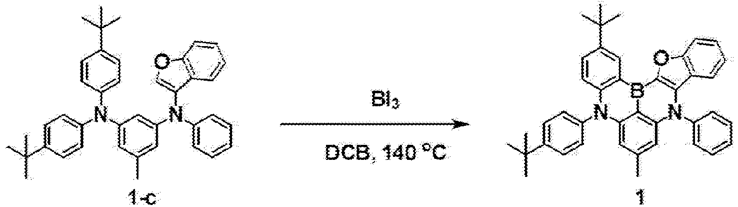

After dissolving Compound 1-c (31.8 mmol, 18.4 g) in 1,2-dichlorobenzene (0.1 M, 320 ml) in a 3-neck flask, boron triiodide (50.9 mmol, 19.9 g) was introduced thereto, and the result was stirred for 3 hours at 140°C under the argon atmosphere. The reaction material was cooled to 0°C, and after adding N,N-diisopropylethylamine (286 mmol, 37 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 1 (3.1 g, yield 17%, MS[M+H]+=587).

### Synthesis Example 2: Synthesis of Compound 2

### Step 1) Synthesis of Compound 2-a

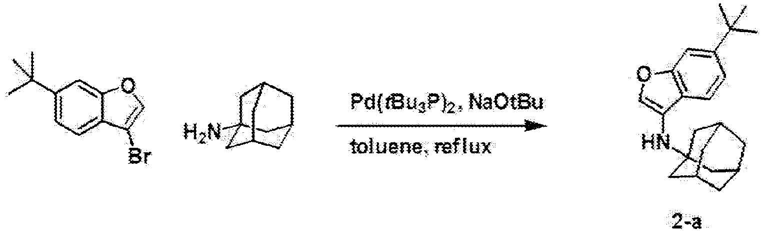

After dissolving 1-adamantylamine (132.2 mmol, 20 g) and 6-tert-butyl-3-bromobenzofuran (132.2 mmol, 33.5 g) in toluene (0.2 M, 661 ml) in a 3-neck flask, sodium tert-butoxide (198 mmol, 19 g) and bis(tri-tert-butylphosphine)palladium(0) (1.32 mmol, 0.68 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 2-a (31.2 g, yield 73%, MS[M+H]+=323).

### Step 2) Synthesis of Compound 2-b

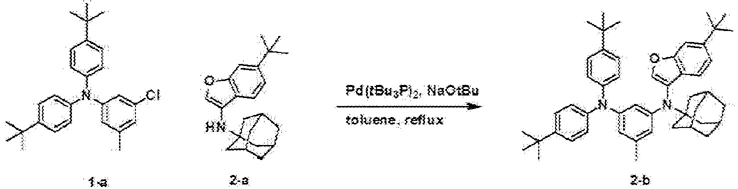

After dissolving Compound 1-a (61.6 mmol, 25 g) and Compound 2-a (61.6 mmol, 19.9 g) in toluene (0.2 M, 307 ml) in a 3-neck flask, sodium tert-butoxide (92.4 mmol, 8.9 g) and bis(tri-tert-butylphosphine)palladium(0) (0.62 mmol, 0.31 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 2-b (24.4 g, yield 57%, MS[M+H]+=693).

### Step 3) Synthesis of Compound 2

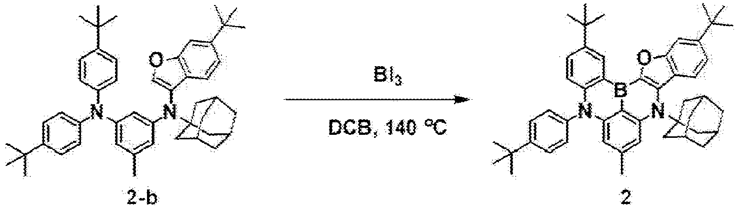

After dissolving Compound 2-b (30.8 mmol, 22.4 g) in 1,2-dichlorobenzene (0.1 M, 308 ml) in a 3-neck flask, boron triiodide (49.3 mmol, 19.3 g) was introduced thereto, and the result was stirred for 3 hours at 140°C under the argon atmosphere. The reaction material was cooled to 0°C, and after adding N,N-diiso propylethylamine (277 mmol, 36 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 2 (3.4 g, yield 16%, MS[M+H]+=701).

### Synthesis Example 3: Synthesis of Compound 3

### Step 1) Synthesis of Compound 3-a

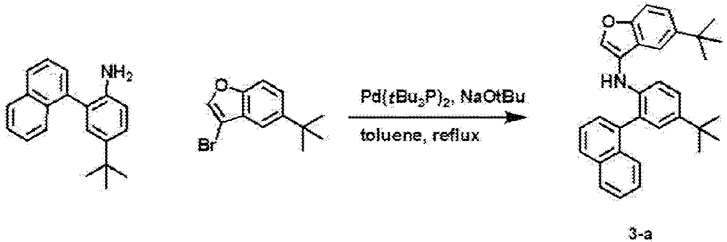

After dissolving 4-tert-butyl-2-(1-naphthalenyl)-aniline (56.5 mmol, 15.5 g) and 5-tert-butyl-3-bromobenzofuran (56.5 mmol, 14.3 g) in toluene (0.2 M, 282 ml) in a 3-neck flask, sodium tert-butoxide (85 mmol, 8.2 g) and bis(tri-tert-butylphosphine)palladium(0) (0.56 mmol, 0.29 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 3-a (21.7 g, yield 86%, MS[M+H]+=448).

### Step 2) Synthesis of Compound 3-b

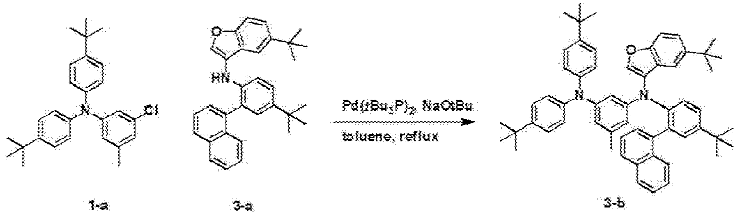

After dissolving Compound 1-a (46.8 mmol, 19 g) and Compound 3-a (46.8 mmol, 20.9 g) in toluene (0.2 M, 234 ml) in a 3-neck flask, sodium tert-butoxide (70.2 mmol, 6.7 g) and bis(tri-tert-butylphosphine)palladium(0) (0.47 mmol, 0.24 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₂ and concentrated, and the sample was purified using silica gel column chromatography to obtain Compound 3-b (24.7 g, yield 65%, MS[M+H]+=817).

### Step 3) Synthesis of Compound 3

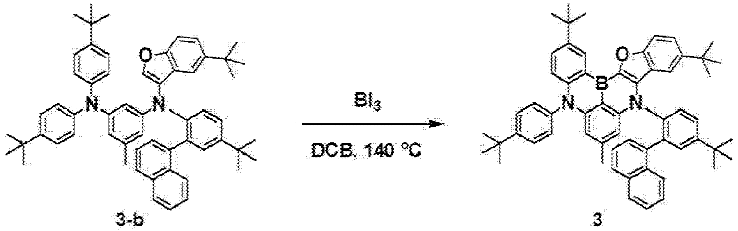

After dissolving Compound 3-b (30.2 mmol, 24.7 g) in 1,2-dichlorobenzene (0.1 M, 303 ml) in a 3-neck flask, boron triiodide (48.4 mmol, 19 g) was introduced thereto, and the result was stirred for 3 hours at 140°C under the argon atmosphere. The reaction material was cooled to 0°C, and after adding N,N-diiso propylethylamine (272 mmol, 35 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 3 (3.9 g, yield 16%, MS[M+H]+=825).

### Synthesis Example 4: Synthesis of Compound 4

### Step 1) Synthesis of Compound 4-a

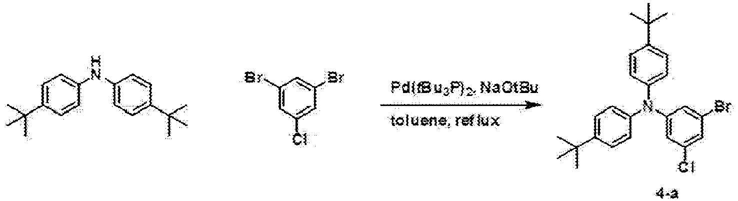

After dissolving 1,3-dibromo-5-chlorobenzene (111 mmol, 30 g) and bis (4-tert-butylphenyl) amine (111 mmol, 31.2 g) in toluene (0.2 M, 555 ml) in a 3-neck flask, sodium tert-butoxide (166.5 mmol, 16 g) and bis(tri-tert-butylphosphine)palladium(0) (1.1 mmol, 0.58 g) were introduced thereto, and the result was stirred for 1 hour under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 4-a (30.6 g, yield 59%, MS[M+H]+=471).

### Step 2) Synthesis of Compound 4-b

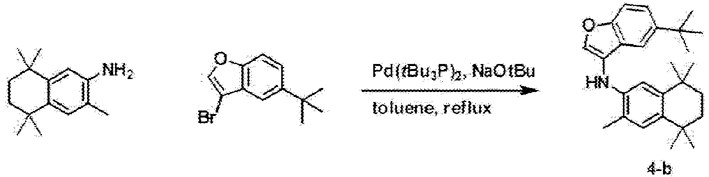

After dissolving 3,5,5,8,8-pentamethyl-5,6,7,8-tetrahydronaphthalen-2-amine (79.1 mmol, 21.7 g) and 5-tert-butyl-3-bromobenzofuran (79.1 mmol, 20 g) in toluene (0.2 M, 395 ml) in a 3-neck flask, sodium tert-butoxide (118.6 mmol, 11.4 g) and bis(tri-tert-butylphosphine)palladium(0) (0.79 mmol, 0.40 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 4-b (25.3 g, yield 82%, MS[M+H]+=390).

### Step 3) Synthesis of Compound 4-c

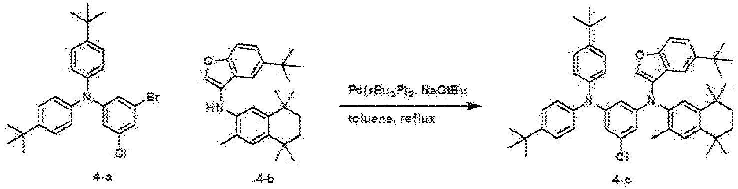

After dissolving Compound 4-a (63.7 mmol, 39 g) and Compound 4-b (63.7 mmol, 24.8 g) in toluene (0.2 M, 319 ml) in a 3-neck flask, sodium tert-butoxide (95.6 mmol, 9.2 g) and bis(tri-tert-butylphosphine)palladium(0) (0.64 mmol, 0.33 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 4-c (27.7 g, yield 56%, MS[M+H]+=780).

### Step 4) Synthesis of Compound 4-d

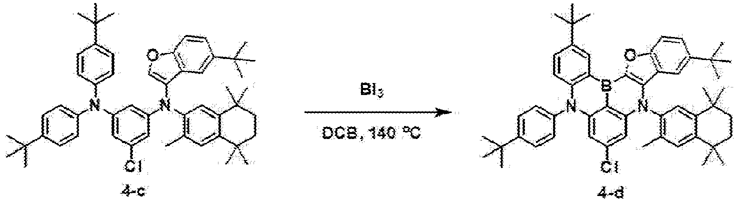

After dissolving Compound 4-c (35.5 mmol, 27.7 g) in 1,2-dichlorobenzene (0.1 M, 355 ml) in a 3-neck flask, boron triiodide (56.9 mmol, 22.3 g) was introduced thereto, and the result was stirred for 3 hours at 140°C under the argon atmosphere. The reaction material was cooled to 0°C, and after adding N,N-diisopropylethylamine (320 mmol, 41 g) thereto, the result was stirred for 1 hour. The result was extracted in a separatory funnel using toluene and H₂O. The extract was dried with MgSO₄ and concentrated, and the sample was purified using silica gel column chromatography to obtain Compound 4-d (6.2 g, yield 22%, MS[M+H]+=787).

### Step 5) Synthesis of Compound 4

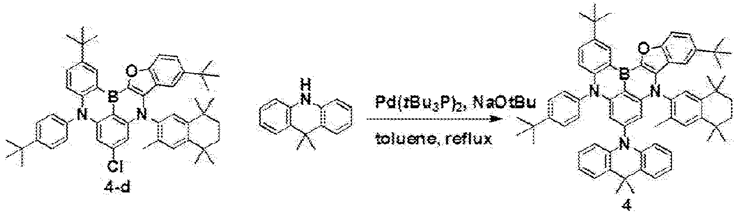

After dissolving Compound 4-d (7.9 mmol, 6.2 g) and 9,9-dimethyl-9,10-dihydroacridine (9.4 mmol, 2 g) in toluene (0.2 M, 47 ml) in a 3-neck flask, sodium tert-butoxide (11.8 mmol, 1.1 g) and bis (tri-tert-butylphosphine)palladium(0) (0.08 mmol, 0.04 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography and then went through sublimation purification to obtain Compound 4 (24.7 g, yield 77%, MS[M+H]+=960).

### Synthesis Example 5: Synthesis of Compound 5

### Step 1) Synthesis of Compound 5-a

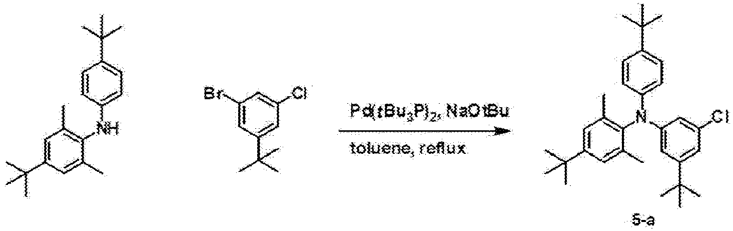

After dissolving 1-bromo-3-chloro-5-tert-butylbenzene (121 mmol, 30 g) and 4-tert-butyl-*N*-(4-tert-butylphenyl)-2,6-dimethylaniline (121 mmol, 37.5 g) in toluene (0.2 M, 605 ml) in a 3-neck flask, sodium tert-butoxide (182 mmol, 17.5 g) and bis(tri-tert-butylphosphine)palladium(0) (1.2 mmol, 0.62 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 5-a (51.2 g, yield 89%, MS[M+H]+=476).

### Step 2) Synthesis of Compound 5-b

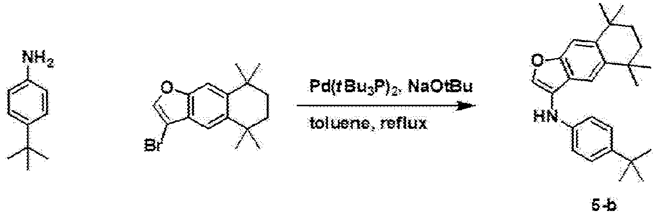

After dissolving 3-bromo-5,5,8,8-tetramethyl-5,6,7,8-tetrahydronaphtho[2,3-*b*]furan (97.6 mmol, 30 g) and 4-tert-butylaniline (97.6 mmol, 14.6 g) in toluene (0.2 M, 488 ml) in a 3-neck flask, sodium tert-butoxide (146.5 mmol, 14.1 g) and bis(tri-tert-butylphosphine)palladium(0) (0.98 mmol, 0.5 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 5-b (35.4 g, yield 97%, MS[M+H]+=376).

### Step 3) Synthesis of Compound 5-c

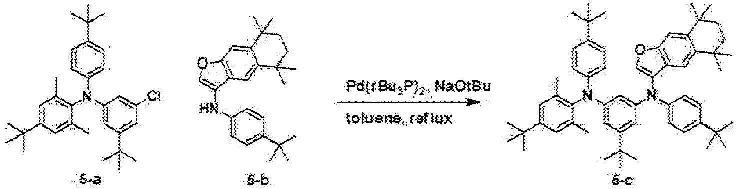

After dissolving Compound 5-a (44.1 mmol, 21 g) and Compound 5-b (44.1 mmol, 16.6 g) in toluene (0.2 M, 220 ml) in a 3-neck flask, sodium tert-butoxide (66.2 mmol, 6.4 g) and bis(tri-tert-butylphosphine)palladium(0) (0.44 mmol, 0.23 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 5-c (21.8 g, yield 610, MS[M+H]+=815).

### Step 4) Synthesis of Compound 5

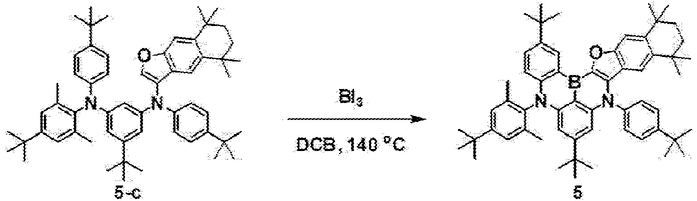

After dissolving Compound 5-c (26.7 mmol, 21.8 g) in 1,2-dichlorobenzene (0.1 M, 267 ml) in a 3-neck flask, boron triiodide (42.8 mmol, 16.8 g) was introduced thereto, and the result was stirred for 3 hours at 140°C under the argon atmosphere. The reaction material was cooled to 0°C, and after adding N,N-diisopropylethylamine (241 mmol, 31 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 5 (3.4 g, yield 15%, MS[M+H]+=823).

### Synthesis Example 6: Synthesis of Compound 6

### Step 1) Synthesis of Compound 6-a

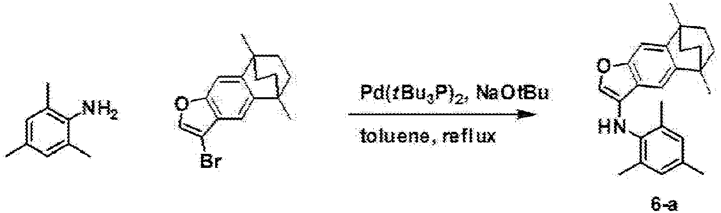

After dissolving 1,4,6-trimethylaniline (74.0 mmol, 10 g) and 3-bromo-5,8-dimethyl-5,6,7,8-tetrahydro-5,8-ethanonaphtho[2,3-*b*]furan (74.0 mmol, 22.6 g) in toluene (0.2 M, 370 ml) in a 3-neck flask, sodium tert-butoxide (111 mmol, 10.7 g) and bis(tri-tert-butylphosphine)palladium(0) (0.740 mmol, 0.378 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 6-a (21.9 g, yield 82%, MS[M+H]+=360).

### Step 2) Synthesis of Compound 6-b

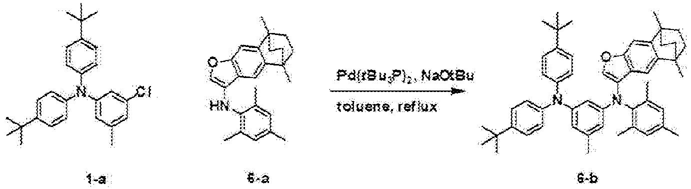

After dissolving Compound 1-a (36.9 mmol, 15.0 g) and Compound 2-a (36.9 mmol, 13.3 g) in toluene (0.2 M, 185 ml) in a 3-neck flask, sodium tert-butoxide (55.4 mmol, 5.33 g) and bis(tri-tert-butylphosphine)palladium(0) (0.369 mmol, 0.189 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 6-b (21.5 g, yield 80%, MS[M+H]+=729).

### Step 3) Synthesis of Compound 6

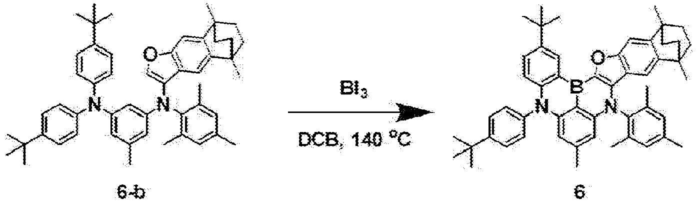

After dissolving Compound 6-b (29.5 mmol, 21.5 g) in 1,2-dichlorobenzene (0.1 M, 295 ml) in a 3-neck flask, boron triiodide (47.2 mmol, 18.5 g) was introduced thereto, and the result was stirred for 3 hours at 140°C under the argon atmosphere. The reaction material was cooled to 0°C, and after adding N,N-diisopropylethylamine (265 mmol, 34.3 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 6 (4.2 g, yield 19%, MS[M+H]+=737).

### Synthesis Example 7: Synthesis of Compound 7

### Step 1) Synthesis of Compound 7-a

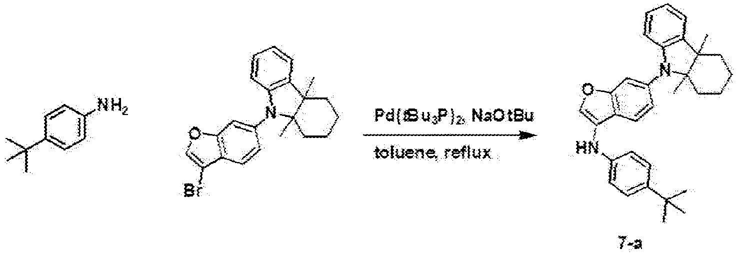

After dissolving 4-tert-butylaniline (33.5 mmol, 5 g) and 9-(3-bromobenzofuran-6-yl)-4a,9a-dimethyl-2,3,4,4a,9,9a-hexahydro-1*H*-carbazole (33.5 mmol, 13.3 g) in toluene (0.2 M, 168 ml) in a 3-neck flask, sodium tert-butoxide (50.3 mmol, 4.83 g) and bis(tri-tert-butylphosphine)palladium(0) (0.335 mmol, 0.171 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 7-a (13.2 g, yield 85%, MS[M+H]+=465).

### Step 2) Synthesis of Compound 7-b

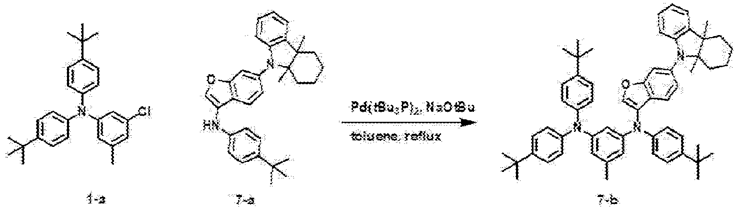

After dissolving Compound 1-a (25.9 mmol, 10.5 g) and Compound 7-a (25.9 mmol, 12.0 g) in toluene (0.2 M, 129 ml) in a 3-neck flask, sodium tert-butoxide (38.8 mmol, 3.73 g) and bis(tri-tert-butylphosphine)palladium(0) (0.259 mmol, 0.132 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 7-b (19.4 g, yield 90%, MS[M+H]+=834).

### Step 3) Synthesis of Compound 7

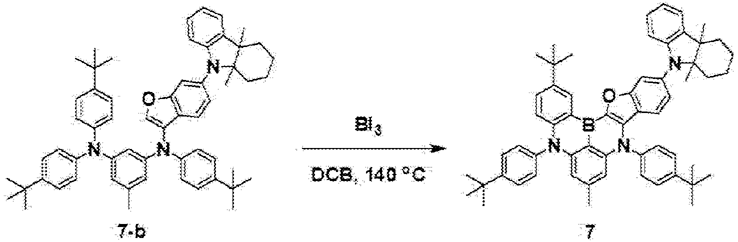

After dissolving Compound 7-b (23.3 mmol, 19.4 g) in 1,2-dichlorobenzene (0.1 M, 233 ml) in a 3-neck flask, boron triiodide (37.2 mmol, 14.6 g) was introduced thereto, and the result was stirred for 3 hours at 140°C under the argon atmosphere. The reaction material was cooled to 0°C, and after adding N,N-diisopropylethylamine (209 mmol, 27.1 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 7 (3.7 g, yield 19%, MS[M+H]+=842).

### Synthesis Example 8: Synthesis of Compound 8

### Step 1) Synthesis of Compound 8-a

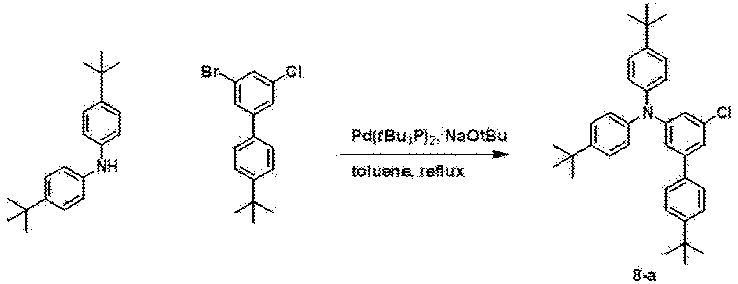

After dissolving bis(4-tert-butylphenyl)amine (46.2 mmol, 13 g) and 3-bromo-4'-tert-butyl-5-chloro-1,1'-biphenyl (46.2 mmol, 15 g) in toluene (0.2 M, 231 ml) in a 3-neck flask, sodium tert-butoxide (69.3 mmol, 6.66 g) and bis(tri-tert-butylphosphine)palladium(0) (0.462 mmol, 0.236 g) were introduced thereto, and the result was stirred for 2 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 8-a (19.4 g, yield 80%, MS[M+H]+=524).

### Step 2) Synthesis of Compound 8-b

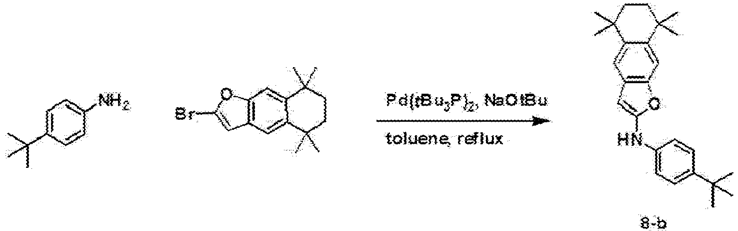

After dissolving 2-bromo-5,5,8,8-tetramethyl-5,6,7,8-tetrahydronaphtho[2,3-*b*]furan (78.1 mmol, 24 g) and 4-tert-butylaniline (78.1 mmol, 11.7 g) in toluene (0.2 M, 390 ml) in a 3-neck flask, sodium tert-butoxide (117.2 mmol, 11.3 g) and bis(tri-tert-butylphosphine)palladium(0) (0.781 mmol, 0.4 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 8-b (20.6 g, yield 70%, MS[M+H]+=376).

### Step 3) Synthesis of Compound 8-c

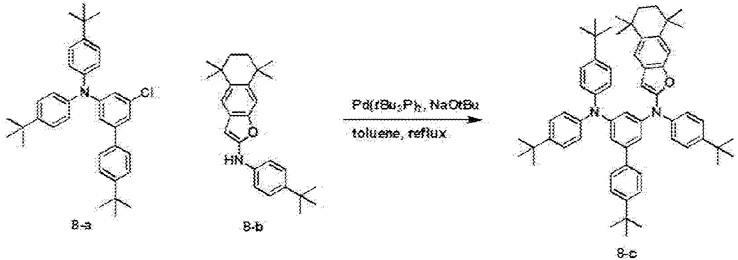

After dissolving Compound 8-a (37.0 mmol, 19.4 g) and Compound 8-b (37.0 mmol, 13.9 g) in toluene (0.2 M, 185 ml) in a 3-neck flask, sodium tert-butoxide (55.5 mmol, 5.33 g) and bis(tri-tert-butylphosphine)palladium(0) (0.37 mmol, 0.189 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 8-c (22.0 g, yield 69%, MS[M+H]+=863).

### Step 4) Synthesis of Compound 8

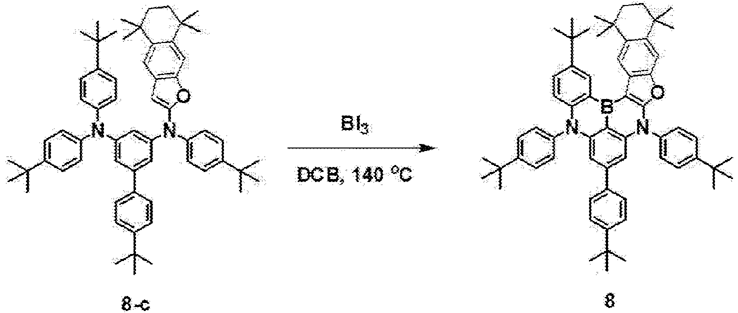

After dissolving Compound 8-c (25.5 mmol, 22.0 g) in 1,2-dichlorobenzene (0.1 M, 255 ml) in a 3-neck flask, boron triiodide (40.8 mmol, 16.0 g) was introduced thereto, and the result was stirred for 3 hours at 140°C under the argon atmosphere. The reaction material was cooled to 0°C, and after adding N,N-diisopropylethylamine (229 mmol, 30 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 8 (2.9 g, yield 13%, MS[M+H]+=871).

### Synthesis Example 9: Synthesis of Compound 9

### Step 1) Synthesis of Compound 9-a

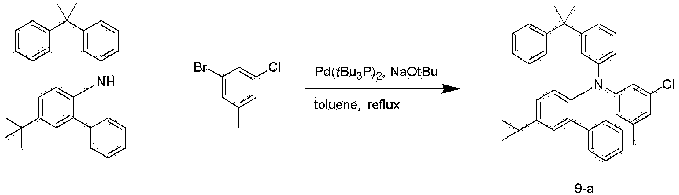

After dissolving 5-tert-butyl-*N*-(3-(2-phenylpropan-2-yl)phenyl)-[1,1'-biphenyl]-2-amine (71.5 mmol, 30 g) and 1-bromo-3-chloro-5-methylbenzene (71.5 mmol, 14.7 g) in toluene (0.2 M, 357 ml) in a 3-neck flask, sodium tert-butoxide (107 mmol, 10.3 g) and bis(tri-tert-butylphosphine)palladium(0) (0.715 mmol, 0.365 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 9-a (34.6 g, yield 89%, MS[M+H]+=544).

### Step 2) Synthesis of Compound 9-b

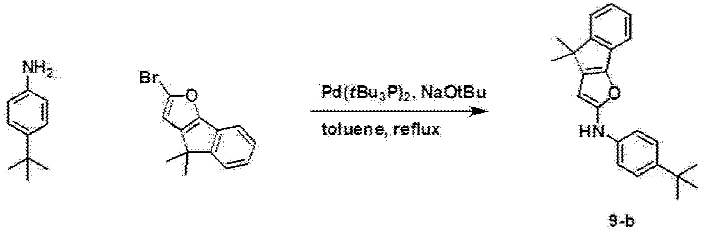

After dissolving 2-bromo-4,4-dimethyl-4*H*-indeno[2,3-b] furan (144 mmol, 38 g) and 4-tert-butylaniline (144 mmol, 21.6 g) in toluene (0.2 M, 722 ml) in a 3-neck flask, sodium tert-butoxide (217 mmol, 20.8 g) and bis(tri-tert-butylphosphine)palladium(0) (1.44 mmol, 0.738 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 9-b (35.8 g, yield 75%, MS[M+H]+=376).

### Step 3) Synthesis of Compound 9-c

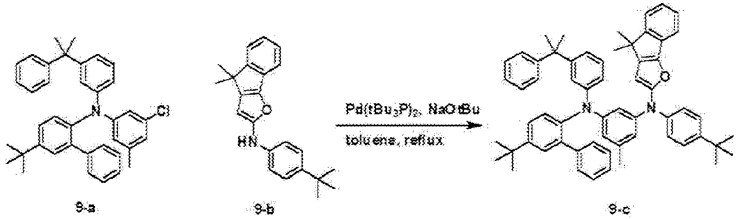

After dissolving Compound 9-a (51.3 mmol, 27.9 g) and Compound 9-b (51.3 mmol, 17 g) in toluene (0.2 M, 256 ml) in a 3-neck flask, sodium tert-butoxide (76.9 mmol, 7.39 g) and bis(tri-tert-butylphosphine)palladium(0) (0.513 mmol, 0.262 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 9-c (27.3 g, yield 63%, MS[M+H]+=839).

### Step 4) Synthesis of Compound 9

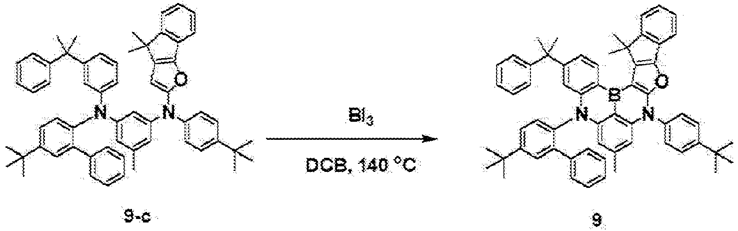

After dissolving Compound 9-c (32.5 mmol, 27.3 g) in 1,2-dichlorobenzene (0.1 M, 325 ml) in a 3-neck flask, boron triiodide (52.1 mmol, 20.4 g) was introduced thereto, and the result was stirred for 3 hours at 140°C under the argon atmosphere. The reaction material was cooled to 0°C, and after adding N,N-diisopropylethylamine (293 mmol, 37.8 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 9 (4.2 g, yield 15%, MS[M+H]+=847).

### Synthesis Example 10: Synthesis of Compound 10

### Step 1) Synthesis of Compound 10-a

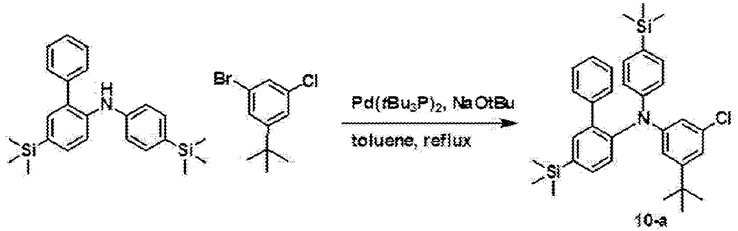

After dissolving 5-trimethylsilyl-*N*-(4-trimethylsilylphenyl)-[1,1'-biphenyl]-2-amine (51.3 mmol, 20 g) and 1-bromo-3-chloro-5-tert-butylbenzene (51.3 mmol, 12.7 g) in toluene (0.2 M, 257 ml) in a 3-neck flask, sodium tert-butoxide (77.0 mmol, 7.40 g) and bis(tri-tert-butylphosphine)palladium(0) (0.513 mmol, 0.262 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 10-a (26.7 g, yield 94%, MS[M+H]+=556).

### Step 2) Synthesis of Compound 10-b

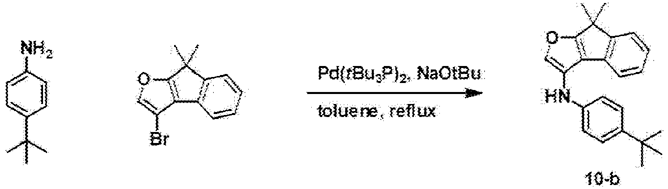

After dissolving 3-bromo-8,8-dimethyl-8*H*-indeno[2,1-b]furan (76.0 mmol, 20 g) and 4-tert-butylaniline (76.0 mmol, 11.3 g) in toluene (0.2 M, 380 ml) in a 3-neck flask, sodium tert-butoxide (114 mmol, 11.0 g) and bis(tri-tert-butylphosphine)palladium(0) (0.760 mmol, 0.388 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 10-b (17.3 g, yield 69%, MS[M+H]+=331).

### Step 3) Synthesis of Compound 10-c

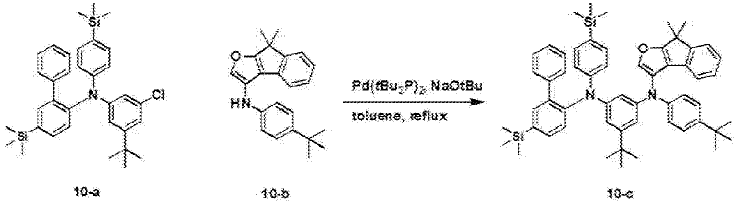

After dissolving Compound 10-a (41.3 mmol, 23 g) and Compound 10-b (41.3 mmol, 13.7 g) in toluene (0.2 M, 207 ml) in a 3-neck flask, sodium tert-butoxide (62.0 mmol, 6.00 g) and bis(tri-tert-butylphosphine)palladium(0) (0.413 mmol, 0.211 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 10-c (23.7 g, yield 67%, MS[M+H]+=839).

### Step 4) Synthesis of Compound 10

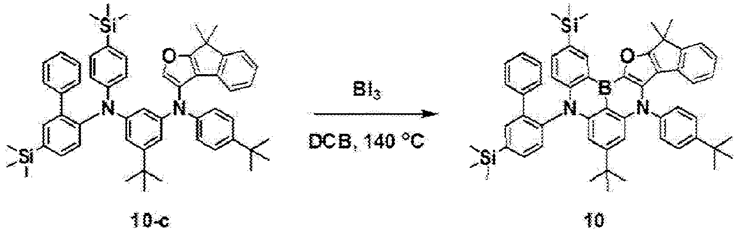

After dissolving Compound 10-c (27.8 mmol, 23.7 g) in 1,2-dichlorobenzene (0.1 M, 280 ml) in a 3-neck flask, boron triiodide (44.5 mmol, 17.4 g) was introduced thereto, and the result was stirred for 3 hours at 140°C under the argon atmosphere. The reaction material was cooled to 0°C, and after adding N,N-diisopropylethylamine (251 mmol, 32.4 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 10 (3.5 g, yield 15%, MS[M+H]+=859).

### Synthesis Example 11: Synthesis of Compound 11

### Step 1) Synthesis of Compound 11-a

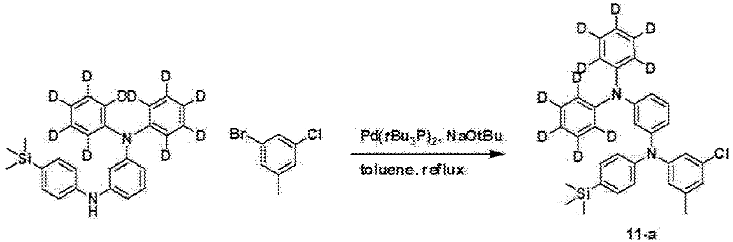

After dissolving *N*¹,*N*¹-bis(phenyl-*d*₅)-*N*³-(4-trimethylsilylphenyl)benzene-1,3-diamine (47.8 mmol, 20 g) and 1-bromo-3-chloro-5-methylbenzene (47.8 mmol, 9.82 g) in toluene (0.2 M, 240 ml) in a 3-neck flask, sodium tert-butoxide (71.7 mmol, 6.89 g) and bis(tri-tert-butylphosphine)palladium(0) (0.478 mmol, 0.244 g) were introduced thereto, and the result was stirred for 2 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 11-a (24.5 g, yield 94%, MS[M+H]+=543).

### Step 2) Synthesis of Compound 11-b

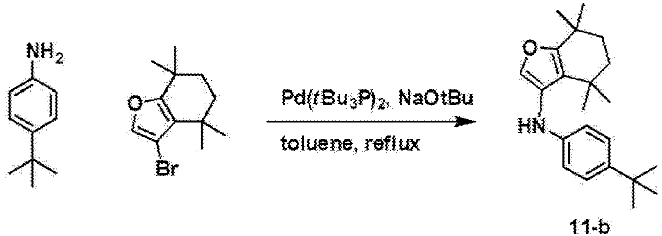

After dissolving 3-bromo-4,4,7,7-tetramethyl-4,5,6,7-tetrahydrofuran (38.9 mmol, 10 g) and 4-tert-butylaniline (38.9 mmol, 5.80 g) in toluene (0.2 M, 195 ml) in a 3-neck flask, sodium tert-butoxide (58.3 mmol, 5.60 g) and bis(tri-tert-butylphosphine)palladium(0) (0.389 mmol, 0.200 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 11-b (11.2 g, yield 88%, MS[M+H]+=331).

### Step 3) Synthesis of Compound 11-c

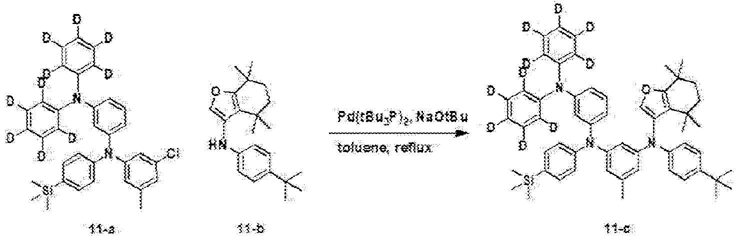

After dissolving Compound 11-a (33.1 mmol, 18 g) and Compound 11-b (33.1 mmol, 10.8 g) in toluene (0.2 M, 165 ml) in a 3-neck flask, sodium tert-butoxide (49.7 mmol, 4.78 g) and bis(tri-tert-butylphosphine)palladium(0) (0.331 mmol, 0.169 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 11-c (21.2 g, yield 77%, MS[M+H]+=832).

### Step 4) Synthesis of Compound 11

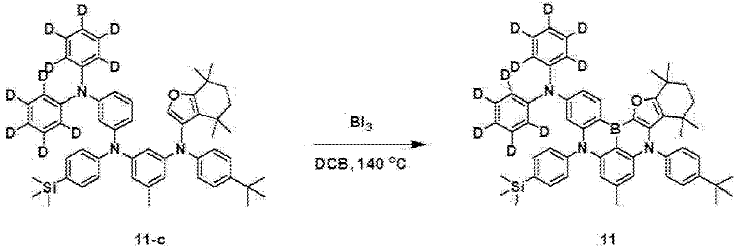

After dissolving Compound 11-c (25.5 mmol, 21.2 g) in 1,2-dichlorobenzene (0.1 M, 255 ml) in a 3-neck flask, boron triiodide (40.8 mmol, 16.0 g) was introduced thereto, and the result was stirred for 3 hours at 140°C under the argon atmosphere. The reaction material was cooled to 0°C, and after adding N,N-diisopropylethylamine (229 mmol, 29.6 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 11 (2.9 g, yield 14%, MS[M+H]+=859).

### Synthesis Example 12: Synthesis of Compound 12

### Step 1) Synthesis of Compound 12-a

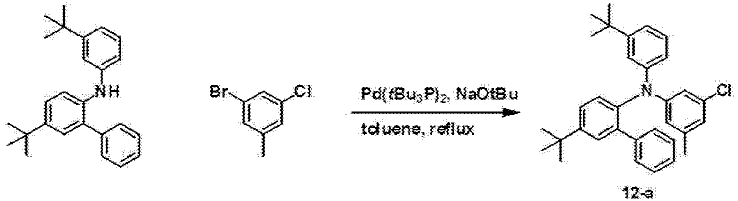

After dissolving 5-tert-butyl-*N*-(3-tert-butylphenyl)-[1,1'-biphenyl]-2-amine (55.9 mmol, 20 g) and 1-bromo-3-chloro-5-methylbenzene (55.9 mmol, 11.5 g) in toluene (0.2 M, 280 ml) in a 3-neck flask, sodium tert-butoxide (83.9 mmol, 8.06 g) and bis(tri-tert-butylphosphine)palladium(0) (0.559 mmol, 0.286 g) were introduced thereto, and the result was stirred for 5 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 12-a (23.8 g, yield 88%, MS[M+H]+=482).

### Step 2) Synthesis of Compound 12-b

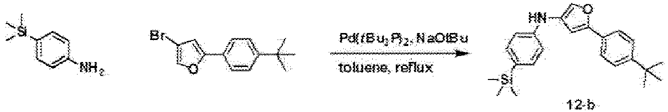

After dissolving 4-bromo-2-(4-tert-butylphenyl)furan (53.7 mmol, 15 g) and 4-trimethylsilylaniline (53.7 mmol, 8.88 g) in toluene (0.2 M, 270 ml) in a 3-neck flask, sodium tert-butoxide (80.6 mmol, 7.75 g) and bis(tri-tert-butylphosphine)palladium(0) (0.537 mmol, 0.275 g) were introduced thereto, and the result was stirred for 10 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 12-b (17.4 g, yield 89%, MS[M+H]+=331).

### Step 3) Synthesis of Compound 12-c

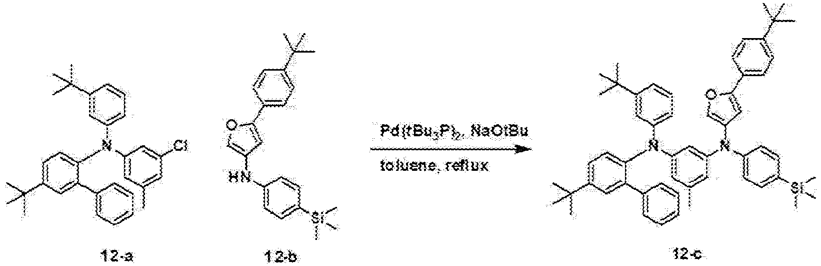

After dissolving Compound 12-a (41.5 mmol, 20 g) and Compound 12-b (41.5 mmol, 19.1 g) in toluene (0.2 M, 305 ml) in a 3-neck flask, sodium tert-butoxide (62.2 mmol, 6.00 g) and bis(tri-tert-butylphosphine)palladium(0) (0.415 mmol, 0.212 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 12-c (24.7 g, yield 74%, MS[M+H]+=832).

### Step 4) Synthesis of Compound 12

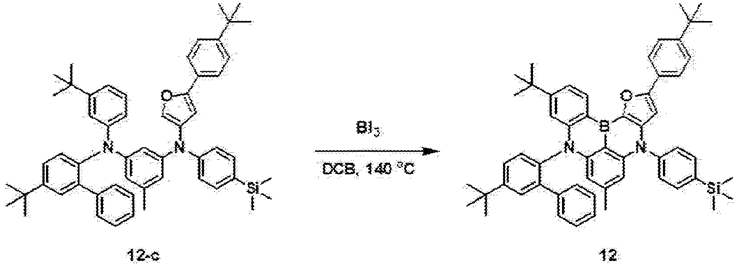

After dissolving Compound 12-c (30.5 mmol, 24.7 g) in 1,2-dichlorobenzene (0.1 M, 305 ml) in a 3-neck flask, boron triiodide (48.8 mmol, 19.1 g) was introduced thereto, and the result was stirred for 3 hours at 140°C under the argon atmosphere. The reaction material was cooled to 0°C, and after adding N,N-diisopropylethylamine (275 mmol, 35.5 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 12 (4.3 g, yield 17%, MS[M+H]+=817).

### Synthesis Example 13: Synthesis of Compound 13

### Step 1) Synthesis of Compound 13-a

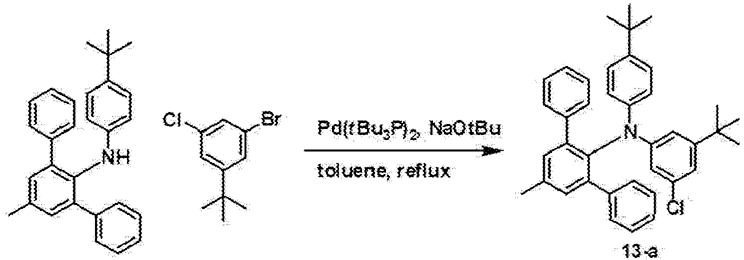

After dissolving *N*-(4-tert-butylphenyl)-5'-methyl-[1,1',3',1"-terphenyl]-2'-amine (76.6 mmol, 30 g) and 1-bromo-3-chloro-5-tert-butylbenzene (76.6 mmol, 19.0 g) in toluene (0.2 M, 380 ml) in a 3-neck flask, sodium tert-butoxide (115 mmol, 11.0 g) and bis(tri-tert-butylphosphine)palladium(0) (0.766 mmol, 0.392 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 13-a (26.4 g, yield 62%, MS[M+H]+=558).

### Step 2) Synthesis of Compound 13-b

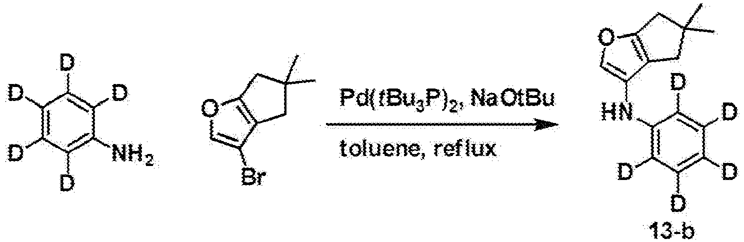

After dissolving 3-bromo-5,5-dimethyl-5,6-dihydro-4*H-*cyclopenta[*b*]furan (93.0 mmol, 20 g) and aniline-*d*₅ (93.0 mmol, 9.13 g) in toluene (0.2 M, 465 ml) in a 3-neck flask, sodium tert-butoxide (139 mmol, 13.4 g) and bis(tri-tert-butylphosphine)palladium(0) (0.93 mmol, 0.475 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 13-b (19.3 g, yield 89%, MS[M+H]+=232).

### Step 3) Synthesis of Compound 13-c

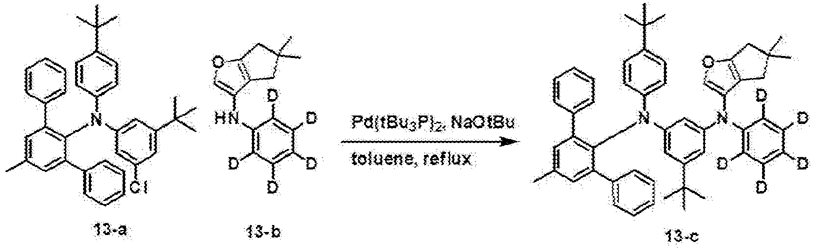

After dissolving Compound 13-a (44.8 mmol, 25 g) and Compound 13-b (44.8 mmol, 10.4 g) in toluene (0.2 M, 225 ml) in a 3-neck flask, sodium tert-butoxide (67.2 mmol, 6.46 g) and bis(tri-tert-butylphosphine)palladium(0) (0.448 mmol, 0.229 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 13-c (24.3 g, yield 72%, MS[M+H]+=754).

### Step 4) Synthesis of Compound 13

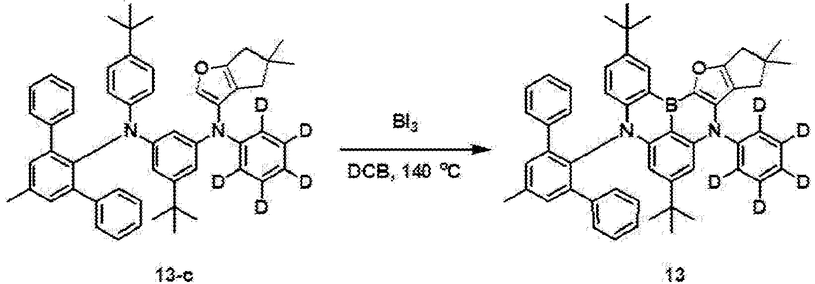

After dissolving Compound 13-c (32.2 mmol, 24.3 g) in 1,2-dichlorobenzene (0.1 M, 322 ml) in a 3-neck flask, boron triiodide (51.6 mmol, 20.2 g) was introduced thereto, and the result was stirred for 3 hours at 140°C under the argon atmosphere. The reaction material was cooled to 0°C, and after adding N,N-diisopropylethylamine (290 mmol, 37.5 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 13 (4.5 g, yield 18%, MS[M+H]+=762).

### Synthesis Example 14: Synthesis of Compound 14

### Step 1) Synthesis of Compound 14-a

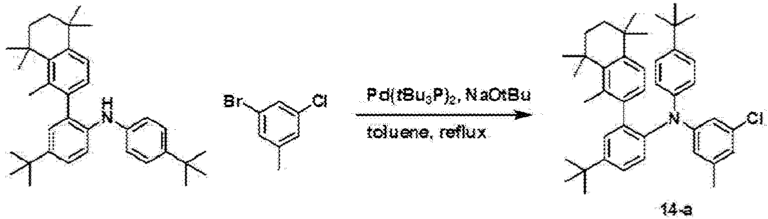

After dissolving 4-tert-butyl-*N*-(4-tert-butylphenyl)-2-(1,5,5,8,8-pentamethyl-5,6,7,8-tetrahydronaphthalen-2-yl)aniline (31.1 mmol, 15 g) and 1-bromo-3-chloro-5-methylbenzene (31.1 mmol, 6.40 g) in toluene (0.2 M, 155 ml) in a 3-neck flask, sodium tert-butoxide (46.7 mmol, 4.49 g) and bis(tri-tert-butylphosphine)palladium(0) (0.311 mmol, 0.159 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 14-a (16.4 g, yield 87%, MS[M+H]+=606).

### Step 2) Synthesis of Compound 14-b

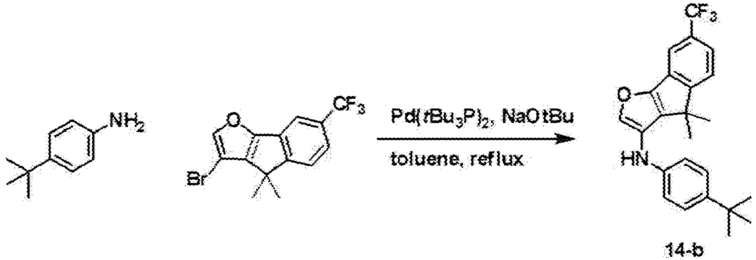

After dissolving 3-bromo-4,4-dimethyl-7-trifluoromethyl-4H-indeno[1,2-*b*]furan (75.5 mmol, 25 g) and 4-tert-butylaniline (75.5 mmol, 11.3 g) in toluene (0.2 M, 380 ml) in a 3-neck flask, sodium tert-butoxide (113 mmol, 10.9 g) and bis(tri-tert-butylphosphine)palladium(0) (0.755 mmol, 0.386 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 14-b (26.9 g, yield 89%, MS[M+H]+=399).

### Step 3) Synthesis of Compound 14-c

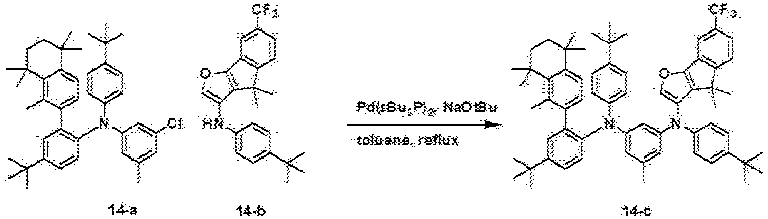

After dissolving Compound 14-a (26.4 mmol, 16 g) and Compound 14-b (26.4 mmol, 10.5 g) in toluene (0.2 M, 130 ml) in a 3-neck flask, sodium tert-butoxide (39.6 mmol, 3.80 g) and bis(tri-tert-butylphosphine)palladium(0) (0.264 mmol, 0.135 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 14-c (20.4 g, yield 80%, MS[M+H]+=969).

### Step 4) Synthesis of Compound 14

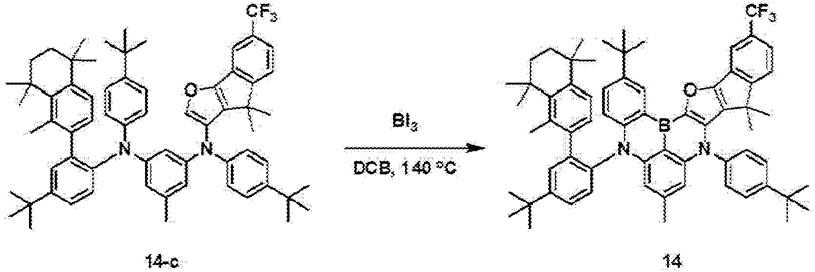

After dissolving Compound 14-c (21.0 mmol, 20.4 g) in 1,2-dichlorobenzene (0.1 M, 210 ml) in a 3-neck flask, boron triiodide (33.7 mmol, 13.2 g) was introduced thereto, and the result was stirred for 3 hours at 140°C under the argon atmosphere. The reaction material was cooled to 0°C, and after adding N,N-diisopropylethylamine (189 mmol, 24.5 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 14 (3.2 g, yield 16%, MS[M+H]+=977).

### Synthesis Example 15: Synthesis of Compound 15

### Step 1) Synthesis of Compound 15-a

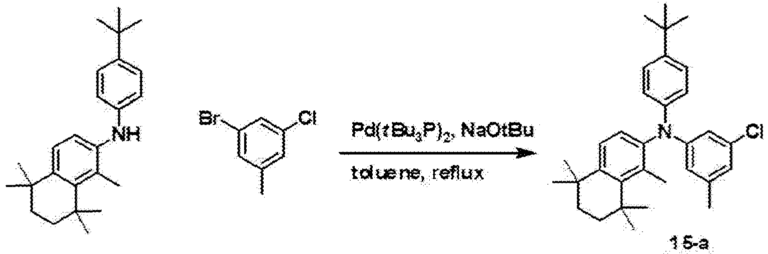

After dissolving *N*-(4-terphenyl)-1,5,5,8,8-pentamethyl-5,6,7,8-tetrahydronaphthalen-2-amine (114 mmol, 40 g) and 1-bromo-3-chloro-5-methylbenzene (114 mmol, 23.5 g) in toluene (0.2 M, 572 ml) in a 3-neck flask, sodium tert-butoxide (172 mmol, 16.5 g) and bis(tri-tert-butylphosphine)palladium(0) (1.14 mmol, 0.585 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 15-a (42.3 g, yield 78%, MS[M+H]+=474).

### Step 2) Synthesis of Compound 15-b

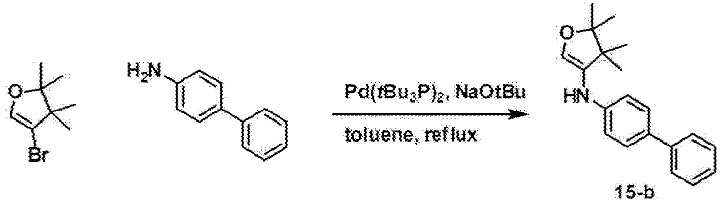

After dissolving 4-bromo-2,2,3,3-tetramethyl-2,3-dihydrofuran (73.1 mmol, 15 g) and [1,1'-biphenyl]-4-amine (73.1 mmol, 12.4 g) in toluene (0.2 M, 370 ml) in a 3-neck flask, sodium tert-butoxide (110 mmol, 10.5 g) and bis(tri-tert-butylphosphine)palladium(0) (0.731 mmol, 0.374 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 15-b (13.6 g, yield 63%, MS[M+H]+=293).

### Step 3) Synthesis of Compound 15-c

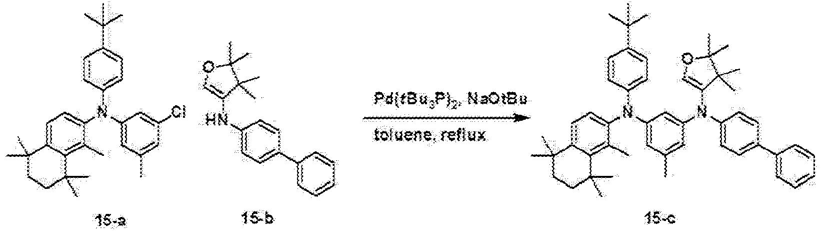

After dissolving Compound 15-a (42.2 mmol, 20 g) and Compound 15-b (42.2 mmol, 12.4 g) in toluene (0.2 M, 210 ml) in a 3-neck flask, sodium tert-butoxide (63.3 mmol, 3.80 g) and bis(tri-tert-butylphosphine)palladium(0) (0.422 mmol, 0.216 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 15-c (19.2 g, yield 62%, MS[M+H]+=731).

### Step 4) Synthesis of Compound 15

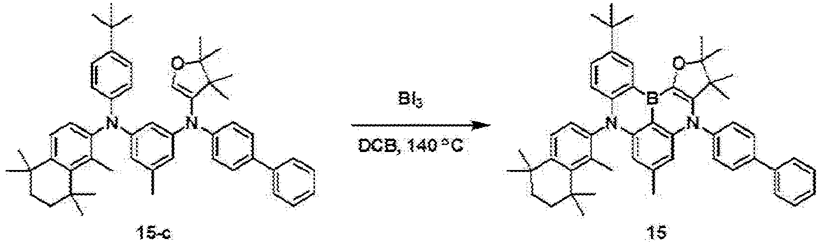

After dissolving Compound 15-c (26.3 mmol, 19.2 g) in 1,2-dichlorobenzene (0.1 M, 263 ml) in a 3-neck flask, boron triiodide (42.0 mmol, 16.5 g) was introduced thereto, and the result was stirred for 3 hours at 140°C under the argon atmosphere. The reaction material was cooled to 0°C, and after adding N,N-diisopropylethylamine (236 mmol, 30.5 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 15 (3.8 g, yield 20%, MS[M+H]+=739).

### Synthesis Example 16: Synthesis of Compound 16

### Step 1) Synthesis of Compound 16-a

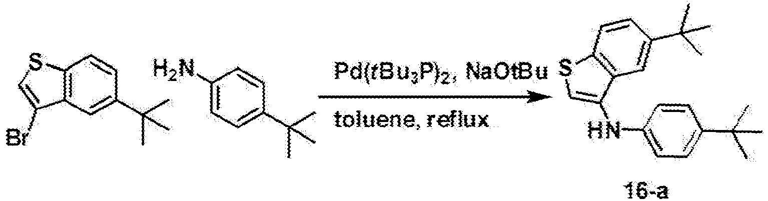

After dissolving 4-tert-butylaniline (134 mmol, 20 g) and 3-bromo-5-tert-butylbenzo[b]thiophene (134 mmol, 36.1 g) in toluene (0.2 M, 670 ml) in a 3-neck flask, sodium tert-butoxide (201 mmol, 19.3 g) and bis(tri-tert-butylphosphine)palladium(0) (1.34 mmol, 0.685 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 16-a (40.7 g, yield 90%, MS[M+H]+=338).

### Step 2) Synthesis of Compound 16-b

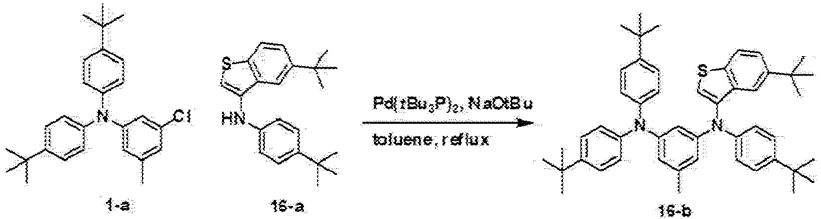

After dissolving Compound 1-a (73.4 mmol, 30 g) and Compound 16-a (73.4 mmol, 24.9 g) in toluene (0.2 M, 370 ml) in a 3-neck flask, sodium tert-butoxide (111 mmol, 10.7 g) and bis(tri-tert-butylphosphine)palladium(0) (0.734 mmol, 0.378 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 16-b (38.7 g, yield 74%, MS[M+H]+=707).

### Step 3) Synthesis of Compound 16

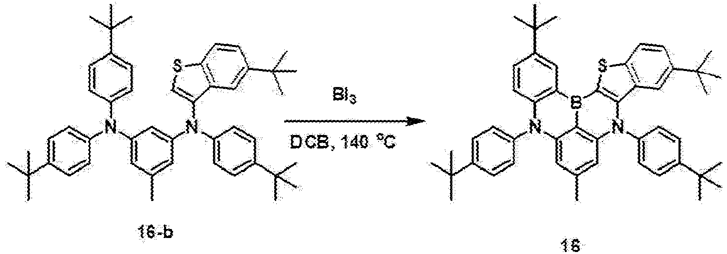

After dissolving Compound 16-b (54.7 mmol, 38.7 g) in 1,2-dichlorobenzene (0.1 M, 550 ml) in a 3-neck flask, boron triiodide (87.6 mmol, 34.3 g) was introduced thereto, and the result was stirred for 3 hours at 140°C under the argon atmosphere. The reaction material was cooled to 0°C, and after adding *N*,*N*-diisopropylethylamine (493 mmol, 63.7 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 16 (6.4 g, yield 16%, MS[M+H]+=715).

### Synthesis Example 17: Synthesis of Compound 17

### Step 1) Synthesis of Compound 17-a

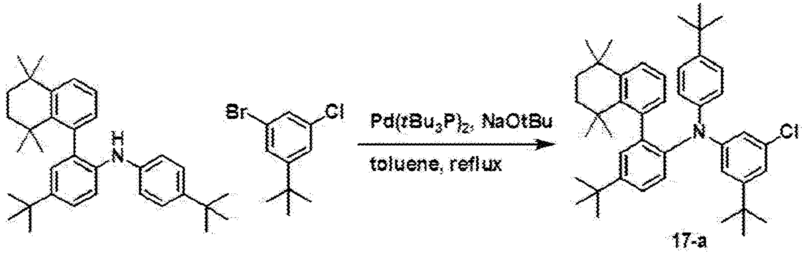

After dissolving 4-tert-butyl-*N*-(4-tert-butylphenyl)-2-(5,5,8,8-tetramethyl-5,6,7,8-tetrahydronaphthalen-1-yl)aniline (42.8 mmol, 20 g) and 1-bromo-3-chloro-5-tert-butylbenzene (42.8 mmol, 10.6 g) in toluene (0.2 M, 215 ml) in a 3-neck flask, sodium tert-butoxide (64.1 mmol, 6.16 g) and bis(tri-tert-butylphosphine)palladium(0) (0.428 mmol, 0.219 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 17-a (22.9 g, yield 84%, MS[M+H]+=634).

### Step 2) Synthesis of Compound 17-b

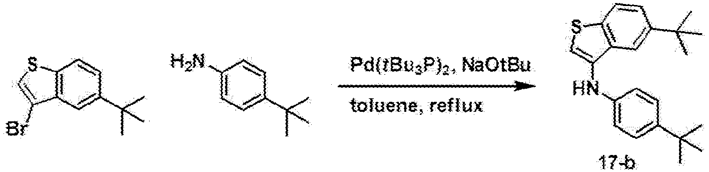

After dissolving 3-bromo-5-tert-butylbenzo[*b*]thiophene (149 mmol, 40 g) and 4-tert-butylaniline (149 mmol, 22.2 g) in toluene (0.2 M, 745 ml) in a 3-neck flask, sodium tert-butoxide (223 mmol, 21.4 g) and bis(tri-tert-butylphosphine)palladium(0) (1.49 mmol, 0.759 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 17-b (45.6 g, yield 910, MS[M+H]+=338).

### Step 3) Synthesis of Compound 17-c

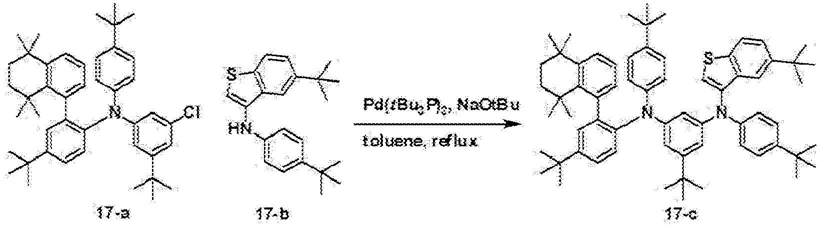

After dissolving Compound 17-a (26.8 mmol, 17 g) and Compound 17-b (26.8 mmol, 9.05 g) in toluene (0.2 M, 135 ml) in a 3-neck flask, sodium tert-butoxide (40.2 mmol, 3.87 g) and bis(tri-tert-butylphosphine)palladium(0) (0.268 mmol, 0.137 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 17-c (20.1 g, yield 80%, MS[M+H]+=935).

### Step 4) Synthesis of Compound 17

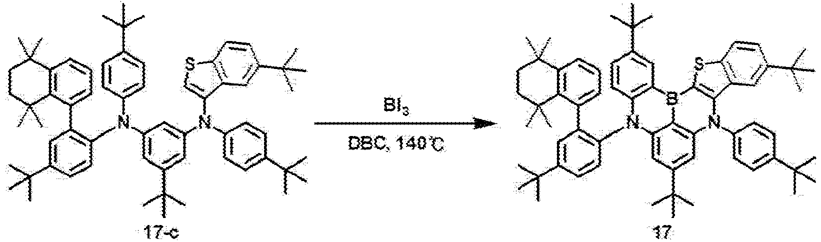

After dissolving Compound 17-c (21.5 mmol, 20.1 g) in 1,2-dichlorobenzene (0.1 M, 215 ml) in a 3-neck flask, boron triiodide (34.4 mmol, 13.5 g) was introduced thereto, and the result was stirred for 3 hours at 140°C under the argon atmosphere. The reaction material was cooled to 0°C, and after adding N,N-diisopropylethylamine (193 mmol, 25 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 17 (3.4 g, yield 17%, MS[M+H]+=943).

### Synthesis Example 18: Synthesis of Compound 18

### Step 1) Synthesis of Compound 18-a

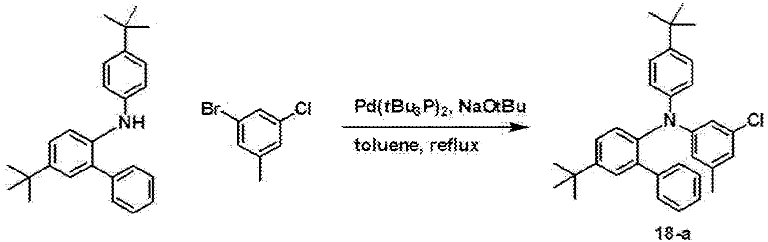

After dissolving 5-tert-butyl-*N*-(4-tert-butylphenyl)-[1,1'-biphenyl]-2-amine (140 mmol, 50 g) and 1-bromo-3-chloro-5-methylbenzene (140 mmol, 28.7 g) in toluene (0.2 M, 700 ml) in a 3-neck flask, sodium tert-butoxide (210 mmol, 20.2 g) and bis(tri-tert-butylphosphine)palladium(0) (1.40 mmol, 0.715 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 18-a (57.1 g, yield 85%, MS[M+H]+=482).

### Step 2) Synthesis of Compound 18-b

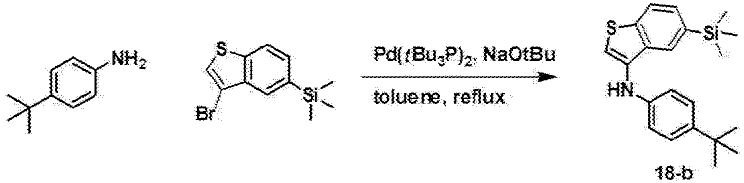

After dissolving 3-bromo-5-trimethylsilylbenzo[b]thiophene (70.1 mmol, 20 g) and 4-tert-butylaniline (70.1 mmol, 10.5 g) in toluene (0.2 M, 350 ml) in a 3-neck flask, sodium tert-butoxide (105 mmol, 10.1 g) and bis(tri-tert-butylphosphine)palladium(0) (0.701 mmol, 0.358 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 18-b (19.2 g, yield 77%, MS[M+H]+=354).

### Step 3) Synthesis of Compound 18-c

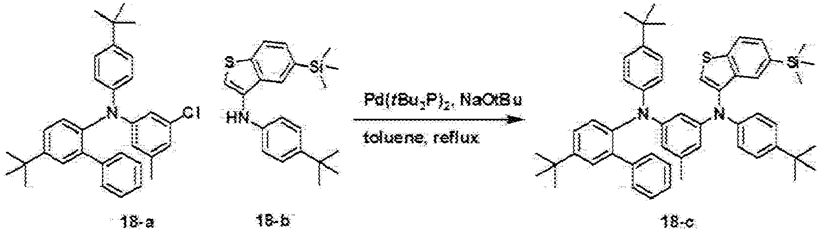

After dissolving Compound 18-a (51.9 mmol, 25 g) and Compound 18-b (51.9 mmol, 18.3 g) in toluene (0.2 M, 260 ml) in a 3-neck flask, sodium tert-butoxide (77.9 mmol, 7.47 g) and bis(tri-tert-butylphosphine)palladium(0) (0.519 mmol, 0.265 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 18-c (33.2 g, yield 80%, MS[M+H]+=799).

### Step 4) Synthesis of Compound 18

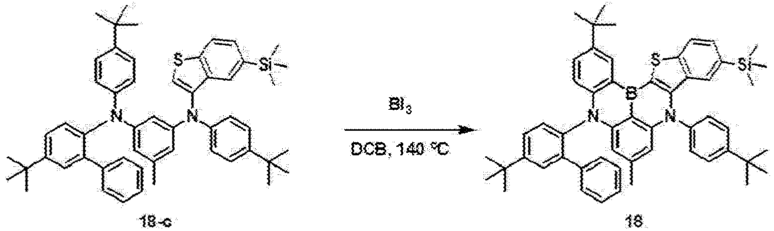

After dissolving Compound 18-c (41.5 mmol, 33.2 g) in 1,2-dichlorobenzene (0.1 M, 415 ml) in a 3-neck flask, boron triiodide (66.5 mmol, 26.0 g) was introduced thereto, and the result was stirred for 3 hours at 140°C under the argon atmosphere. The reaction material was cooled to 0°C, and after adding N,N-diisopropylethylamine (374 mmol, 48.3 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 18 (6.1 g, yield 18%, MS[M+H]+=807).

### Synthesis Example 19: Synthesis of Compound 19

### Step 1) Synthesis of Compound 19-a

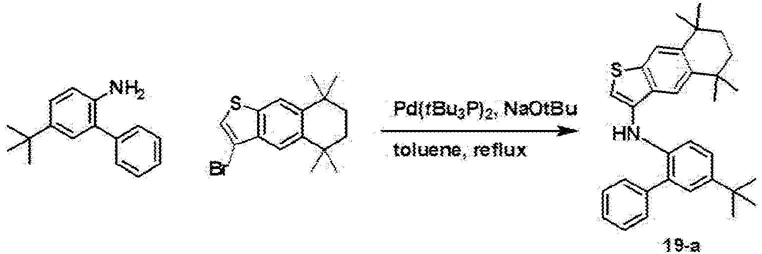

After dissolving 5-tert-butyl-[1,1'-biphenyl]-2-amine (66.6 mmol, 15 g) and 3-bromo-5,5,8,8-tetramethyl-5,6,7,8-tetrahydronaphtho[2,3-*b*]thiophene (66.6 mmol, 21.5 g) in toluene (0.2 M, 335 ml) in a 3-neck flask, sodium tert-butoxide (99.9 mmol, 9.60 g) and bis(tri-tert-butylphosphine)palladium(0) (0.666 mmol, 0.340 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 19-a (24.2 g, yield 78%, MS[M+H]+=468).

### Step 2) Synthesis of Compound 19-b

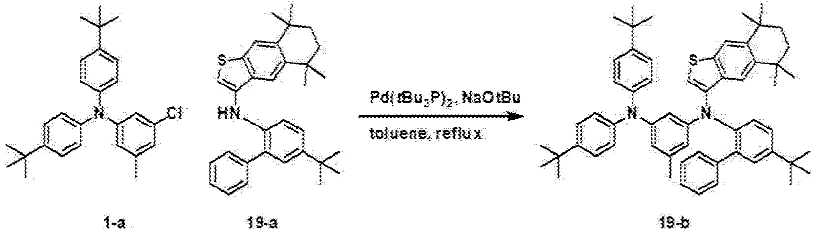

After dissolving Compound 1-a (49.3 mmol, 20 g) and Compound 19-a (49.3 mmol, 23.0 g) in toluene (0.2 M, 250 ml) in a 3-neck flask, sodium tert-butoxide (73.9 mmol, 7.10 g) and bis(tri-tert-butylphosphine)palladium(0) (0.493 mmol, 0.252 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 19-b (36.1 g, yield 88%, MS[M+H]+=837).

### Step 3) Synthesis of Compound 19

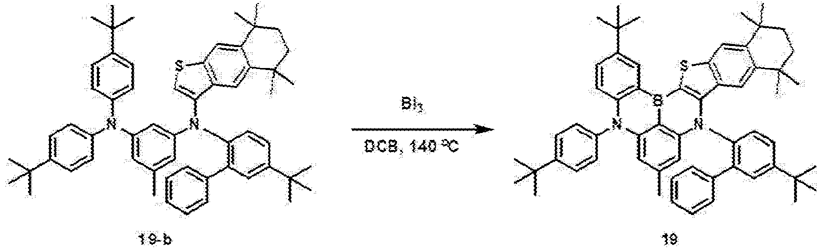

After dissolving Compound 19-b (43.1 mmol, 36.1 g) in 1,2-dichlorobenzene (0.1 M, 430 ml) in a 3-neck flask, boron triiodide (69.0 mmol, 27.0 g) was introduced thereto, and the result was stirred for 3 hours at 140°C under the argon atmosphere. The reaction material was cooled to 0°C, and after adding N,N-diisopropylethylamine (388 mmol, 50.2 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 19 (7.1 g, yield 19%, MS[M+H]+=603).

### Synthesis Example 20: Synthesis of Compound 20

### Step 1) Synthesis of Compound 20-a

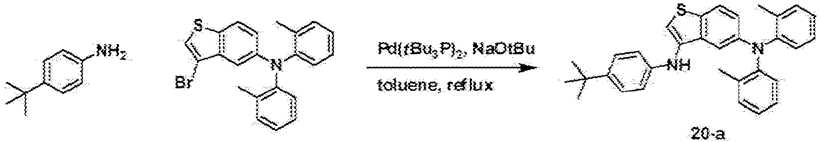

After dissolving 4-tert-butylamine (36.7 mmol, 5.48 g) and 3-bromo-*N*,*N*-di-o-tolylbenzo[*b*]thiophen-5-amine (36.7 mmol, 15 g) in toluene (0.2 M, 185 ml) in a 3-neck flask, sodium tert-butoxide (55.1 mmol, 5.29 g) and bis(tri-tert-butylphosphine)palladium(0) (0.367 mmol, 0.188 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 20-a (15.9 g, yield 910, MS[M+H]+=477).

### Step 2) Synthesis of Compound 20-b

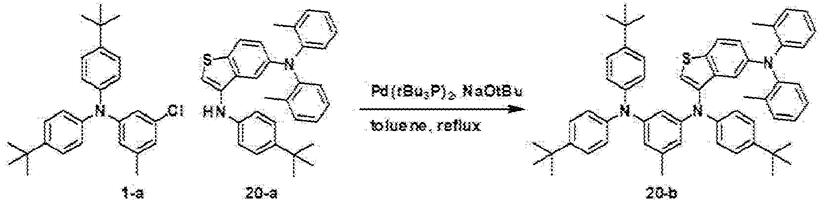

After dissolving Compound 1-a (29.6 mmol, 12 g) and Compound 20-a (29.6 mmol, 14.1 g) in toluene (0.2 M, 150 ml) in a 3-neck flask, sodium tert-butoxide (44.3 mmol, 4.26 g) and bis(tri-tert-butylphosphine)palladium(0) (0.296 mmol, 0.151 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 20-b (19.1 g, yield 76%, MS[M+H]+=846).

### Step 3) Synthesis of Compound 20

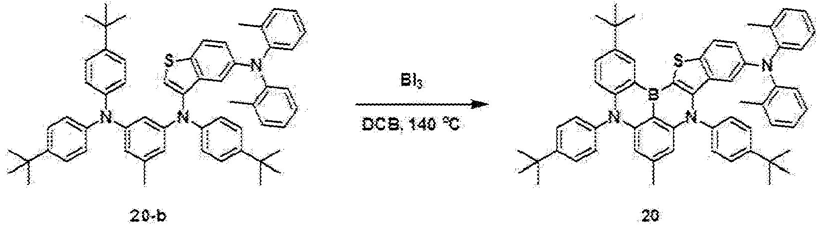

After dissolving Compound 20-b (22.6 mmol, 19.1 g) in 1,2-dichlorobenzene (0.1 M, 226 ml) in a 3-neck flask, boron triiodide (36.1 mmol, 14.1 g) was introduced thereto, and the result was stirred for 3 hours at 140°C under the argon atmosphere. The reaction material was cooled to 0°C, and after adding N,N-diisopropylethylamine (203 mmol, 26.3 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 20 (3.2 g, yield 17%, MS[M+H]+=854).

### Synthesis Example 21: Synthesis of Compound 21

### Step 1) Synthesis of Compound 21-a

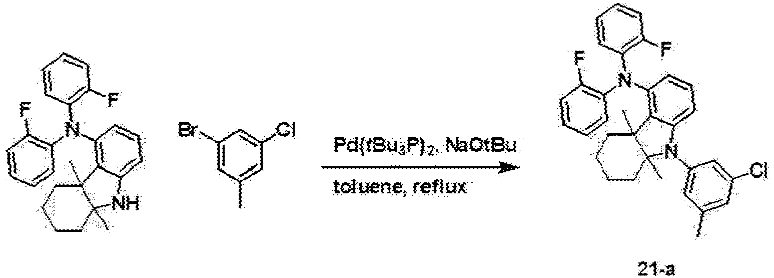

After dissolving *N,N*-bis(2-fluorophenyl)-4a,9a-dimethyl-2,3,4,4a,9,9a-hexahydro-1*H*-carbazol-5-amine (34.1 mmol, 13.8 g) and 1-bromo-3-chloro-5-methylbenzene (34.1 mmol, 7 g) in toluene (0.2 M, 170 ml) in a 3-neck flask, sodium tert-butoxide (51.1 mmol, 4.91 g) and bis(tri-tert-butylphosphine)palladium(0) (0.341 mmol, 0.174 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 21-a (15.3 g, yield 85%, MS[M+H]+=15.3).

### Step 2) Synthesis of Compound 21-b

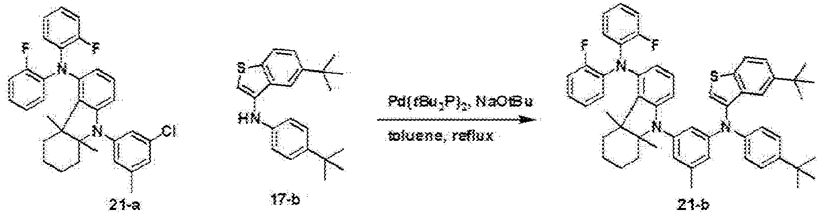

After dissolving Compound 21-a (28.4 mmol, 15 g) and Compound 17-b (28.4 mmol, 9.57 g) in toluene (0.2 M, 140 ml) in a 3-neck flask, sodium tert-butoxide (42.5 mmol, 4.09 g) and bis(tri-tert-butylphosphine)palladium(0) (0.284 mmol, 0.145 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 21-b (18.4 g, yield 78%, MS[M+H]+=830).

### Step 3) Synthesis of Compound 21

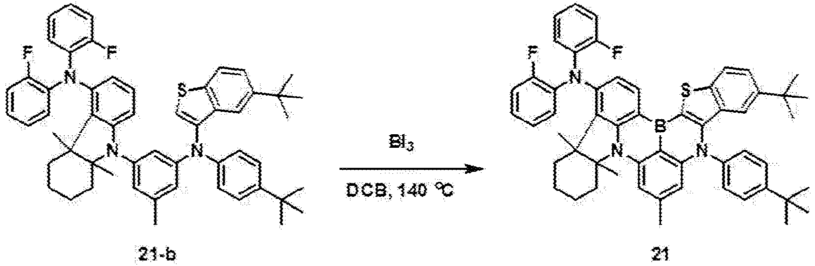

After dissolving Compound 21-b (22.2 mmol, 18.4 g) in 1,2-dichlorobenzene (0.1 M, 220 ml) in a 3-neck flask, boron triiodide (35.5 mmol, 13.9 g) was introduced thereto, and the result was stirred for 3 hours at 140°C under the argon atmosphere. The reaction material was cooled to 0°C, and after adding N,N-diisopropylethylamine (199 mmol, 25.8 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 21 (3.8 g, yield 20%, MS[M+H]+=838).

### Synthesis Example 22: Synthesis of Compound 22

### Step 1) Synthesis of Compound 22-a

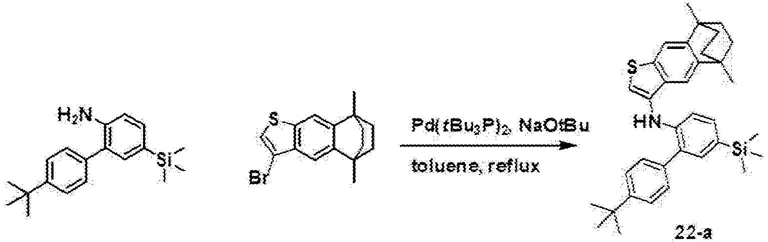

After dissolving 4'-tert-butyl-5-trimethylsilyl-[1,1'-biphenyl]-2-amine (43.6 mmol, 13 g) and 3-bromo-5,8-dimethyl-5,6,7,8-tetrahydro-5,8-ethanonaphtho[2,3-b]thiophene (43.6 mmol, 14 g) in toluene (0.2 M, 220 ml) in a 3-neck flask, sodium tert-butoxide (65.4 mmol, 6.28 g) and bis(tri-tert-butylphosphine)palladium(0) (0.436 mmol, 0.223 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 22-a (14.6 g, yield 62%, MS[M+H]+=538).

### Step 2) Synthesis of Compound 22-b

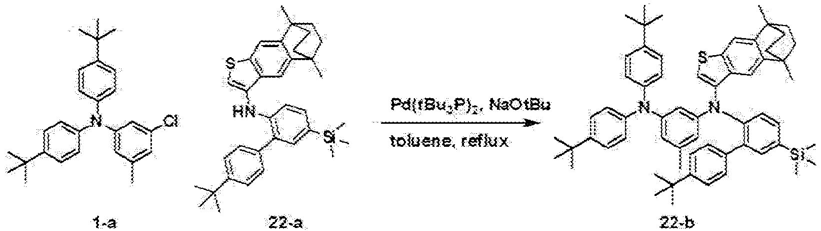

After dissolving Compound 1-a (24.6 mmol, 10 g) and Compound 22-a (24.6 mmol, 13.2 g) in toluene (0.2 M, 125 ml) in a 3-neck flask, sodium tert-butoxide (36.9 mmol, 3.55 g) and bis(tri-tert-butylphosphine)palladium(0) (0.246 mmol, 0.126 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 22-b (16.5 g, yield 74%, MS[M+H]+=907).

### Step 3) Synthesis of Compound 22

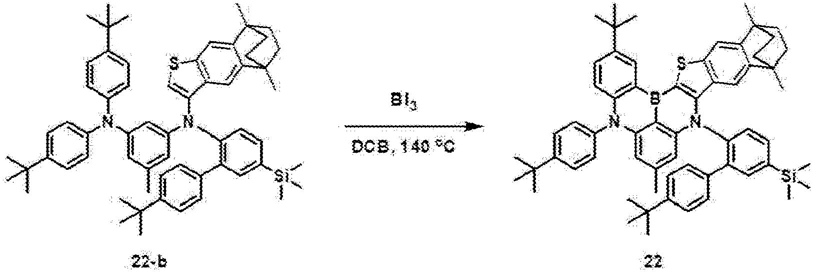

After dissolving Compound 22-b (18.2 mmol, 16.5 g) in 1,2-dichlorobenzene (0.1 M, 180 ml) in a 3-neck flask, boron triiodide (29.1 mmol, 11.4 g) was introduced thereto, and the result was stirred for 3 hours at 140°C under the argon atmosphere. The reaction material was cooled to 0°C, and after adding N,N-diisopropylethylamine (164 mmol, 21.1 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 22 (2.8 g, yield 17%, MS[M+H]+=915).

### Synthesis Example 23: Synthesis of Compound 23

### Step 1) Synthesis of Compound 23-a

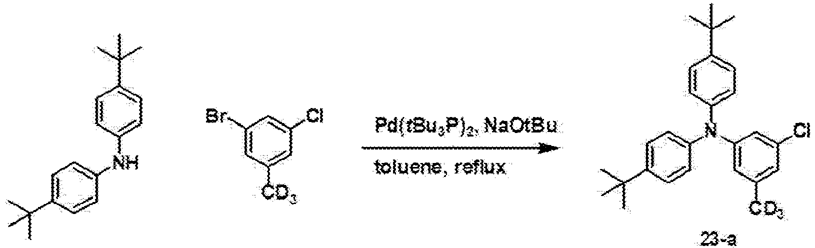

After dissolving bis-(4-tert-butylphenyl)-amine (107 mmol, 30 g) and 1-bromo-3-chloro-5-(methyl-*d*-3)benzene (107 mmol, 22.2 g) in toluene (0.2 M, 535 ml) in a 3-neck flask, sodium tert-butoxide (160 mmol, 15.4 g) and bis(tri-tert-butylphosphine)palladium(0) (1.07 mmol, 0.545 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 23-a (36.2 g, yield 83%, MS[M+H]+=409).

### Step 2) Synthesis of Compound 23-b

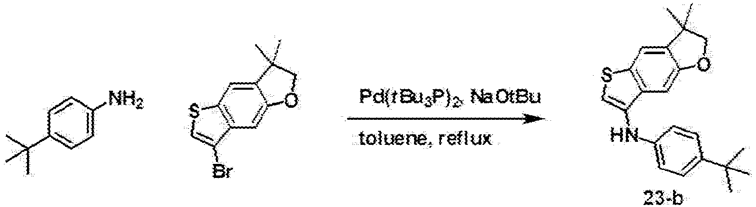

After dissolving 7-bromo-3,3-dimethyl-2,3-dihydrothieno[2,3-*f*]benzofuran (53.0 mmol, 15 g) and 4-tert-butylaniline (53.0 mmol, 7.9 g) in toluene (0.2 M, 265 ml) in a 3-neck flask, sodium tert-butoxide (79.5 mmol, 7.64 g) and bis(tri-tert-butylphosphine)palladium(0) (0.530 mmol, 0.271 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 23-b (12.7 g, yield 68%, MS[M+H]+=352).

### Step 3) Synthesis of Compound 23-c

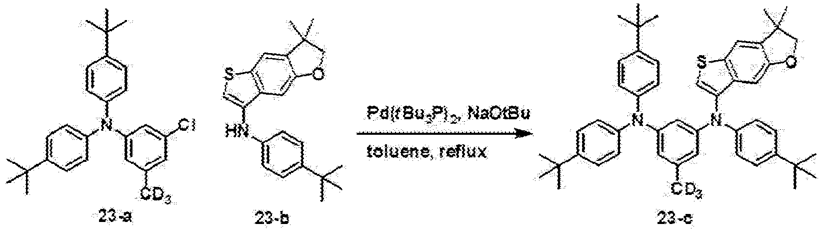

After dissolving Compound 23-a (34.2 mmol, 14 g) and Compound 23-b (34.2 mmol, 12 g) in toluene (0.2 M, 170 ml) in a 3-neck flask, sodium tert-butoxide (51.3 mmol, 4.93 g) and bis(tri-tert-butylphosphine)palladium(0) (0.342 mmol, 0.175 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 23-c (20.3 g, yield 82%, MS[M+H]+=724).

### Step 4) Synthesis of Compound 23

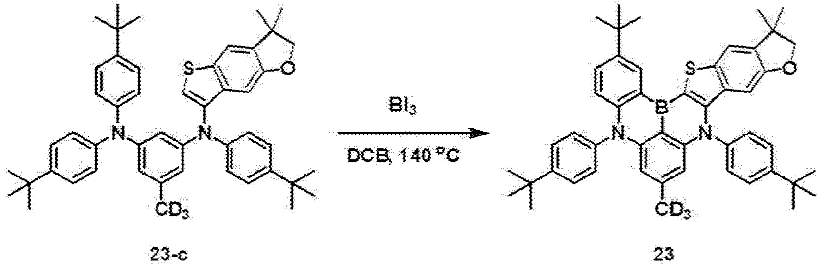

After dissolving Compound 23-c (28.0 mmol, 20.3 g) in 1,2-dichlorobenzene (0.1 M, 280 ml) in a 3-neck flask, boron triiodide (44.9 mmol, 17.6 g) was introduced thereto, and the result was stirred for 3 hours at 140°C under the argon atmosphere. The reaction material was cooled to 0°C, and after adding N,N-diisopropylethylamine (252 mmol, 32.6 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 23 (4.1 g, yield 20%, MS[M+H]+=732).

### Synthesis Example 24: Synthesis of Compound 24

### Step 1) Synthesis of Compound 24-a

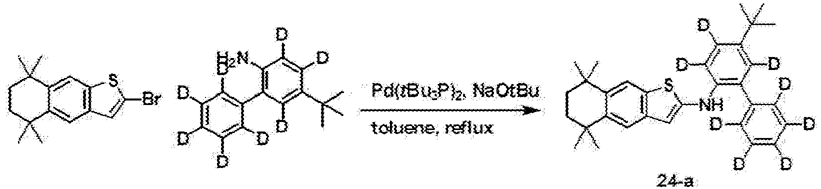

After dissolving 5-tert-butyl-[1,1'-biphenyl]-2',3,3',4,4',5',6,6'-d8-2-amine (40.2 mmol, 9.38 g) and 2-bromo-5,5,8,8-tetramethyl-5,6,7,8-tetrahydronaphtho[2,3-b]thiophene (40.2 mmol, 13 g) in toluene (0.2 M, 200 ml) in a 3-neck flask, sodium tert-butoxide (60.3 mmol, 5.80 g) and bis(tri-tert-butylphosphine)palladium(0) (0.402 mmol, 0.206 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 24-a (18.2 g, yield 95%, MS[M+H]+=476).

### Step 2) Synthesis of Compound 24-b

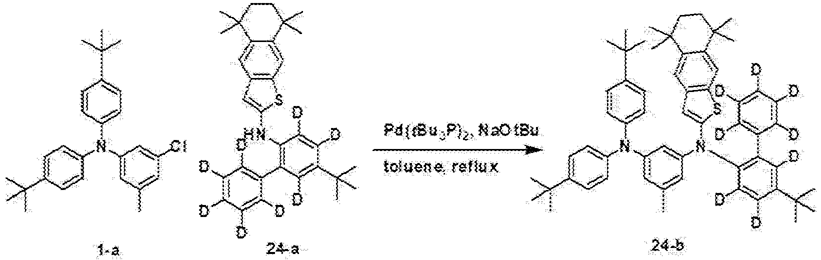

After dissolving Compound 1-a (36.9 mmol, 15 g) and Compound 24-a (36.9 mmol, 17.6 g) in toluene (0.2 M, 185 ml) in a 3-neck flask, sodium tert-butoxide (55.4 mmol, 5.33 g) and bis(tri-tert-butylphosphine)palladium(0) (0.369 mmol, 0.189 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 24-b (18.4 g, yield 59%, MS[M+H]+=845).

### Step 3) Synthesis of Compound 24

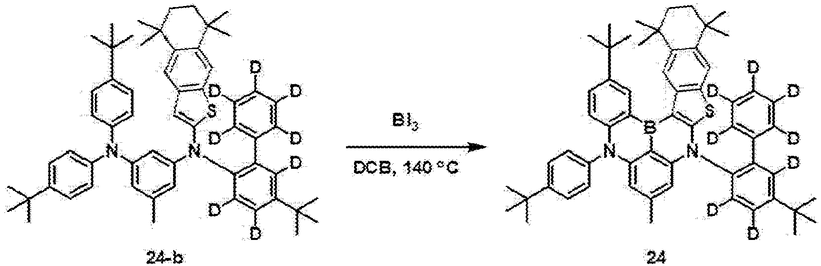

After dissolving Compound 24-b (21.8 mmol, 18.4 g) in 1,2-dichlorobenzene (0.1 M, 220 ml) in a 3-neck flask, boron triiodide (34.8 mmol, 13.6 g) was introduced thereto, and the result was stirred for 3 hours at 140°C under the argon atmosphere. The reaction material was cooled to 0°C, and after adding N,N-diisopropylethylamine (196 mmol, 25.3 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 24 (3.2 g, yield 17%, MS[M+H]+=853).

### Synthesis Example 25: Synthesis of Compound 25

### Step 1) Synthesis of Compound 25-a

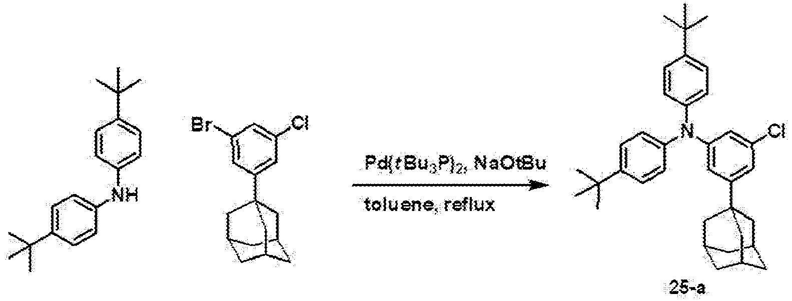

After dissolving bis-(4-tert-butylphenyl)-amine (142 mmol, 40 g) and (3r,5r,7r)-1-(3-bromo-5-chlorophenyl)adamantane (142 mmol, 46.3 g) in toluene (0.2 M, 710 ml) in a 3-neck flask, sodium tert-butoxide (213 mmol, 20.5 g) and bis(tri-tert-butylphosphine)palladium(0) (1.42 mmol, 0.726 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 25-a (63.8 g, yield 85%, MS[M+H]+=526).

### Step 2) Synthesis of Compound 25-b

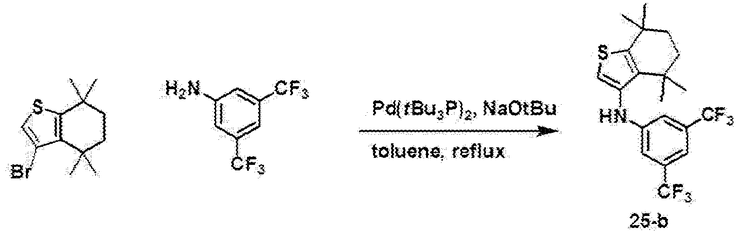

After dissolving 3-bromo-4,4,7,7-tetramethyl-4,5,6,7-tetrahydrobenzo[b]thiophene (54.9 mmol, 15 g) and 3,5-bis(trifluoromethyl)aniline (54.9 mmol, 12.6 g) in toluene (0.2 M, 275 ml) in a 3-neck flask, sodium tert-butoxide (82.3 mmol, 7.91 g) and bis(tri-tert-butylphosphine)palladium(0) (0.549 mmol, 0.281 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 25-b (16.7 g, yield 72%, MS[M+H]+=421).

### Step 3) Synthesis of Compound 25-c

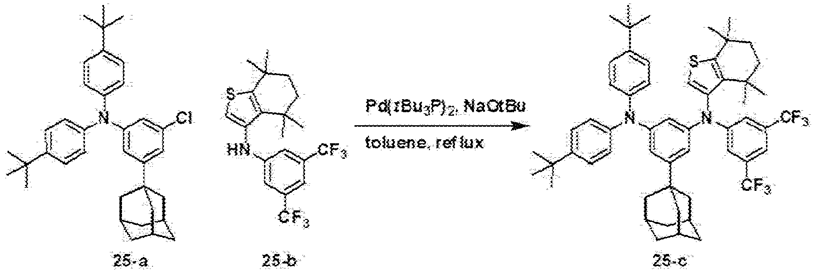

After dissolving Compound 25-a (38.0 mmol, 20 g) and Compound 25-b (38.0 mmol, 16 g) in toluene (0.2 M, 190 ml) in a 3-neck flask, sodium tert-butoxide (57.0 mmol, 5.48 g) and bis(tri-tert-butylphosphine)palladium(0) (0.380 mmol, 0.194 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 25-c (22.7 g, yield 66%, MS[M+H]+=911).

### Step 4) Synthesis of Compound 25

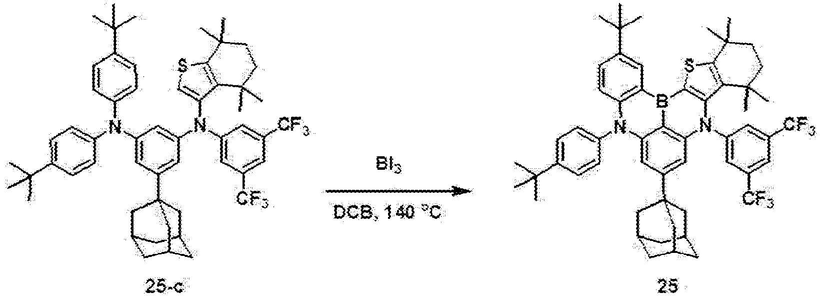

After dissolving Compound 25-c (24.9 mmol, 22.7 g) in 1,2-dichlorobenzene (0.1 M, 250 ml) in a 3-neck flask, boron triiodide (39.9 mmol, 15.6 g) was introduced thereto, and the result was stirred for 3 hours at 140°C under the argon atmosphere. The reaction material was cooled to 0°C, and after adding N,N-diisopropylethylamine (224 mmol, 29.0 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 25 (3.9 g, yield 17%, MS[M+H]+=919).

### Synthesis Example 26: Synthesis of Compound 26

### Step 1) Synthesis of Compound 26-a

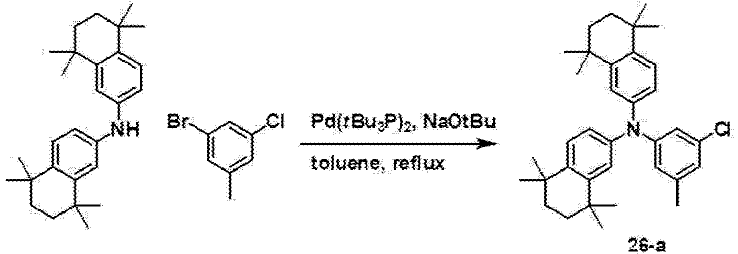

After dissolving bis-(5,5,8,8-tetramethyl-5,6,7,8-tetrahydronaphthalen-2-yl)amine (77.0 mmol, 30 g) and 1-bromo-3-chloro-5-methylbenzene (77.0 mmol, 15.8 g) in toluene (0.2 M, 385 ml) in a 3-neck flask, sodium tert-butoxide (115 mmol, 11.1 g) and bis(tri-tert-butylphosphine)palladium(0) (0.77 mmol, 0.393 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 26-a (36.6 g, yield 92%, MS[M+H]+=514).

### Step 2) Synthesis of Compound 26-b

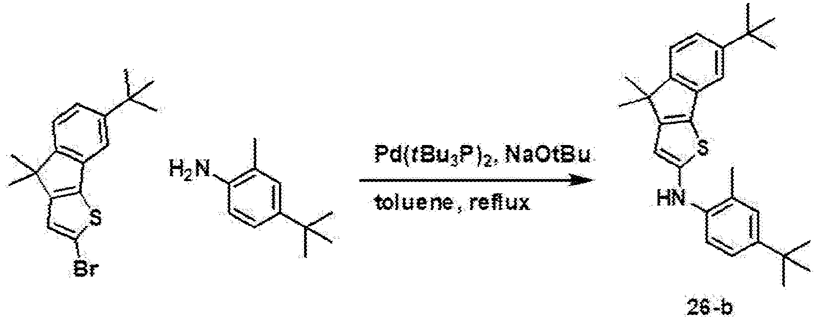

After dissolving 2-bromo-7-tert-butyl-4,4-dimethyl-4*H-*indeno[1,2-b]thiophene (35.8 mmol, 12 g) and 4-tert-butyl-2-methylaniline (35.8 mmol, 5.84 g) in toluene (0.2 M, 180 ml) in a 3-neck flask, sodium tert-butoxide (53.7 mmol, 5.16 g) and bis(tri-tert-butylphosphine)palladium(0) (0.358 mmol, 0.183 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 26-b (13.4 g, yield 90%, MS[M+H]+=418).

### Step 3) Synthesis of Compound 26-c

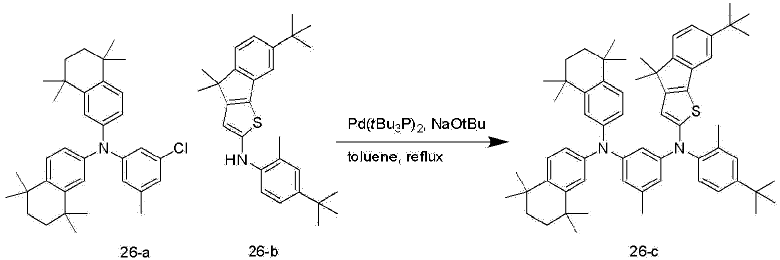

After dissolving Compound 26-a (29.2 mmol, 15 g) and Compound 26-b (29.2 mmol, 12.2 g) in toluene (0.2 M, 145 ml) in a 3-neck flask, sodium tert-butoxide (43.8 mmol, 4.21 g) and bis(tri-tert-butylphosphine)palladium(0) (0.292 mmol, 0.149 g) were introduced thereto, and the result was stirred for 15 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 26-c (19.2 g, yield 74%, MS[M+H]+=895).

### Step 4) Synthesis of Compound 26

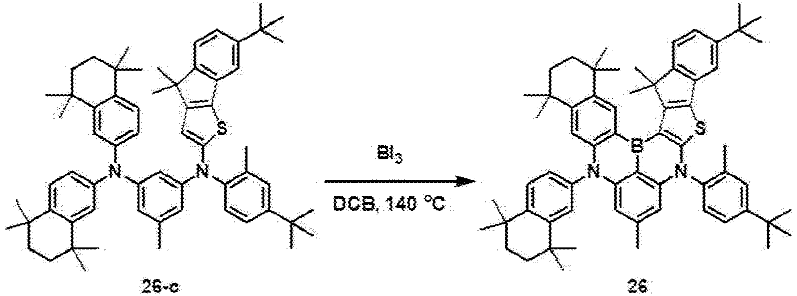

After dissolving Compound 26-c (21.4 mmol, 19.2 g) in 1,2-dichlorobenzene (0.1 M, 215 ml) in a 3-neck flask, boron triiodide (34.3 mmol, 13.4 g) was introduced thereto, and the result was stirred for 3 hours at 140°C under the argon atmosphere. The reaction material was cooled to 0°C, and after adding N,N-diisopropylethylamine (193 mmol, 24.9 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 26 (4.5 g, yield 23%, MS[M+H]+=903).

### Synthesis Example 27: Synthesis of Compound 27

### Step 1) Synthesis of Compound 27-a

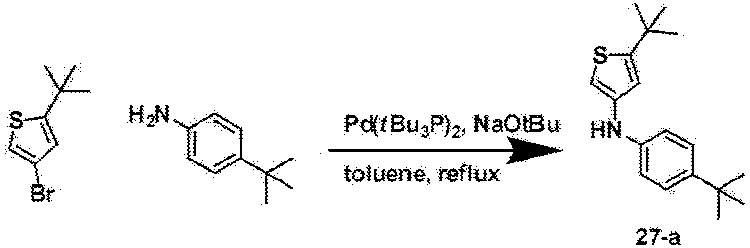

After dissolving 4-tert-butylaniline (80.4 mmol, 12 g) and 4-bromo-2-tert-butylthiophene (80.4 mmol, 17.6 g) in toluene (0.2 M, 400 ml) in a 3-neck flask, sodium tert-butoxide (121 mmol, 11.6 g) and bis(tri-tert-butylphosphine)palladium(0) (0.804 mmol, 0.411 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 27-a (19.2 g, yield 83%, MS[M+H]+=287).

### Step 2) Synthesis of Compound 27-b

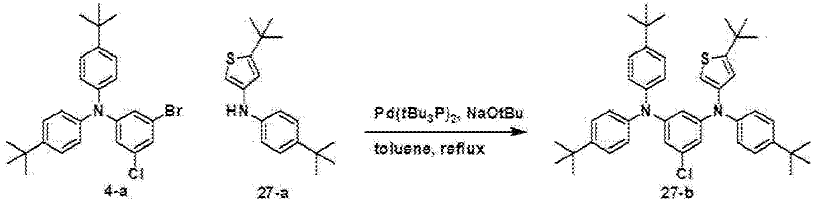

After dissolving Compound 4-a (38.2 mmol, 18 g) and Compound 27-a (38.2 mmol, 11 g) in toluene (0.2 M, 190 ml) in a 3-neck flask, sodium tert-butoxide (57.3 mmol, 5.51 g) and bis(tri-tert-butylphosphine)palladium(0) (0.382 mmol, 0.195 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 27-b (21.4 g, yield 83%, MS[M+H]+=677).

### Step 3) Synthesis of Compound 27-c

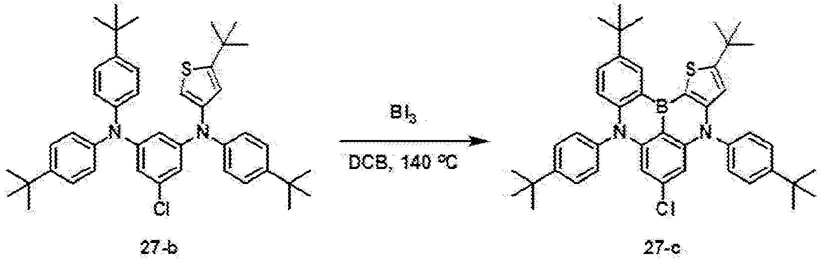

After dissolving Compound 27-b (31.6 mmol, 21.4 g) in 1,2-dichlorobenzene (0.1 M, 315 ml) in a 3-neck flask, boron triiodide (50.5 mmol, 19.8 g) was introduced thereto, and the result was stirred for 3 hours at 140°C under the argon atmosphere. The reaction material was cooled to 0°C, and after adding N,N-diisopropylethylamine (284 mmol, 36.7 g) thereto, the result was stirred for 1 hour. The result was extracted in a separatory funnel using toluene and H₂O. The extract was dried with MgSO₄ and concentrated, and the sample was purified using silica gel column chromatography to obtain Compound 27-c (4.4 g, yield 20%, MS[M+H]+=685).

### Step 4) Synthesis of Compound 27

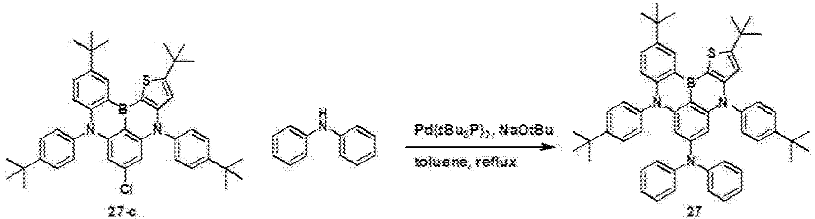

After dissolving Compound 27-c (6.42 mmol, 4.4 g) and diphenylamine (7.7 mmol, 1.30 g) in toluene (0.2 M, 40 ml) in a 3-neck flask, sodium tert-butoxide (9.63 mmol, 0.926 g) and bis(tri-tert-butylphosphine)palladium(0) (0.06 mmol, 0.033 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography and then went through sublimation purification to obtain Compound 27 (4.1 g, yield 78%, MS[M+H]+=818).

### Synthesis Example 28: Synthesis of Compound 28

### Step 1) Synthesis of Compound 28-a

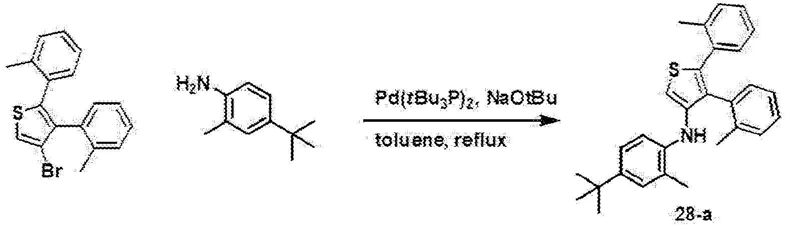

After dissolving 4-tert-butyl-2-methylaniline (30.6 mmol, 5 g) and 4-bromo-2,3-di-o-tolylthiophene (30.6 mmol, 10.5 g) in toluene (0.2 M, 155 ml) in a 3-neck flask, sodium tert-butoxide (45.9 mmol, 4.41 g) and bis(tri-tert-butylphosphine)palladium(0) (0.306 mmol, 0.157 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 28-a (10.4 g, yield 80%, MS[M+H]+=426).

### Step 2) Synthesis of Compound 28-b

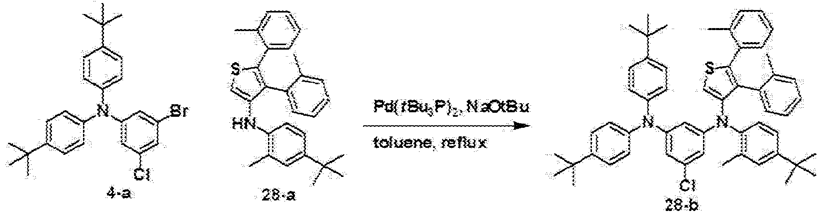

After dissolving Compound 4-a (23.4 mmol, 11 g) and Compound 28-a (23.4 mmol, 9.94 g) in toluene (0.2 M, 120 ml) in a 3-neck flask, sodium tert-butoxide (35.0 mmol, 3.37 g) and bis(tri-tert-butylphosphine)palladium(0) (0.234 mmol, 0.119 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 28-b (11.8 g, yield 62%, MS[M+H]+=816).

### Step 3) Synthesis of Compound 28-c

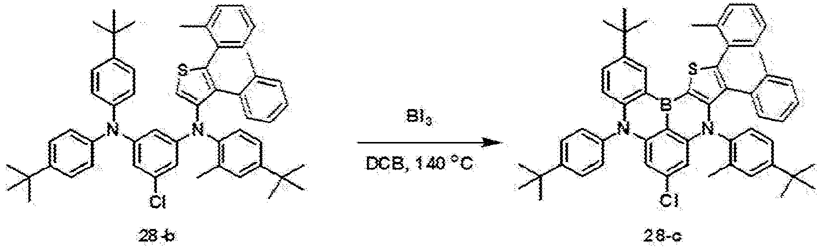

After dissolving Compound 28-b (14.5 mmol, 11.8 g) in 1,2-dichlorobenzene (0.1 M, 145 ml) in a 3-neck flask, boron triiodide (23.1 mmol, 9.06 g) was introduced thereto, and the result was stirred for 3 hours at 140°C under the argon atmosphere. The reaction material was cooled to 0°C, and after adding N,N-diisopropylethylamine (130 mmol, 16.8 g) thereto, the result was stirred for 1 hour. The result was extracted in a separatory funnel using toluene and H₂O. The extract was dried with MgSO₄ and concentrated, and the sample was purified using silica gel column chromatography to obtain Compound 28-c (2.9 g, yield 24%, MS[M+H]+=823).

### Step 4) Synthesis of Compound 28

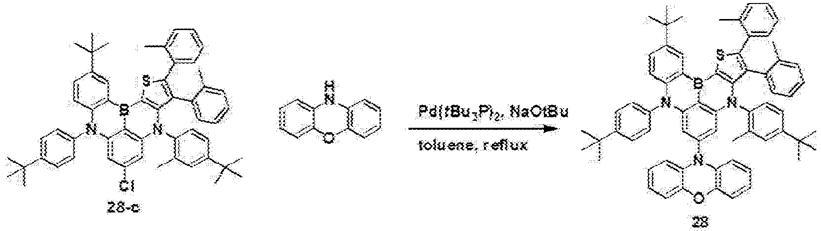

After dissolving Compound 28-c (3.52 mmol, 2.9 g) and 10H-phenoxazine (4.23 mmol, 0.774 g) in toluene (0.2 M, 21 ml) in a 3-neck flask, sodium tert-butoxide (5.28 mmol, 0.508 g) and bis(tri-tert-butylphosphine)palladium(0) (0.035 mmol, 0.018 g) were introduced thereto, and the result was stirred for 18 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography and then went through sublimation purification to obtain Compound 28 (2.9 g, yield 85%, MS[M+H]+=970).

### Synthesis Example 29: Synthesis of Compound 29

### Step 1) Synthesis of Compound 29-a

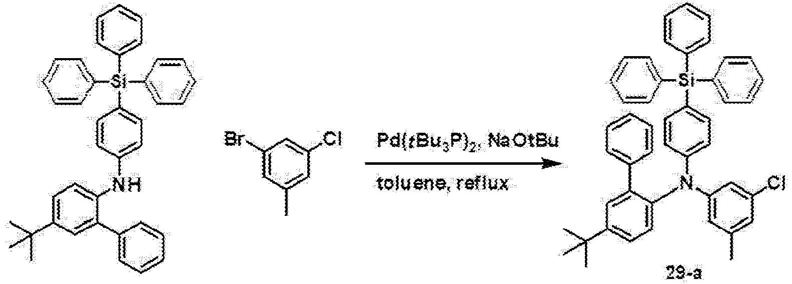

After dissolving 5-tert-butyl-*N*-(4-triphenylsilylphenyl)-[1,1'-biphenyl]-2-amine (53.6 mmol, 30 g) and 1-bromo-3-chloro-5-methylbenzene (53.6 mmol, 11.0 g) in toluene (0.2 M, 270 ml) in a 3-neck flask, sodium tert-butoxide (80.4 mmol, 7.72 g) and bis(tri-tert-butylphosphine)palladium(0) (0.536 mmol, 0.274 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 29-a (27.1 g, yield 74%, MS[M+H]+=684).

### Step 2) Synthesis of Compound 29-b

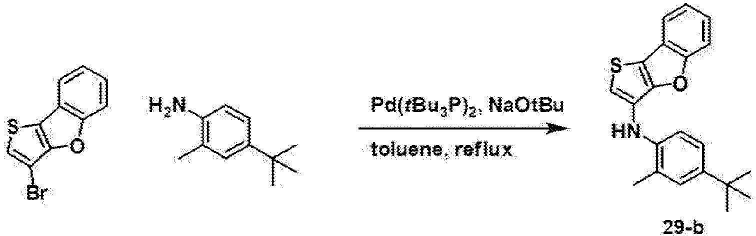

After dissolving 3-bromothieno[2,3-*b*]benzofuran (39.5 mmol, 10 g) and 4-tert-butyl-2-methylaniline (39.5 mmol, 6.45 g) in toluene (0.2 M, 200 ml) in a 3-neck flask, sodium tert-butoxide (59.3 mmol, 5.70 g) and bis(tri-tert-butylphosphine)palladium(0) (0.358 mmol, 0.183 g) were introduced thereto, and the result was stirred for 15 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 29-b (9.8 g, yield 74%, MS[M+H]+=335).

### Step 3) Synthesis of Compound 29-c

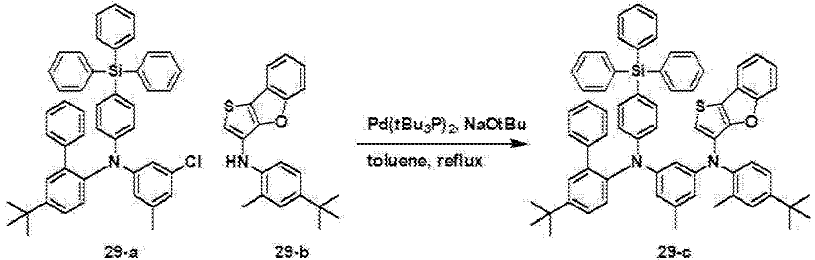

After dissolving Compound 29-a (29.2 mmol, 20 g) and Compound 29-b (29.2 mmol, 9.80 g) in toluene (0.2 M, 145 ml) in a 3-neck flask, sodium tert-butoxide (43.8 mmol, 4.21 g) and bis(tri-tert-butylphosphine)palladium(0) (0.292 mmol, 0.149 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 29-c (23.1 g, yield 80%, MS[M+H]+=983).

### Step 4) Synthesis of Compound 29

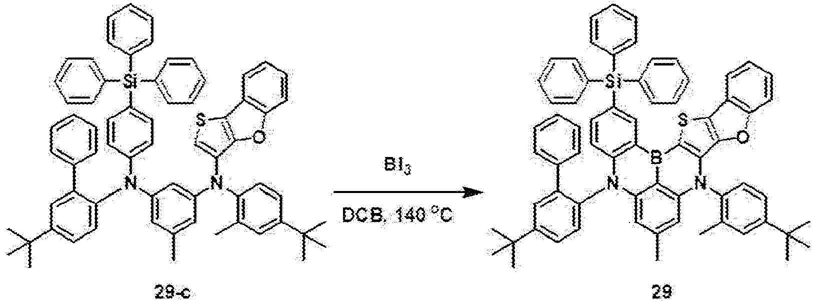

After dissolving Compound 29-c (23.5 mmol, 23.1 g) in 1,2-dichlorobenzene (0.1 M, 235 ml) in a 3-neck flask, boron triiodide (37.6 mmol, 14.7 g) was introduced thereto, and the result was stirred for 3 hours at 140°C under the argon atmosphere. The reaction material was cooled to 0°C, and after adding N,N-diisopropylethylamine (211 mmol, 27.3 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 29 (3.7 g, yield 16%, MS[M+H]+=991).

### Synthesis Example 30: Synthesis of Compound 30

### Step 1) Synthesis of Compound 30-a

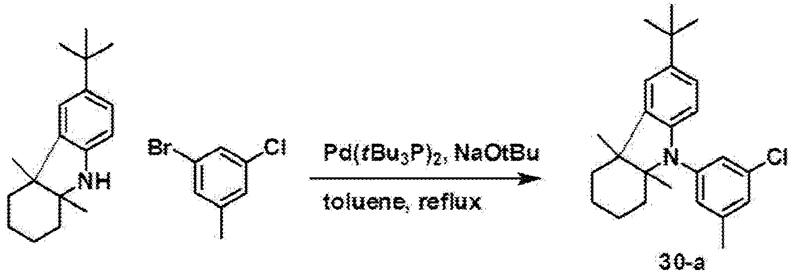

After dissolving 6-tert-butyl-4a,9a-dimethyl-2,3,4,4a,9,9a-hexahydro-1*H*-carbazole (194 mmol, 50 g) and 1-bromo-3-chloro-5-methylbenzene (194 mmol, 39.9 g) in toluene (0.2 M, 970 ml) in a 3-neck flask, sodium tert-butoxide (291 mmol, 28.0 g) and bis(tri-tert-butylphosphine)palladium(0) (1.94 mmol, 0.993 g) were introduced thereto, and the result was stirred for 8 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 30-a (67.2 g, yield 910, MS[M+H]+=382).

### Step 2) Synthesis of Compound 30-b

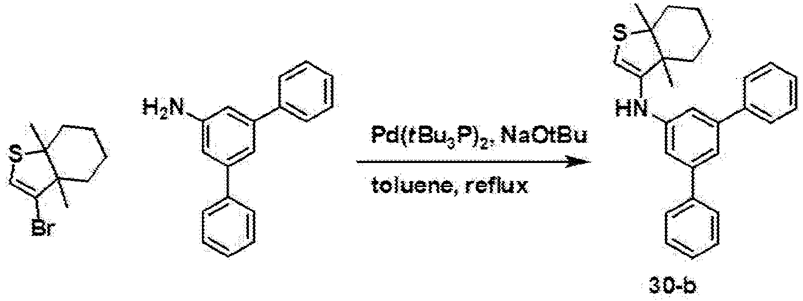

After dissolving 3-bromo-3a,7a-dimethyl-3a,4,5,6,7,7a-hexahydro[*b*]thiophene (56.6 mmol, 14 g) and [1,1':3',1"-terphenyl]-5'-amine (56.6 mmol, 13.9 g) in toluene (0.2 M, 280 ml) in a 3-neck flask, sodium tert-butoxide (85.0 mmol, 8.16 g) and bis(tri-tert-butylphosphine)palladium(0) (0.566 mmol, 0.289 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 30-b (17.4 g, yield 75%, MS[M+H]+=412).

### Step 3) Synthesis of Compound 30-c

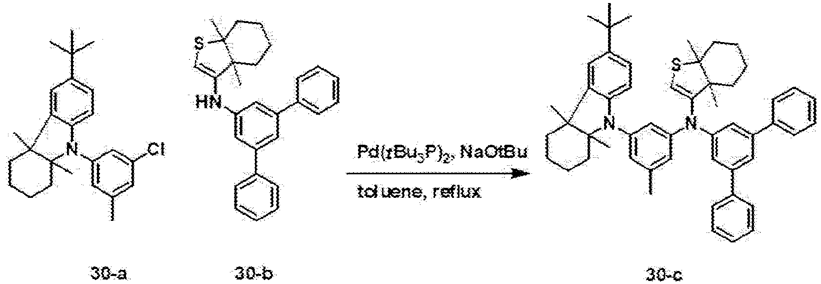

After dissolving Compound 30-a (39.3 mmol, 15 g) and Compound 30-b (39.3 mmol, 16.2 g) in toluene (0.2 M, 200 ml) in a 3-neck flask, sodium tert-butoxide (58.9 mmol, 5.66 g) and bis(tri-tert-butylphosphine)palladium(0) (0.393 mmol, 0.2 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 30-c (24.8 g, yield 83%, MS[M+H]+=757).

### Step 4) Synthesis of Compound 30

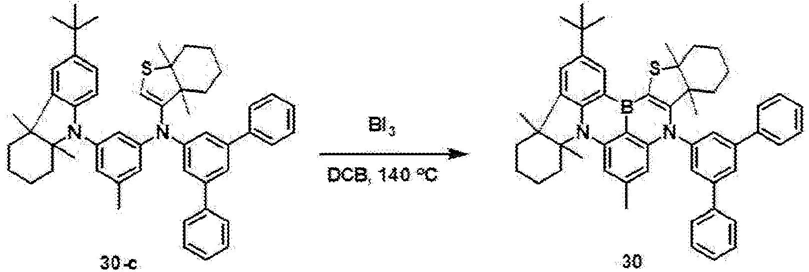

After dissolving Compound 30-c (32.8 mmol, 24.8 g) in 1,2-dichlorobenzene (0.1 M, 330 ml) in a 3-neck flask, boron triiodide (52.4 mmol, 20.5 g) was introduced thereto, and the result was stirred for 3 hours at 140°C under the argon atmosphere. The reaction material was cooled to 0°C, and after adding N,N-diisopropylethylamine (295 mmol, 38.1 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 30 (4.1 g, yield 16%, MS[M+H]+=765).

### Synthesis Example 31: Synthesis of Compound 31

### Step 1) Synthesis of Compound 31-a

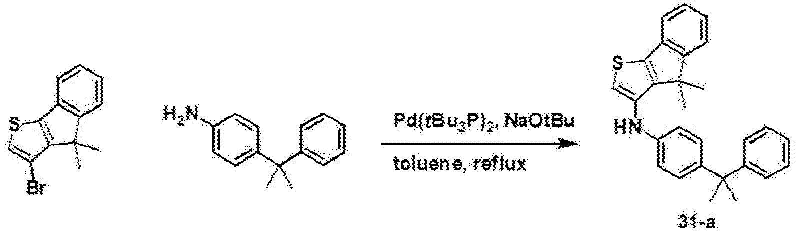

After dissolving 3-bromo-4,4-dimethyl-4*H*-indeno[1,2-b]thiophene (36.1 mmol, 10 g) and 4-(2-phenylpropan-2-yl)aniline (36.1 mmol, 8 g) in toluene (0.2 M, 180 ml) in a 3-neck flask, sodium tert-butoxide (54.2 mmol, 5.21 g) and bis(tri-tert-butylphosphine)palladium(0) (0.361 mmol, 0.185 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 31-a (12.5 g, yield 84%, MS[M+H]+=410).

### Step 2) Synthesis of Compound 31-b

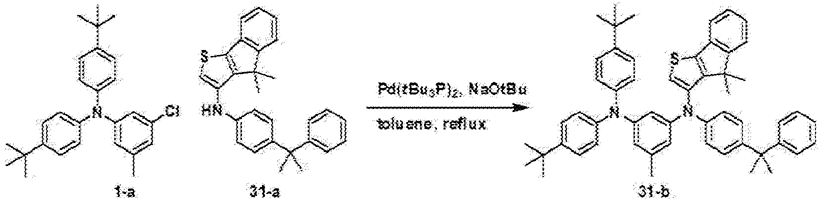

After dissolving Compound 1-a (29.6 mmol, 12 g) and Compound 31-a (29.6 mmol, 12.1 g) in toluene (0.2 M, 150 ml) in a 3-neck flask, sodium tert-butoxide (44.3 mmol, 4.26 g) and bis(tri-tert-butylphosphine)palladium(0) (0.296 mmol, 0.151 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 31-b (17.3 g, yield 75%, MS[M+H]+=779).

### Step 3) Synthesis of Compound 31

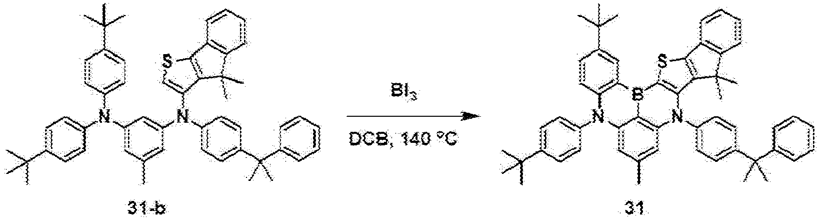

After dissolving Compound 31-b (22.2 mmol, 17.3 g) in 1,2-dichlorobenzene (0.1 M, 220 ml) in a 3-neck flask, boron triiodide (35.5 mmol, 13.9 g) was introduced thereto, and the result was stirred for 3 hours at 140°C under the argon atmosphere. The reaction material was cooled to 0°C, and after adding N,N-diisopropylethylamine (200 mmol, 25.8 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 31 (3.8 g, yield 22%, MS[M+H]+=787).

### Synthesis Example 32: Synthesis of Compound 32

### Step 1) Synthesis of Compound 32-a

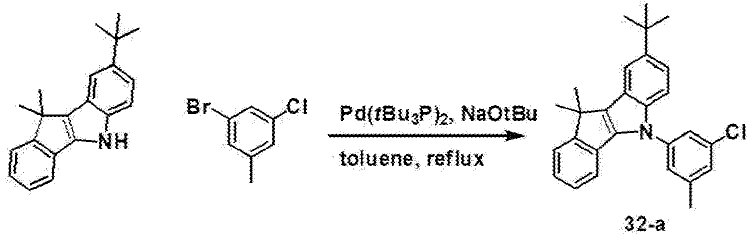

After dissolving 8-tert-butyl-10,10-dimethyl-5,10-dihydroindeno[1,2-*b*]indole (104 mmol, 30 g) and 1-bromo-3-chloro-5-methylbenzene (104 mmol, 21.3 g) in toluene (0.2 M, 520 ml) in a 3-neck flask, sodium tert-butoxide (155 mmol, 14.9 g) and bis(tri-tert-butylphosphine)palladium(0) (1.04 mmol, 0.530 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 32-a (39.2 g, yield 910, MS[M+H]+=414).

### Step 2) Synthesis of Compound 32-b

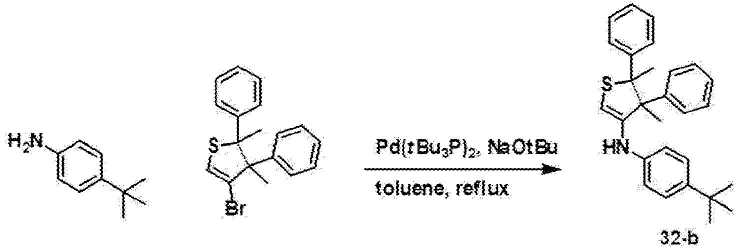

After dissolving 4-bromo-2,3-dimethyl-2,3-diphenyl-2,3-dihydrothiophene (57.9 mmol, 20 g) and 4-tert-butylaniline (57.9 mmol, 8.64 g) in toluene (0.2 M, 290 ml) in a 3-neck flask, sodium tert-butoxide (86.9 mmol, 8.35 g) and bis(tri-tert-butylphosphine)palladium(0) (0.579 mmol, 0.296 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 32-b (12.3 g, yield 510, MS[M+H]+=414).

### Step 3) Synthesis of Compound 32-c

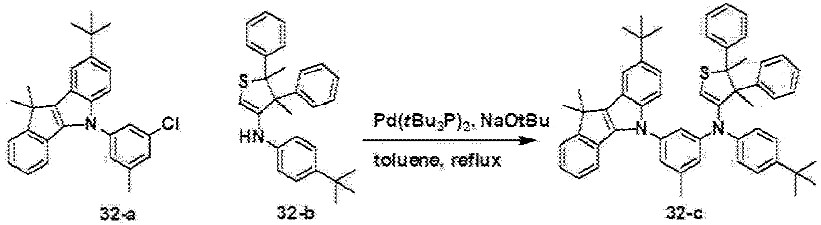

After dissolving Compound 32-a (29.0 mmol, 12 g) and Compound 32-b (29.0 mmol, 12 g) in toluene (0.2 M, 145 ml) in a 3-neck flask, sodium tert-butoxide (43.5 mmol, 4.18 g) and bis(tri-tert-butylphosphine)palladium(0) (0.290 mmol, 0.148 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 32-c (18.8 g, yield 82%, MS[M+H]+=791).

### Step 4) Synthesis of Compound 32

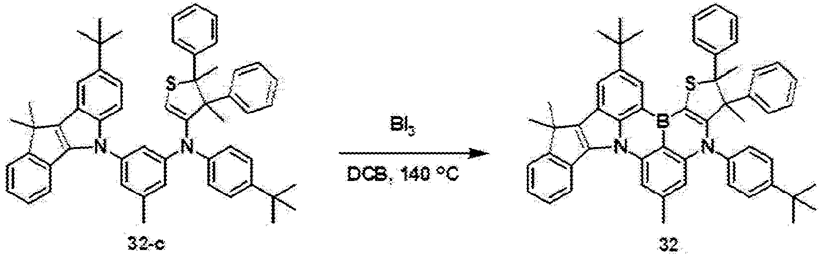

After dissolving Compound 32-c (23.8 mmol, 18.8 g) in 1,2-dichlorobenzene (0.1 M, 240 ml) in a 3-neck flask, boron triiodide (38.0 mmol, 14.9 g) was introduced thereto, and the result was stirred for 3 hours at 140°C under the argon atmosphere. The reaction material was cooled to 0°C, and after adding N,N-diisopropylethylamine (214 mmol, 27.6 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 32 (3.1 g, yield 16%, MS[M+H]+=799).

### Synthesis Example 33: Synthesis of Compound 33

### Step 1) Synthesis of Compound 33-a

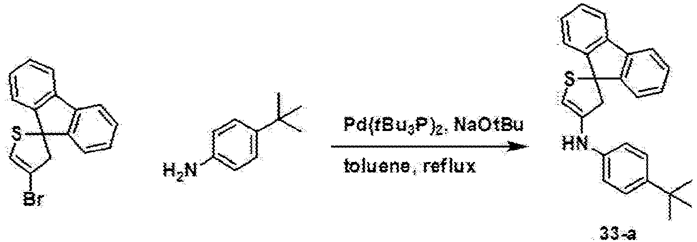

After dissolving 4-tert-butylaniline (67.0 mmol, 10 g) and 4'-bromo-3'*H*-spiro[fluorene-9,2'-thiophene] (67.0 mmol, 21.1 g) in toluene (0.2 M, 335 ml) in a 3-neck flask, sodium tert-butoxide (101 mmol, 9.66 g) and bis(tri-tert-butylphosphine)palladium(0) (0.670 mmol, 0.342 g) were introduced thereto, and the result was stirred for 15 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 33-a (16.8 g, yield 65%, MS[M+H]+=384).

### Step 2) Synthesis of Compound 33-b

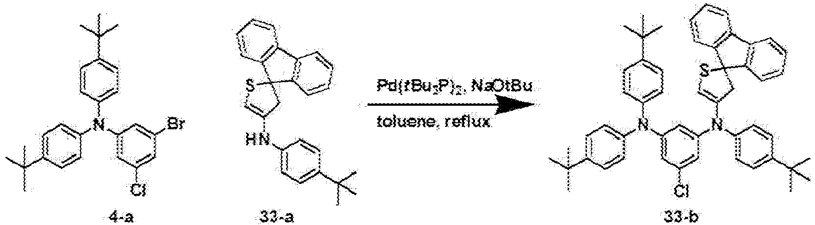

After dissolving Compound 4-a (42.5 mmol, 20 g) and Compound 33-a (42.5 mmol, 16.3 g) in toluene (0.2 M, 210 ml) in a 3-neck flask, sodium tert-butoxide (63.7 mmol, 6.12 g) and bis(tri-tert-butylphosphine)palladium(0) (0.425 mmol, 0.217 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 33-b (23.0 g, yield 70%, MS[M+H]+=774).

### Step 3) Synthesis of Compound 33-c

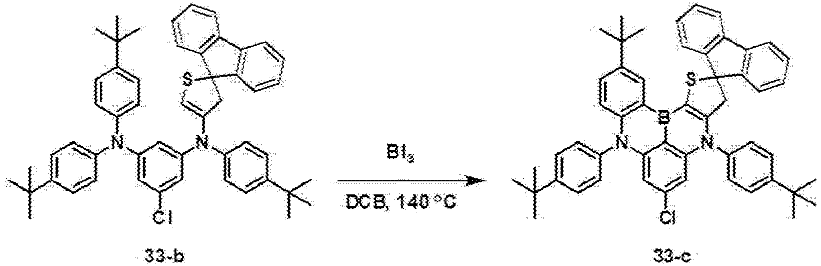

After dissolving Compound 33-b (29.7 mmol, 23 g) in 1,2-dichlorobenzene (0.1 M, 300 ml) in a 3-neck flask, boron triiodide (47.5 mmol, 18.6 g) was introduced thereto, and the result was stirred for 3 hours at 140°C under the argon atmosphere. The reaction material was cooled to 0°C, and after adding N,N-diisopropylethylamine (268 mmol, 34.6 g) thereto, the result was stirred for 1 hour. The result was extracted in a separatory funnel using toluene and H₂O. The extract was dried with MgSO₄ and concentrated, and the sample was purified using silica gel column chromatography to obtain Compound 33-c (3.9 g, yield 17%, MS[M+H]+=781).

### Step 4) Synthesis of Compound 33

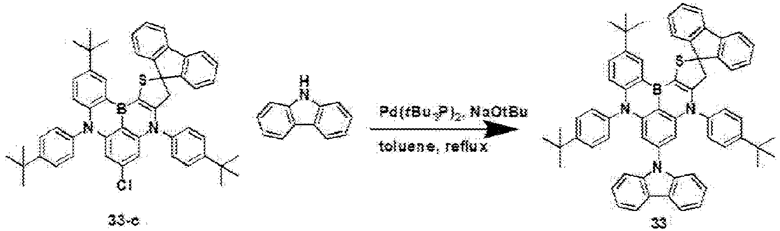

After dissolving Compound 33-c (4.99 mmol, 3.9 g) and 9*H-*carbazole (5.99 mmol, 1.00 g) in toluene (0.2 M, 30 ml) in a 3-neck flask, sodium tert-butoxide (7.49 mmol, 0.719 g) and bis(tri-tert-butylphosphine)palladium(0) (0.05 mmol, 0.026 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography and then went through sublimation purification to obtain Compound 33 (3.7 g, yield 810, MS[M+H]+=912).

### Synthesis Example 34: Synthesis of Compound 34

### Step 1) Synthesis of Compound 34-a

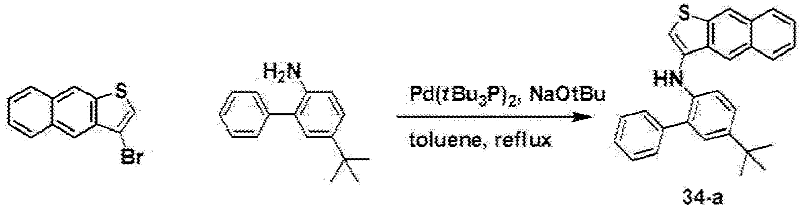

After dissolving 3-bromonaphtho[2,3-*b*]thiophene (88.8 mmol, 23.4 g) and 5-tert-butyl-[1,1'-biphenyl]-2-amine (88.8 mmol, 20 g) in toluene (0.2 M, 445 ml) in a 3-neck flask, sodium tert-butoxide (133 mmol, 12.8 g) and bis(tri-tert-butylphosphine)palladium(0) (0.888 mmol, 0.454 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 34-a (21.9 g, yield 610, MS[M+H]+=408).

### Step 2) Synthesis of Compound 34-b

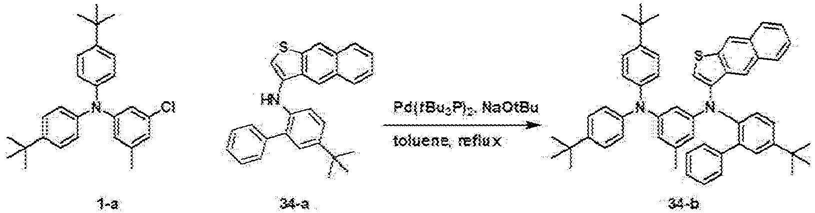

After dissolving Compound 1-a (24.6 mmol, 10 g) and Compound 34-a (24.6 mmol, 10 g) in toluene (0.2 M, 125 ml) in a 3-neck flask, sodium tert-butoxide (36.9 mmol, 3.55 g) and bis(tri-tert-butylphosphine)palladium(0) (0.246 mmol, 0.126 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 34-b (16.3 g, yield 85%, MS[M+H]+=777).

### Step 3) Synthesis of Compound 34

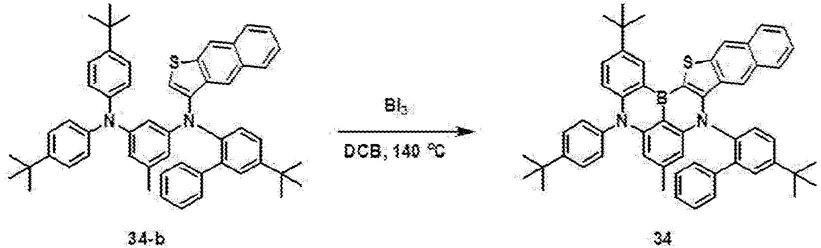

After dissolving Compound 34-b (21.0 mmol, 16.3 g) in 1,2-dichlorobenzene (0.1 M, 210 ml) in a 3-neck flask, boron triiodide (33.6 mmol, 13.1 g) was introduced thereto, and the result was stirred for 3 hours at 140°C under the argon atmosphere. The reaction material was cooled to 0°C, and after adding N,N-diisopropylethylamine (189 mmol, 24.4 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 34 (4.8 g, yield 29%, MS[M+H]+=785).

### Synthesis Example 35: Synthesis of Compound 35

### Step 1) Synthesis of Compound 35-a

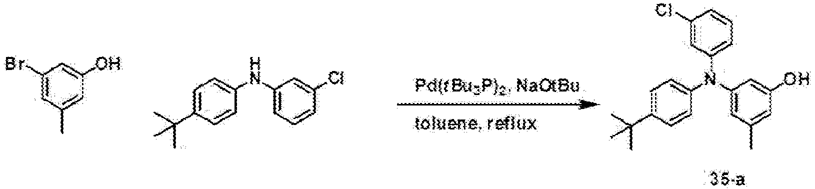

After dissolving 3-bromo-5-methylphenol (53.5 mmol, 10 g) and *N*-(4-tert-butylphenyl)-3-chloroaniline (53.5 mmol, 13.9 g) in toluene (0.2 M, 270 ml) in a 3-neck flask, sodium tert-butoxide (80.2 mmol, 7.71 g) and bis(tri-tert-butylphosphine)palladium(0) (0.535 mmol, 0.273 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 35-a (17.6 g, yield 90%, MS[M+H]+=366).

### Step 2) Synthesis of Compound 35-b

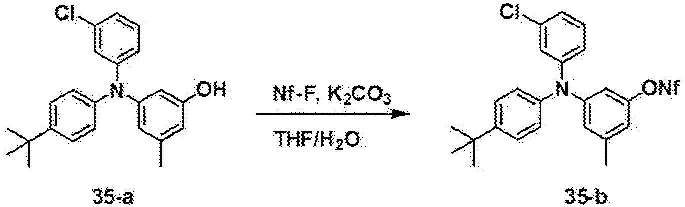

After dissolving Compound 35-a (48.1 mmol, 17.6 g) and potassium carbonate (144 mmol, 20 g) in tetrahydrofuran and water (0.1 M, 480 ml) in a 3-neck flask, perfluoro-1-butanesulfone fluoride (144 mmol, 43.6 g) was introduced thereto, and the result was stirred for 5 hours at room temperature. When the reaction was finished, toluene and water were introduced thereto, and the reaction solution was transferred to a separatory funnel and extracted. The extract was dried with MgSO₄ and concentrated, and the sample was purified using silica gel column chromatography to obtain Compound 35-b (30.2 g, yield 97%, MS[M+H]+=648).

### Step 3) Synthesis of Compound 35-c

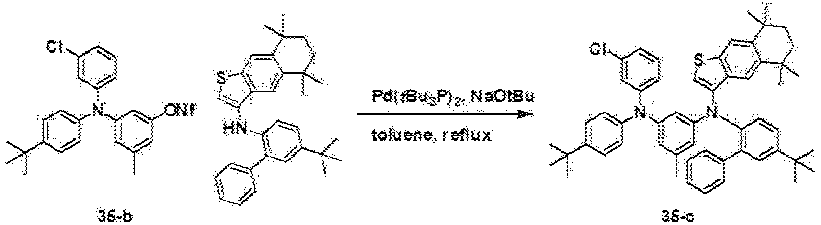

After dissolving Compound 35-b (46.6 mmol, 30.2 g) and N-(5-tert-butyl-[1,1'-biphenyl]-2-yl)-5, 5, 8, 8-tetramethyl-5,6,7,8-tetranaphtho[2,3-*b*]thiophen-3-amine (46.6 mmol, 21.8 g) in toluene (0.2 M, 233 ml) in a 3-neck flask, sodium tert-butoxide (69.9 mmol, 6.72 g) and bis(tri-tert-butylphosphine)palladium(0) (0.466 mmol, 0.238 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 35-c (27.4 g, yield 72%, MS[M+H]+=816).

### Step 4) Synthesis of Compound 35-d

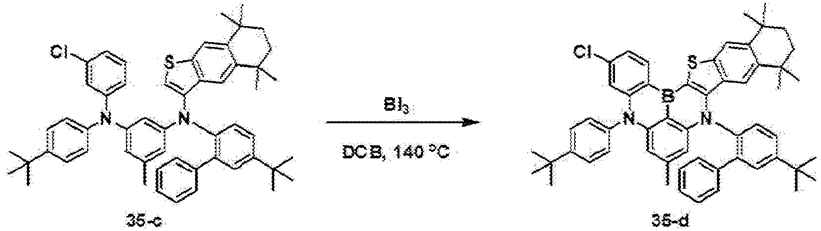

After dissolving Compound 35-c (33.6 mmol, 27.4 g) in 1,2-dichlorobenzene (0.1 M, 335 ml) in a 3-neck flask, boron triiodide (53.8 mmol, 21.0 g) was introduced thereto, and the result was stirred for 8 hours at 140°C under the argon atmosphere. The reaction material was cooled to 0°C, and after adding N,N-diisopropylethylamine (302 mmol, 39.1 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 35-d (5.3 g, yield 19%, MS[M+H]+=824).

### Step 5) Synthesis of Compound 35

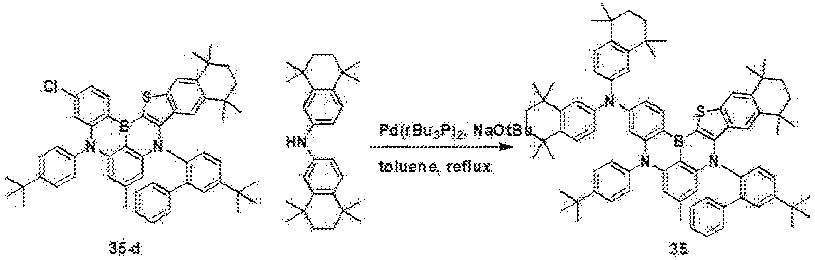

After dissolving Compound 35-d (6.44 mmol, 5.3 g) and bis(5,5,8,8-tetramethyl-5,6,7,8-tetrahydronaphthalen-2-yl)amine (7.72 mmol, 3 g) in toluene (0.2 M, 39 ml) in a 3-neck flask, sodium tert-butoxide (9.66 mmol, 0.928 g) and bis(tri-tert-butylphosphine)palladium(0) (0.064 mmol, 0.033 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography and then went through sublimation purification to obtain Compound 35 (4.1 g, yield 54%, MS[M+H]+=1177).

### Synthesis Example 36: Synthesis of Compound 36

### Step 1) Synthesis of Compound 36-a

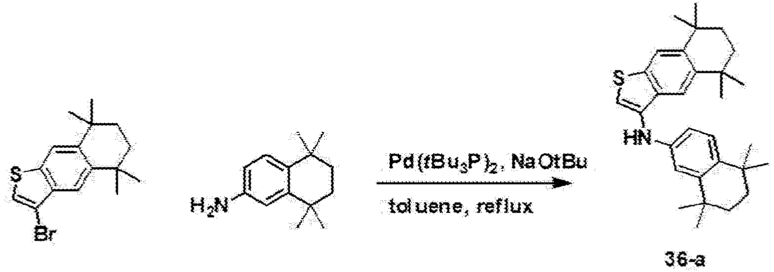

After dissolving 3-bromo-5,5,8,8-tetramethyl-5,6,7,8-tetrahydronaphtho[2,3-b]thiophene (155 mmol, 50 g) and 5,5,8,8-tetramethyl-5,6,7,8-tetrahydronaphthalen-2-amine (155 mmol, 31.4 g) in toluene (0.2 M, 775 ml) in a 3-neck flask, sodium tert-butoxide (232 mmol, 22.3 g) and bis(tri-tert-butylphosphine)palladium(0) (1.55 mmol, 0.79 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 36-a (47.6 g, yield 69%, MS[M+H]+=446).

### Step 2) Synthesis of Compound 36-b

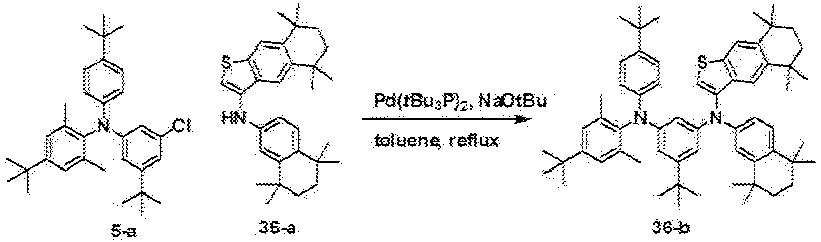

After dissolving Compound 5-a (35.7 mmol, 17 g) and Compound 36-a (35.7 mmol, 15.9 g) in toluene (0.2 M, 180 ml) in a 3-neck flask, sodium tert-butoxide (53.6 mmol, 5.15 g) and bis(tri-tert-butylphosphine)palladium(0) (0.357 mmol, 0.182 g) were introduced thereto, and the result was stirred for 13 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 36-b (22.1 g, yield 70%, MS[M+H]+=886).

### Step 3) Synthesis of Compound 36

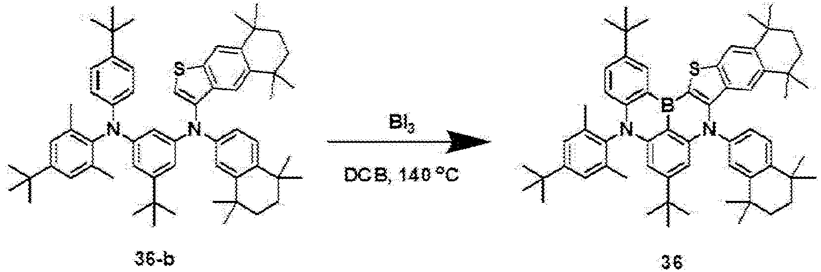

After dissolving Compound 36-b (25.0 mmol, 22.1 g) in 1,2-dichlorobenzene (0.1 M, 250 ml) in a 3-neck flask, boron triiodide (39.9 mmol, 15.6 g) was introduced thereto, and the result was stirred for 8 hours at 140°C under the argon atmosphere. The reaction material was cooled to 0°C, and after adding N,N-diisopropylethylamine (225 mmol, 29 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 36 (2.9 g, yield 13%, MS[M+H]+=893).

### Synthesis Example 37: Synthesis of Compound 37

### Step 1) Synthesis of Compound 37-a

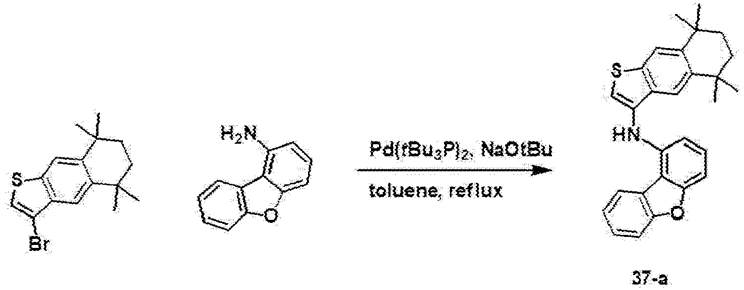

After dissolving 3-bromo-5,5,8,8-tetramethyl-5,6,7,8-tetrahydronaphtho[2,3-b]thiophene (46.4 mmol, 15 g) and dibenzo[*b*,*d*]furan-1-amine (46.4 mmol, 8.5 g) in toluene (0.2 M, 230 ml) in a 3-neck flask, sodium tert-butoxide (69.6 mmol, 6.69 g) and bis(tri-tert-butylphosphine)palladium(0) (0.464 mmol, 0.237 g) were introduced thereto, and the result was stirred for 10 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 37-a (17.4 g, yield 88%, MS[M+H]+=426).

### Step 2) Synthesis of Compound 37-b

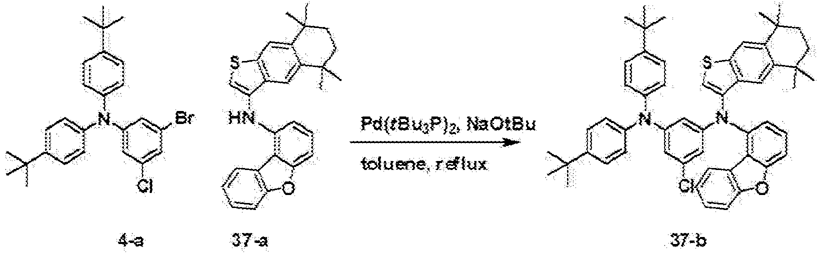

After dissolving Compound 4-a (36.1 mmol, 17 g) and Compound 37-a (36.1 mmol, 15.4 g) in toluene (0.2 M, 180 ml) in a 3-neck flask, sodium tert-butoxide (54.2 mmol, 5.2 g) and bis(tri-tert-butylphosphine)palladium(0) (0.361 mmol, 0.185 g) were introduced thereto, and the result was stirred for 9 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 37-b (24.6 g, yield 84%, MS[M+H]+=816).

### Step 3) Synthesis of Compound 37-c

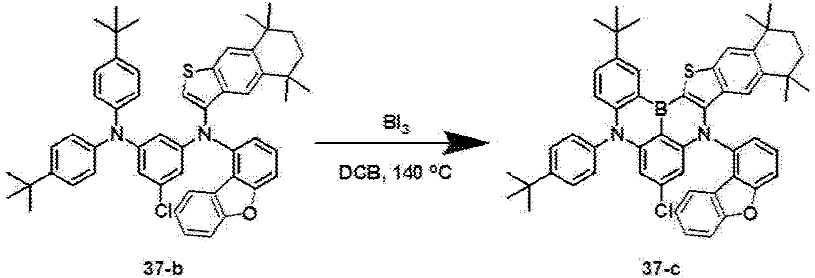

After dissolving Compound 37-b (30.2 mmol, 24.6 g) in 1,2-dichlorobenzene (0.1 M, 300 ml) in a 3-neck flask, boron triiodide (48.3 mmol, 18.9 g) was introduced thereto, and the result was stirred for 6 hours at 140°C under the argon atmosphere. The reaction material was cooled to 0°C, and after adding N,N-diisopropylethylamine (271 mmol, 35 g) thereto, the result was stirred for 1 hour. The result was extracted in a separatory funnel using toluene and H₂O. The extract was dried with MgSO₄ and concentrated, and the sample was purified using silica gel column chromatography to obtain Compound 37-c (5.2 g, yield 210, MS[M+H]+=823).

### Step 4) Synthesis of Compound 37

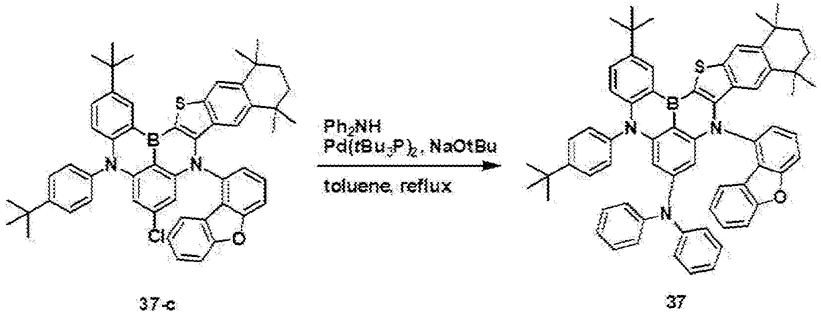

After dissolving Compound 37-c (6.32 mmol, 5.2 g) and diphenylamine (7.58 mmol, 1.3 g) in toluene (0.2 M, 38 ml) in a 3-neck flask, sodium tert-butoxide (9.47 mmol, 0.91 g) and bis(tri-tert-butylphosphine)palladium(0) (0.06 mmol, 0.032 g) were introduced thereto, and the result was stirred for 12 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography and then went through sublimation purification to obtain Compound 37 (3.3 g, yield 55%, MS[M+H]+=956).

### Synthesis Example 38: Synthesis of Compound 38

### Step 1) Synthesis of Compound 38-a

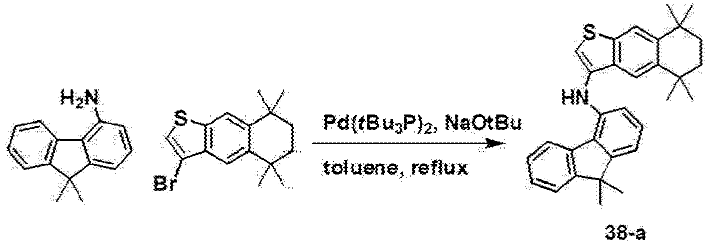

After dissolving 9,9-dimethyl-9H-fluoren-4-amine (92.8 mmol, 30 g) and 3-bromo-5,5,8,8-tetramethyl-5,6,7,8-tetrahydronaphtho[2,3-b]thiophene (92.8 mmol, 19.4 g) in toluene (0.2 M, 465 ml) in a 3-neck flask, sodium tert-butoxide (139 mmol, 13.4 g) and bis(tri-tert-butylphosphine)palladium(0) (0.928 mmol, 0.474 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 38-a (37.4 g, yield 89%, MS[M+H]+=452).

### Step 2) Synthesis of Compound 38-b

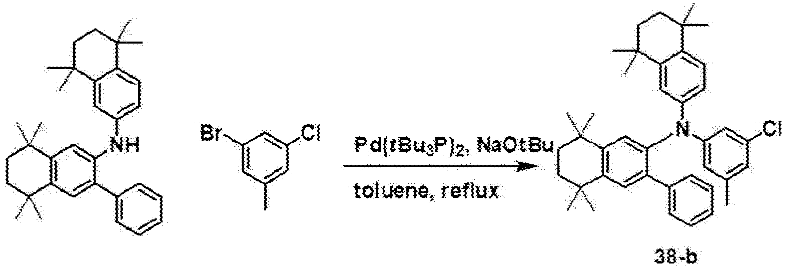

After dissolving 1-bromo-3-chloro-5-methylbenzene (29.2 mmol, 6 g) and 5,5,8,8-tetramethyl-3-phenyl-N-(5,5,8,8-tetramethyl-5,6,7,8-tetrahydronaphthalen-2-yl)-5,6,7,8-tetrahydronaphthalen-2-amine (29.2 mmol, 13.6 g) in toluene (0.2 M, 150 ml) in a 3-neck flask, sodium tert-butoxide (30.5 mmol, 2.93 g) and bis(tri-tert-butylphosphine)palladium(0) (0.292 mmol, 0.15 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 38-b (12.3 g, yield 710, MS[M+H]+=590).

### Step 3) Synthesis of Compound 38-c

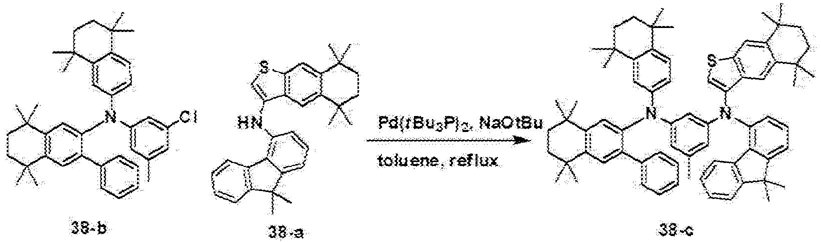

After dissolving Compound 38-a (20.3 mmol, 9.18 g) and Compound 38-b (20.3 mmol, 12 g) in toluene (0.2 M, 100 ml) in a 3-neck flask, sodium tert-butoxide (30.5 mmol, 2.93 g) and bis(tri-tert-butylphosphine)palladium(0) (0.2 mmol, 0.1 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 38-c (14.2 g, yield 69%, MS[M+H]+=1006).

### Step 4) Synthesis of Compound 38

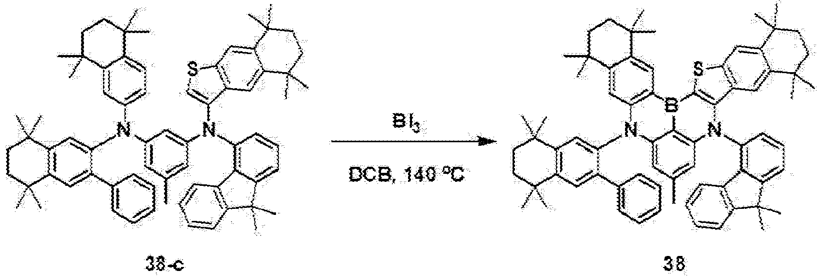

After dissolving Compound 38-c (14.1 mmol, 14.2 g) in 1,2-dichlorobenzene (0.1 M, 140 ml) in a 3-neck flask, boron triiodide (22.6 mmol, 8.85 g) was introduced thereto, and the result was stirred for 10 hours at 140°C under the argon atmosphere. The reaction material was cooled to 0°C, and after adding N,N-diisopropylethylamine (127 mmol, 16.5 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 38 (2.6 g, yield 18%, MS[M+H]+=1013) .

### Synthesis Example 39: Synthesis of Compound 39

### Step 1) Synthesis of Compound 39-a

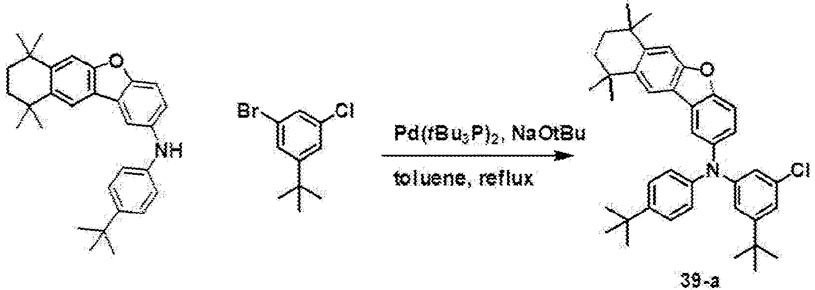

After dissolving N-(4-tert-butylphenyl)-7,7,10,10-tetramethyl-7,8,9,10-tetrahydronaphtho[2,3-b]benzofuran (35.2 mmol, 15 g) and 1-bromo-3-chloro-5-tert-butylbenzene (35.2 mmol, 8.7 g) in toluene (0.2 M, 175 ml) in a 3-neck flask, sodium tert-butoxide (52.9 mmol, 5.08 g) and bis(tri-tert-butylphosphine)palladium(0) (0.352 mmol, 0.18 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 39-a (18.2 g, yield 87%, MS[M+H]+=592).

### Step 2) Synthesis of Compound 39-b

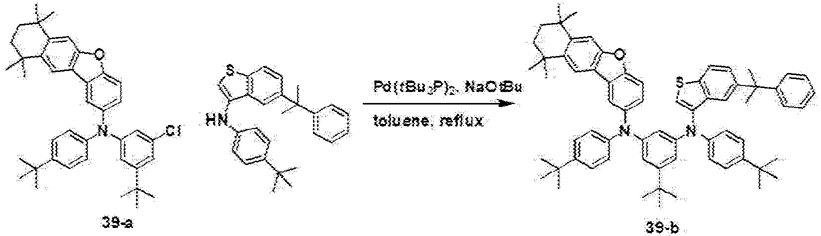

After dissolving Compound 39-a (30.7 mmol, 18.2 g) and N-(4-tert-butylphenyl)-5-(2-phenylpropan-2-yl)benzo[b]thiophen-3-amine (41.6 mmol, 19.6 g) in toluene (0.2 M, 155 ml) in a 3-neck flask, sodium tert-butoxide (46.1 mmol, 4.43 g) and bis(tri-tert-butylphosphine)palladium(0) (0.307 mmol, 0.157 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 39-b (21.5 g, yield 73%, MS[M+H]+=955).

### Step 3) Synthesis of Compound 39

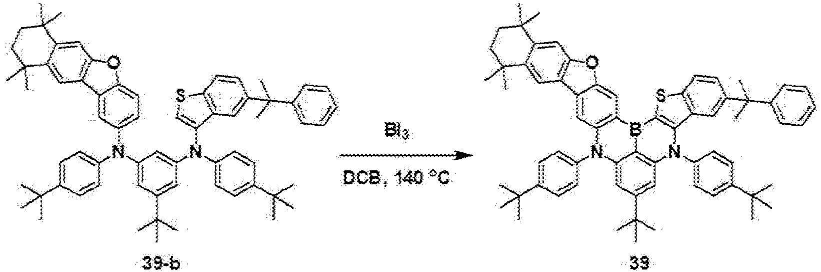

After dissolving Compound 39-b (22.5 mmol, 21.5 g) in 1,2-dichlorobenzene (0.1 M, 225 ml) in a 3-neck flask, boron triiodide (36 mmol, 14.1 g) was introduced thereto, and the result was stirred for 3 hours at 140°C under the argon atmosphere. The reaction material was cooled to 0°C, and after adding N,N-diisopropylethylamine (203 mmol, 26.2 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 39 (3.8 g, yield 18%, MS[M+H]+=963).

### Synthesis Example 40: Synthesis of Compound 40

### Step 1) Synthesis of Compound 40-a

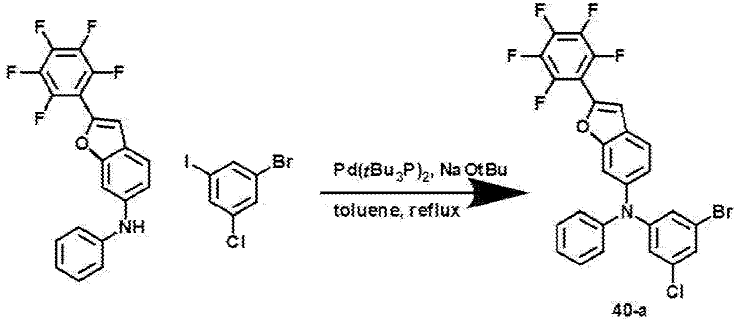

After dissolving 2-(perfluorophenyl)-N-phenylbenzofuran-6-amine (53.3 mmol, 20 g) and 1-bromo-3-chloro-5-iodobenzene (79.9 mmol, 7.68 g) in toluene (0.2 M, 270 ml) in a 3-neck flask, sodium tert-butoxide (80 mmol, 7.68 g) and bis(tri-tert-butylphosphine)palladium(0) (0.532 mmol, 0.272 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 40-a (20.7 g, yield 69%, MS[M+H]+=565).

### Step 2) Synthesis of Compound 40-b

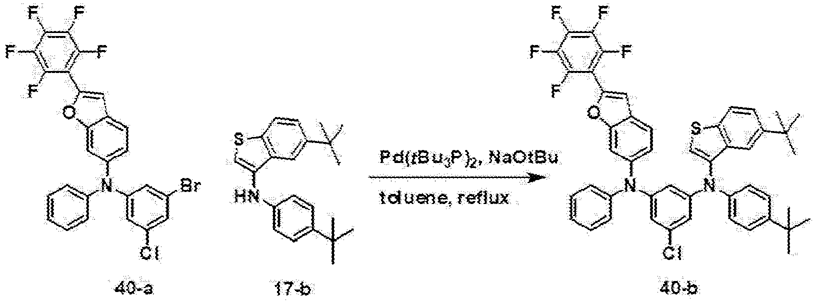

After dissolving Compound 40-a (35.6 mmol, 20.1 g) and Compound 17-b (35.6 mmol, 12 g) in toluene (0.2 M, 180 ml) in a 3-neck flask, sodium tert-butoxide (53.3 mmol, 5.12 g) and bis(tri-tert-butylphosphine)palladium(0) (0.356 mmol, 0.182 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 40-b (26.1 g, yield 89%, MS[M+H]+=821).

### Step 3) Synthesis of Compound 40-c

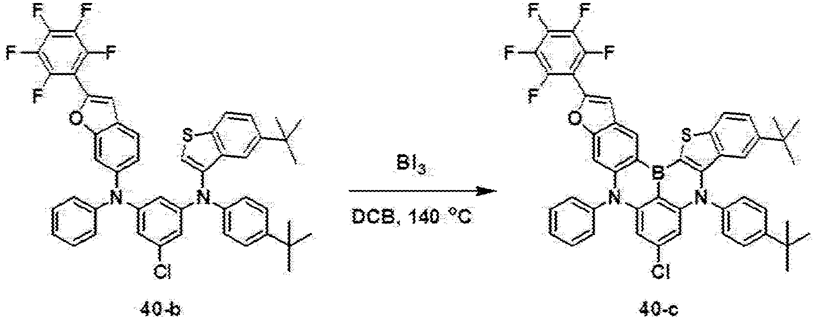

After dissolving Compound 40-b (31.8 mmol, 26.1 g) in 1,2-dichlorobenzene (0.1 M, 320 ml) in a 3-neck flask, boron triiodide (50.8 mmol, 20 g) was introduced thereto, and the result was stirred for 3 hours at 140°C under the argon atmosphere. The reaction material was cooled to 0°C, and after adding N,N-diisopropylethylamine (286 mmol, 37 g) thereto, the result was stirred for 1 hour. The result was extracted in a separatory funnel using toluene and H₂O. The extract was dried with MgSO₄ and concentrated, and the sample was purified using silica gel column chromatography to obtain Compound 40-c (4.6 g, yield 17%, MS[M+H]+=829).

### Step 4) Synthesis of Compound 40

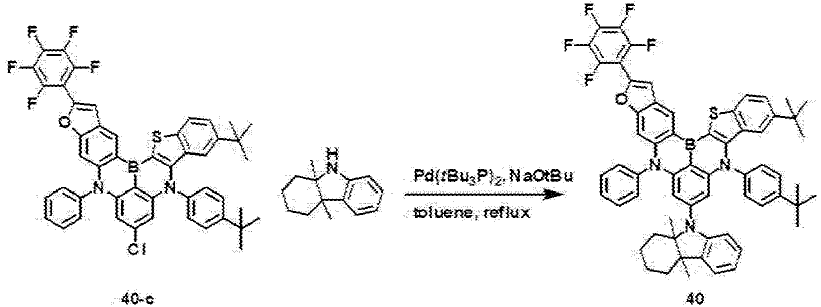

After dissolving Compound 40-c (5.55 mmol, 4.6 g) and 4a,9a-dimethyl-2,3,4,4a,9,9a-hexahydro-1H-carbazole (6.66 mmol, 1.34 g) in toluene (0.2 M, 33 ml) in a 3-neck flask, sodium tert-butoxide (8.32 mmol, 0.8 g) and bis(tri-tert-butylphosphine)palladium(0) (0.055 mmol, 0.028 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography and then went through sublimation purification to obtain Compound 40 (3.8 g, yield 69%, MS[M+H]+=994).

### Synthesis Example 41: Synthesis of Compound 41

### Step 1) Synthesis of Compound 41-a

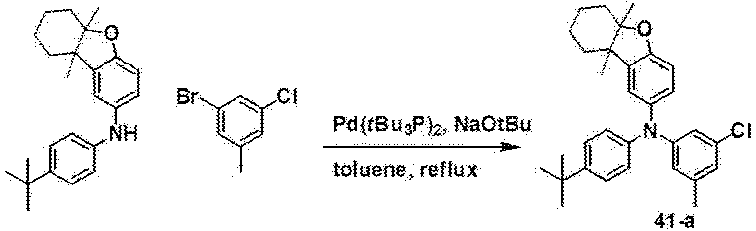

After dissolving N-(4-tert-butylphenyl)-5a,9a-dimethyl-5a,6,7,8,9,9a-hexahydrodibenzo[*b*,*d*]furan-2-amine (51.5 mmol, 18 g) and 1-bromo-3-chloro-5-methylbenzene (51.5 mmol, 10.6 g) in toluene (0.2 M, 260 ml) in a 3-neck flask, sodium tert-butoxide (77.2 mmol, 7.42 g) and bis(tri-tert-butylphosphine)palladium(0) (0.515 mmol, 0.263 g) were introduced thereto, and the result was stirred for 2 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 41-a (20.5 g, yield 84%, MS[M+H]+=474).

### Step 2) Synthesis of Compound 41-b

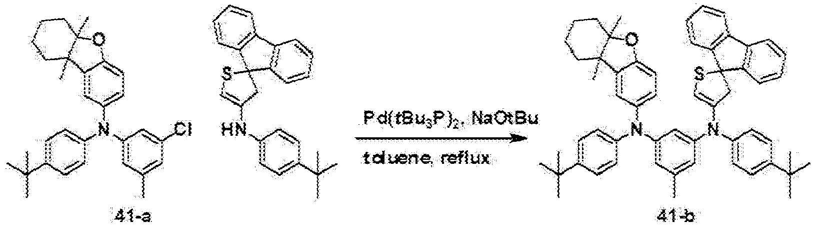

After dissolving Compound 41-a (43.2 mmol, 20.5 g) and N-(4-tert-butylphenyl)-3'*H*-spiro[fluorene-9,2'-thiophen]-4'-amine (43.2 mmol, 16.6 g) in toluene (0.2 M, 220 ml) in a 3-neck flask, sodium tert-butoxide (64.9 mmol, 6.23 g) and bis(tri-tert-butylphosphine)palladium(0) (0.432 mmol, 0.221 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 41-b (22.9 g, yield 64%, MS[M+H]+=821).

### Step 3) Synthesis of Compound 41

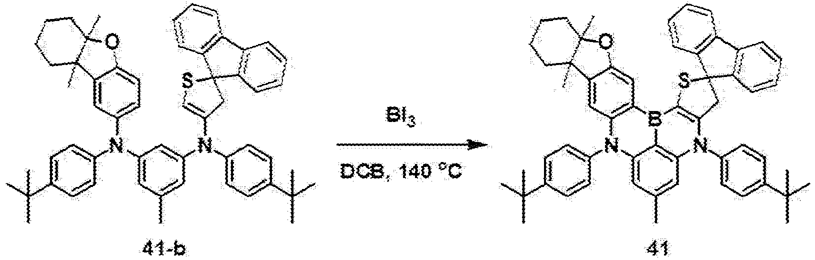

After dissolving Compound 41-b (27.9 mmol, 22.9 g) in 1,2-dichlorobenzene (0.1 M, 280 ml) in a 3-neck flask, boron triiodide (44.6 mmol, 17.5 g) was introduced thereto, and the result was stirred for 3 hours at 140°C under the argon atmosphere. The reaction material was cooled to 0°C, and after adding N,N-diisopropylethylamine (251 mmol, 32.4 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 41 (4.1 g, yield 18%, MS[M+H]+=829).

### Synthesis Example 42: Synthesis of Compound 42

### Step 1) Synthesis of Compound 42-a

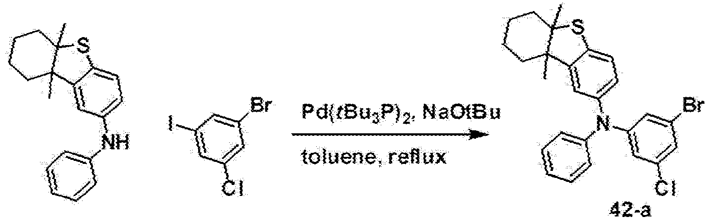

After dissolving 5a,9a-dimethyl-N-phenyl-5a,6,7,8,9,9a-hexahydrobenzo[b,d] thiophen-2-amine (48.5 mmol, 15 g) and 1-bromo-3-chloro-5-iodobenzene (48.5 mmol, 15.4 g) in toluene (0.2 M, 240 ml) in a 3-neck flask, sodium tert-butoxide (72.7 mmol, 7 g) and bis(tri-tert-butylphosphine)palladium(0) (0.485 mmol, 0.248 g) were introduced thereto, and the result was stirred for 3 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 42-a (15.6 g, yield 65%, MS[M+H]+=499).

### Step 2) Synthesis of Compound 42-b

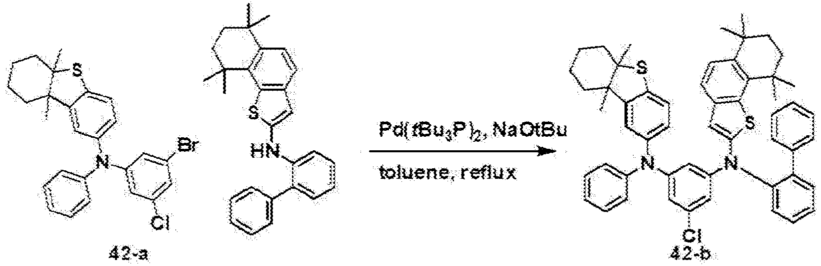

After dissolving Compound 42-a (31.3 mmol, 15.6 g) and N-([1,1'-biphenyl]-2-yl)-6,6,9,9-tetramethyl-6,7,8,9-tetrahydronaphtho[1,2-b]thiophen-2-amine (31.3 mmol, 12.9 g) in toluene (0.2 M, 160 ml) in a 3-neck flask, sodium tert-butoxide (46.9 mmol, 4.51 g) and bis(tri-tert-butylphosphine)palladium(0) (0.313 mmol, 0.16 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 42-b (19.2 g, yield 74%, MS[M+H]+=830).

### Step 3) Synthesis of Compound 42-c

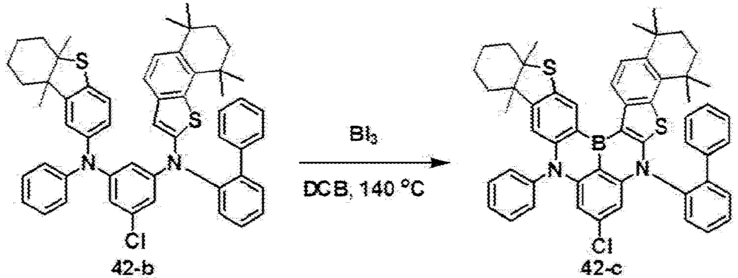

After dissolving Compound 42-b (23.1 mmol, 19.2 g) in 1,2-dichlorobenzene (0.1 M, 230 ml) in a 3-neck flask, boron triiodide (37.0 mmol, 14.5 g) was introduced thereto, and the result was stirred for 3 hours at 140°C under the argon atmosphere. The reaction material was cooled to 0°C, and after adding N,N-diisopropylethylamine (208 mmol, 27 g) thereto, the result was stirred for 1 hour. The result was extracted in a separatory funnel using toluene and H₂O. The extract was dried with MgSO₄ and concentrated, and the sample was purified using silica gel column chromatography to obtain Compound 42-c (2.7 g, yield 14%, MS[M+H]+=837).

### Step 4) Synthesis of Compound 42

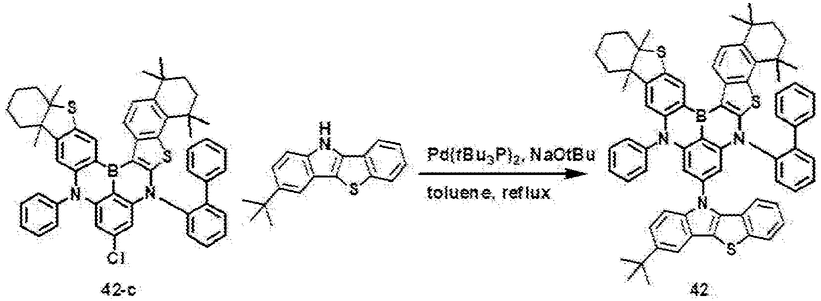

After dissolving Compound 42-c (3.22 mmol, 2.7 g) and 3-tert-butyl-10H-benzo[4,5]thieno[3,2-b]indole (3.87 mmol, 1.08 g) in toluene (0.2 M, 19 ml) in a 3-neck flask, sodium tert-butoxide (4.84 mmol, 0.46 g) and bis(tri-tert-butylphosphine)palladium(0) (0.032 mmol, 0.016 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography and then went through sublimation purification to obtain Compound 42 (2.5 g, yield 72%, MS[M+H]+=1080).

### Synthesis Example 43: Synthesis of Compound 43

### Step 1) Synthesis of Compound 43-a

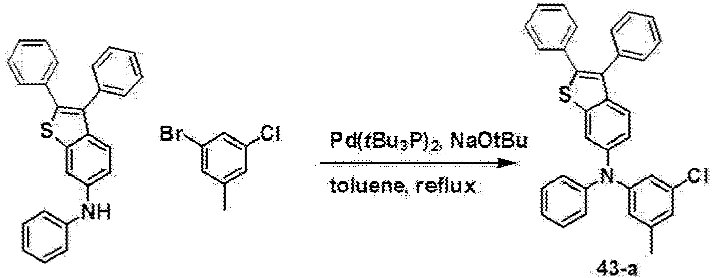

After dissolving N,2,3-triphenylbenzo[b]thiophen-6-amine (53.0 mmol, 20 g) and 1-bromo-3-chloro-5-methylbenzene (53.0 mmol, 10.9 g) in toluene (0.2 M, 265 ml) in a 3-neck flask, sodium tert-butoxide (79.5 mmol, 7.64 g) and bis(tri-tert-butylphosphine)palladium(0) (0.53 mmol, 0.271 g) were introduced thereto, and the result was stirred for 6 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 43-a (19.7 g, yield 74%, MS[M+H]+=502).

### Step 2) Synthesis of Compound 43-b

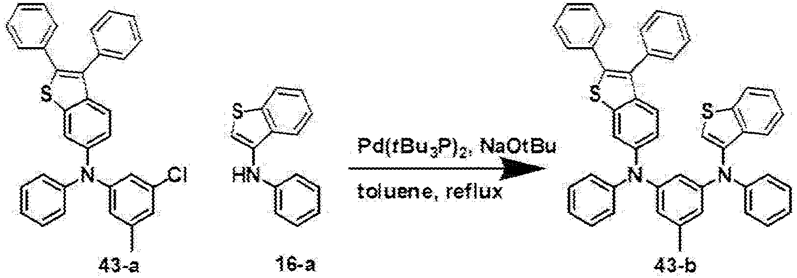

After dissolving Compound 43-a (39.2 mmol, 19.7 g) and Compound 16-a (39.2 mmol, 8.84 g) in toluene (0.2 M, 200 ml) in a 3-neck flask, sodium tert-butoxide (58.9 mmol, 5.66 g) and bis(tri-tert-butylphosphine)palladium(0) (0.392 mmol, 0.2 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 43-b (23.4 g, yield 86%, MS[M+H]+=691).

### Step 3) Synthesis of Compound 43

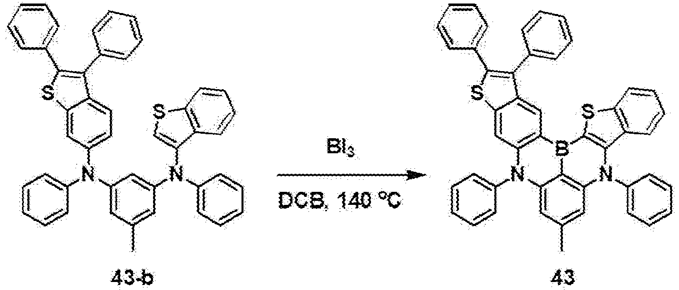

After dissolving Compound 43-b (33.9 mmol, 23.4 g) in 1,2-dichlorobenzene (0.1 M, 340 ml) in a 3-neck flask, boron triiodide (54.2 mmol, 21.2 g) was introduced thereto, and the result was stirred for 3 hours at 140°C under the argon atmosphere. The reaction material was cooled to 0°C, and after adding N,N-diisopropylethylamine (305 mmol, 39.4 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 43 (5.2 g, yield 22%, MS[M+H]+=699).

### Synthesis Example 44: Synthesis of Compound 44

### Step 1) Synthesis of Compound 44-a

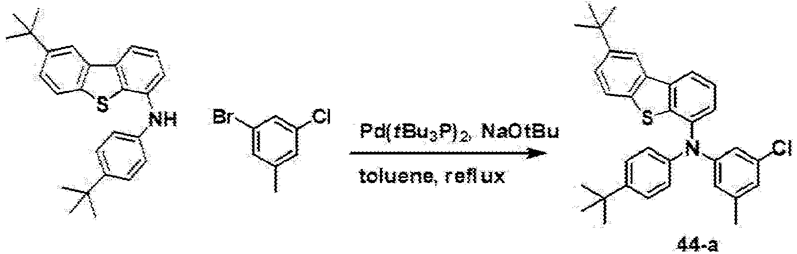

After dissolving 8-tert-butyl-N-(4-tert-butylphenyl)dibenzo[*b*,*d*]thiophen-4-amine (38.7 mmol, 15 g) and 1-bromo-3-chloro-5-methylbenzene (38.7 mmol, 8 g) in toluene (0.2 M, 195 ml) in a 3-neck flask, sodium tert-butoxide (58.1 mmol, 5.58 g) and bis(tri-tert-butylphosphine)palladium(0) (0.387 mmol, 0.198 g) were introduced thereto, and the result was stirred for 2 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 44-a (18.8 g, yield 95%, MS[M+H]+=512).

### Step 2) Synthesis of Compound 44-b

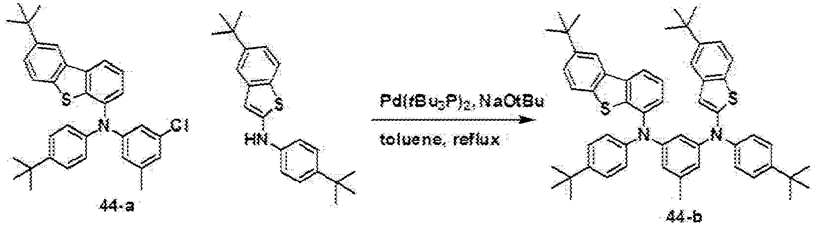

After dissolving Compound 44-a (36.7 mmol, 18.8 g) and 5-tert-butyl-*N*-(4-tert-butylphenyl)benzo[*b*]thiophen-2-amine (36.7 mmol, 12.4 g) in toluene (0.2 M, 180 ml) in a 3-neck flask, sodium tert-butoxide (55.1 mmol, 5.29 g) and bis(tri-tert-butylphosphine)palladium(0) (0.367 mmol, 0.187 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 44-b (27.4 g, yield 92%, MS[M+H]+=813).

### Step 3) Synthesis of Compound 44

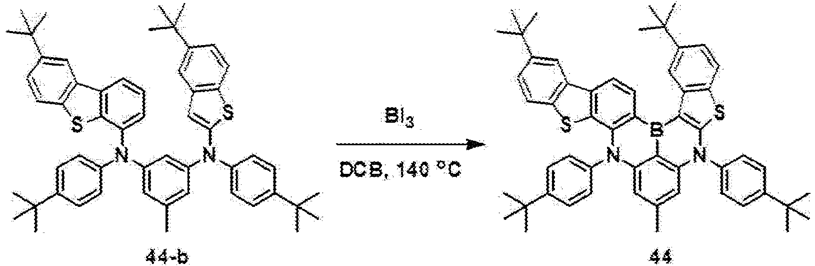

After dissolving Compound 44-b (33.7 mmol, 27.4 g) in 1,2-dichlorobenzene (0.1 M, 340 ml) in a 3-neck flask, boron triiodide (53.9 mmol, 21.1 g) was introduced thereto, and the result was stirred for 3 hours at 140°C under the argon atmosphere. The reaction material was cooled to 0°C, and after adding *N*,*N*-diisopropylethylamine (303 mmol, 39.2 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 44 (4.6 g, yield 17%, MS[M+H]+=821).

### Synthesis Example 45: Synthesis of Compound 45

### Step 1) Synthesis of Compound 45-a

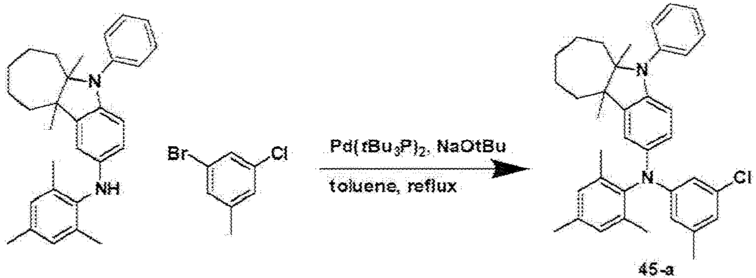

After dissolving N-mesityl-5a,10a-dimethyl-5-phenyl-5,5a,6,7,8,9,10,10a-octahydrocyclopenta[b]indol-2-amine (47.1 mmol, 20 g) and 1-bromo-3-chloro-5-methylbenzene (47.1 mmol, 9.68 g) in toluene (0.2 M, 235 ml) in a 3-neck flask, sodium tert-butoxide (70.6 mmol, 6.79 g) and bis(tri-tert-butylphosphine)palladium(0) (0.471 mmol, 0.241 g) were introduced thereto, and the result was stirred for 8 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 45-a (21.5 g, yield 83%, MS[M+H]+=549).

### Step 2) Synthesis of Compound 45-b

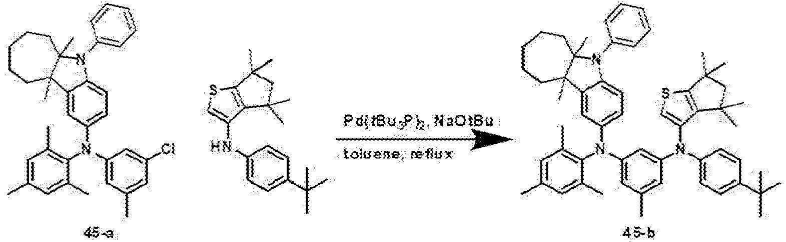

After dissolving Compound 45-a (39.1 mmol, 21.5 g) and N-(4-tert-butylphenyl)-4,4,6,6-tetramethyl-5,6-dihydro-4H-cyclopenta[b]thiophen-3-amine (39.1 mmol, 12.8 g) in toluene (0.2 M, 195 ml) in a 3-neck flask, sodium tert-butoxide (58.7 mmol, 5.64 g) and bis(tri-tert-butylphosphine)palladium(0) (0.391 mmol, 0.2 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 45-b (21.9 g, yield 67%, MS[M+H]+=840).

### Step 3) Synthesis of Compound 45

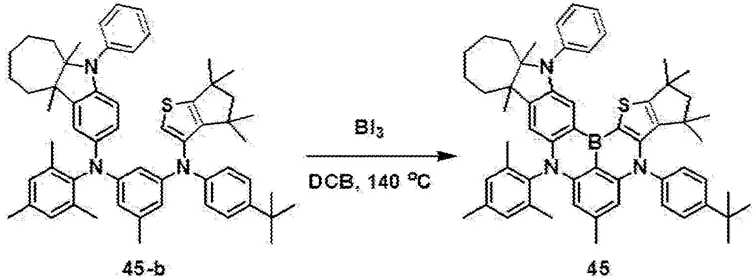

After dissolving Compound 45-b (26.1 mmol, 21.9 g) in 1,2-dichlorobenzene (0.1 M, 260 ml) in a 3-neck flask, boron triiodide (41.7 mmol, 16.3 g) was introduced thereto, and the result was stirred for 3 hours at 140°C under the argon atmosphere. The reaction material was cooled to 0°C, and after adding *N*,*N*-diisopropylethylamine (235 mmol, 30.3 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 45 (3.5 g, yield 16%, MS[M+H]+=848) .

### Synthesis Example 46: Synthesis of Compound 46

### Step 1) Synthesis of Compound 46-a

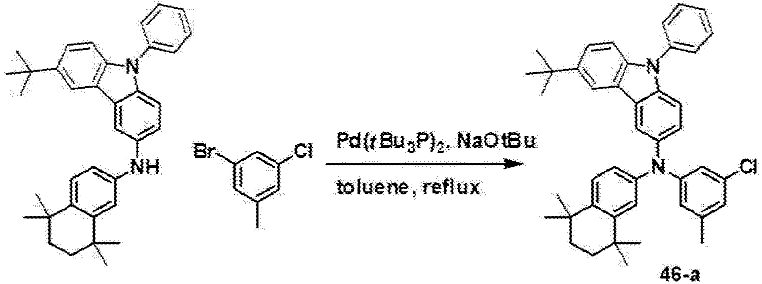

After dissolving 6-tert-butyl-9-phenyl-*N*-(5,5,8,8-tetramethyl-5,6,7,8-tetrahydronaphthalen-2-yl-9H-carbazol-3-amine (39.9 mmol, 20 g) and 1-bromo-3-chloro-5-methylbenzene (39.9 mmol, 8.21 g) in toluene (0.2 M, 200 ml) in a 3-neck flask, sodium tert-butoxide (60 mmol, 5.76 g) and bis(tri-tert-butylphosphine)palladium(0) (0.4 mmol, 0.204 g) were introduced thereto, and the result was stirred for 5 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 46-a (20.9 g, yield 84%, MS[M+H]+=625).

### Step 2) Synthesis of Compound 46-b

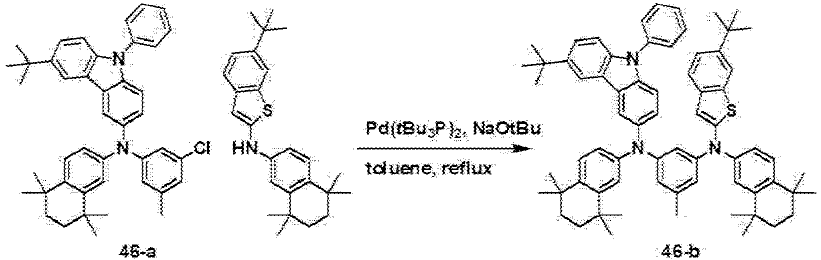

After dissolving Compound 46-a (33.4 mmol, 20.9 g) and 6-tert-butyl-N-(5,5,8,8-tetramethyl-5,6,7,8-tetrahydronaphthalen-2-yl)benzo[b]thiophen-2-amine (33.4 mmol, 13.1 g) in toluene (0.2 M, 170 ml) in a 3-neck flask, sodium tert-butoxide (50.1 mmol, 4.82 g) and bis(tri-tert-butylphosphine)palladium(0) (0.334 mmol, 0.171 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 46-b (25.2 g, yield 77%, MS[M+H]+=980).

### Step 3) Synthesis of Compound 46

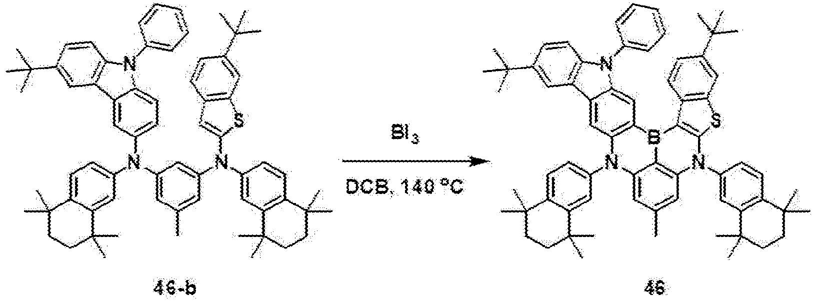

After dissolving Compound 46-b (25.7 mmol, 25.2 g) in 1,2-dichlorobenzene (0.1 M, 260 ml) in a 3-neck flask, boron triiodide (41.1 mmol, 16.1 g) was introduced thereto, and the result was stirred for 3 hours at 140°C under the argon atmosphere. The reaction material was cooled to 0°C, and after adding N,N-diisopropylethylamine (231 mmol, 30 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 46 (4.1 g, yield 16%, MS[M+H]+=988).

### Synthesis Example 47: Synthesis of Compound 47

### Step 1) Synthesis of Compound 47-a

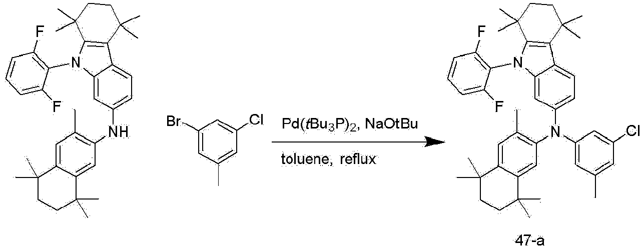

After dissolving 9-(2,6,-difluorophenyl)-1,1,4,4-tetramethyl-N-(3,5,5,8,8-pentamethyl-5,6,7,8-tetrahydronaphthalen-2-yl)-2,3,4,9-tetrahydro-1H-carbazol-7-amine (36.1 mmol, 20 g) and 1-bromo-3-chloro-5-methylbenzene (36.1 mmol, 7.41 g) in toluene (0.2 M, 180 ml) in a 3-neck flask, sodium tert-butoxide (54.1 mmol, 5.20 g) and bis(tri-tert-butylphosphine)palladium(0) (0.36 mmol, 0.184 g) were introduced thereto, and the result was stirred for 8 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 47-a (21.7 g, yield 89%, MS[M+H]+=679).

### Step 2) Synthesis of Compound 47-b

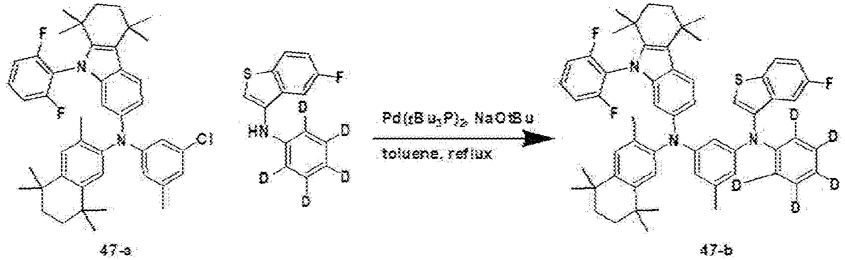

After dissolving Compound 47-a (31.9 mmol, 21.7 g) and 5-fluoro-N-(phenyl-d5)benzo[b]thiophen-3-amine (31.9 mmol, 7.93 g) in toluene (0.2 M, 160 ml) in a 3-neck flask, sodium tert-butoxide (47.9 mmol, 4.6 g) and bis(tri-tert-butylphosphine)palladium(0) (0.319 mmol, 0.163 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 47-b (20.1 g, yield 710, MS[M+H]+=891).

### Step 3) Synthesis of Compound 47

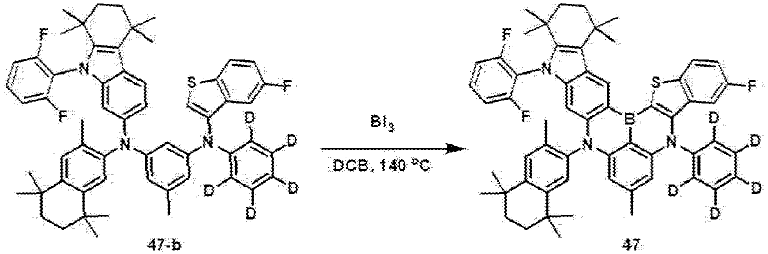

After dissolving Compound 47-b (22.6 mmol, 20.1 g) in 1,2-dichlorobenzene (0.1 M, 225 ml) in a 3-neck flask, boron triiodide (36.1 mmol, 14.1 g) was introduced thereto, and the result was stirred for 3 hours at 140°C under the argon atmosphere. The reaction material was cooled to 0°C, and after adding *N*,*N*-diisopropylethylamine (203 mmol, 36.2 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 47 (2.5 g, yield 12%, MS[M+H]+=899).

### Synthesis Example 48: Synthesis of Compound 48

### Step 1) Synthesis of Compound 48-a

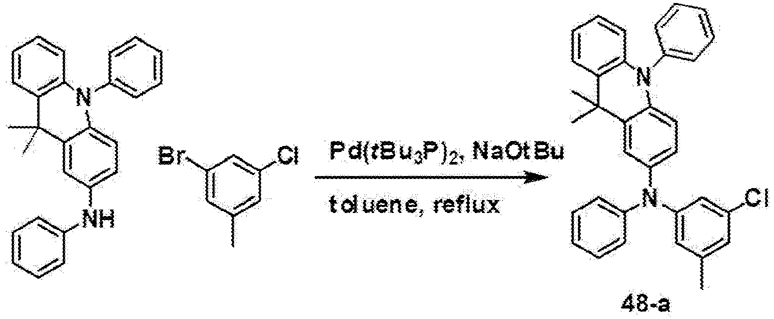

After dissolving 9-(2,6,-difluorophenyl)-1,1,4,4-tetramethyl-N-(3,5,5,8,8-pentamethyl-5,6,7,8-tetrahydronaphthalen-2-yl)-2,3,4,9-tetrahydro-1H-carbazol-7-amine (53.1 mmol, 20 g) and 1-bromo-3-chloro-5-methylbenzene (53.1 mmol, 10.9 g) in toluene (0.2 M, 265 ml) in a 3-neck flask, sodium tert-butoxide (79.7 mmol, 10.9 g) and bis(tri-tert-butylphosphine)palladium(0) (0.531 mmol, 0.271 g) were introduced thereto, and the result was stirred for 2 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 48-a (22.8 g, yield 86%, MS[M+H]+=501).

### Step 2) Synthesis of Compound 48-b

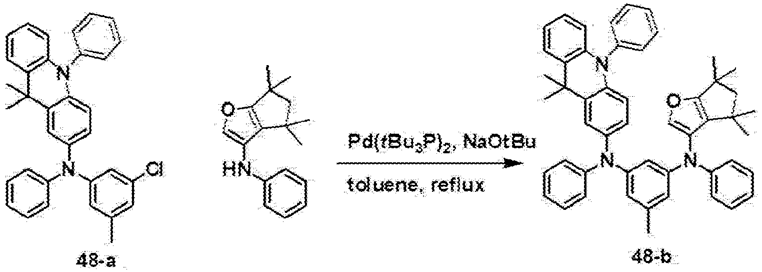

After dissolving Compound 48-a (45.5 mmol, 22.8 g) and 4,4,6,6-tetramethyl-N-phenyl-5,6-dihydro-4H-cyclopenta[b]furan-3-amine (45.5 mmol, 11.6 g) in toluene (0.2 M, 230 ml) in a 3-neck flask, sodium tert-butoxide (68.3 mmol, 6.56 g) and bis(tri-tert-butylphosphine)palladium(0) (0.455 mmol, 0.233 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 48-b (23.1 g, yield 710, MS[M+H]+=720).

### Step 3) Synthesis of Compound 48

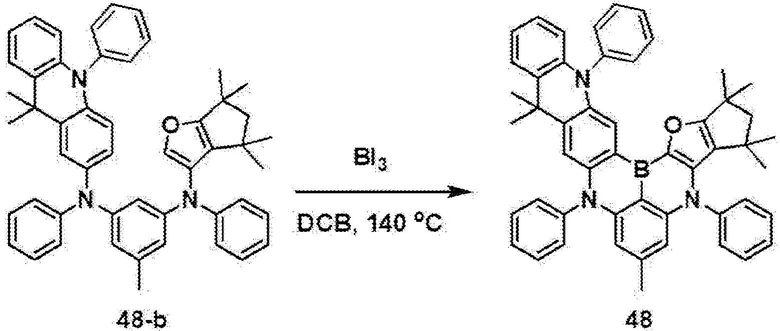

After dissolving Compound 48-b (32.1 mmol, 23.1 g) in 1,2-dichlorobenzene (0.1 M, 320 ml) in a 3-neck flask, boron triiodide (51.3 mmol, 20.1 g) was introduced thereto, and the result was stirred for 3 hours at 140°C under the argon atmosphere. The reaction material was cooled to 0°C, and after adding *N*,*N*-diisopropylethylamine (289 mmol, 37.3 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 48 (3.6 g, yield 15%, MS[M+H]+=728) .

### Synthesis Example 49: Synthesis of Compound 49

### Step 1) Synthesis of Compound 49-a

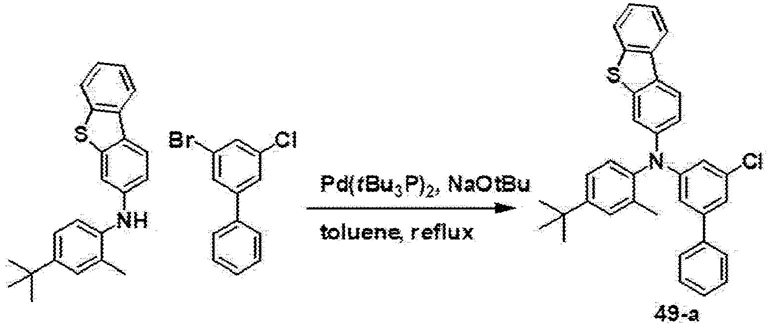

After dissolving N-(4-tert-butyl-2-methylphenyl)dibenzo[b,d]thiophen-3-amine (43.4 mmol, 15 g) and 3-bromo-5-chloro-1,1'-biphenyl (43.4 mmol, 11.6 g) in toluene (0.2 M, 220 ml) in a 3-neck flask, sodium tert-butoxide (65.1 mmol, 6.26 g) and bis(tri-tert-butylphosphine)palladium(0) (0.434 mmol, 0.222 g) were introduced thereto, and the result was stirred for 2 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 49-a (20.1 g, yield 87%, MS[M+H]+=532).

### Step 2) Synthesis of Compound 49-b

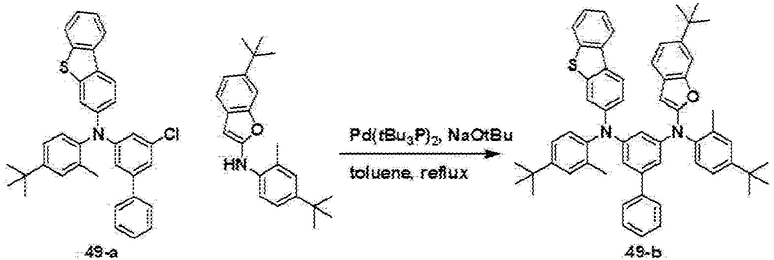

After dissolving Compound 49-a (37.8 mmol, 20.1 g) and 6-tert-butyl-*N*-(4-tert-butyl-2-methylphenyl)benzofuran-2-amine (37.8 mmol, 12.7 g) in toluene (0.2 M, 190 ml) in a 3-neck flask, sodium tert-butoxide (56.7 mmol, 5.4 g) and bis(tri-tert-butylphosphine)palladium(0) (0.378 mmol, 0.193 g) were introduced thereto, and the result was stirred for 24 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 49-b (21.7 g, yield 69%, MS[M+H]+=831).

### Step 3) Synthesis of Compound 49

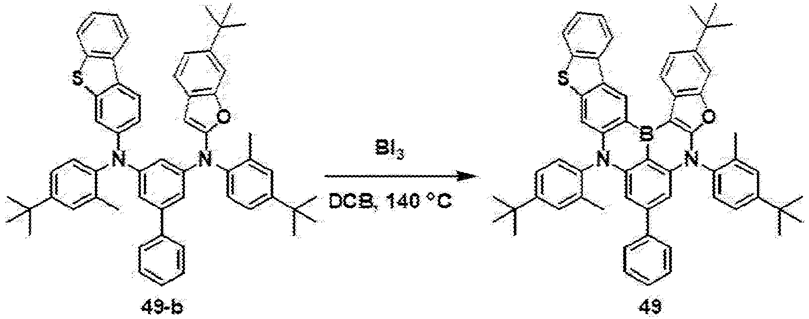

After dissolving Compound 49-b (26.1 mmol, 21.7 g) in 1,2-dichlorobenzene (0.1 M, 260 ml) in a 3-neck flask, boron triiodide (41.8 mmol, 16.4 g) was introduced thereto, and the result was stirred for 3 hours at 140°C under the argon atmosphere. The reaction material was cooled to 0°C, and after adding N,N-diisopropylethylamine (235 mmol, 30.4 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 49 (3.3 g, yield 15%, MS[M+H]+=839).

### Synthesis Example 50: Synthesis of Compound 50

### Step 1) Synthesis of Compound 50-a

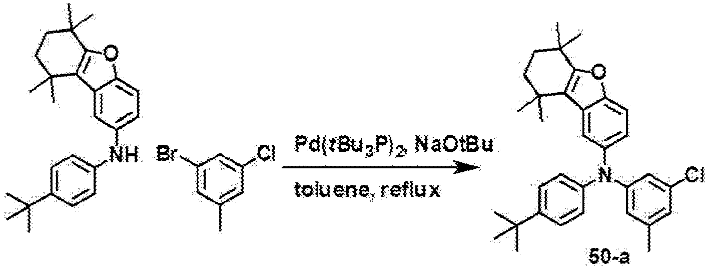

After dissolving N-(4-tert-butylphenyl)-6,6,9,9-tetramethyl-6,7,8,9-tetrahydrodibenzo[*b,d*]furan-2-amine (39.9 mmol, 15 g) and 1-bromo-3-chloro-5-methylbenzene (39.9 mmol, 8.21 g) in toluene (0.2 M, 200 ml) in a 3-neck flask, sodium tert-butoxide (59.9 mmol, 5.76 g) and bis(tri-tert-butylphosphine)palladium(0) (0.399 mmol, 0.204 g) were introduced thereto, and the result was stirred for 4 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 50-a (18.5 g, yield 93%, MS[M+H]+=500).

### Step 2) Synthesis of Compound 50-b

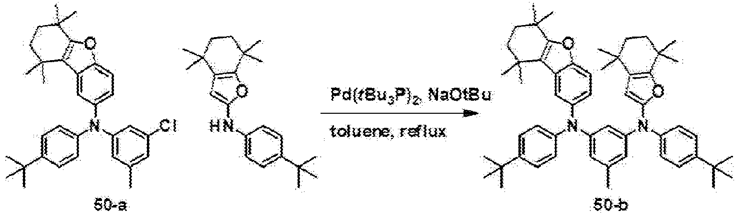

After dissolving Compound 50-a (37.0 mmol, 18.5 g) and *N-*(4-tert-butylphenyl)-4,4,7,7-tetramethyl-4,5,6,7-tetrahydrobenzofuran-2-amine (37.0 mmol, 12.0 g) in toluene (0.2 M, 185 ml) in a 3-neck flask, sodium tert-butoxide (55.5 mmol, 5.33 g) and bis(tri-tert-butylphosphine)palladium(0) (0.370 mmol, 0.189 g) were introduced thereto, and the result was stirred for 15 hours under reflux under the argon atmosphere. When the reaction was finished, the result was cooled to room temperature, then H₂O was introduced thereto, and the reaction solution was transferred to a separatory funnel and extracted. The extract was dried with MgSO₄ and concentrated, and the sample was purified using silica gel column chromatography to obtain Compound 50-b (22.8 g, yield 78%, MS[M+H]+=789).

### Step 3) Synthesis of Compound 50

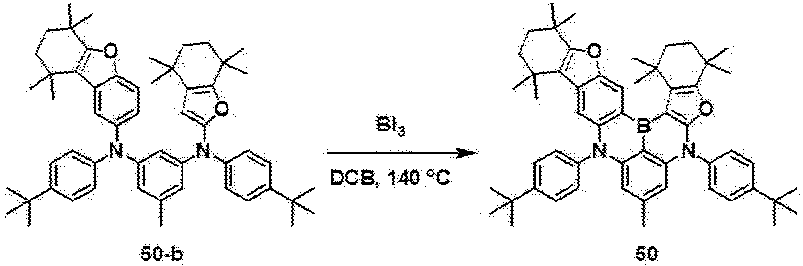

After dissolving Compound 50-b (28.9 mmol, 22.8 g) in 1,2-dichlorobenzene (0.1 M, 290 ml) in a 3-neck flask, boron triiodide (46.2 mmol, 18.1 g) was introduced thereto, and the result was stirred for 3 hours at 140°C under the argon atmosphere. The reaction material was cooled to 0°C, and after adding *N*,*N*-diisopropylethylamine (260 mmol, 33.6 g) thereto, the result was stirred for 1 hour. The result was extracted in a separatory funnel using toluene and H₂O. The extract was dried with MgSO₄ and concentrated, and the sample was purified using silica gel column chromatography and then went through sublimation purification to obtain Compound 50 (4.1 g, yield 18%, MS[M+H]+=797).

### Experimental Example 1: Experiment of Photoluminescence Analysis

When a boron compound is substituted with an aromatic 5-membered ring including oxygen or sulfur in a symmetrical structure, a boron compound having a flatter structure compared to a boron compound substituted with three 6-membered aromatic rings may be prepared according to Korean Patent No. 10-2030309, and it was seen that a device with high color purity, high efficiency and long lifetime was able to be obtained using the same.

It was identified that, by including a layer containing the compound of Chemical Formula 1 according to one embodiment of the present specification, that is, an asymmetric boron compound including one aromatic 5-membered ring in an organic material layer of an organic light emitting device, an organic light emitting device having superior effects compared to Korean Patent No. 10-2030309 was obtained.

Through the following experiment of photoluminescence analysis, fluorescence spectra of a symmetric compound in the art and the compound of Chemical Formula 1 according to one embodiment of the present specification, that is, an asymmetric compound, were identified. In the experiment of photoluminescence analysis, BD-A in which boron is substituted with three benzene rings, BD-C that is a symmetric compound in which boron is substituted with two benzothiophene and one benzene ring, and Compound 16 synthesized from Synthesis Example 16, which is Chemical Formula 1 according to one embodiment of the present specification, were used.

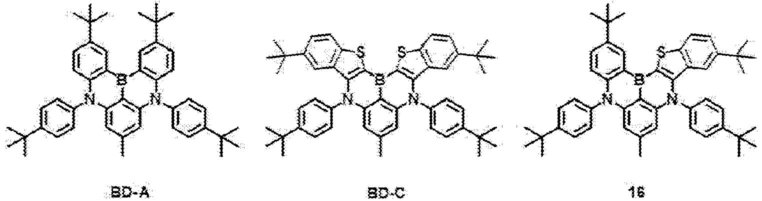

A JASCO FP-8600 fluorescence spectrophotometer was used in the experiment of photoluminescence analysis, and a maximum emission wavelength was measured using the following method.

Using toluene as a solvent, BD-A, BD-C and Compound 16, which are compounds to measure, were each dissolved in a concentration of 1×10⁻⁵ M to prepare a sample for measurement. The sample solution was introduced to a quartz cell, and degassed using nitrogen gas (N₂) to remove oxygen in the solution, and using the measurement device, a fluorescence spectrum was measured at room temperature (300 K). Herein, a wavelength value (nm) of the maximum emission peak was obtained, and a spread width of the graph at half the height from the maximum emission peak (FWHM, full width at half maximum, nm) was obtained. The results are shown in the following Table 1, and the measurement graphs are shown in FIG. 4.

**[Table 1]**

| Compound | Maximum Emission Wavelength (nm) | Full Width at Half Maximum (nm) | CIEy |
|---|---|---|---|
| BD-A | 456.1 | 23.4 | 0.0859 |
| BD-C | 463.0 | 20.9 | 0.1777 |
| Compound 16 | 456.1 | 21.9 | 0.1043 |

From Table 1 and FIG. 4, it was identified that, in each of the compounds of BD-A, BD-C and Compound 16, the second peak formed in a long wavelength region became stronger compared to the main peak as the heterocyclic group increased by one. As the second peak became stronger, there is a disadvantage in that color purity becomes poor in an organic light emitting device, and asymmetric Compound 16 of Chemical Formula 1 of the present specification had a weaker second peak compared to Compound BD-C. Accordingly, it was seen that, due to the light emission properties, a device with high color purity and high efficiency was formed with the compound of Chemical Formula 1 according to one embodiment of the present specification, which is an asymmetric compound, compared to with a symmetric compound.

### Experimental Example 2: Experiment of Quantum Chemistry Calculation

In order to determine a cause of the results of the photoluminescence analysis experiment of Experimental Example 1, quantum calculations were conducted on a symmetric thiophene-boron compound (following DABNA and BD-F) and the compound of Chemical Formula 1 according to one embodiment of the present specification, that is, an asymmetric thiophene-boron Compound (following BD-E). The quantum calculation was conducted based on a density functional theory (DFT) calculation and a time-dependent density functional theory calculation of the Schrodinger Material Science Suite program, and herein, B3LYP was used as the function, and 6-31G* was used as the basis function. A Franck-Condon analysis of the emission wavelength was conducted using the Schrodinger Material Science Suite program with reference to prior literatures, and the results are shown in the following Tables 2 to 4 (for example, refer to literature ^{┌}Santoro, F., Lami, A., Improta, R., Bloino, J., Barone, V. J. Chem. Phys. 128, 224311 (2008). _{┘} and literature ^{┌}Kondo, Y., Yoshiura, K., Kitera, S. et al. Nat. Photonics 13, 678 (2019)._{┘} ).

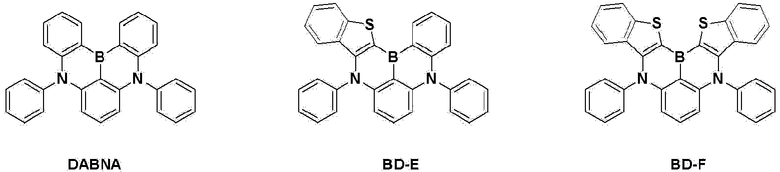

**[Table 2]**

| DABNA | Transition (S1→S0) | Relative frequency (cm⁻¹) | Intensity (a.u.) |
|---|---|---|---|
| Main peak | 0→0 | 0.00 | 1.945 |
| | 0→7¹ | 78.79 | 2.186 |
| | 0→10¹ | 128.18 | 0.320 |
| | 0→7² | 157.58 | 0.880 |
| | 0→7¹10¹ | 206.97 | 0.360 |
| | 0→13¹ | 207.98 | 0.522 |
| | 0→7¹13¹ | 286.77 | 0.587 |
| 2nd peak | 0→110¹ | 1353.27 | 0.071 |
| | 0→7¹110¹ | 1432.06 | 0.079 |
| | 0→121¹ | 1507.61 | 0.123 |
| | 0→7¹121¹ | 1586.40 | 0.138 |
| | 0→129¹ | 1638.95 | 0.159 |
| | 0→7¹129¹ | 1717.74 | 0.178 |
| | 0→7²129¹ | 1796.53 | 0.072 |

**[Table 3]**

| BD-E | Transition (S1→S0) | Relative frequency (cm⁻¹) | Intensity (a.u.) |
|---|---|---|---|
| Main peak | 0→0 | 0.00 | 5.872 |
| | 0→5¹ | 57.94 | 0.398 |
| | 0→11¹ | 127.17 | 0.942 |
| | 0→13¹ | 188.07 | 0.603 |
| | 0→18¹ | 247.80 | 0.384 |
| | 0→27¹ | 523.89 | 0.171 |
| | 0→55¹ | 746.19 | 0.522 |
| 2nd peak | 0→99¹ | 1183.40 | 0.169 |
| | 0→124¹ | 1449.13 | 0.447 |
| | 0→130¹ | 1502.27 | 0.418 |
| | 0→138¹ | 1638.06 | 0.246 |
| | 0→141¹ | 1648.25 | 0.185 |

**[Table 4]**

| BD-F | Transition (S1→S0) | Relative frequency (cm⁻¹) | Intensity (a.u.) |
|---|---|---|---|
| Main peak | 0→0 | 0.00 | 6.710 |
| | 0→5¹ | 58.68 | 0.221 |
| | 0→10¹ | 93.01 | 0.868 |
| | 0→21¹ | 262.00 | 0.477 |
| | 0→46¹ | 603.10 | 0.267 |
| | 0→63¹ | 754.06 | 0.259 |
| 2nd peak | 0→130¹ | 1412.99 | 0.195 |
| | 0→132¹ | 1453.63 | 0.507 |
| | 0→135¹ | 1491.20 | 0.642 |
| | 0→139¹ | 1501.46 | 0.193 |
| | 0→151¹ | 1648.52 | 0.278 |

From Tables 2 to 4, it was identified that the compounds of BD-E and BD-F including a heterocyclic group had a second peak with higher intensity formed in a longer wavelength region compared to DABNA including an aromatic hydrocarbon ring (benzene). However, it was expected that the second peak of asymmetric BD-E, the compound according to one embodiment of the present specification, appeared to have lower intensity compared to the second peak of BD-F, and this result indicates the cause of the difference in color purity between Compound 16 and BD-C in Experimental Example 1. From the results of Experimental Examples 1 and 2, it was identified that a device with more superior light emission properties was obtained when using Chemical Formula 1 according to one embodiment of the present specification, which is an asymmetric boron compound, in an organic material layer of an organic light emitting device compared to when using a symmetric boron compound.

### Experimental Example 3: Experiment of Thermos Gravimetric Analysis

A thermos gravimetric analyzer (TGA) is a device measuring, after applying a temperature to a sample, changes in the mass of the sample as a function of time or temperature. A mass loss of a material is caused by evaporation or a chemical reaction producing gaseous products. Using Q-500, 3 mg or more and less than 5 mg of compounds of the following Table 5 completed with sublimation purification were each put on a Pt pan, and heated from room temperature to 700°C at a rate of 10°C/min. Herein, a temperature at which the mass of the compound was reduced by 5% with respect to the total weight (=Td-5% loss) and the amount (percent) of the residue remaining on the pan after heating to 700°C were measured. The thermos gravimetric analysis graph of Compound 19 prepared in Synthesis Example 19 is shown in FIG. 5, and the thermos gravimetric analysis graphs of BD-A and BD-C are respectively shown in FIG. 6 and FIG. 7.

**[Table 5]**

| Compound | Molecular Weight | Td (5% loss) | Residue (%) |
|---|---|---|---|
| BD-A | 658.78 | 368 | 0.3 |
| BD-C | 770.95 | 459 | 30.1 |
| Compound 19 | 845.05 | 375 | 4.2 |

From Table 5 and FIG. 5 to FIG. 7, it was identified that Chemical Formula 1 according to one embodiment of the present specification, that is, Compound 19 that is an asymmetric boron compound including a 5-membered ring including O or S, had a lower Td-5% loss value even with a higher molecular weight compared to BD-C that is a boron Compound including a 5-membered ring including O or S but having a symmetric structure. In addition, the percentage of the compound remaining on the pan after the analysis was also within 5%, which is similar to BD-A that is a boron compound that does not have a heteroaryl group. Through this experiment, it was identified that Chemical Formula 1 according to one embodiment of the present specification, that is, an asymmetric boron compound including a 5-membered ring including O or S, was superior in terms of thermal stability by having a low a Td-5% loss value compared to compounds with similar molecular weights and thereby having a low sublimation temperature, and was an organic material suited for a deposition device as well.

### Experimental Example 4. Manufacture of Organic Light Emitting Device

### Example 1

parts by weight of the light emitting layer, and vacuum depositing the following BH-A as a host. Then, on the light emitting layer, the following Compound ET-A was vacuum deposited to 50 Å as a first electron transfer layer, and subsequently, the following ET-B and LiQ were vacuum deposited in a weight ratio of 1:1 to a thickness of 360 Å to form a second electron transfer layer. An electron injection layer was formed on the second electron transfer layer by vacuum depositing LiQ to a thickness of 5 Å. On the electron injection layer, a cathode was formed by depositing aluminum and silver in a weight ratio of 10:1 to a thickness of 220 Å, and then depositing aluminum thereon to a thickness of 1000 Å.

In the above-described process, the deposition rates of the organic materials were maintained at 0.4 Å/sec to 0.9 Å/sec, the deposition rate of the aluminum of the cathode was maintained at 2 Å/sec, and the degree of vacuum during the deposition was maintained at 6.67×10⁻³ mPa (5×10⁻⁸ torr) to 13.33×10⁻³ mPa (1×10⁻⁷ torr), and as a result, an organic light emitting device was manufactured.
parts by weight of the light emitting layer, and vacuum depositing the following BH-A as a host. Then, on the light emitting layer, the following Compound ET-A was vacuum deposited to 50 Å as a first electron transfer layer, and subsequently, the following ET-B and LiQ were vacuum deposited in a weight ratio of 1:1 to a thickness of 360 Å to form a second electron transfer layer. An electron injection layer was formed on the second electron transfer layer by vacuum depositing LiQ to a thickness of 5 Å. On the electron injection layer, a cathode was formed by depositing aluminum and silver in a weight ratio of 10:1 to a thickness of 220 Å, and then depositing aluminum thereon to a thickness of 1000 Å.

In the above-described process, the deposition rates of the organic materials were maintained at 0.4 Å/sec to 0.9 Å/sec, the deposition rate of the aluminum of the cathode was maintained at 2 Å/sec, and the degree of vacuum during the deposition was maintained at 5×10⁻⁸ torr to 1×10⁻⁷ torr (wherein 1 torr = 133.322 Pa), and as a result, an organic light emitting device was manufactured.

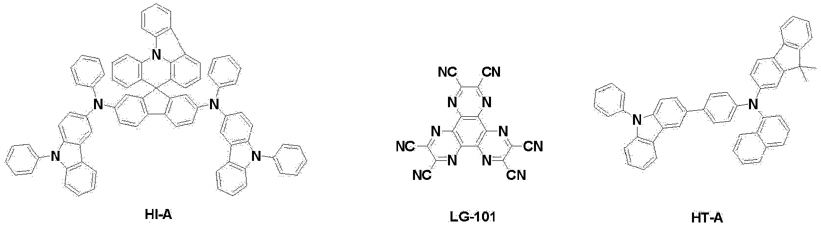

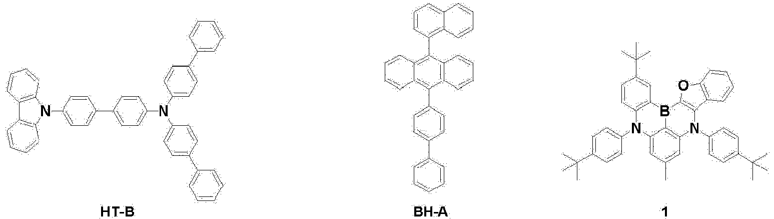

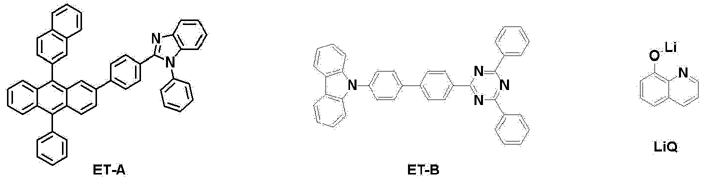

### Examples 2 to 50 and Comparative Examples 1 to 3

Organic light emitting devices of Example 2 to Example 50 and Comparative Example 1 to Comparative Example 3 were each manufactured in the same manner as in Example 1 except that compounds described in the following Table 6 were each used as the dopant of the light emitting layer instead of Compound 1.

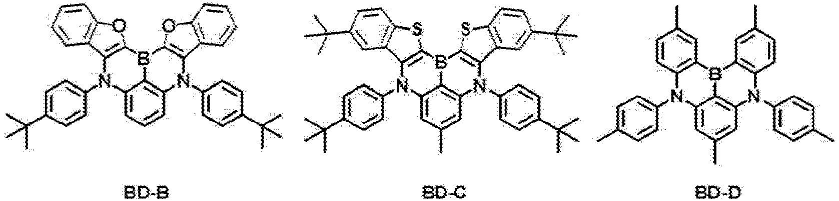

For each of the organic light emitting devices of Examples 1 to 50 and Comparative Examples 1 to 3, voltage and efficiency when applying current density of 10 mA/cm² and a lifetime (T95) when applying current density of 20 mA/cm² were measured, and the results are shown in the following Table 6. Herein, T95 means time taken for luminance to decrease to 95% when employing initial luminance at current density of 20 mA/cm² as 100%, and the percentage is shown based on Comparative Example 1 (100%).

**[Table 6]**

| | Dopant | 10 mA/cm² | | | 20 mA/cm² |
|---|---|---|---|---|---|
| | | Voltage (V) | Quantum Efficiency (QE) | Color Coordinate CIEy | LT95 (%) |
| Example 1 | Compound 1 | 3.80 | 8.48 | 0.0989 | 123 |
| Example 2 | Compound 2 | 3.76 | 8.33 | 0.1027 | 117 |
| Example 3 | Compound 3 | 3.73 | 8.22 | 0.0859 | 114 |
| Example 4 | Compound 4 | 3.70 | 8.59 | 0.0855 | 111 |
| Example 5 | Compound 5 | 3.78 | 8.23 | 0.0809 | 121 |
| Example 6 | Compound 6 | 3.75 | 8.44 | 0.1042 | 116 |
| Example 7 | Compound 7 | 3.74 | 8.11 | 0.0891 | 117 |
| Example 8 | Compound 8 | 3.80 | 8.19 | 0.0863 | 119 |
| Example 9 | Compound 9 | 3.74 | 8.41 | 0.1025 | 110 |
| Example 10 | Compound 10 | 3.78 | 8.34 | 0.0934 | 110 |
| Example 11 | Compound | 3.77 | 8.44 | 0.1071 | 120 |
| | 11 | | | | |
| Example 12 | Compound 12 | 3.80 | 8.32 | 0.0789 | 112 |
| Example 13 | Compound 13 | 3.72 | 8.15 | 0.1068 | 124 |
| Example 14 | Compound 14 | 3.78 | 8.35 | 0.0939 | 110 |
| Example 15 | Compound 15 | 3.76 | 8.40 | 0.1030 | 132 |
| Example 16 | Compound 16 | 3.79 | 8.21 | 0.1043 | 125 |
| Example 17 | Compound 17 | 3.81 | 8.26 | 0.0929 | 122 |
| Example 18 | Compound 18 | 3.76 | 8.27 | 0.0979 | 124 |
| Example 19 | Compound 19 | 3.79 | 8.75 | 0.1067 | 134 |
| Example 20 | Compound 20 | 3.75 | 8.28 | 0.0834 | 120 |
| Example 21 | Compound 21 | 3.76 | 8.68 | 0.1061 | 127 |
| Example 22 | Compound 22 | 3.81 | 8.31 | 0.1022 | 128 |
| Example 23 | Compound 23 | 3.85 | 8.13 | 0.0933 | 119 |
| Example 24 | Compound 24 | 3.82 | 8.06 | 0.0888 | 132 |
| Example 25 | Compound 25 | 3.77 | 8.37 | 0.1008 | 136 |
| Example 26 | Compound 26 | 3.78 | 8.12 | 0.0782 | 135 |
| Example 27 | Compound 27 | 3.80 | 8.40 | 0.1021 | 115 |
| Example 28 | Compound 28 | 3.77 | 8.10 | 0.0954 | 121 |
| Example 29 | Compound 29 | 3.80 | 8.34 | 0.1045 | 120 |
| Example 30 | Compound 30 | 3.85 | 8.14 | 0.0797 | 137 |
| Example 31 | Compound 31 | 3.78 | 8.32 | 0.0862 | 121 |
| Example 32 | Compound 32 | 3.85 | 8.41 | 0.0915 | 119 |
| Example 33 | Compound 33 | 3.77 | 8.21 | 0.0811 | 123 |
| Example 34 | Compound 34 | 3.75 | 8.43 | 0.0906 | 124 |
| Example 35 | Compound 35 | 3.76 | 8.65 | 0.0804 | 113 |
| Example 36 | Compound 36 | 3.77 | 8.59 | 0.1025 | 121 |
| Example 37 | Compound 37 | 3.74 | 8.44 | 0.0791 | 109 |
| Example 38 | Compound 38 | 3.77 | 8.80 | 0.1086 | 118 |
| Example 39 | Compound 39 | 3.79 | 8.32 | 0.0848 | 126 |
| Example 40 | Compound 40 | 3.80 | 8.05 | 0.1053 | 123 |
| Example 41 | Compound 41 | 3.85 | 8.26 | 0.1091 | 126 |
| Example 42 | Compound 42 | 3.77 | 8.49 | 0.0988 | 125 |
| Example 43 | Compound 43 | 3.83 | 8.30 | 0.0928 | 111 |
| Example 44 | Compound 44 | 3.80 | 8.13 | 0.1089 | 119 |
| Example 45 | Compound 45 | 3.77 | 8.42 | 0.1006 | 120 |
| Example 46 | Compound 46 | 3.78 | 8.80 | 0.0934 | 117 |
| Example 47 | Compound 47 | 3.75 | 8.16 | 0.0789 | 130 |
| Example 48 | Compound 48 | 3.78 | 8.35 | 0.0807 | 120 |
| Example 49 | Compound 49 | 3.73 | 8.36 | 0.0979 | 116 |
| Example 50 | Compound | 3.80 | 8.08 | 0.1007 | 121 |
| | 50 | | | | |
| Comparative Example 1 | BD-B | 3.89 | 7.59 | 0.1404 | 100 |
| Comparative Example 2 | BD-C | 3.92 | 7.82 | 0.1777 | 123 |
| Comparative Example 3 | BD-D | 3,99 | 7.40 | 0.0859 | 88 |

### Examples 51 to 54 and Comparative Example 4

Organic light emitting devices of Examples 51 to 54 and Comparative Example 4 were each manufactured in the same manner as in Example 1 except that compounds described in the following Table 7 were used as the dopant of the light emitting layer instead of Compound 1, and compounds described in the following Table 7 were used as the host material instead of BH-A.

In the light emitting layer, the first host and the second host had a weight ratio of 50:50.

For each of the organic light emitting devices of Examples 51 to 54 and Comparative Example 4, voltage and efficiency when applying current density of 10 mA/cm² and a lifetime (T95) when applying current density of 20 mA/cm² were measured, and the results are shown in the following Table 7. Herein, T95 means time taken for luminance to decrease to 95% when employing initial luminance at current density of 20 mA/cm² as 100%, and the percentage is shown based on Comparative Example 1 (100%) of Table 6.

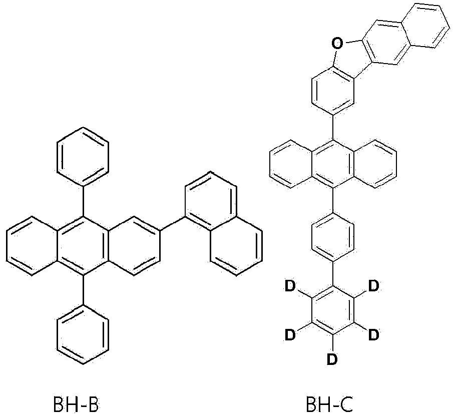

**[Table 7]**

| | First Host | Second Host | Dopant | 10 mA/cm² | | 20 mA/cm² |
|---|---|---|---|---|---|---|
| | | | | Voltage (V) | Quantum Efficiency (QE) | LT95 |
| Example 51 | BH-A | BH-B | Compound 5 | 3.82 | 8.13 | 130 |
| Example 52 | BH-A | BH-B | Compound 19 | 3.79 | 8.53 | 142 |
| Example 53 | BH-A | BH-C | Compound 26 | 3.72 | 8.22 | 128 |
| Example 54 | BH-B | BH-C | Compound 48 | 3.68 | 8.57 | 116 |
| Comparative Example 4 | BH-A | BH-B | BD-D | 3.94 | 7.52 | 93 |

### Examples 55 to 57 and Comparative Example 5

Organic light emitting devices of Examples 55 to 57 and Comparative Example 5 were each manufactured in the same manner as in Example 1 except that compounds described in the following Table 8 were used as the dopant of the light emitting layer instead of Compound 1, and compounds described in the following Table 8 were used as the host material instead of BH-A.

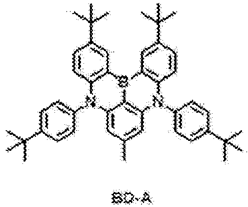

In the light emitting layer, the first dopant and the second dopant had a weight ratio of 50:50.

For each of the organic light emitting devices of Examples 55 to 57 and Comparative Example 5, voltage and efficiency when applying current density of 10 mA/cm² and a lifetime (T95) when applying current density of 20 mA/cm² were measured, and the results are shown in the following Table 8. Herein, T95 means time taken for luminance to decrease to 95% when employing initial luminance at current density of 20 mA/cm² as 100%, and the percentage is shown based on Comparative Example 1 (100%) of Table 6.

**[Table 8]**

| | Host | First Dopant | Second Dopant | 10 mA/cm² | | 20 mA/cm² |
|---|---|---|---|---|---|---|
| | | | | Voltage (V) | Quantum Efficienc y (QE) | LT95 |
| Example 55 | BH-A | Compound 17 | BD-A | 3.87 | 8.47 | 121 |
| Example 56 | BH-A | Compound 37 | Compound 50 | 3.75 | 8.36 | 119 |
| Example 57 | BH-C | Compound 14 | Compound 39 | 3.81 | 8.28 | 124 |
| Comparative Example 5 | BH-A | BD-A | BD-B | 3.90 | 7.08 | 109 |

In Tables 6 to 8, it was seen that Examples 1 to 57 including Chemical Formula 1 according to one embodiment of the present specification, that is, an asymmetric boron compound including a 5-membered ring including O or S, in the organic light emitting device were effective in obtaining lower driving voltage, excellent efficiency, favorable color purity and long lifetime compared to Comparative Examples 1 to 5 including a boron compound with a symmetric structure.

## Claims

1. A compound represented by the following Chemical Formula 1: 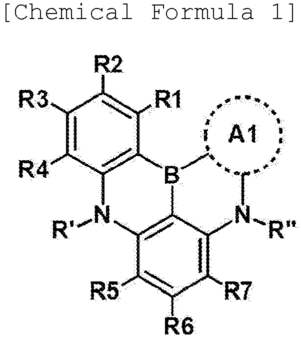 in Chemical Formula 1,
A1 is a monocyclic or polycyclic heteroring substituted or unsubstituted, and including a 5-membered ring including O or S;
R1 to R7, R' and R" are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring, and
wherein "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

2. The compound of Claim 1, wherein A1 is represented by the following Chemical Formula A1-1 or A1-2: 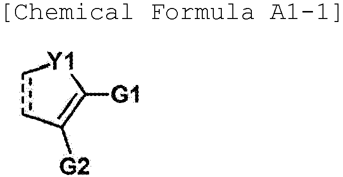 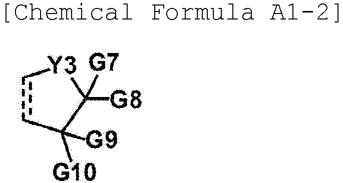 in Chemical Formulae A1-1 and A1-2,
Y1 and Y3 are the same as or different from each other, and each independently O; or S;
G1, G2 and G7 to G10 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring; means a site bonding to Chemical Formula 1, and
wherein "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

3. The compound of Claim 1, wherein A1 is represented by any one of the following Chemical Formulae A1-3 to Al-5: 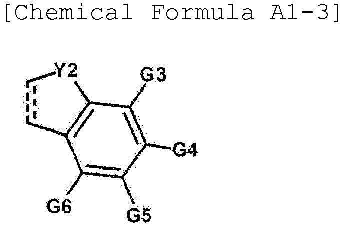 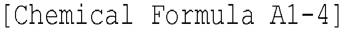 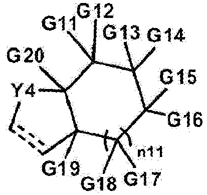 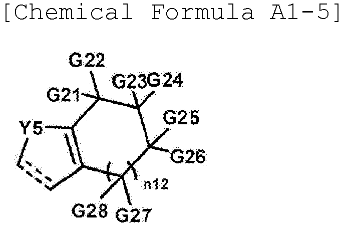 in Chemical Formulae A1-3 to A1-5,
Y2, Y4 and Y5 are the same as or different from each other, and each independently O; or S;
G3 to G6 and G11 to G28 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring;
n11 and n12 are each an integer of 0 to 2;
when n11 and n12 are 2, structures in the two parentheses are the same as or different from each other; 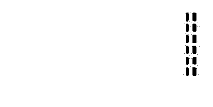 means a site bonding to Chemical Formula 1, and
wherein "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

4. The compound of Claim 1, wherein Chemical Formula 1 is represented by any one of the following Chemical Formulae 1-1 to 1-5: 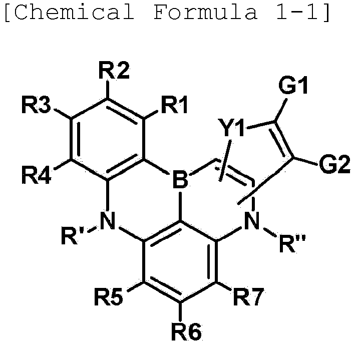 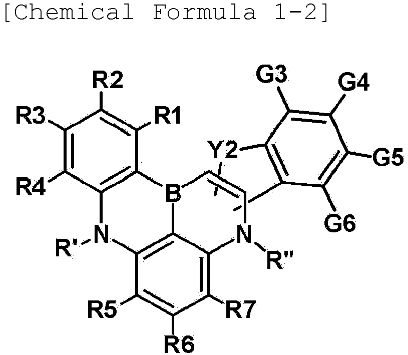 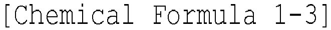 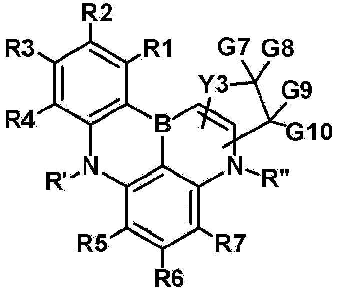 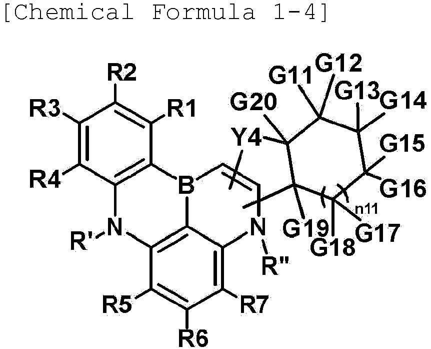 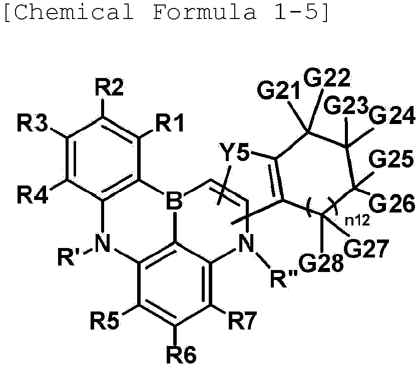 in Chemical Formulae 1-1 to 1-5,
R', R" and R1 to R7 have the same definitions as in Chemical Formula 1;
Y1 to Y5 are the same as or different from each other, and each independently O; or S;
G1 to G28 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring;
n11 and n12 are each an integer of 0 to 2;
when n11 and n12 are 2, structures in the two parentheses are the same as or different from each other, and
wherein "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

5. The compound of Claim 1, wherein adjacent groups among R1 to R4 bond to each other to form a ring represented by the following Chemical Formula B1-1 or B1-2: 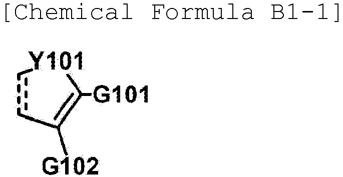 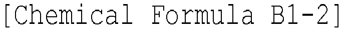 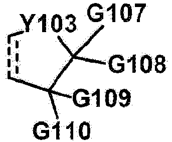 in Chemical Formulae B1-1 and B1-2,
Y101 and Y103 are the same as or different from each other, and each independently O; S; or NR‴;
R‴, G101, G102 and G107 to G110 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring; 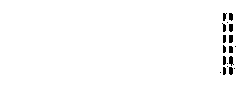 means a site bonding to Chemical Formula 1, and
wherein "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

6. The compound of Claim 1, wherein adjacent groups among R1 to R4 bond to each other to form a ring represented by any one of the following Chemical Formulae B1-3 to Bl-5: 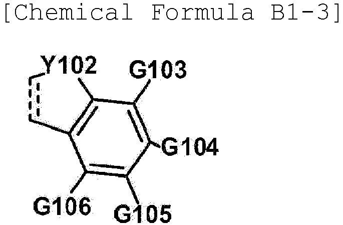 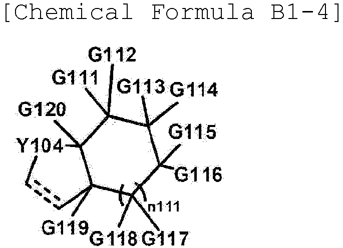 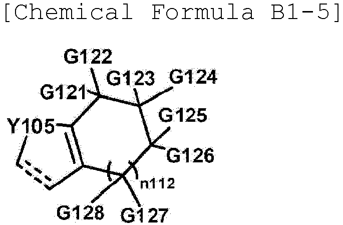 in Chemical Formulae B1-3 to B1-5,
Y102, Y104 and Y105 are the same as or different from each other, and each independently O; S; or NR‴;
R‴, G103 to G106 and G111 to G128 are the same as or different from each other, and each independently hydrogen; deuterium; a halogen group; a cyano group; a substituted or unsubstituted alkyl group; a substituted or unsubstituted haloalkyl group; a substituted or unsubstituted cycloalkyl group; a substituted or unsubstituted alkoxy group; a substituted or unsubstituted haloalkoxy group; a substituted or unsubstituted alkylthioxy group; a substituted or unsubstituted aryloxy group; a substituted or unsubstituted arylthioxy group; a substituted or unsubstituted alkenyl group; a substituted or unsubstituted boron group; a substituted or unsubstituted amine group; a substituted or unsubstituted arylalkyl group; a substituted or unsubstituted silyl group; a substituted or unsubstituted hydrocarbon ring group; or a substituted or unsubstituted heterocyclic group, or bond to adjacent groups to form a substituted or unsubstituted ring;
n111 and n112 are each an integer of 0 to 2;
when n111 and n112 are 2, structures in the two parentheses are the same as or different from each other; 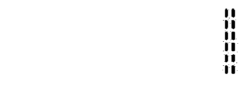 means a site bonding to Chemical Formula 1, and
wherein "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

7. The compound of Claim 1, wherein R1 to R7 are the same as or different from each other, and each independently hydrogen; deuterium; a cyano group; a linear or branched alkyl group having 1 to 30 carbon atoms; a monocyclic or polycyclic cycloalkyl group having 3 to 30 carbon atoms; a linear or branched alkylsilyl group having 1 to 30 carbon atoms; a linear or branched haloalkyl group having 1 to 30 carbon atoms; a linear or branched haloalkoxy group having 1 to 30 carbon atoms; an arylalkyl group having 6 to 30 carbon atoms; a monocyclic or polycyclic diarylamine group having 6 to 30 carbon atoms; a monocyclic or polycyclic aryl group having 6 to 30 carbon atoms; or a monocyclic or polycyclic heterocyclic group having 2 to 30 carbon atoms, and the substituent is unsubstituted or substituted with one or more selected from the group consisting of deuterium, a halogen group, a cyano group, a linear or branched alkyl group having 1 to 30 carbon atoms, a linear or branched alkylsilyl group having 1 to 30 carbon atoms, a monocyclic or polycyclic aryl group having 6 to 30 carbon atoms, and combinations thereof.

8. The compound of Claim 1, wherein adjacent groups among R1 to R4 bond to each other to form a substituted or unsubstituted monocyclic or polycyclic hydrocarbon ring having 3 to 30 carbon atoms; or a substituted or unsubstituted monocyclic or polycyclic heteroring having 2 to 30 carbon atoms, and
wherein "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

9. The compound of Claim 1, wherein R' and R" are the same as or different from each other, and each independently a linear or branched alkyl group having 1 to 30 carbon atoms; an arylalkyl group having 6 to 30 carbon atoms; a monocyclic or polycyclic hydrocarbon ring having 3 to 30 carbon atoms group; or a monocyclic or polycyclic heterocyclic group having 2 to 30 carbon atoms, and the substituent is unsubstituted or substituted with one or more selected from the group consisting of deuterium, a halogen group, a cyano group, a linear or branched alkyl group having 1 to 30 carbon atoms, a monocyclic or polycyclic cycloalkyl group having 3 to 30 carbon atoms, a linear or branched alkylsilyl group having 1 to 30 carbon atoms, an arylsilyl group having 3 to 30 carbon atoms, a linear or branched alkoxy group having 1 to 30 carbon atoms, a linear or branched haloalkyl group having 1 to 30 carbon atoms, a linear or branched haloalkoxy group having 1 to 30 carbon atoms, an aryloxy group having 6 to 30 carbon atoms, a monocyclic or polycyclic arylalkyl group having 6 to 30 carbon atoms, a monocyclic or polycyclic diarylamine group having 6 to 30 carbon atoms, a monocyclic or polycyclic aryl group having 6 to 30 carbon atoms, a monocyclic or polycyclic heterocyclic group having 2 to 30 carbon atoms, and combinations thereof.

10. The compound of Claim 1, wherein Chemical Formula 1 is any one selected from among the following compounds: 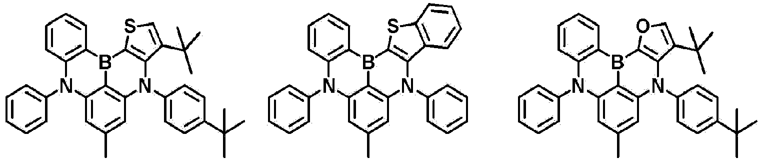 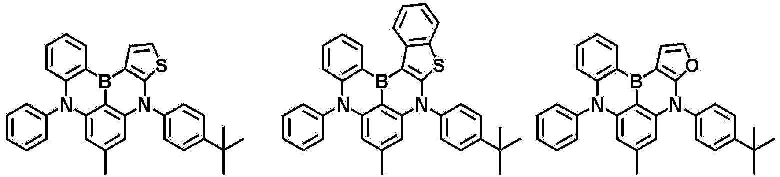 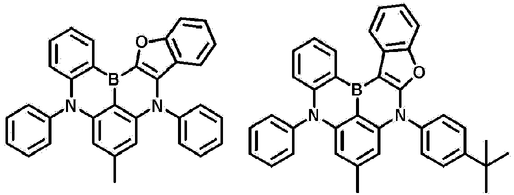 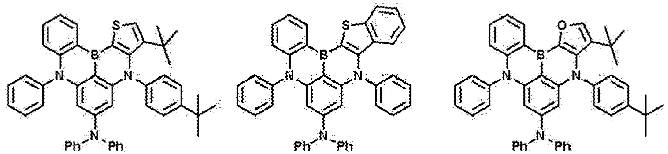 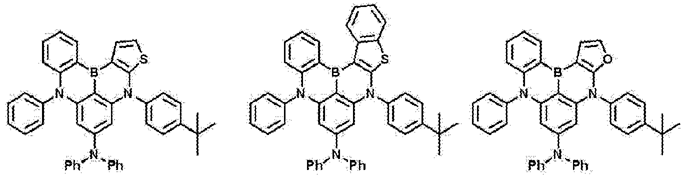 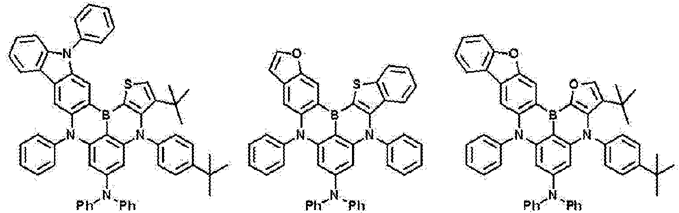 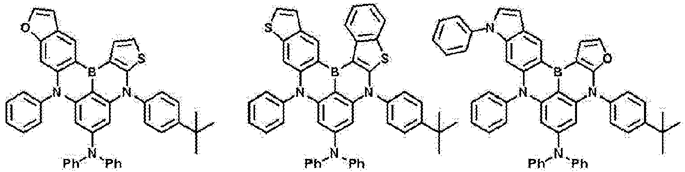 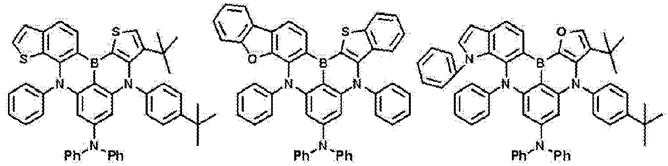 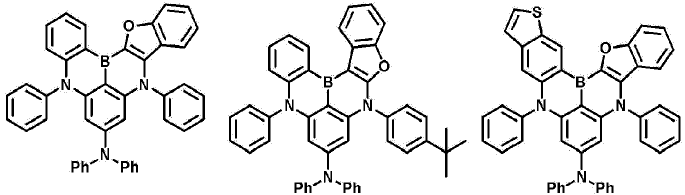 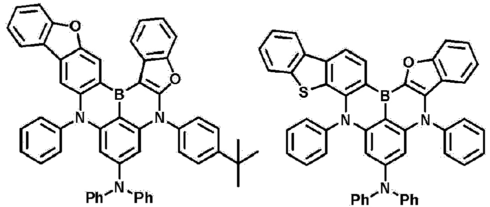 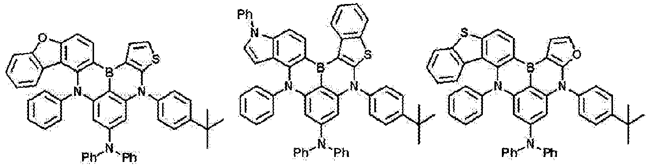 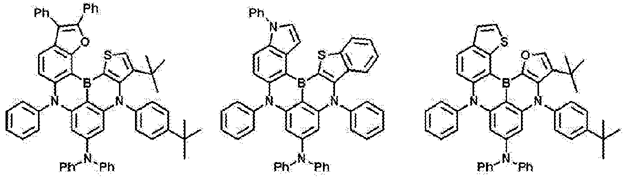 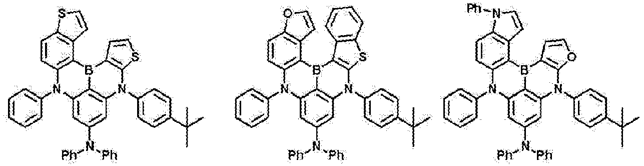 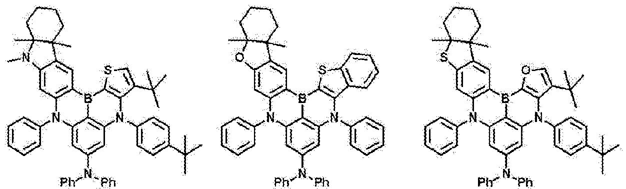 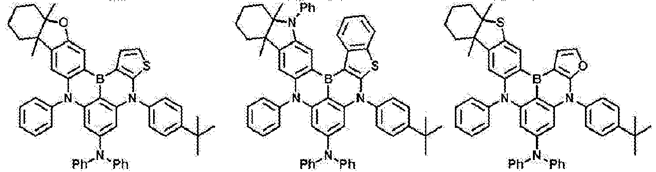 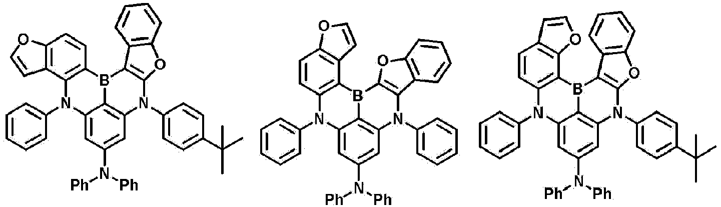 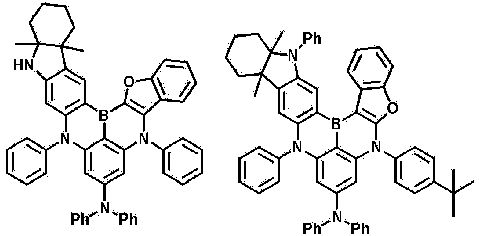 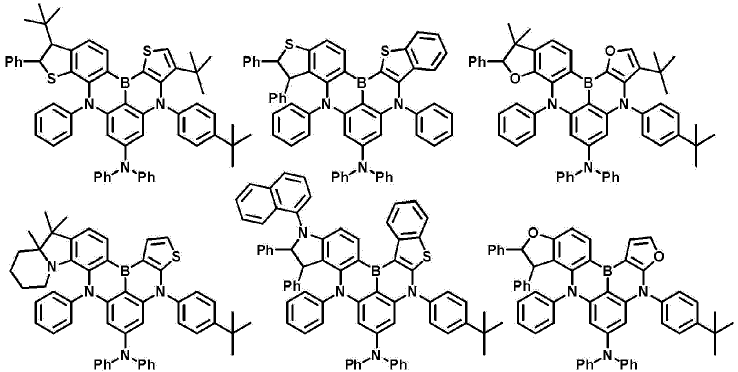 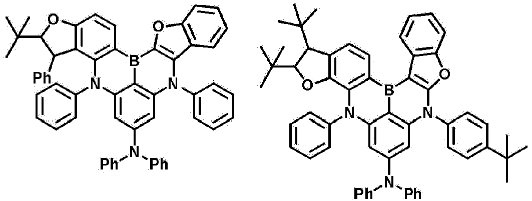 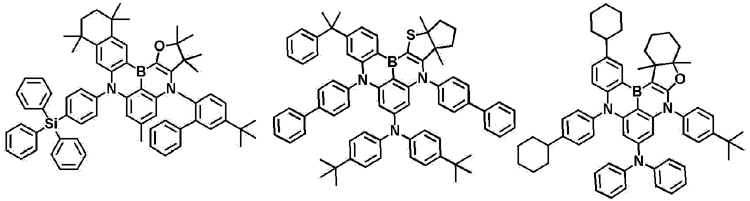 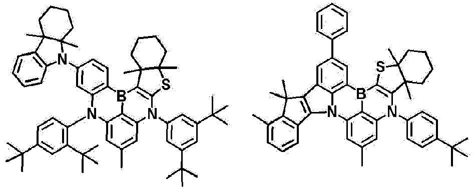 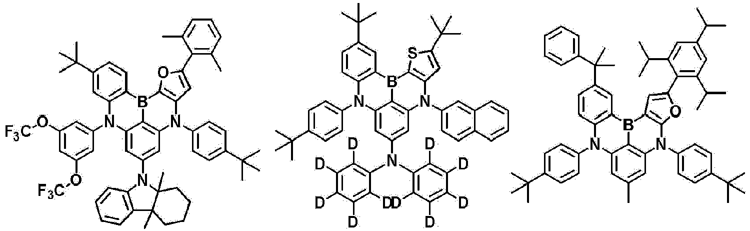 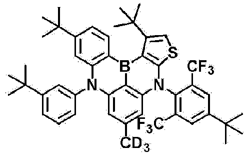 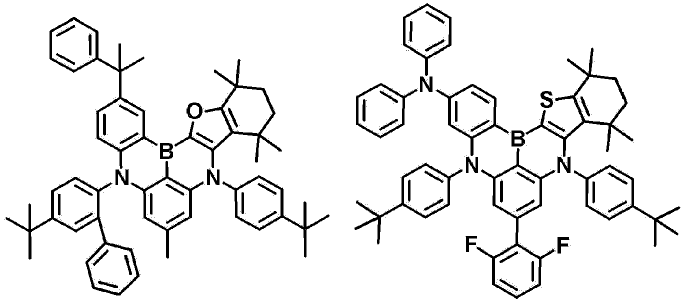 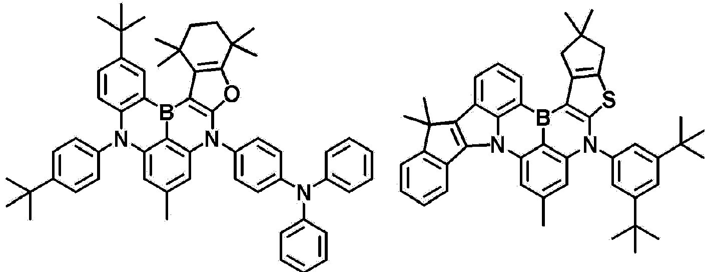 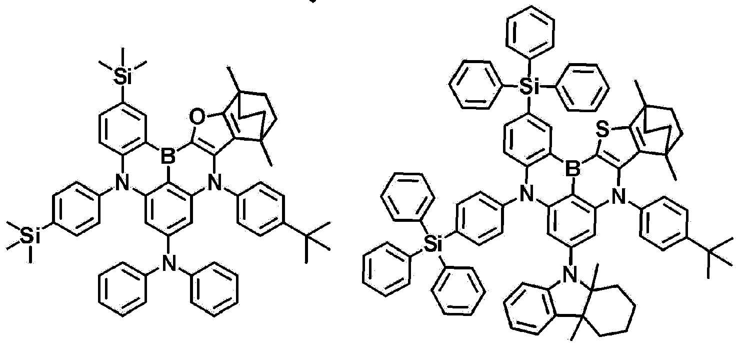 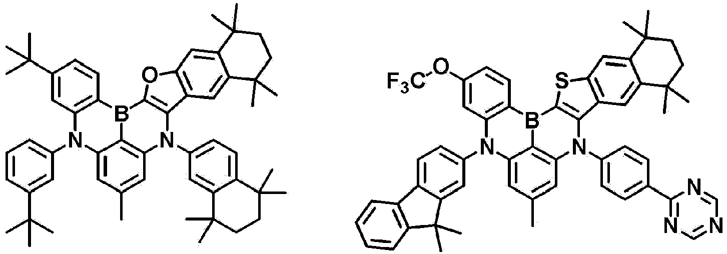 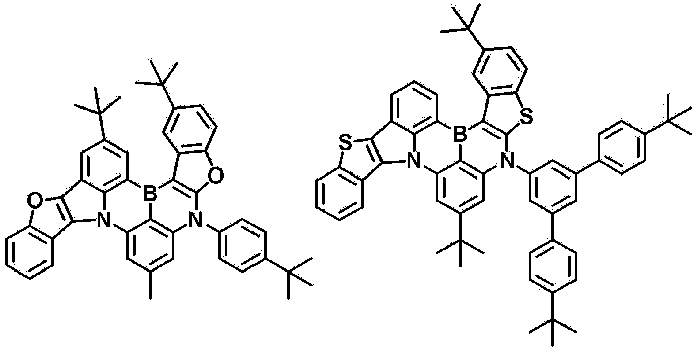 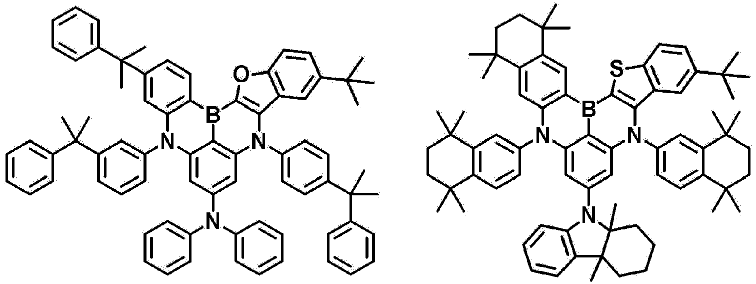 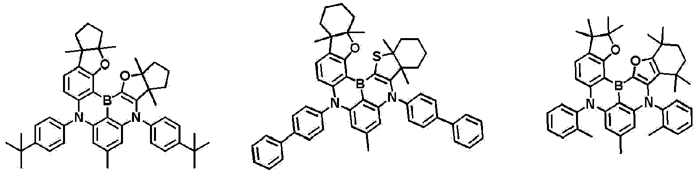 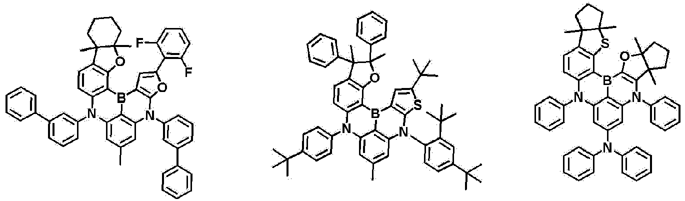 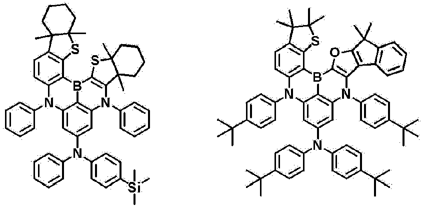 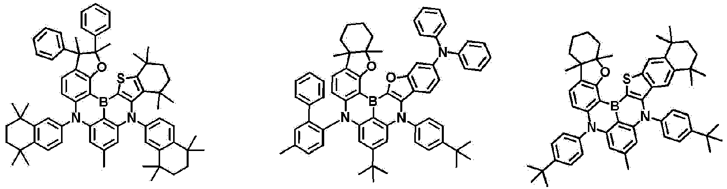 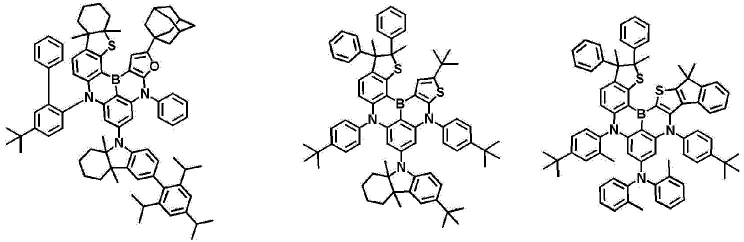 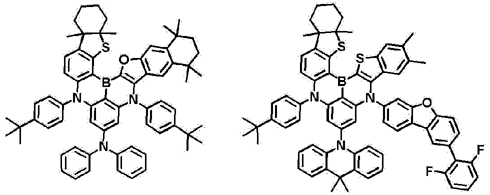 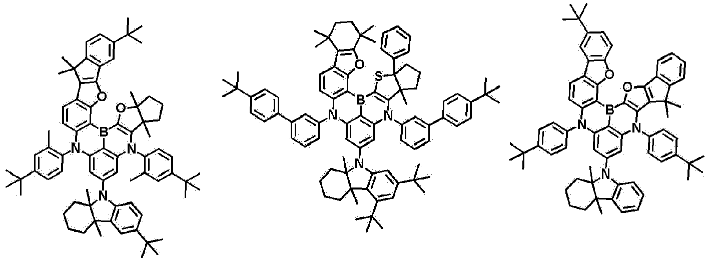 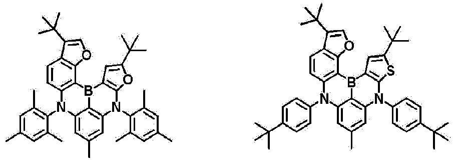 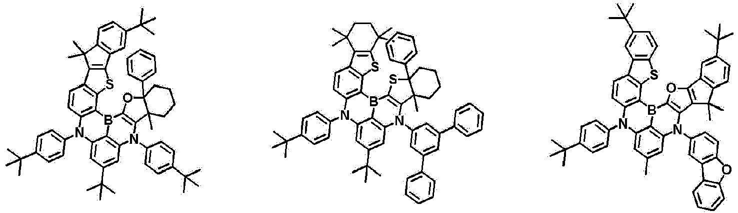 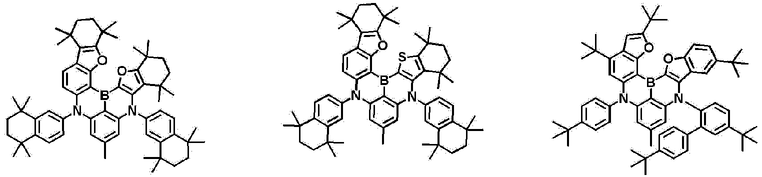 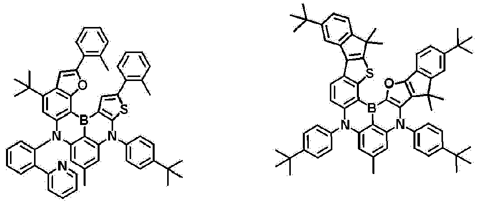 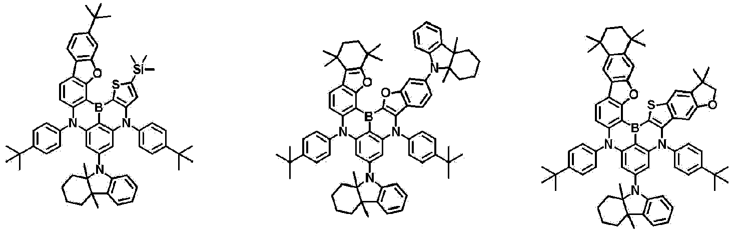 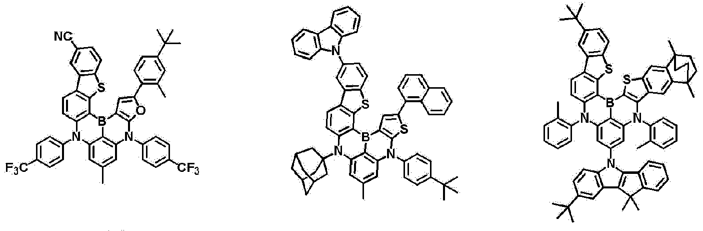 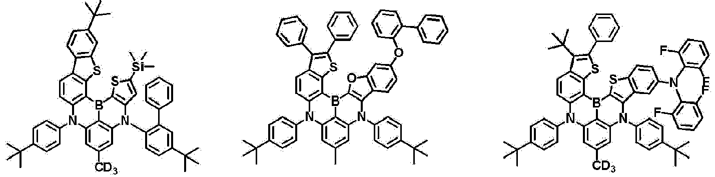 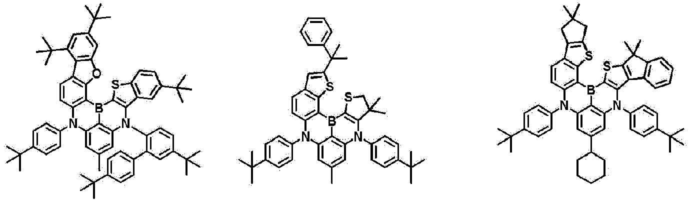 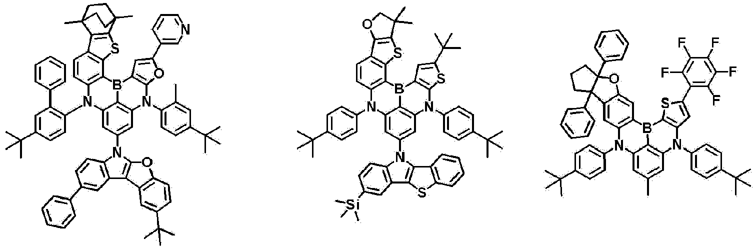 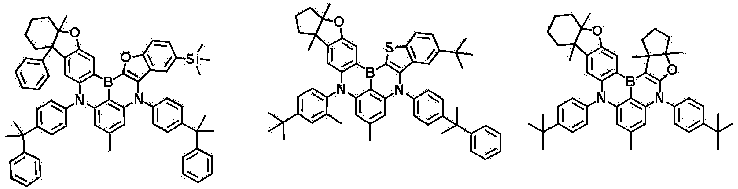 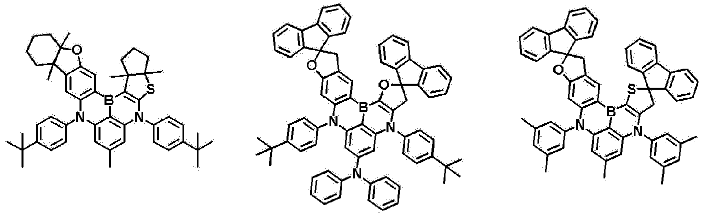 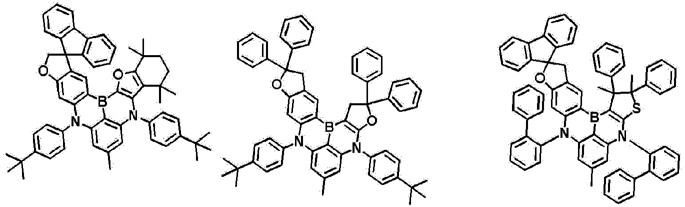 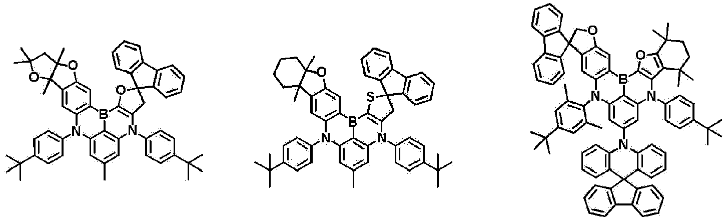 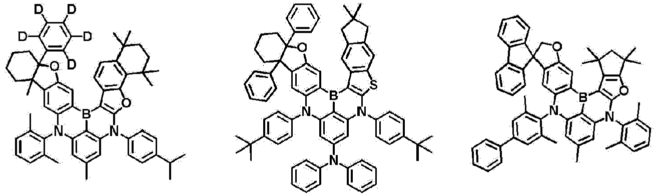 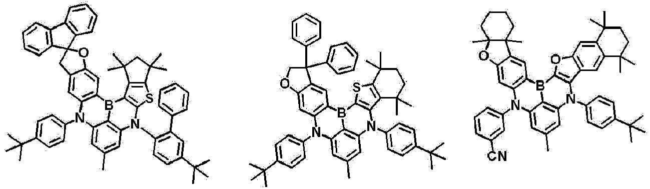 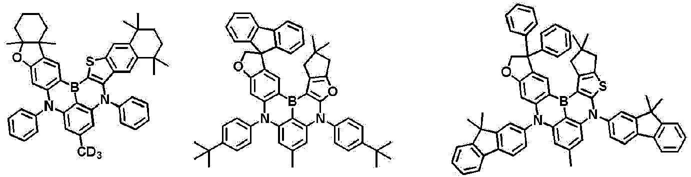 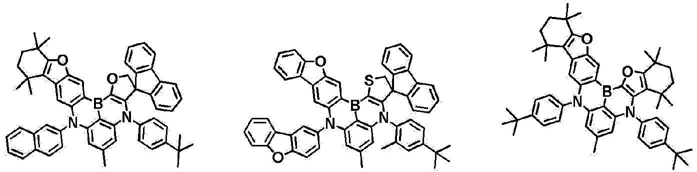 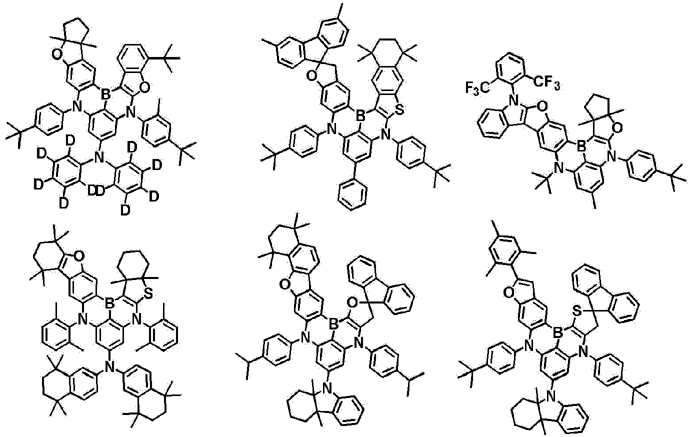 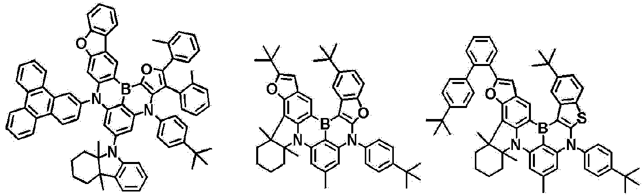 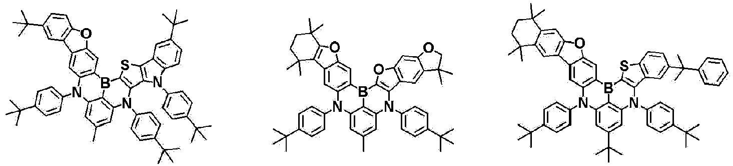 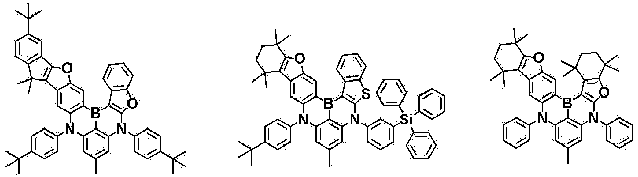 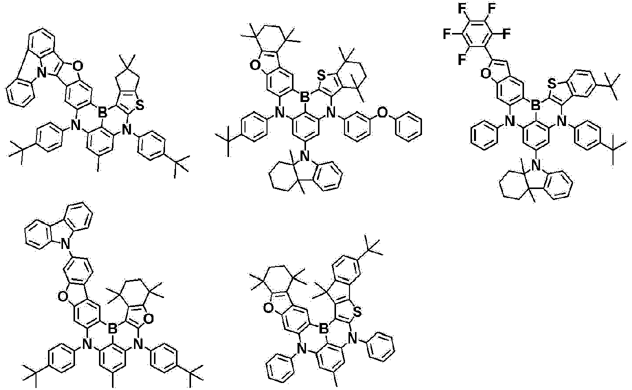 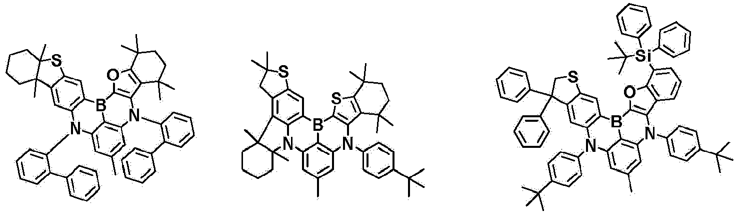 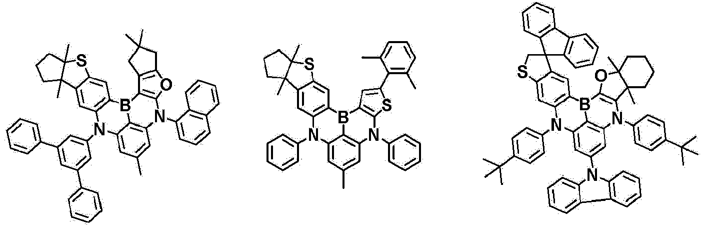 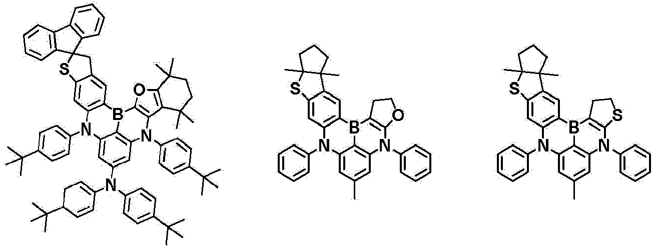 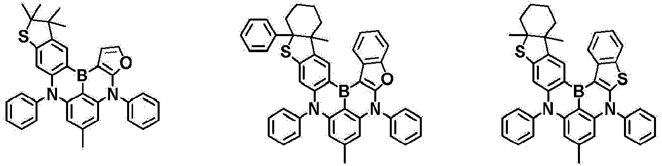 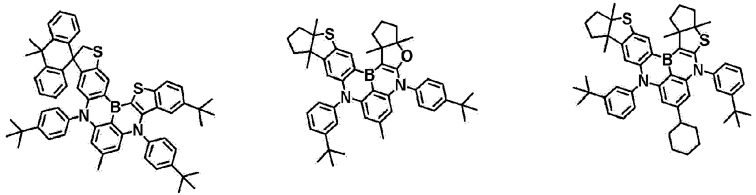 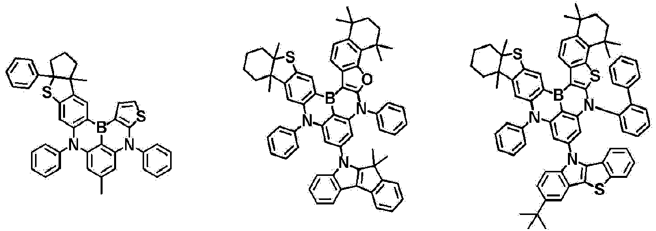 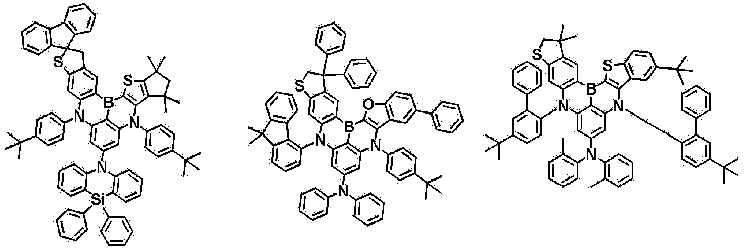 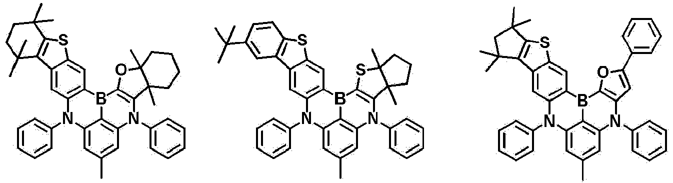 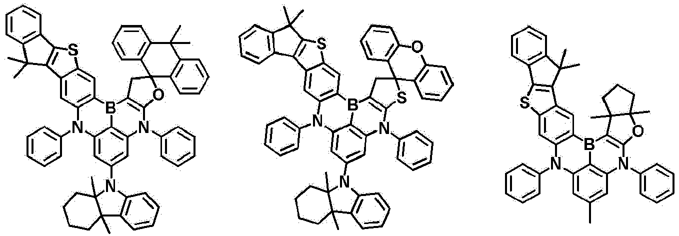 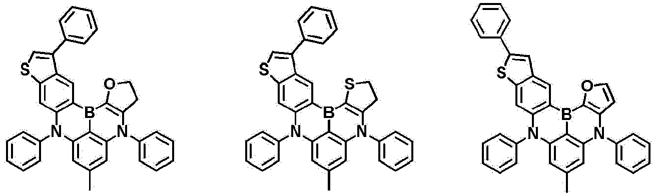 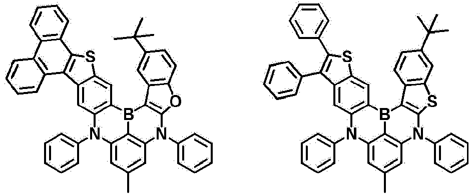 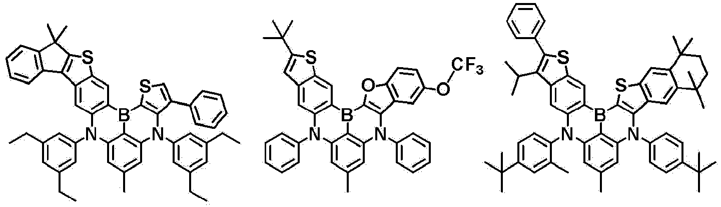 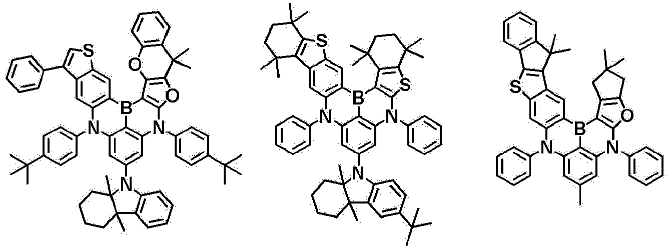 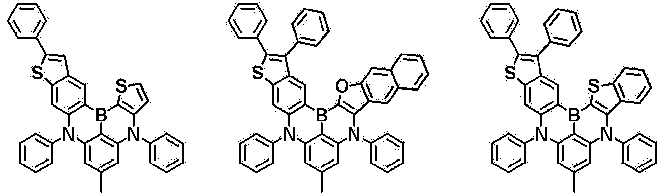 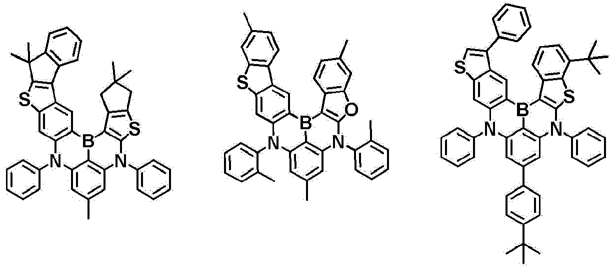 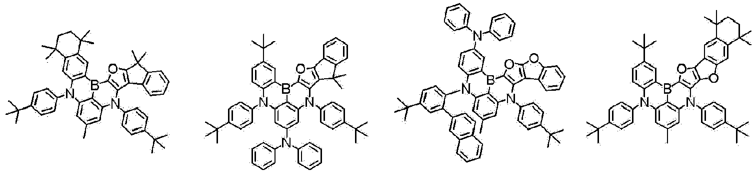 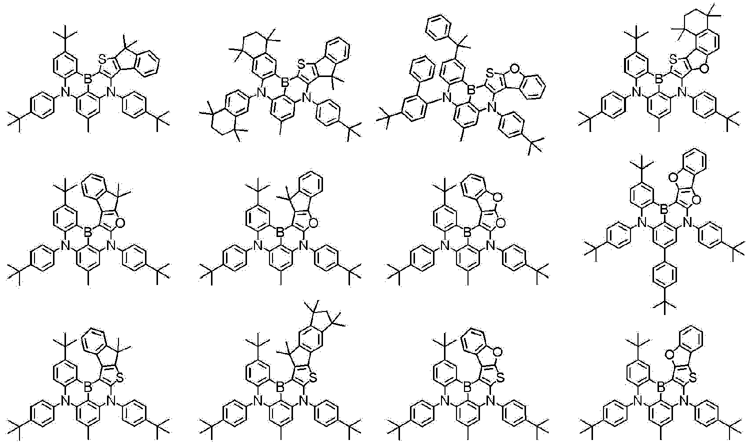 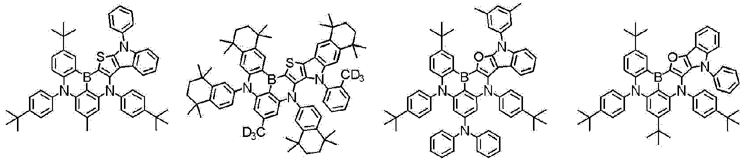 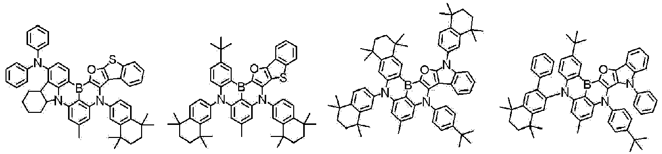 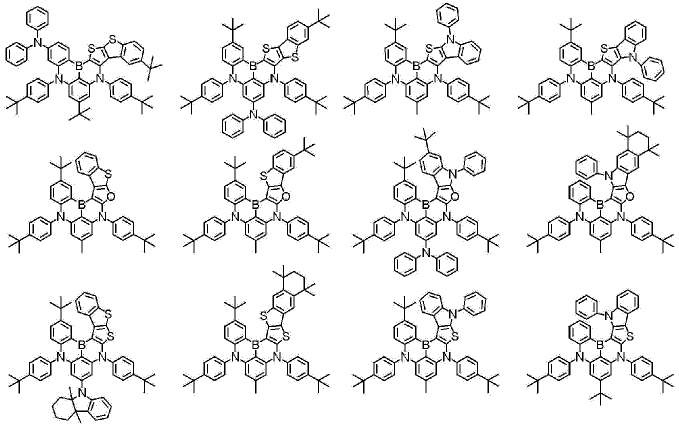 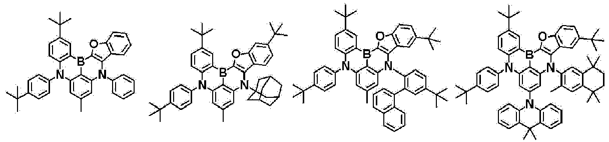 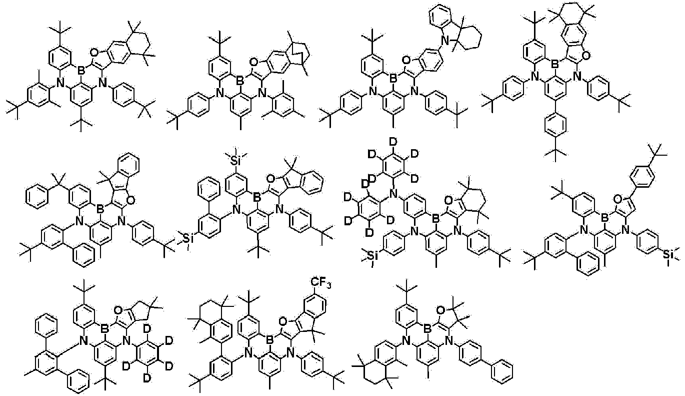 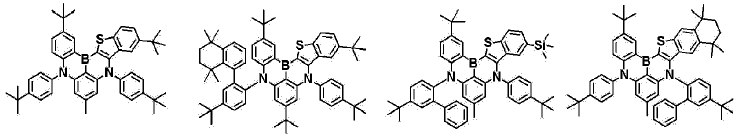 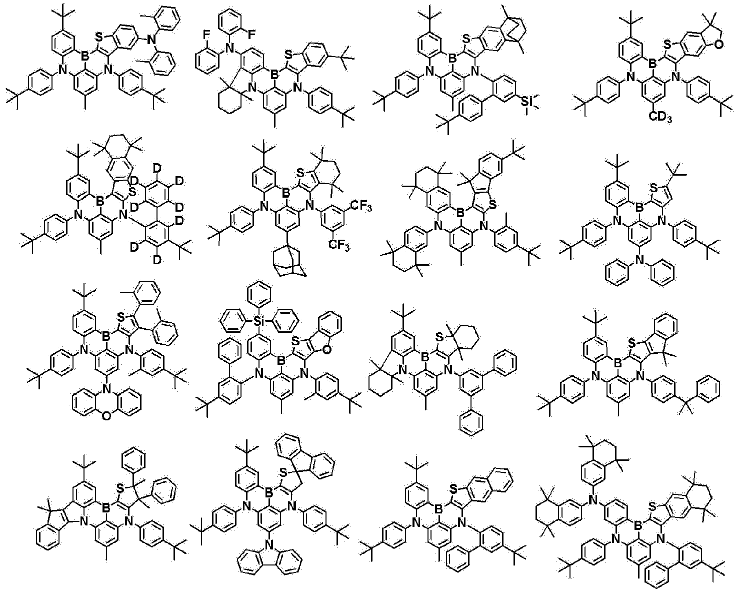 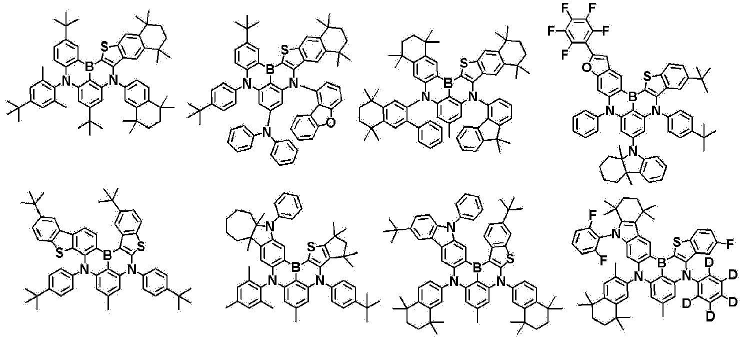 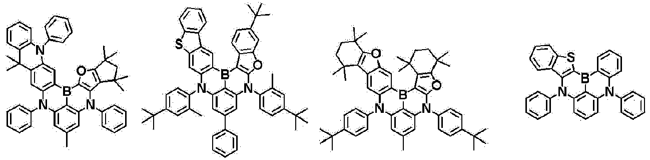 in the compounds,
Ph means a phenyl group.

11. An organic light emitting device comprising:
a first electrode;
a second electrode; and
one or more organic material layers provided between the first electrode and the second electrode,
wherein one or more layers of the organic material layers include the compound of any one of Claims 1 to 10.

12. The organic light emitting device of Claim 11, wherein the organic material layer includes a light emitting layer, and the light emitting layer includes the compound.

13. The organic light emitting device of Claim 11, wherein the organic material layer includes a light emitting layer, the light emitting layer includes a dopant material, and the dopant material includes the compound.

14. The organic light emitting device of Claim 11, wherein the organic material layer includes a light emitting layer, and the light emitting layer further includes a compound represented by the following Chemical Formula H: 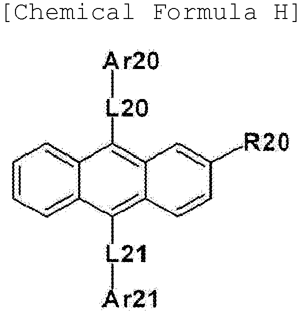 in Chemical Formula H,
L20 and L21 are the same as or different from each other, and each independently a direct bond; a substituted or unsubstituted arylene group; or a substituted or unsubstituted heteroarylene group;
Ar20 and Ar21 are the same as or different from each other, and each independently hydrogen; deuterium; a substituted or unsubstituted aryl group; or a substituted or unsubstituted heterocyclic group;
R20 is hydrogen; deuterium; a halogen group; a substituted or unsubstituted alkyl group; a substituted or unsubstituted cycloalkyl group; a substituted or unsubstituted aryl group; or a substituted or unsubstituted heterocyclic group, and
wherein "substituted or unsubstituted" means being substituted with one or more substituents selected from the group consisting of deuterium; a halogen group; a cyano group; an alkyl group; a cycloalkyl group; an alkoxy group; an aryloxy group; an alkylthioxy group; an arylthioxy group; an alkenyl group; a haloalkyl group; a haloalkoxy group; an arylalkyl group; a silyl group; a boron group; an amine group; an aryl group; a fused ring group of aromatic hydrocarbon and aliphatic hydrocarbon; and a heterocyclic group, or being substituted with a substituent linking two or more substituents among the substituents illustrated above, or having no substituents.

15. The organic light emitting device of Claim 11, wherein the organic material layer includes a light emitting layer, and the light emitting layer includes one or more types of hosts and a dopant.

## Patentansprüche

1. Verbindung dargestellt durch die folgende chemische Formel 1: 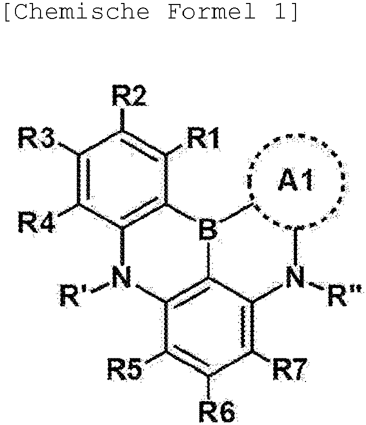 in der chemischen Formel 1
ist A1 ein monocyclischer oder polycyclischer Heteroring, der substituiert oder unsubstituiert ist und einen 5-gliedrigen Ring einschließlich O oder S einschließt,
R1 bis R7, R' und R" sind gleich oder verschieden voneinander und jeweils unabhängig Wasserstoff, Deuterium, eine Halogengruppe, eine Cyanogruppe, eine substituierte oder unsubstituierte Alkylgruppe, eine substituierte oder unsubstituierte Halogenalkylgruppe, eine substituierte oder unsubstituierte Cycloalkylgruppe, eine substituierte oder unsubstituierte Alkoxygruppe, eine substituierte oder unsubstituierte Halogenalkoxygruppe, eine substituierte oder unsubstituierte Alkylthioxygruppe, eine substituierte oder unsubstituierte Aryloxygruppe, eine substituierte oder unsubstituierte Arylthioxygruppe, eine substituierte oder unsubstituierte Alkenylgruppe, eine substituierte oder unsubstituierte Borgruppe, eine substituierte oder unsubstituierte Amingruppe eine substituierte oder unsubstituierte Arylalkylgruppe, eine substituierte oder unsubstituierte Silylgruppe, eine substituierte oder unsubstituierte Kohlenwasserstoffringgruppe oder eine substituierte oder unsubstituierte heterocyclische Gruppe oder binden an benachbarte Gruppen, um einen substituierten oder unsubstituierten Ring zu bilden, und
wobei "substituiert oder unsubstituiert" substituiert mit einem oder mehreren Substituenten, ausgewählt aus der Gruppe, bestehend aus Deuterium, einer Halogengruppe, einer Cyanogruppe, einer Alkylgruppe, einer Cycloalkylgruppe, einer Alkoxygruppe, einer Aryloxygruppe, einer Alkylthioxygruppe, einer Arylthioxygruppe, einer Alkenylgruppe, einer Halogenalkylgruppe, einer Halogenalkoxygruppe, einer Arylalkylgruppe, einer Silylgruppe, einer Borgruppe, einer Amingruppe, einer Arylgruppe, einer kondensierten Ringgruppe aus aromatischem Kohlenwasserstoff und aliphatischem Kohlenwasserstoff und einer heterocyclischen Gruppe, oder substituiert mit einem Substituenten, der zwei oder mehr Substituenten unter den oben veranschaulichten Substituenten verknüpft, oder ohne Substituenten bedeutet.

2. Verbindung nach Anspruch 1, worin A1 durch die folgende chemische Formel A1-1 oder A1-2 dargestellt ist: 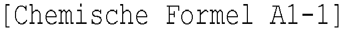 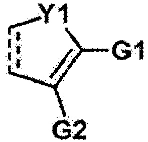 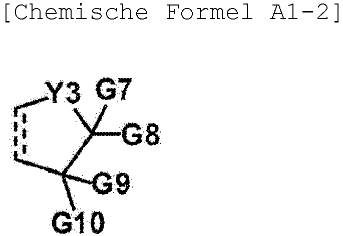 in den chemischen Formeln A1-1 und A1-2
sind Y1 und Y3 gleich oder verschieden voneinander und jeweils unabhängig O oder S,
G1, G2 und G7 bis G10 sind gleich oder verschieden voneinander und jeweils unabhängig Wasserstoff, Deuterium, eine Halogengruppe, eine Cyanogruppe, eine substituierte oder unsubstituierte Alkylgruppe, eine substituierte oder unsubstituierte Halogenalkylgruppe, eine substituierte oder unsubstituierte Cycloalkylgruppe, eine substituierte oder unsubstituierte Alkoxygruppe, eine substituierte oder unsubstituierte Halogenalkoxygruppe, eine substituierte oder unsubstituierte Alkylthioxygruppe, eine substituierte oder unsubstituierte Aryloxygruppe, eine substituierte oder unsubstituierte Arylthioxygruppe, eine substituierte oder unsubstituierte Alkenylgruppe, eine substituierte oder unsubstituierte Borgruppe, eine substituierte oder unsubstituierte Amingruppe eine substituierte oder unsubstituierte Arylalkylgruppe, eine substituierte oder unsubstituierte Silylgruppe, eine substituierte oder unsubstituierte Kohlenwasserstoffringgruppe oder eine substituierte oder unsubstituierte heterocyclische Gruppe oder binden an benachbarte Gruppen, um einen substituierten oder unsubstituierten Ring zu bilden, 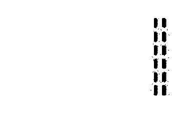
bedeutet eine Stelle, die an die chemische Formel 1 bindet und
wobei "substituiert oder unsubstituiert" substituiert mit einem oder mehreren Substituenten, ausgewählt aus der Gruppe, bestehend aus Deuterium, einer Halogengruppe, einer Cyanogruppe, einer Alkylgruppe, einer Cycloalkylgruppe, einer Alkoxygruppe, einer Aryloxygruppe, einer Alkylthioxygruppe, einer Arylthioxygruppe, einer Alkenylgruppe, einer Halogenalkylgruppe, einer Halogenalkoxygruppe, einer Arylalkylgruppe, einer Silylgruppe, einer Borgruppe, einer Amingruppe, einer Arylgruppe, einer kondensierten Ringgruppe aus aromatischem Kohlenwasserstoff und aliphatischem Kohlenwasserstoff und einer heterocyclischen Gruppe, oder substituiert mit einem Substituenten, der zwei oder mehr unter den oben veranschaulichten Substituenten verknüpft, oder ohne Substituenten bedeutet.

3. Verbindung nach Anspruch 1, worin A1 durch eine der folgenden chemischen Formeln A1-3 bis A1-5 dargestellt ist: 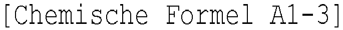 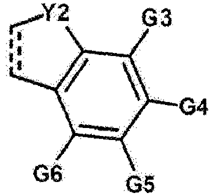 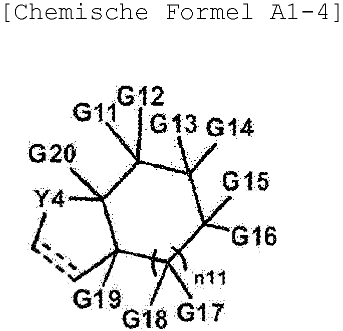 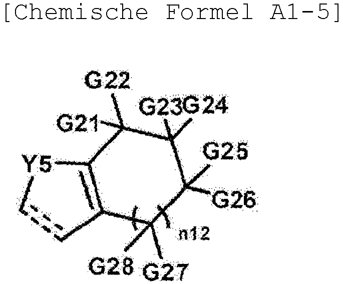 in den chemischen Formeln A1-3 bis A1-5
sind Y2, Y4 und Y5 gleich oder verschieden voneinander und jeweils unabhängig O oder S,
G3 bis G6 und G11 bis G28 sind gleich oder verschieden voneinander und jeweils unabhängig Wasserstoff, Deuterium, eine Halogengruppe, eine Cyanogruppe, eine substituierte oder unsubstituierte Alkylgruppe, eine substituierte oder unsubstituierte Halogenalkylgruppe, eine substituierte oder unsubstituierte Cycloalkylgruppe, eine substituierte oder unsubstituierte Alkoxygruppe, eine substituierte oder unsubstituierte Halogenalkoxygruppe, eine substituierte oder unsubstituierte Alkylthioxygruppe, eine substituierte oder unsubstituierte Aryloxygruppe, eine substituierte oder unsubstituierte Arylthioxygruppe, eine substituierte oder unsubstituierte Alkenylgruppe, eine substituierte oder unsubstituierte Borgruppe, eine substituierte oder unsubstituierte Amingruppe eine substituierte oder unsubstituierte Arylalkylgruppe, eine substituierte oder unsubstituierte Silylgruppe, eine substituierte oder unsubstituierte Kohlenwasserstoffringgruppe oder eine substituierte oder unsubstituierte heterocyclische Gruppe oder binden an benachbarte Gruppen, um einen substituierten oder unsubstituierten Ring zu bilden,
n11 und n12 sind jeweils eine ganze Zahl von 0 bis 2,
wenn n11 und n12 2 sind, sind Strukturen in den beiden Klammern gleich oder verschieden voneinander, 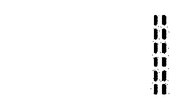
bedeutet eine Stelle, die an chemische Formel 1 bindet und
wobei "substituiert oder unsubstituiert" substituiert mit einem oder mehreren Substituenten, ausgewählt aus der Gruppe, bestehend aus Deuterium, einer Halogengruppe, einer Cyanogruppe, einer Alkylgruppe, einer Cycloalkylgruppe, einer Alkoxygruppe, einer Aryloxygruppe, einer Alkylthioxygruppe, einer Arylthioxygruppe, einer Alkenylgruppe, einer Halogenalkylgruppe, einer Halogenalkoxygruppe, einer Arylalkylgruppe, einer Silylgruppe, einer Borgruppe, einer Amingruppe, einer Arylgruppe, einer kondensierten Ringgruppe aus aromatischem Kohlenwasserstoff und aliphatischem Kohlenwasserstoff und einer heterocyclischen Gruppe, oder substituiert mit einem Substituenten, der zwei oder mehr Substituenten unter den oben veranschaulichten Substituenten verknüpft, oder ohne Substituenten bedeutet.

4. Verbindung nach Anspruch 1, wobei die chemische Formel 1 durch eine der folgenden chemischen Formeln 1-1 bis 1-5 dargestellt ist: 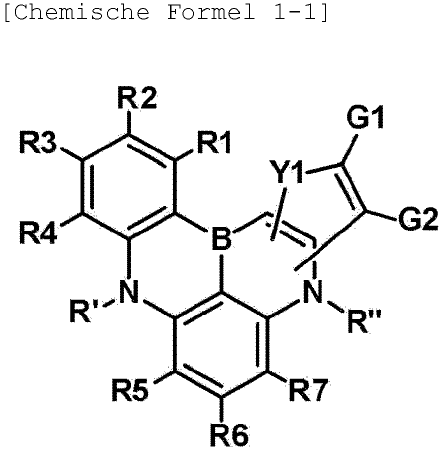 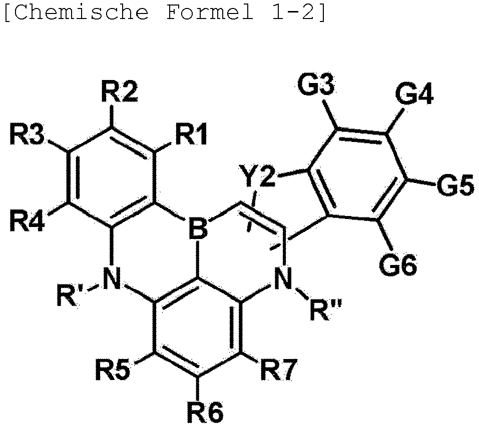 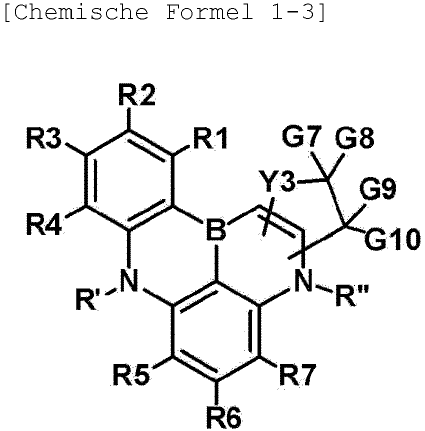 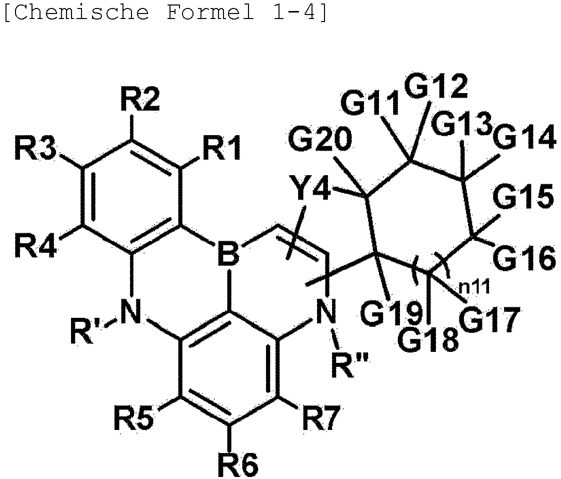 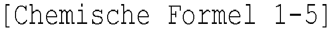 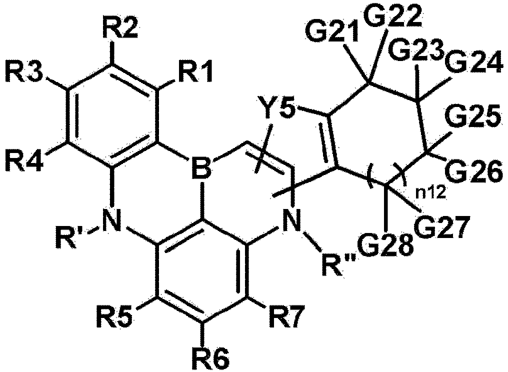 in den chemischen Formeln 1-1 bis 1-5
weisen R', R" und R1 bis R7 dieselben Definitionen wie in der chemischen Formel 1 auf,
Y1 bis Y5 sind gleich oder verschieden voneinander und jeweils unabhängig O oder S,
G1 bis G28 sind gleich oder verschieden voneinander und jeweils unabhängig Wasserstoff, Deuterium, eine Halogengruppe, eine Cyanogruppe, eine substituierte oder unsubstituierte Alkylgruppe, eine substituierte oder unsubstituierte Halogenalkylgruppe, eine substituierte oder unsubstituierte Cycloalkylgruppe, eine substituierte oder unsubstituierte Alkoxygruppe, eine substituierte oder unsubstituierte Halogenalkoxygruppe, eine substituierte oder unsubstituierte Alkylthioxygruppe, eine substituierte oder unsubstituierte Aryloxygruppe, eine substituierte oder unsubstituierte Arylthioxygruppe, eine substituierte oder unsubstituierte Alkenylgruppe, eine substituierte oder unsubstituierte Borgruppe, eine substituierte oder unsubstituierte Amingruppe eine substituierte oder unsubstituierte Arylalkylgruppe, eine substituierte oder unsubstituierte Silylgruppe, eine substituierte oder unsubstituierte Kohlenwasserstoffringgruppe oder eine substituierte oder unsubstituierte heterocyclische Gruppe oder binden an benachbarte Gruppen, um einen substituierten oder unsubstituierten Ring zu bilden,
n11 und n12 sind jeweils eine ganze Zahl von 0 bis 2,
wenn n11 und n12 2 sind, sind Strukturen in den beiden Klammern gleich oder verschieden voneinander, und
wobei "substituiert oder unsubstituiert" substituiert mit einem oder mehreren Substituenten, ausgewählt aus der Gruppe, bestehend aus Deuterium, einer Halogengruppe, einer Cyanogruppe, einer Alkylgruppe, einer Cycloalkylgruppe, einer Alkoxygruppe, einer Aryloxygruppe, einer Alkylthioxygruppe, einer Arylthioxygruppe, einer Alkenylgruppe, einer Halogenalkylgruppe, einer Halogenalkoxygruppe, einer Arylalkylgruppe, einer Silylgruppe, einer Borgruppe, einer Amingruppe, einer Arylgruppe, einer kondensierten Ringgruppe aus aromatischem Kohlenwasserstoff und aliphatischem Kohlenwasserstoff und einer heterocyclischen Gruppe, oder substituiert mit einem Substituenten, der zwei oder mehr Substituenten unter den oben veranschaulichten Substituenten verknüpft, oder ohne Substituenten bedeutet.

5. Verbindung nach Anspruch 1, wobei benachbarte Gruppen unter R1 bis R4 aneinander binden, um einen Ring zu bilden, der durch die folgende chemische Formel B1-1 oder B1-2 dargestellt ist: 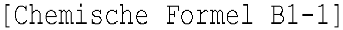 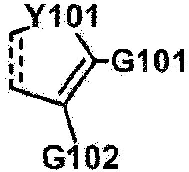 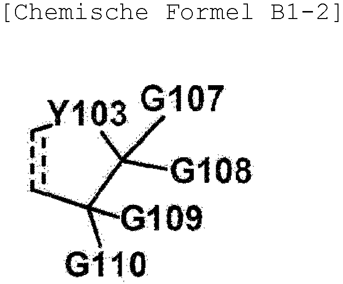 in den chemischen Formeln B1-1 und B1-2
sind Y101 und Y103 gleich oder verschieden voneinander und jeweils unabhängig O, S oder NR‴,
R‴, G101, G102 und G107 bis G110 sind gleich oder verschieden voneinander und jeweils unabhängig Wasserstoff, Deuterium, eine Halogengruppe, eine Cyanogruppe, eine substituierte oder unsubstituierte Alkylgruppe, eine substituierte oder unsubstituierte Halogenalkylgruppe, eine substituierte oder unsubstituierte Cycloalkylgruppe, eine substituierte oder unsubstituierte Alkoxygruppe, eine substituierte oder unsubstituierte Halogenalkoxygruppe, eine substituierte oder unsubstituierte Alkylthioxygruppe, eine substituierte oder unsubstituierte Aryloxygruppe, eine substituierte oder unsubstituierte Arylthioxygruppe, eine substituierte oder unsubstituierte Alkenylgruppe, eine substituierte oder unsubstituierte Borgruppe, eine substituierte oder unsubstituierte Amingruppe eine substituierte oder unsubstituierte Arylalkylgruppe, eine substituierte oder unsubstituierte Silylgruppe, eine substituierte oder unsubstituierte Kohlenwasserstoffringgruppe oder eine substituierte oder unsubstituierte heterocyclische Gruppe oder binden an benachbarte Gruppen, um einen substituierten oder unsubstituierten Ring zu bilden, 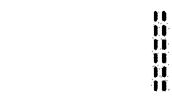
bedeutet eine Stelle, die an chemische Formel 1 bindet und
wobei "substituiert oder unsubstituiert" substituiert mit einem oder mehreren Substituenten, ausgewählt aus der Gruppe, bestehend aus Deuterium, einer Halogengruppe, einer Cyanogruppe, einer Alkylgruppe, einer Cycloalkylgruppe, einer Alkoxygruppe, einer Aryloxygruppe, einer Alkylthioxygruppe, einer Arylthioxygruppe, einer Alkenylgruppe, einer Halogenalkylgruppe, einer Halogenalkoxygruppe, einer Arylalkylgruppe, einer Silylgruppe, einer Borgruppe, einer Amingruppe, einer Arylgruppe, einer kondensierten Ringgruppe aus aromatischem Kohlenwasserstoff und aliphatischem Kohlenwasserstoff und einer heterocyclischen Gruppe, oder substituiert mit einem Substituenten, der zwei oder mehr Substituenten unter den oben veranschaulichten Substituenten verknüpft, oder ohne Substituenten bedeutet.

6. Verbindung nach Anspruch 1, wobei benachbarte Gruppen unter R1 bis R4 aneinander binden, um einen Ring zu bilden, der durch eine der folgenden chemischen Formeln B1-3 bis B1-5 dargestellt ist: 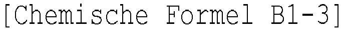 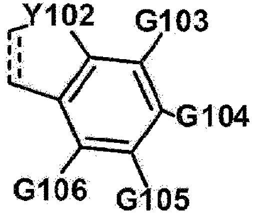 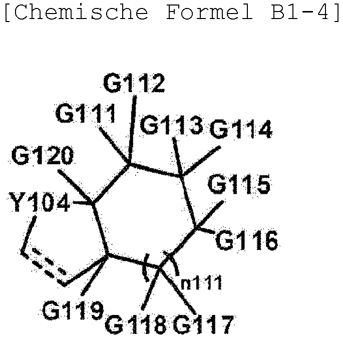 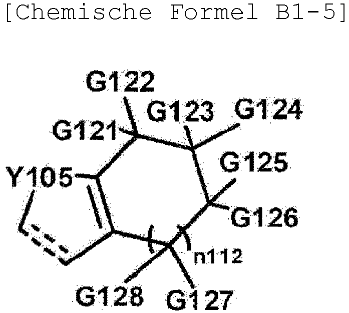 in den chemischen Formeln B1-3 bis B1-5
sind Y102, Y104 und Y105 gleich oder verschieden voneinander und jeweils unabhängig O, S oder NR‴,
R‴, G103 bis G106 und G111 bis G128 sind gleich oder verschieden voneinander und jeweils unabhängig Wasserstoff, Deuterium, eine Halogengruppe, eine Cyanogruppe, eine substituierte oder unsubstituierte Alkylgruppe, eine substituierte oder unsubstituierte Halogenalkylgruppe, eine substituierte oder unsubstituierte Cycloalkylgruppe, eine substituierte oder unsubstituierte Alkoxygruppe, eine substituierte oder unsubstituierte Halogenalkoxygruppe, eine substituierte oder unsubstituierte Alkylthioxygruppe, eine substituierte oder unsubstituierte Aryloxygruppe, eine substituierte oder unsubstituierte Arylthioxygruppe, eine substituierte oder unsubstituierte Alkenylgruppe, eine substituierte oder unsubstituierte Borgruppe, eine substituierte oder unsubstituierte Amingruppe eine substituierte oder unsubstituierte Arylalkylgruppe, eine substituierte oder unsubstituierte Silylgruppe, eine substituierte oder unsubstituierte Kohlenwasserstoffringgruppe oder eine substituierte oder unsubstituierte heterocyclische Gruppe oder binden an benachbarte Gruppen, um einen substituierten oder unsubstituierten Ring zu bilden,
n111 und n112 sind jeweils eine ganze Zahl von 0 bis 2,
wenn n111 und n112 2 sind, sind Strukturen in den beiden Klammern gleich oder verschieden voneinander, 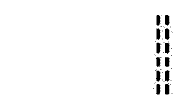
bedeutet eine Stelle, die an chemische Formel 1 bindet und
wobei "substituiert oder unsubstituiert" substituiert mit einem oder mehreren Substituenten, ausgewählt aus der Gruppe, bestehend aus Deuterium, einer Halogengruppe, einer Cyanogruppe, einer Alkylgruppe, einer Cycloalkylgruppe, einer Alkoxygruppe, einer Aryloxygruppe, einer Alkylthioxygruppe, einer Arylthioxygruppe, einer Alkenylgruppe, einer Halogenalkylgruppe, einer Halogenalkoxygruppe, einer Arylalkylgruppe, einer Silylgruppe, einer Borgruppe, einer Amingruppe, einer Arylgruppe, einer kondensierten Ringgruppe aus aromatischem Kohlenwasserstoff und aliphatischem Kohlenwasserstoff und einer heterocyclischen Gruppe, oder substituiert mit einem Substituenten, der zwei oder mehr Substituenten unter den oben veranschaulichten Substituenten verknüpft, oder ohne Substituenten bedeutet.

7. Verbindung nach Anspruch 1, worin R1 bis R7 gleich oder verschieden voneinander sind und jeweils unabhängig Wasserstoff, Deuterium, eine Cyanogruppe, eine lineare oder verzweigte Alkylgruppe mit 1 bis 30 Kohlenstoffatomen, eine monocyclische oder polycyclische Cycloalkylgruppe mit 3 bis 30 Kohlenstoffatomen, eine lineare oder verzweigte Alkylsilylgruppe mit 1 bis 30 Kohlenstoffatomen, eine lineare oder verzweigte Halogenalkylgruppe mit 1 bis 30 Kohlenstoffatomen, eine lineare oder verzweigte Halogenalkoxygruppe mit 1 bis 30 Kohlenstoffatomen, eine Arylalkylgruppe mit 6 bis 30 Kohlenstoffatomen, eine monocyclische oder polycyclische Diarylamingruppe mit 6 bis 30 Kohlenstoffatomen, eine monocyclische oder polycyclische Arylgruppe mit 6 bis 30 Kohlenstoffatomen oder eine monocyclische oder polycyclische heterocyclische Gruppe mit 2 bis 30 Kohlenstoffatomen sind und der Substituent unsubstituiert oder mit einem oder mehreren, ausgewählt aus der Gruppe, bestehend aus Deuterium, einer Halogengruppe, einer Cyanogruppe, einer linearen oder verzweigten Alkylgruppe mit 1 bis 30 Kohlenstoffatomen, einer linearen oder verzweigten Alkylsilylgruppe mit 1 bis 30 Kohlenstoffatomen, einer monocyclischen oder polycyclischen Arylgruppe mit 6 bis 30 Kohlenstoffatomen und Kombinationen davon, substituiert ist.

8. Verbindung nach Anspruch 1, wobei benachbarte Gruppen unter R1 bis R4 aneinander binden, um einen substituierten oder unsubstituierten monocyclischen oder polycyclischen Kohlenwasserstoffring mit 3 bis 30 Kohlenstoffatomen oder einen substituierten oder unsubstituierten monocyclischen oder polycyclischen Heteroring mit 2 bis 30 Kohlenstoffatomen zu bilden, und
wobei "substituiert oder unsubstituiert" substituiert mit einem oder mehreren Substituenten, ausgewählt aus der Gruppe, bestehend aus Deuterium, einer Halogengruppe, einer Cyanogruppe, einer Alkylgruppe, einer Cycloalkylgruppe, einer Alkoxygruppe, einer Aryloxygruppe, einer Alkylthioxygruppe, einer Arylthioxygruppe, einer Alkenylgruppe, einer Halogenalkylgruppe, einer Halogenalkoxygruppe, einer Arylalkylgruppe, einer Silylgruppe, einer Borgruppe, einer Amingruppe, einer Arylgruppe, einer kondensierten Ringgruppe aus aromatischem Kohlenwasserstoff und aliphatischem Kohlenwasserstoff und einer heterocyclischen Gruppe, oder substituiert mit einem Substituenten, der zwei oder mehr Substituenten unter den oben veranschaulichten Substituenten verknüpft, oder ohne Substituenten bedeutet.

9. Verbindung nach Anspruch 1, worin R' und R" gleich oder verschieden voneinander sind und jeweils unabhängig eine lineare oder verzweigte Alkylgruppe mit 1 bis 30 Kohlenstoffatomen, eine Arylgruppe mit 6 bis 30 Kohlenstoffatomen, ein monocyclischer oder polycyclischer Kohlenwasserstoffring mit 3 bis 30 Kohlenstoffatomen Gruppe oder eine monocyclische oder polycyclische heterocyclische Gruppe mit 2 bis 30 Kohlenstoffatomen sind und der Substituent unsubstituiert oder substituiert mit einem oder mehreren, ausgewählt aus der Gruppe, bestehend aus Deuterium, einer Halogengruppe, einer Cyanogruppe, einer linearen oder verzweigten Alkylgruppe mit 1 bis 30 Kohlenstoffatomen, einer monocyclischen oder polycyclischen Cycloalkylgruppe mit 3 bis 30 Kohlenstoffatomen, einer linearen oder verzweigten Alkylsilylgruppe mit 1 bis 30 Kohlenstoffatome, einer Arylsilylgruppe mit 3 bis 30 Kohlenstoffatomen, einer linearen oder verzweigten Alkoxygruppe mit 1 bis 30 Kohlenstoffatome, einer linearen oder verzweigten Halogenalkylgruppe mit 1 bis 30 Kohlenstoffatome, einer linearen oder verzweigten Halogenalkoxygruppe mit 1 bis 30 Kohlenstoffatomen, einer Aryloxygruppe mit 6 bis 30 Kohlenstoffatomen, einer monocyclischen oder polycyclischen Arylalkylgruppe mit 6 bis 30 Kohlenstoffatomen, einer monocyclischen oder polycyclischen Diarylamingruppe mit 6 bis 30 Kohlenstoffatomen, einer monocyclischen oder polycyclischen Arylgruppe mit 6 bis 30 Kohlenstoffatomen, einer monocyclischen oder polycyclischen heterocyclischen Gruppe mit 2 bis 30 Kohlenstoffatomen und Kombinationen davon, substituiert ist.

10. Verbindung nach Anspruch 1, wobei die chemische Formel 1 eine, ausgewählt unter den folgenden Verbindungen, ist: 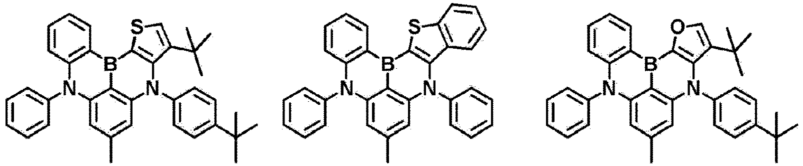 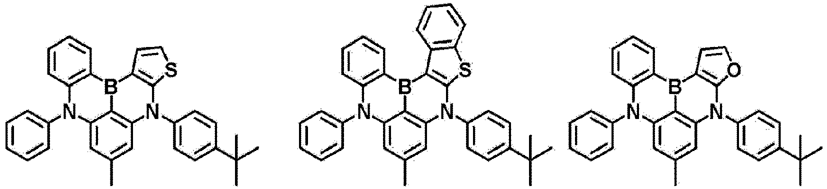 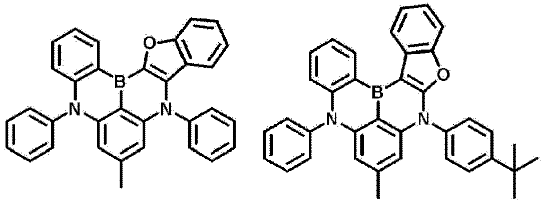 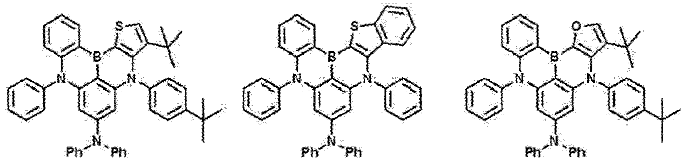 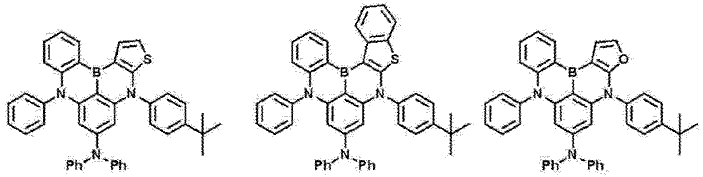 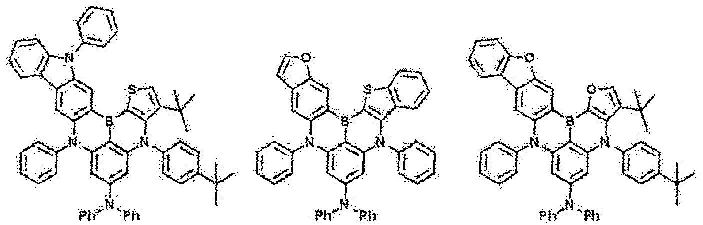 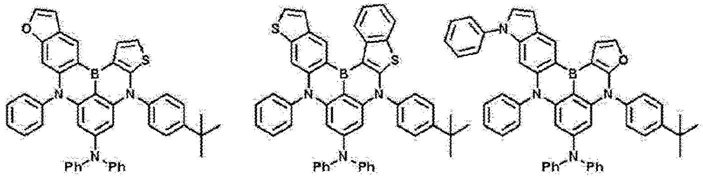 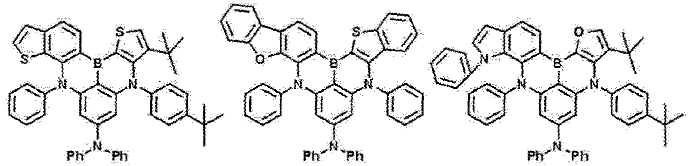 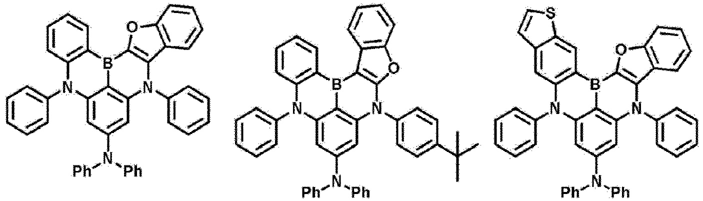 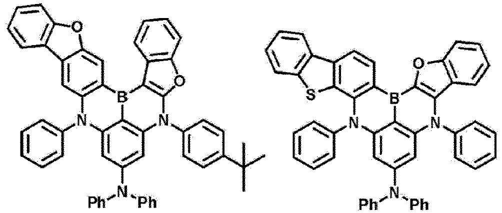 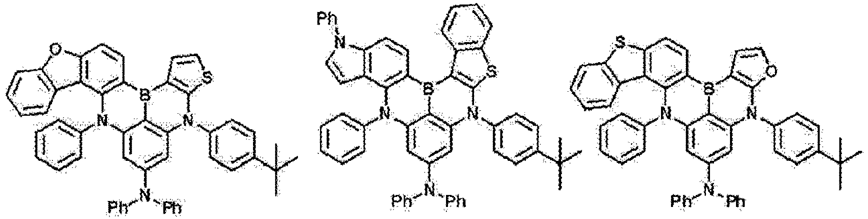 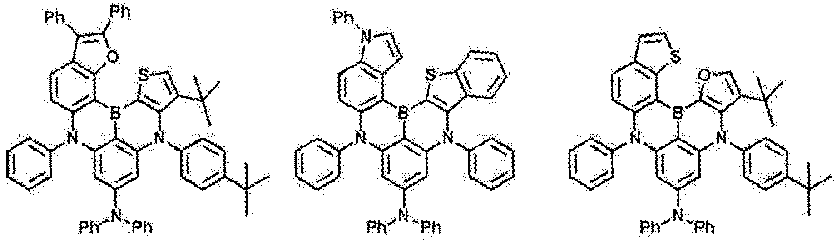 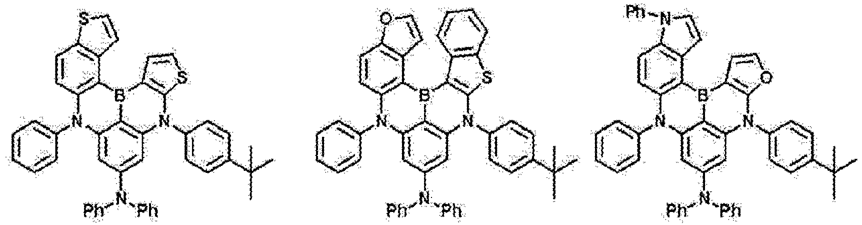 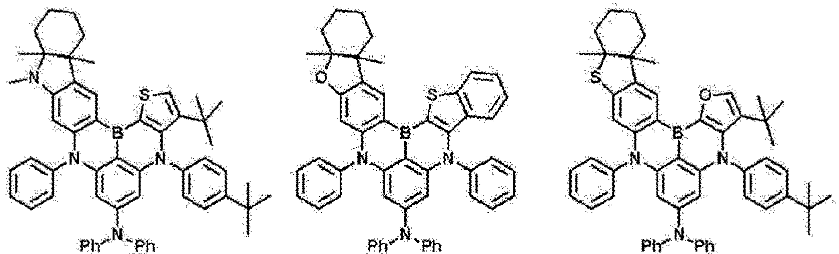 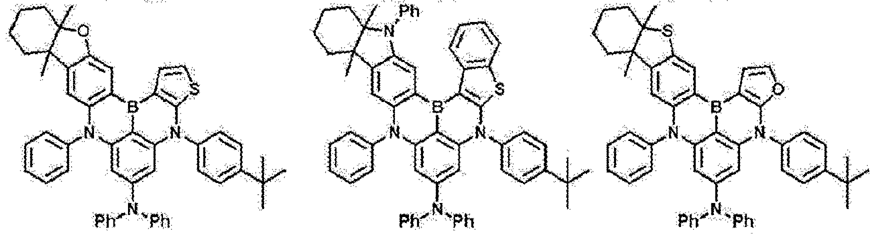 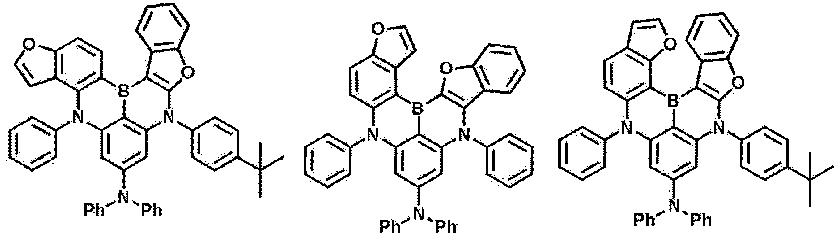 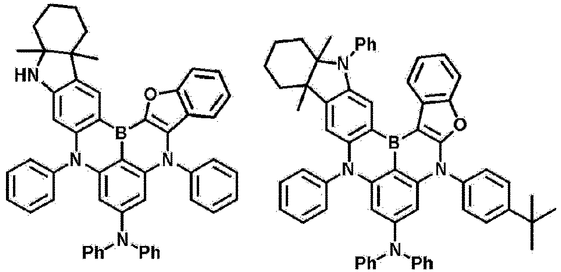 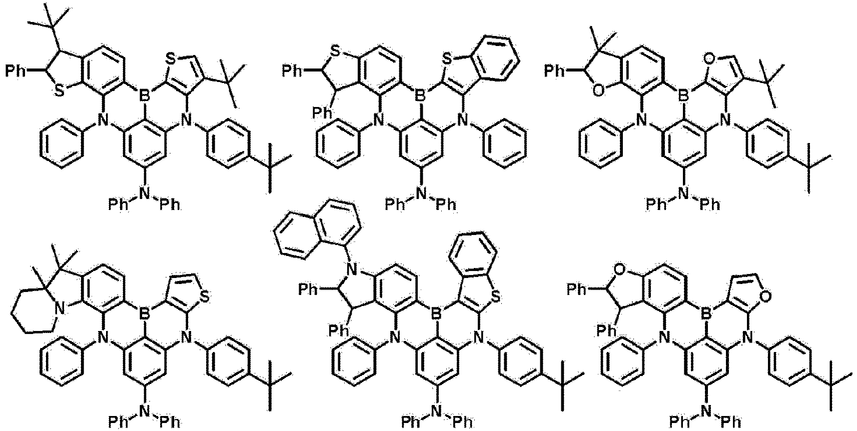 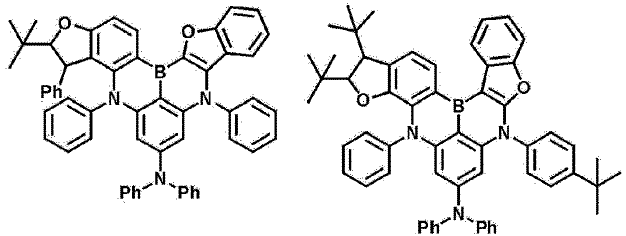 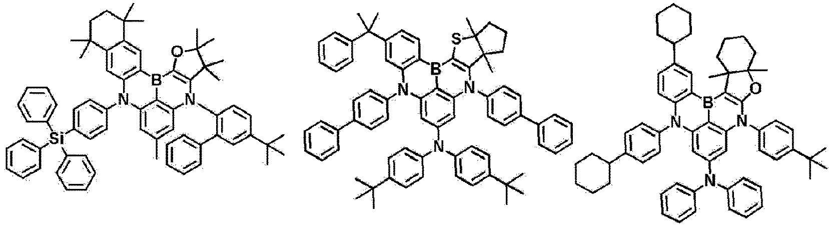 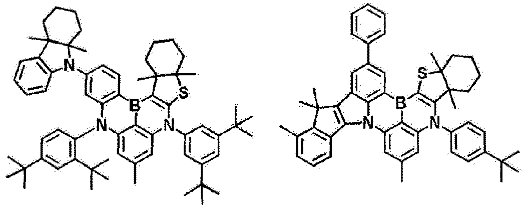 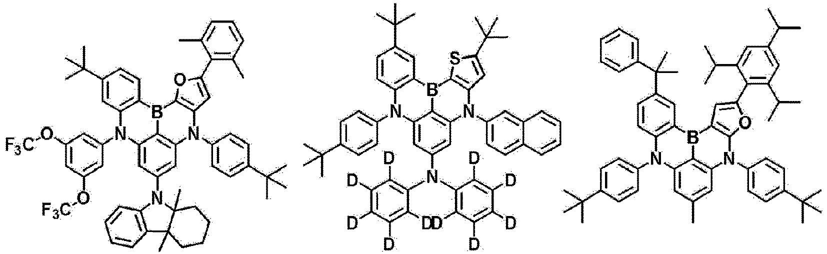 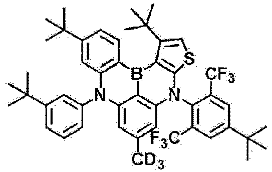 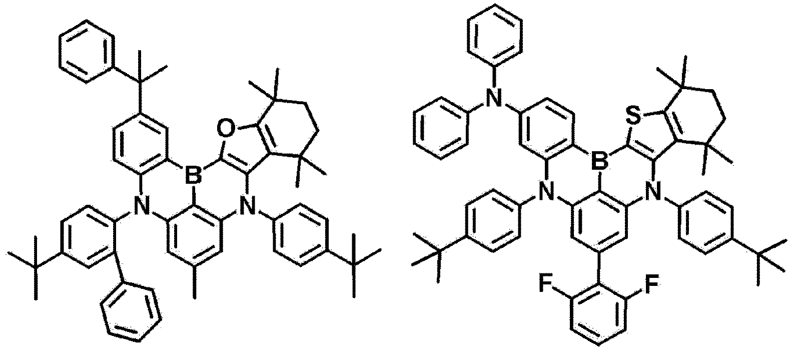 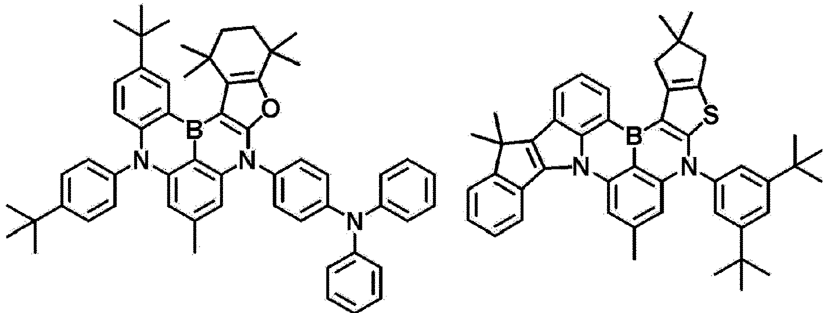 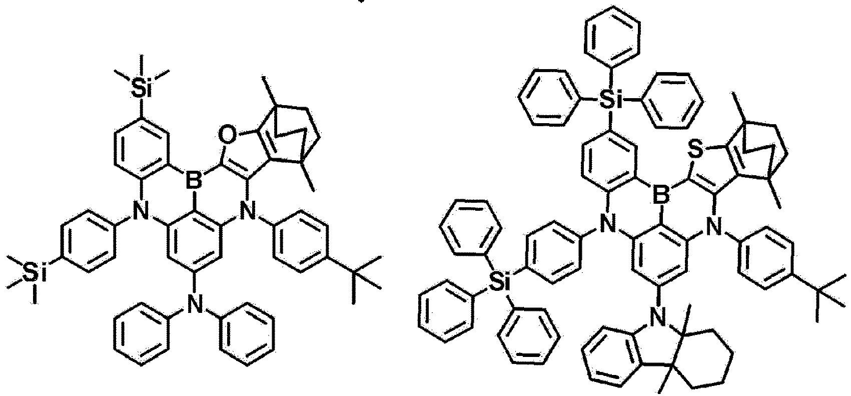 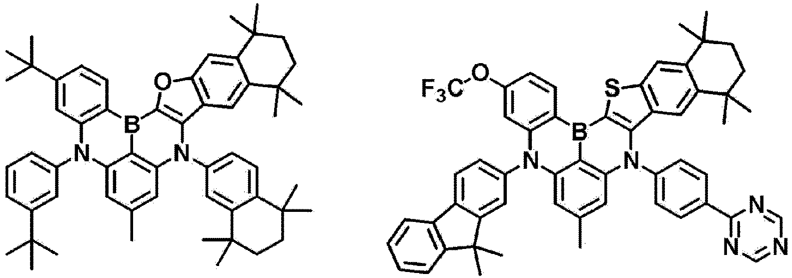 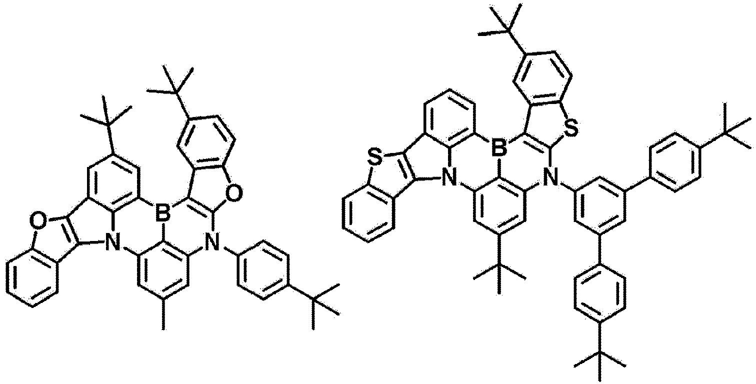 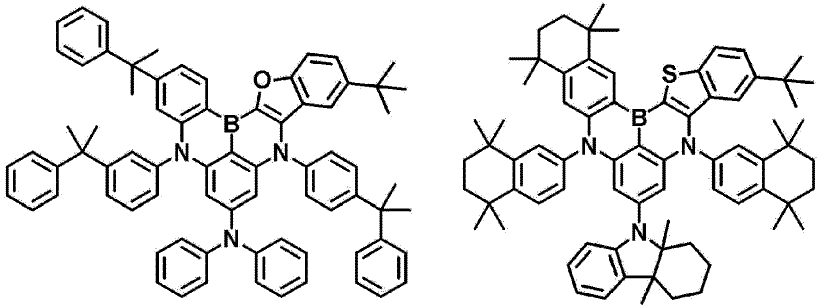 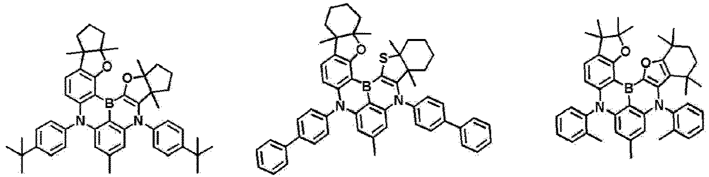 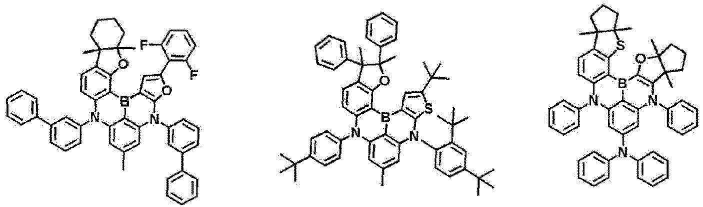 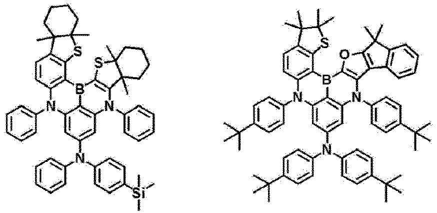 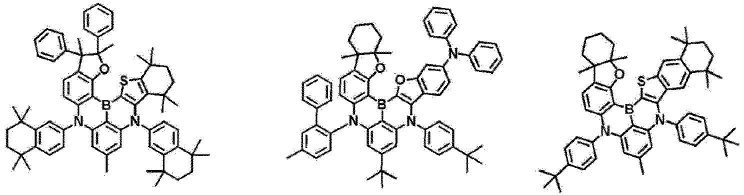 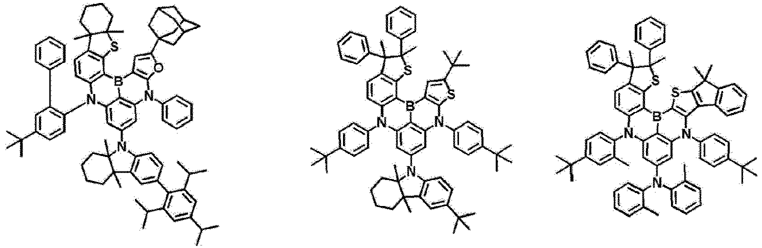 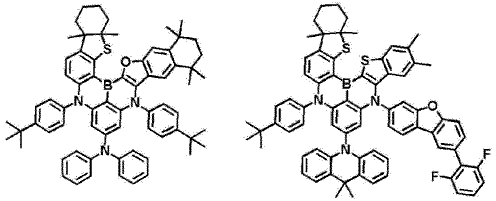 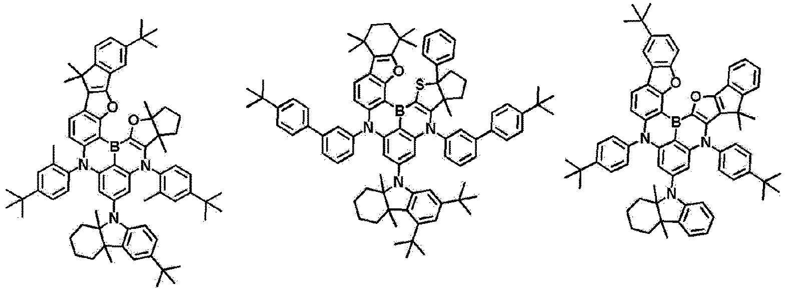 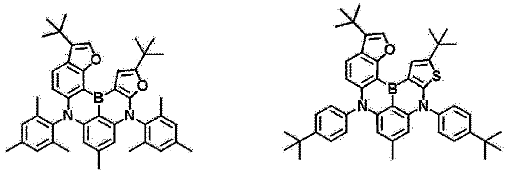 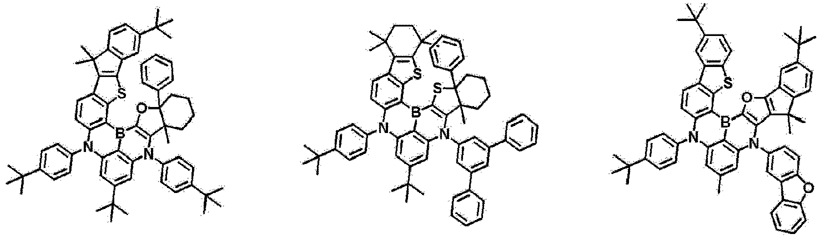 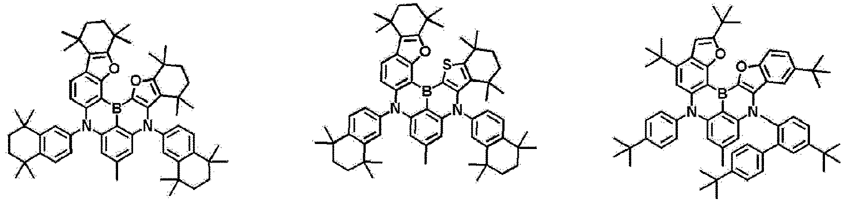 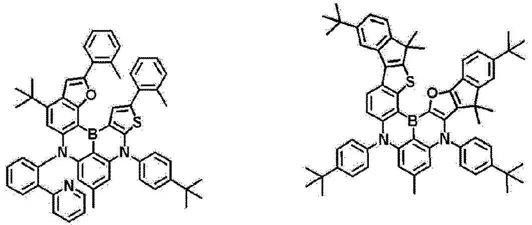 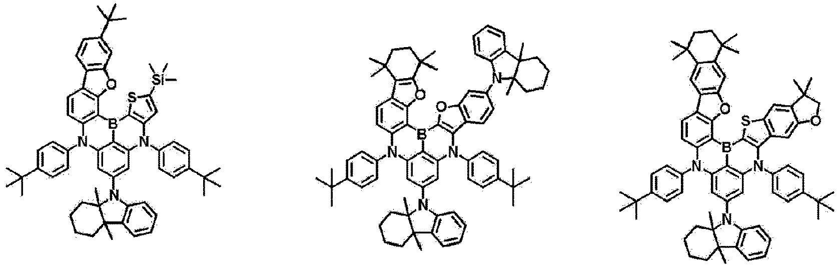 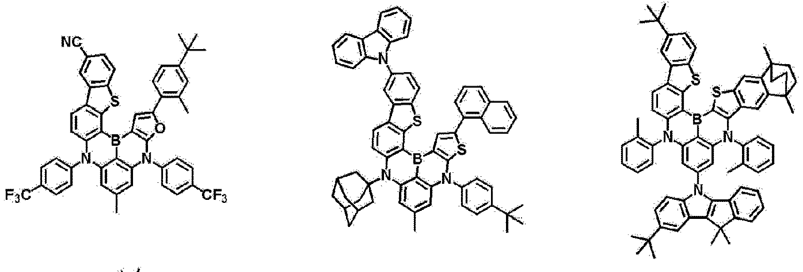 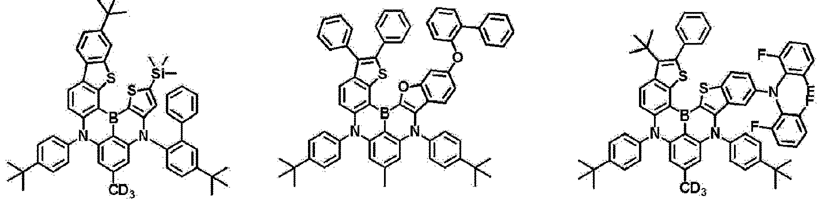 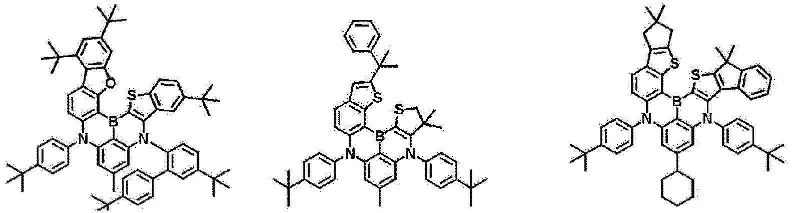 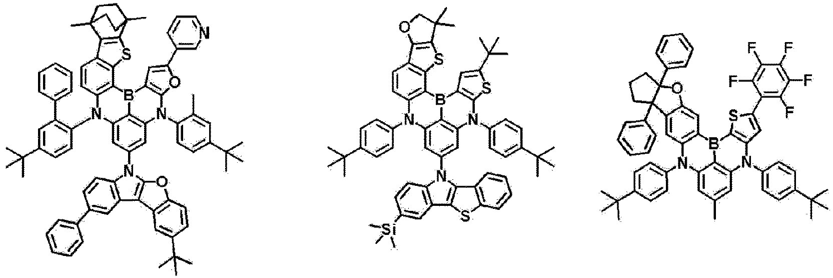 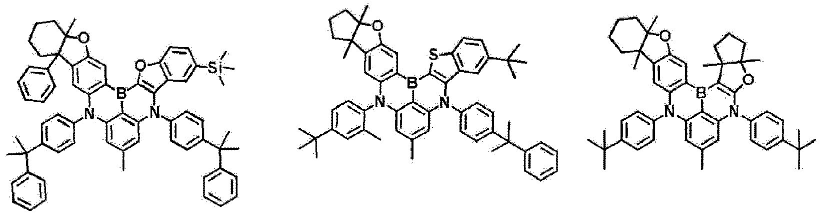 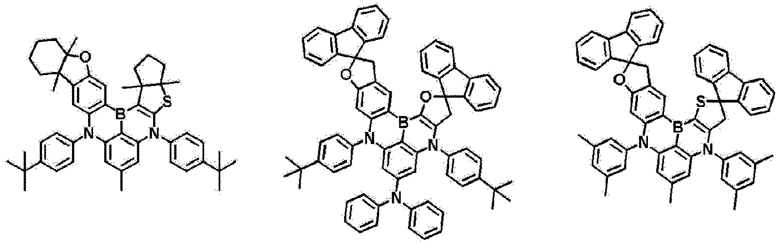 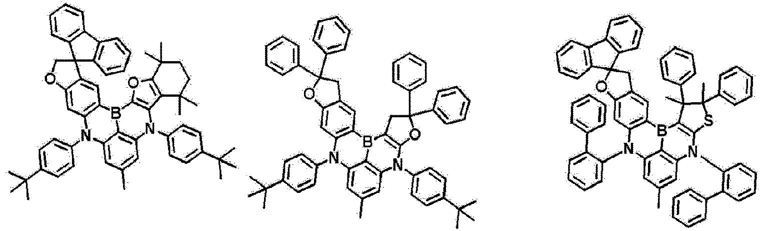 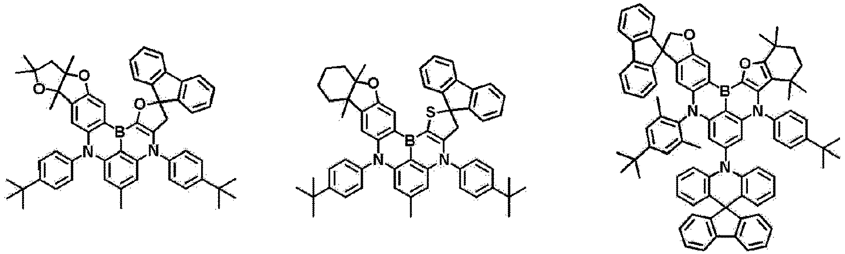 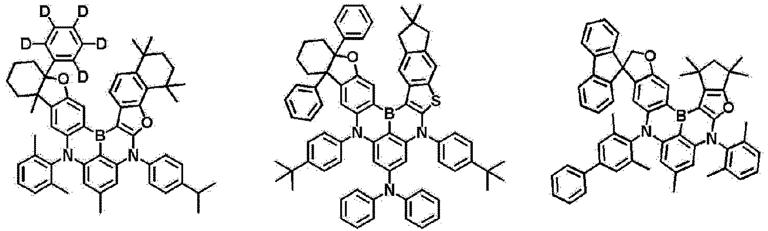 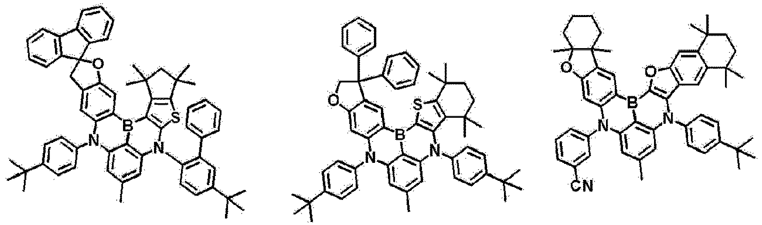 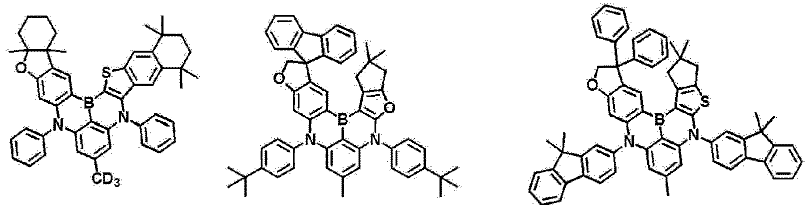 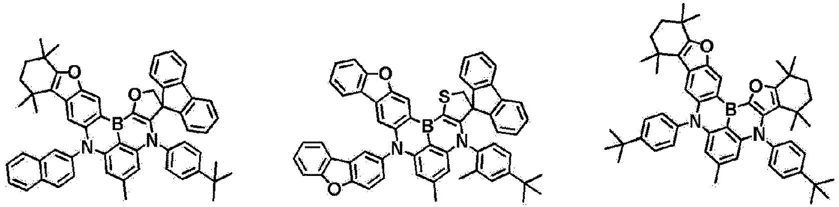 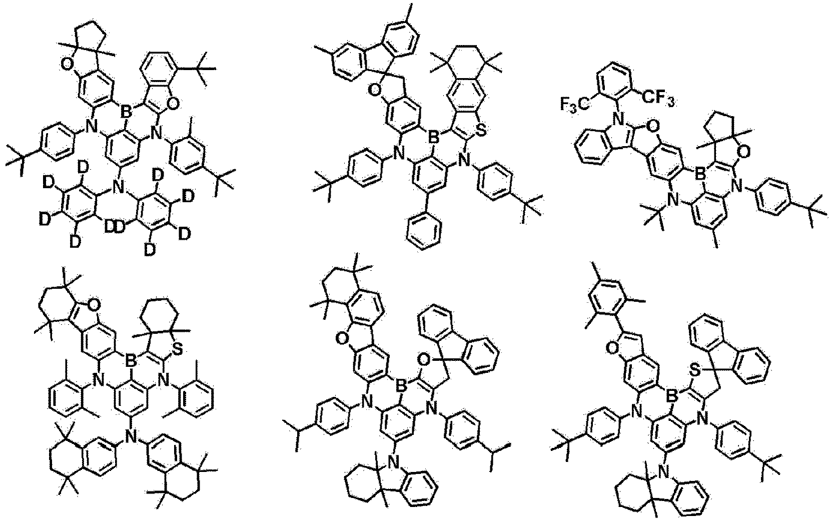 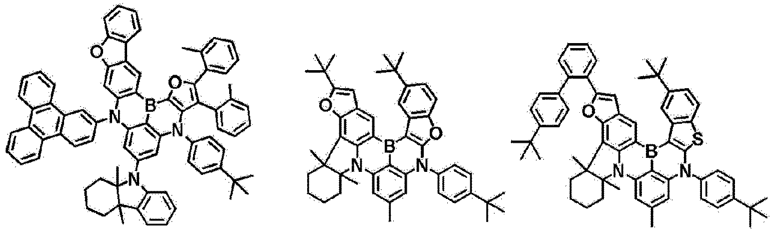 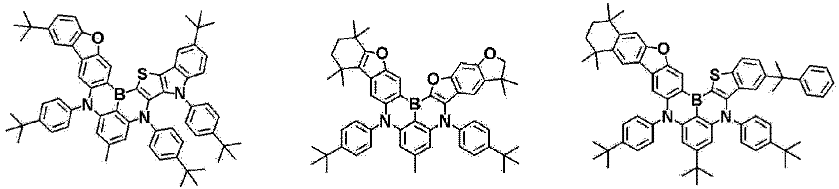 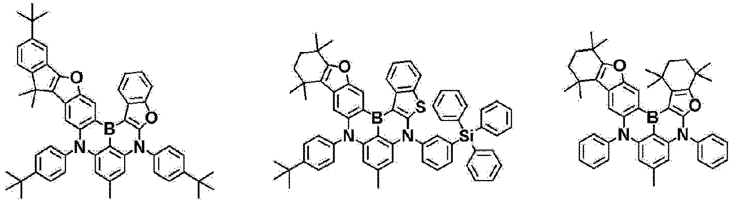 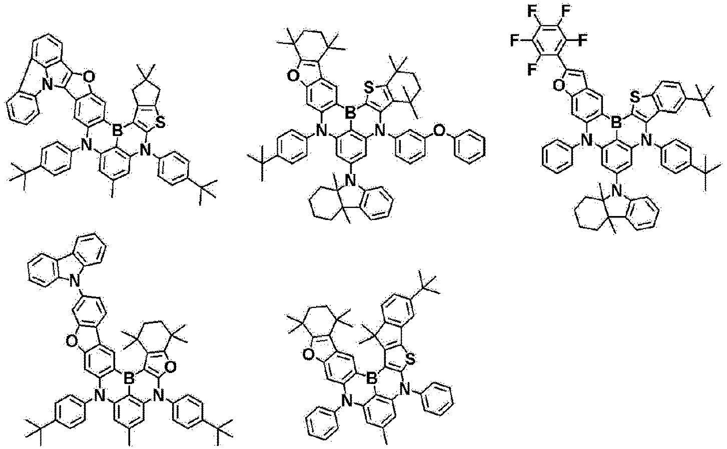 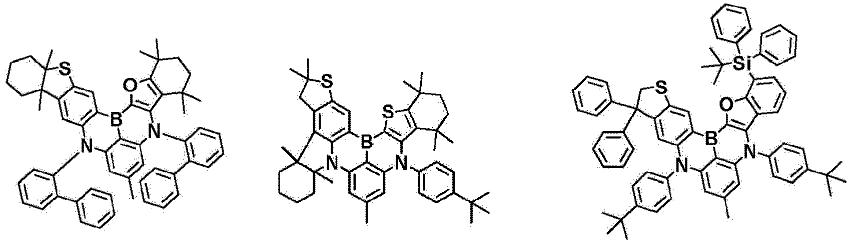 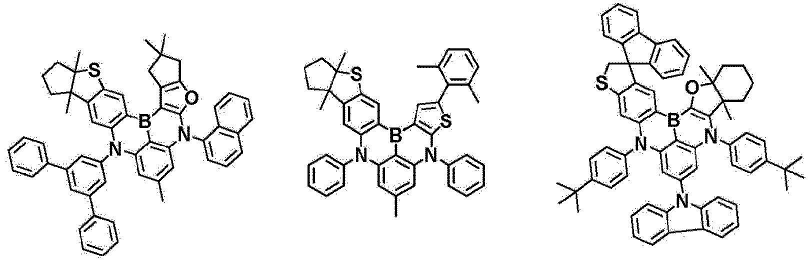 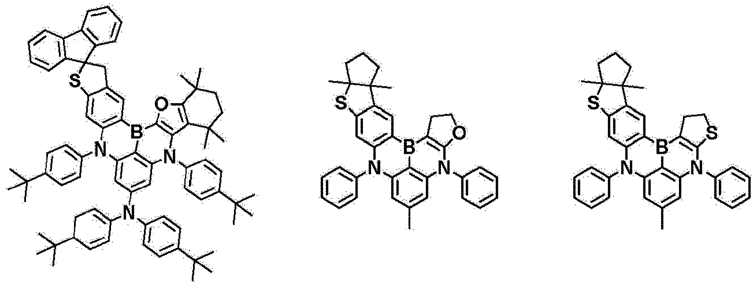 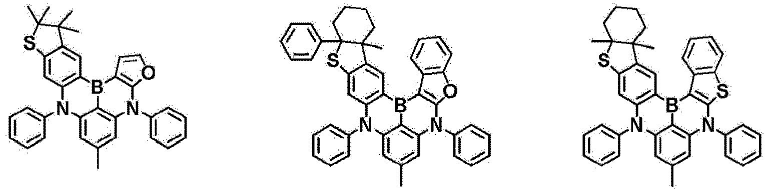 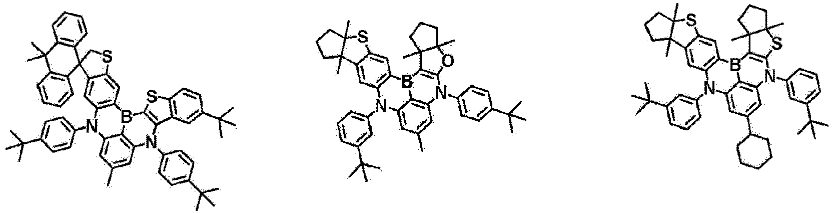 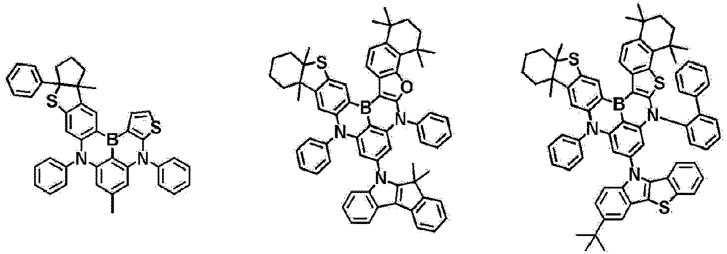 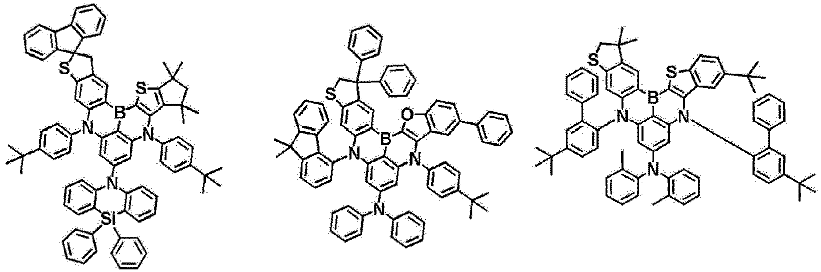 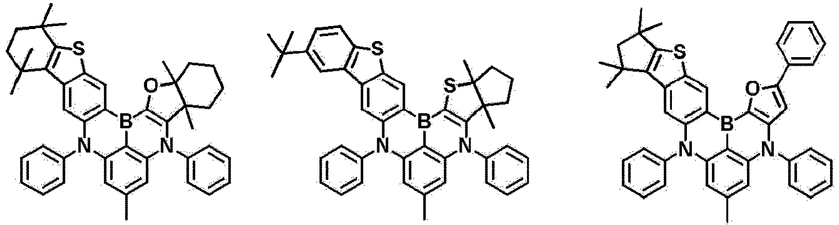 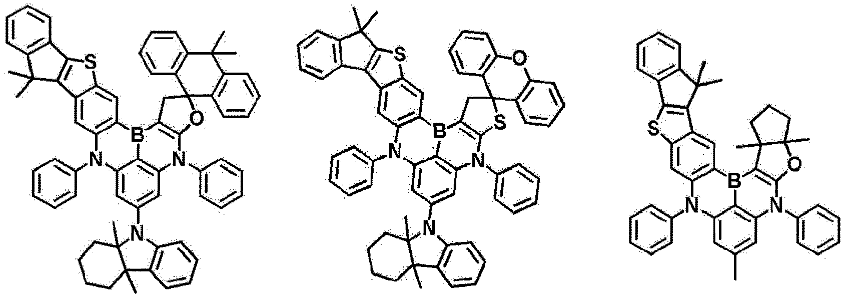 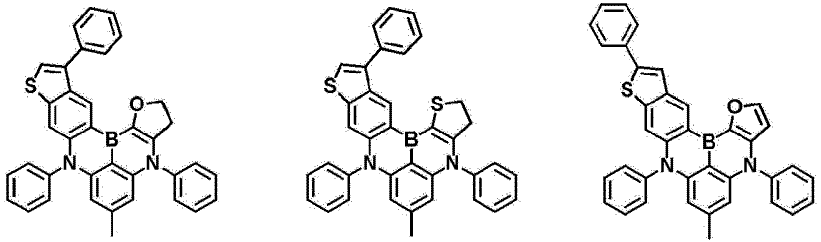 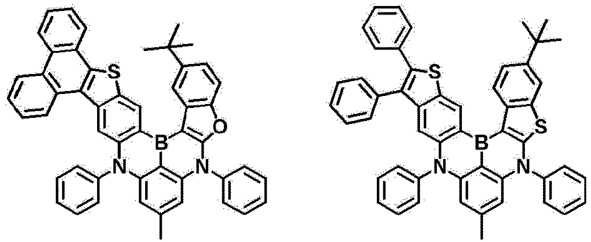 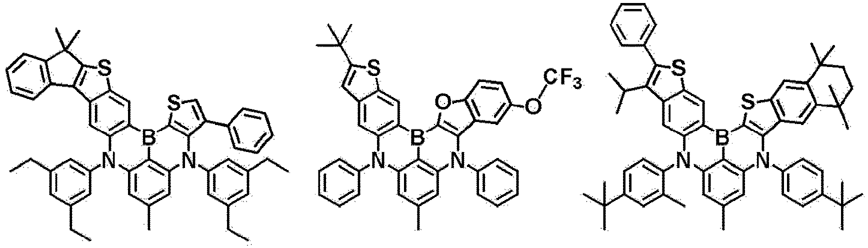 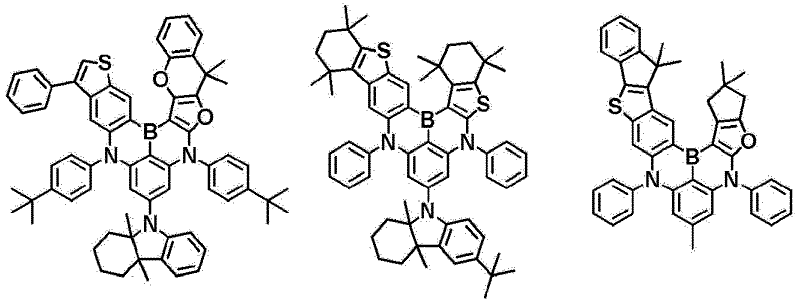 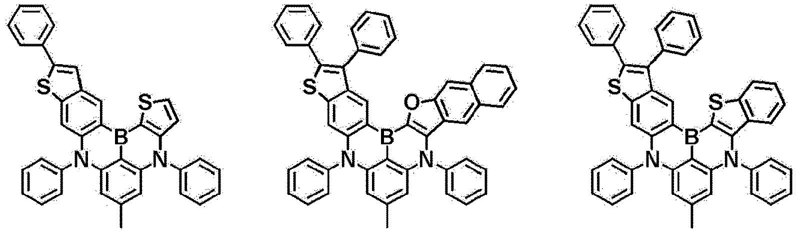 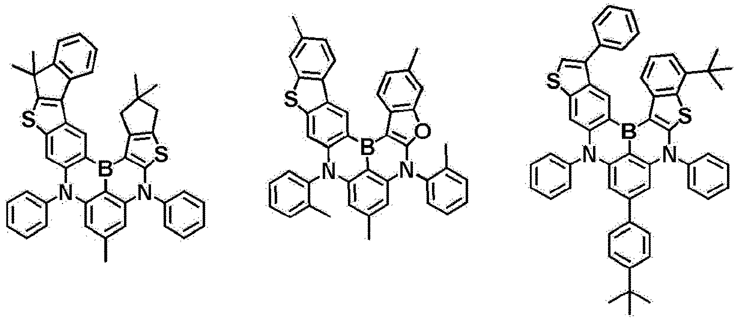 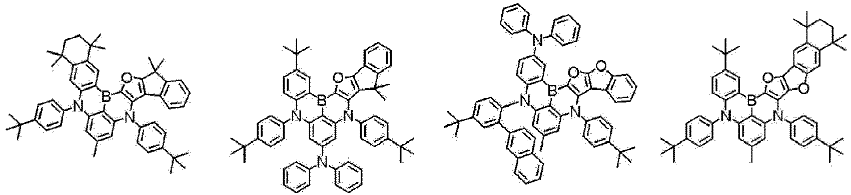 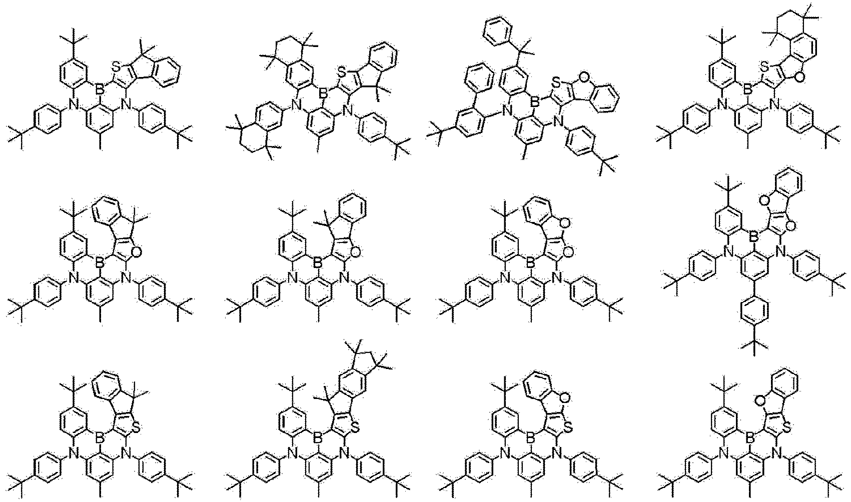 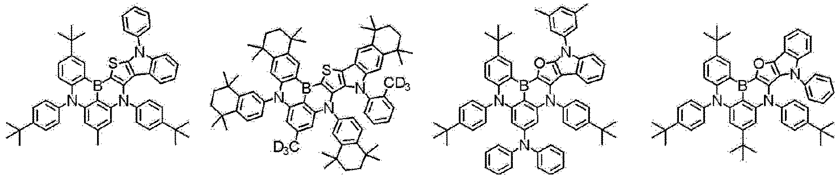 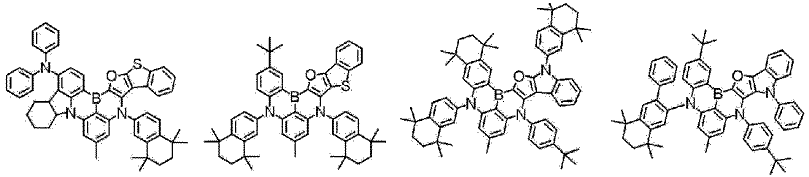 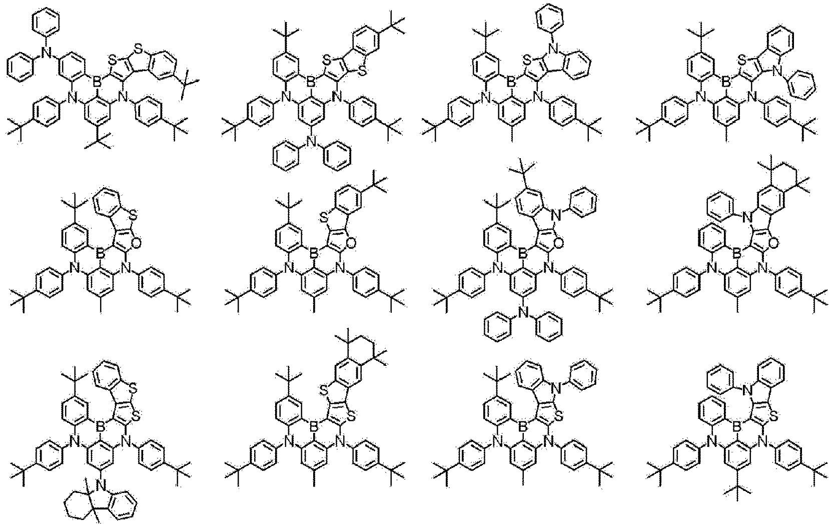 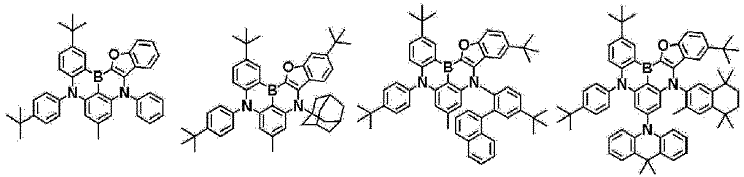 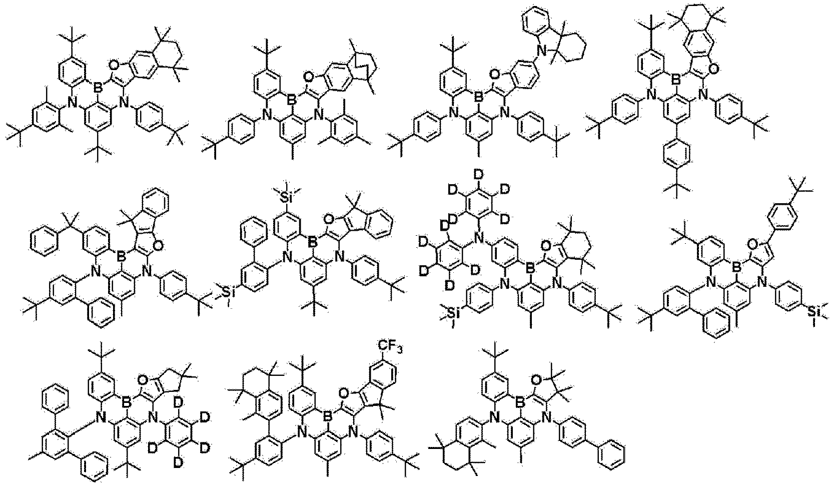 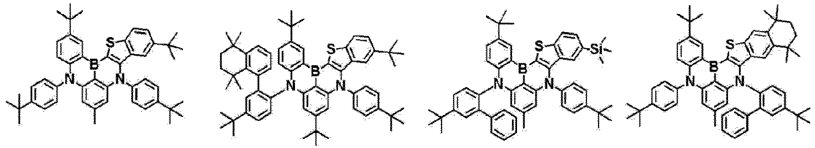 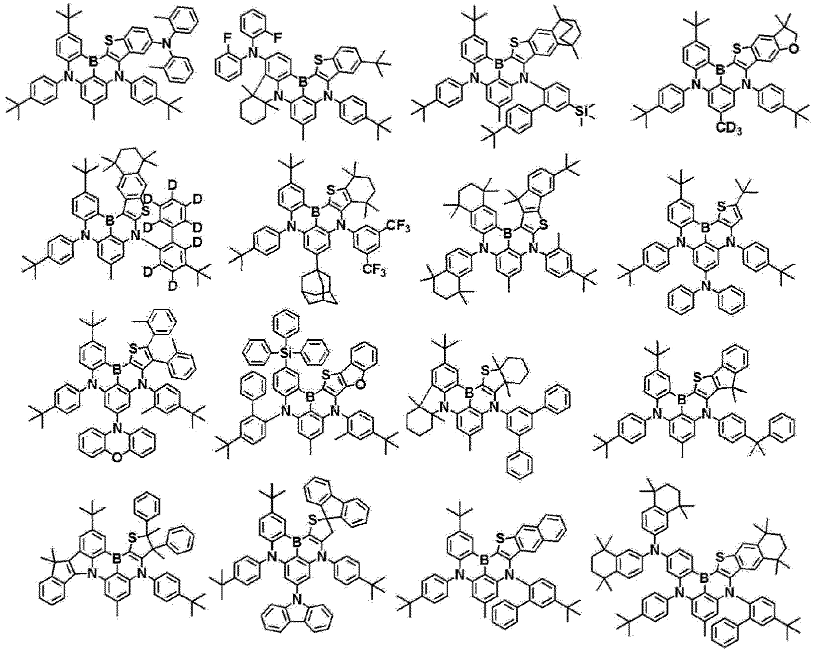 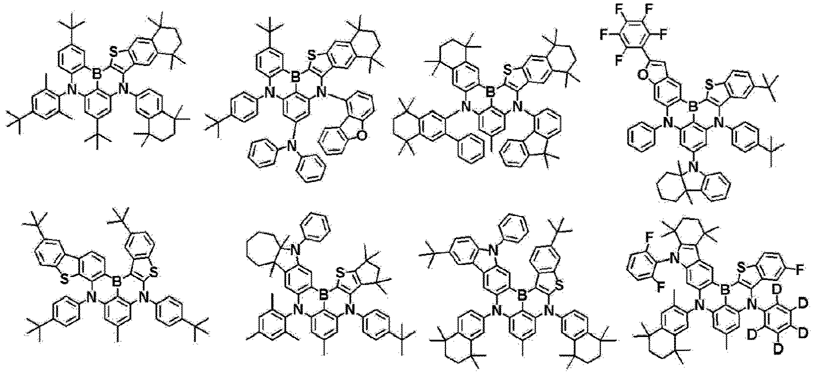 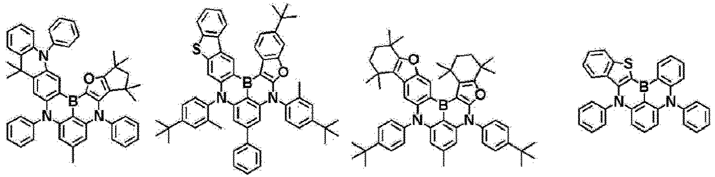 in den Verbindungen
bedeutet Ph eine Phenylgruppe.

11. Organische lichtemittierende Vorrichtung, umfassend:
eine erste Elektrode,
eine zweite Elektrode und
eine oder mehrere organische Materialschichten, die sich zwischen der ersten Elektrode und der zweiten Elektrode befinden,
wobei eine oder mehrere Schichten der organischen Materialschichten die Verbindung nach einem der Ansprüche 1 bis 10 einschließen.

12. Organische lichtemittierende Vorrichtung nach Anspruch 11, wobei die organische Materialschicht eine lichtemittierende Schicht einschließt und die lichtemittierende Schicht die Verbindung einschließt.

13. Organische lichtemittierende Vorrichtung nach Anspruch 11, wobei die organische Materialschicht eine lichtemittierende Schicht einschließt und die lichtemittierende Schicht ein Dotierungsmaterial einschließt und das Dotierungsmaterial die Verbindung einschließt.

14. Organische lichtemittierende Vorrichtung nach Anspruch 11, wobei die organische Materialschicht eine lichtemittierende Schicht einschließt und die lichtemittierende Schicht ferner eine durch die folgende chemische Formel H dargestellte Verbindung einschließt: 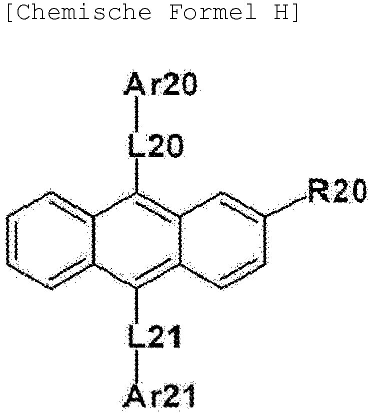 in der chemischen Formel H
sind L20 und L21 gleich oder verschieden voneinander und jeweils unabhängig eine direkte Bindung, eine substituierte oder unsubstituierte Arylengruppe oder eine substituierte oder unsubstituierte Heteroarylengruppe,
Ar20 und Ar21 sind gleich oder verschieden voneinander und jeweils unabhängig Wasserstoff, Deuterium, eine substituierte oder unsubstituierte Arylgruppe oder eine substituierte oder unsubstituierte heterocyclische Gruppe,
R20 ist Wasserstoff, Deuterium, eine Halogengruppe, eine substituierte oder unsubstituierte Arylgruppe, eine substituierte oder unsubstituierte Cycloalkylgruppe, eine substituierte oder unsubstituierte Arylgruppe oder eine substituierte oder unsubstituierte heterocyclische Gruppe und
wobei "substituiert oder unsubstituiert" substituiert mit einem oder mehreren Substituenten, ausgewählt aus der Gruppe, bestehend aus Deuterium, einer Halogengruppe, einer Cyanogruppe, einer Alkylgruppe, einer Cycloalkylgruppe, einer Alkoxygruppe, einer Aryloxygruppe, einer Alkylthioxygruppe, einer Arylthioxygruppe, einer Alkenylgruppe, einer Halogenalkylgruppe, einer Halogenalkoxygruppe, einer Arylalkylgruppe, einer Silylgruppe, einer Borgruppe, einer Amingruppe, einer Arylgruppe, einer kondensierten Ringgruppe aus aromatischem Kohlenwasserstoff und aliphatischem Kohlenwasserstoff und einer heterocyclischen Gruppe, oder substituiert mit einem Substituenten, der zwei oder mehr Substituenten unter den oben veranschaulichten Substituenten verknüpft, oder ohne Substituenten bedeutet.

15. Organische lichtemittierende Vorrichtung nach Anspruch 11, wobei die organische Materialschicht eine lichtemittierende Schicht einschließt und die lichtemittierende Schicht zwei oder mehr Typen von Wirten und ein Dotiermittel einschließt.

## Revendications

1. Composé représenté par la formule chimique 1 suivante : 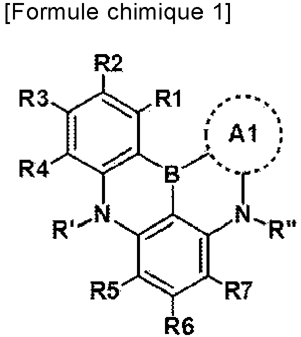 dans la formule chimique 1,
A1 est un hétérocycle monocyclique ou polycyclique substitué ou non substitué, et incluant un cycle à 5 membres incluant O ou S ;
R1 à R7, R' et R" sont identiques ou différents les uns des autres et chacun indépendamment de l'hydrogène ; du deutérium ; un groupe halogène ; un groupe cyano ; un groupe alkyle substitué ou non substitué ; un groupe halogénoalkyle substitué ou non substitué ; un groupe cycloalkyle substitué ou non substitué ; un groupe alcoxy substitué ou non substitué ; un groupe halogénoalcoxy substitué ou non substitué ; un groupe alkylthioxy substitué ou non substitué ; un groupe aryloxy substitué ou non substitué ; un groupe arylthioxy substitué ou non substitué ; un groupe alcényle substitué ou non substitué ; un groupe bore substitué ou non substitué ; un groupe amine substitué ou non substitué ; un groupe arylalkyle substitué ou non substitué ; un groupe silyle substitué ou non substitué ; un groupe cycle hydrocarboné substitué ou non substitué ; ou un groupe hétérocyclique substitué ou non substitué, ou se lient à des groupes adjacents pour former un cycle substitué ou non substitué, et
dans lequel "substitué ou non substitué" signifie étant substitué par un ou plusieurs substituants sélectionnés dans le groupe consistant en du deutérium ; un groupe halogène ; un groupe cyano ; un groupe alkyle ; un groupe cycloalkyle ; un groupe alcoxy ; un groupe aryloxy ; un groupe alkylthioxy ; un groupe arylthioxy ; un groupe alcényle ; un groupe halogénoalkyle ; un groupe halogénoalcoxy ; un groupe arylalkyle ; un groupe silyle ; un groupe bore ; un groupe amine ; un groupe aryle ; un groupe cycle fusionné d'hydrocarbures aromatiques et d'hydrocarbures aliphatiques ; et un groupe hétérocyclique, ou étant substitué par un substituant liant deux substituants ou plus parmi les substituants illustrés ci-dessus, ou ne présentant aucun substituant.

2. Composé selon la revendication 1, dans lequel A1 est représenté par la formule chimique A1-1 ou A1-2 suivante : 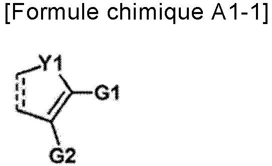 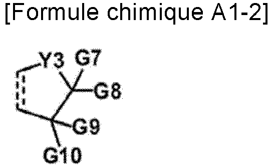 dans les formules chimiques A1-1 et A1-2,
Y1 et Y3 sont identiques ou différents l'un de l'autre, et chacun indépendamment O ; ou S ;
G1, G2 et G7 à G10 sont identiques ou différents les uns des autres et chacun indépendamment de l'hydrogène ; du deutérium ; un groupe halogène ; un groupe cyano ; un groupe alkyle substitué ou non substitué ; un groupe halogénoalkyle substitué ou non substitué ; un groupe cycloalkyle substitué ou non substitué ; un groupe alcoxy substitué ou non substitué ; un groupe halogénoalcoxy substitué ou non substitué ; un groupe alkylthioxy substitué ou non substitué ; un groupe aryloxy substitué ou non substitué ; un groupe arylthioxy substitué ou non substitué ; un groupe alcényle substitué ou non substitué ; un groupe bore substitué ou non substitué ; un groupe amine substitué ou non substitué ; un groupe arylalkyle substitué ou non substitué ; un groupe silyle substitué ou non substitué ; un groupe cycle hydrocarboné substitué ou non substitué ; ou un groupe hétérocyclique substitué ou non substitué, ou se lient à des groupes adjacents pour former un cycle substitué ou non substitué ; 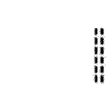
désigne un site se liant à la formule chimique 1, et
dans lequel "substitué ou non substitué" signifie étant substitué par un ou plusieurs substituants sélectionnés dans le groupe consistant en du deutérium ; un groupe halogène ; un groupe cyano ; un groupe alkyle ; un groupe cycloalkyle ; un groupe alcoxy ; un groupe aryloxy ; un groupe alkylthioxy ; un groupe arylthioxy ; un groupe alcényle ; un groupe halogénoalkyle ; un groupe halogénoalcoxy ; un groupe arylalkyle ; un groupe silyle ; un groupe bore ; un groupe amine ; un groupe aryle ; un groupe cycle fusionné d'hydrocarbures aromatiques et d'hydrocarbures aliphatiques ; et un groupe hétérocyclique, ou étant substitué par un substituant liant deux substituants ou plus parmi les substituants illustrés ci-dessus, ou ne présentant aucun substituant.

3. Composé selon la revendication 1, dans lequel A1 est représentée par l'une quelconque des formules chimiques A1-3 à A1-5 suivantes : 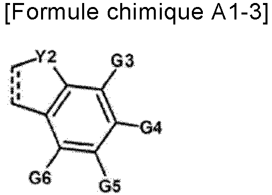 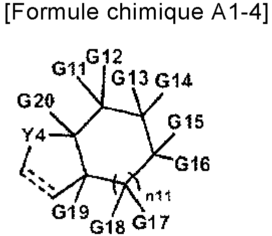 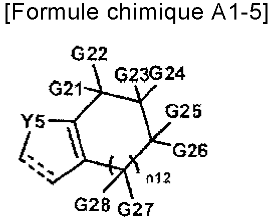
dans les formules chimiques A1-3 à A1-5,
Y2, Y4 et Y5 sont identiques ou différents les uns des autres, et chacun indépendamment O ; ou S ;
G3 à G6 et G11 à G28 sont identiques ou différents les uns des autres et chacun indépendamment de l'hydrogène ; du deutérium ; un groupe halogène ; un groupe cyano ; un groupe alkyle substitué ou non substitué ; un groupe halogénoalkyle substitué ou non substitué ; un groupe cycloalkyle substitué ou non substitué ; un groupe alcoxy substitué ou non substitué ; un groupe halogénoalcoxy substitué ou non substitué ; un groupe alkylthioxy substitué ou non substitué ; un groupe aryloxy substitué ou non substitué ; un groupe arylthioxy substitué ou non substitué ; un groupe alcényle substitué ou non substitué ; un groupe bore substitué ou non substitué ; un groupe amine substitué ou non substitué ; un groupe arylalkyle substitué ou non substitué ; un groupe silyle substitué ou non substitué ; un groupe cycle hydrocarboné substitué ou non substitué ; ou un groupe hétérocyclique substitué ou non substitué, ou se lient à des groupes adjacents pour former un cycle substitué ou non substitué ;
n11 et n12 sont chacun un nombre entier de 0 à 2 ;
lorsque n11 et n12 valent 2, des structures dans les deux parenthèses sont identiques ou différentes l'une de l'autre ; 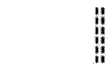
désigne un site se liant à la formule chimique 1, et dans lequel "substitué ou non substitué" signifie étant substitué par un ou plusieurs substituants sélectionnés dans le groupe consistant en du deutérium ; un groupe halogène ; un groupe cyano ; un groupe alkyle ; un groupe cycloalkyle ; un groupe alcoxy ; un groupe aryloxy ; un groupe alkylthioxy ; un groupe arylthioxy ; un groupe alcényle ; un groupe halogénoalkyle ; un groupe halogénoalcoxy ; un groupe arylalkyle ; un groupe silyle ; un groupe bore ; un groupe amine ; un groupe aryle ; un groupe cycle fusionné d'hydrocarbures aromatiques et d'hydrocarbures aliphatiques ; et un groupe hétérocyclique, ou étant substitué par un substituant liant deux substituants ou plus parmi les substituants illustrés ci-dessus, ou ne présentant aucun substituant.

4. Composé selon la revendication 1, dans lequel la formule chimique 1 est représentée par l'une quelconque des formules chimiques 1-1 à 1-5 suivantes : 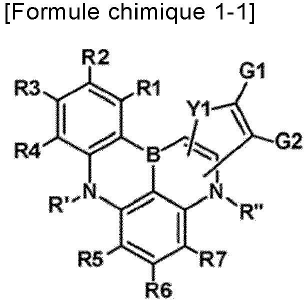 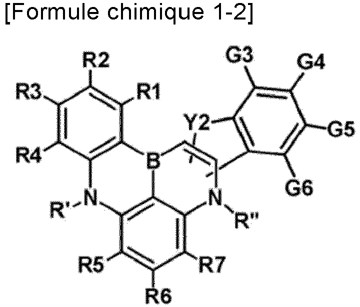 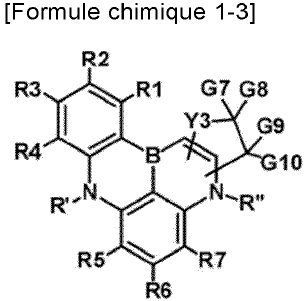 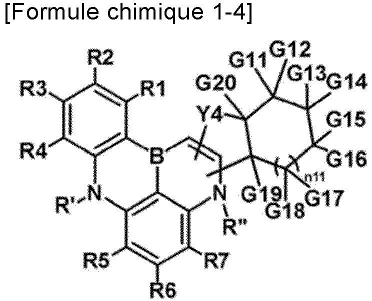 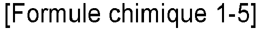 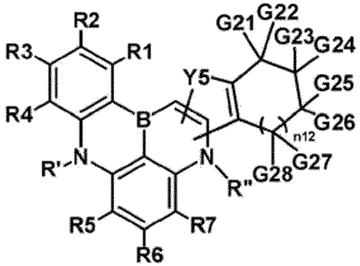 dans les formules chimiques 1-1 à 1-5,
R', R" et R1 à R7 présentent les mêmes définitions que dans la formule chimique 1 ;
Y1 à Y5 sont identiques ou différents les uns des autres, et chacun indépendamment O ; ou S ;
G1 à G28 sont identiques ou différents les uns des autres et chacun indépendamment de l'hydrogène ; du deutérium ; un groupe halogène ; un groupe cyano ; un groupe alkyle substitué ou non substitué ; un groupe halogénoalkyle substitué ou non substitué ; un groupe cycloalkyle substitué ou non substitué ; un groupe alcoxy substitué ou non substitué ; un groupe halogénoalcoxy substitué ou non substitué ; un groupe alkylthioxy substitué ou non substitué ; un groupe aryloxy substitué ou non substitué ; un groupe arylthioxy substitué ou non substitué ; un groupe alcényle substitué ou non substitué ; un groupe bore substitué ou non substitué ; un groupe amine substitué ou non substitué ; un groupe arylalkyle substitué ou non substitué ; un groupe silyle substitué ou non substitué ; un groupe cycle hydrocarboné substitué ou non substitué ; ou un groupe hétérocyclique substitué ou non substitué, ou se lient à des groupes adjacents pour former un cycle substitué ou non substitué ;
n11 et n12 sont chacun un nombre entier de 0 à 2 ;
lorsque n11 et n12 valent 2, des structures dans les deux parenthèses sont identiques ou différentes l'une de l'autre, et
dans lequel "substitué ou non substitué" signifie étant substitué par un ou plusieurs substituants sélectionnés dans le groupe consistant en du deutérium ; un groupe halogène ; un groupe cyano ; un groupe alkyle ; un groupe cycloalkyle ; un groupe alcoxy ; un groupe aryloxy ; un groupe alkylthioxy ; un groupe arylthioxy ; un groupe alcényle ; un groupe halogénoalkyle ; un groupe halogénoalcoxy ; un groupe arylalkyle ; un groupe silyle ; un groupe bore ; un groupe amine ; un groupe aryle ; un groupe cycle fusionné d'hydrocarbures aromatiques et d'hydrocarbures aliphatiques ; et un groupe hétérocyclique, ou étant substitué par un substituant liant deux substituants ou plus parmi les substituants illustrés ci-dessus, ou ne présentant aucun substituant.

5. Composé selon la revendication 1, dans lequel des groupes adjacents parmi R1 à R4 se lient entre eux pour former un cycle représenté par la formule chimique B1-1 ou B1-2 suivante : 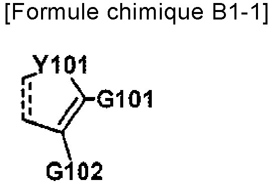 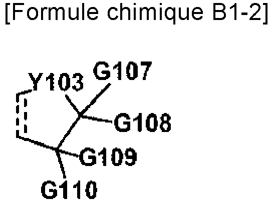 dans les formules chimiques B1-1 et B1-2,
Y101 et Y103 sont identiques ou différents l'un de l'autre, et chacun indépendamment O ; S ; ou NR‴ ;
R‴, G101, G102 et G107 à G110 sont identiques ou différents les uns des autres et chacun indépendamment de l'hydrogène ; du deutérium ; un groupe halogène ; un groupe cyano ; un groupe alkyle substitué ou non substitué ; un groupe halogénoalkyle substitué ou non substitué ; un groupe cycloalkyle substitué ou non substitué ; un groupe alcoxy substitué ou non substitué ; un groupe halogénoalcoxy substitué ou non substitué ; un groupe alkylthioxy substitué ou non substitué ; un groupe aryloxy substitué ou non substitué ; un groupe arylthioxy substitué ou non substitué ; un groupe alcényle substitué ou non substitué ; un groupe bore substitué ou non substitué ; un groupe amine substitué ou non substitué ; un groupe arylalkyle substitué ou non substitué ; un groupe silyle substitué ou non substitué ; un groupe cycle hydrocarboné substitué ou non substitué ; ou un groupe hétérocyclique substitué ou non substitué, ou se lient à des groupes adjacents pour former un cycle substitué ou non substitué ; 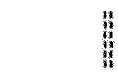
désigne un site se liant à la formule chimique 1, et
dans lequel "substitué ou non substitué" signifie étant substitué par un ou plusieurs substituants sélectionnés dans le groupe consistant en du deutérium ; un groupe halogène ; un groupe cyano ; un groupe alkyle ; un groupe cycloalkyle ; un groupe alcoxy ; un groupe aryloxy ; un groupe alkylthioxy ; un groupe arylthioxy ; un groupe alcényle ; un groupe halogénoalkyle ; un groupe halogénoalcoxy ; un groupe arylalkyle ; un groupe silyle ; un groupe bore ; un groupe amine ; un groupe aryle ; un groupe cycle fusionné d'hydrocarbures aromatiques et d'hydrocarbures aliphatiques ; et un groupe hétérocyclique, ou étant substitué par un substituant liant deux substituants ou plus parmi les substituants illustrés ci-dessus, ou ne présentant aucun substituant.

6. Composé selon la revendication 1, dans lequel des groupes adjacents parmi R1 à R4 se lient entre eux pour former un cycle représenté par l'une quelconque des formule chimiques B1-3 à B1-5 suivantes : 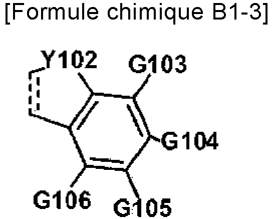 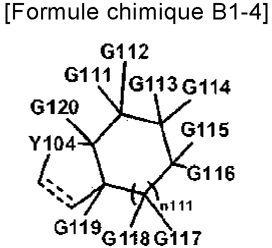 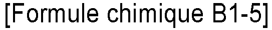 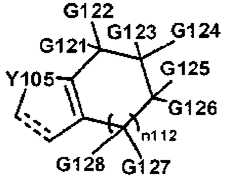 dans les formules chimiques B1-3 à B1-5,
Y102, Y104 et Y105 sont identiques ou différents les uns des autres, et chacun indépendamment O ; S ; ou NR‴ ;
R‴, G103 à G106 et G111 à G128 sont identiques ou différents les uns des autres et chacun indépendamment de l'hydrogène ; du deutérium ; un groupe halogène ; un groupe cyano ; un groupe alkyle substitué ou non substitué ; un groupe halogénoalkyle substitué ou non substitué ; un groupe cycloalkyle substitué ou non substitué ; un groupe alcoxy substitué ou non substitué ; un groupe halogénoalcoxy substitué ou non substitué ; un groupe alkylthioxy substitué ou non substitué ; un groupe aryloxy substitué ou non substitué ; un groupe arylthioxy substitué ou non substitué ; un groupe alcényle substitué ou non substitué ; un groupe bore substitué ou non substitué ; un groupe amine substitué ou non substitué ; un groupe arylalkyle substitué ou non substitué ; un groupe silyle substitué ou non substitué ; un groupe cycle hydrocarboné substitué ou non substitué ; ou un groupe hétérocyclique substitué ou non substitué, ou se lient à des groupes adjacents pour former un cycle substitué ou non substitué ;
n111 et n112 sont chacun un nombre entier de 0 à 2 ;
lorsque n111 et n112 valent 2, des structures dans les deux parenthèses sont identiques ou différentes l'une de l'autre ; 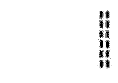
désigne un site se liant à la formule chimique 1, et
dans lequel "substitué ou non substitué" signifie étant substitué par un ou plusieurs substituants sélectionnés dans le groupe consistant en du deutérium ; un groupe halogène ; un groupe cyano ; un groupe alkyle ; un groupe cycloalkyle ; un groupe alcoxy ; un groupe aryloxy ; un groupe alkylthioxy ; un groupe arylthioxy ; un groupe alcényle ; un groupe halogénoalkyle ; un groupe halogénoalcoxy ; un groupe arylalkyle ; un groupe silyle ; un groupe bore ; un groupe amine ; un groupe aryle ; un groupe cycle fusionné d'hydrocarbures aromatiques et d'hydrocarbures aliphatiques ; et un groupe hétérocyclique, ou étant substitué par un substituant liant deux substituants ou plus parmi les substituants illustrés ci-dessus, ou ne présentant aucun substituant.

7. Composé selon la revendication 1, dans lequel R1 à R7 sont identiques ou différents les uns des autres, et chacun indépendamment de l'hydrogène ; du deutérium ; un groupe cyano ; un groupe alkyle linéaire ou ramifié présentant 1 à 30 atomes de carbone ; un groupe cycloalkyle monocyclique ou polycyclique présentant 3 à 30 atomes de carbone ; un groupe alkylsilyle linéaire ou ramifié présentant 1 à 30 atomes de carbone ; un groupe halogénoalkyle linéaire ou ramifié présentant 1 à 30 atomes de carbone ; un groupe halogénoalcoxy linéaire ou ramifié présentant 1 à 30 atomes de carbone ; un groupe arylalkyle présentant 6 à 30 atomes de carbone ; un groupe diarylamine monocyclique ou polycyclique présentant 6 à 30 atomes de carbone ; un groupe aryle monocyclique ou polycyclique présentant 6 à 30 atomes de carbone ; ou un groupe hétérocyclique monocyclique ou polycyclique présentant 2 à 30 atomes de carbone, et le substituant est non substitué ou substitué par un ou plusieurs éléments sélectionnés dans le groupe consistant en du deutérium, un groupe halogène, un groupe cyano, un groupe alkyle linéaire ou ramifié présentant 1 à 30 atomes de carbone, un groupe alkylsilyle linéaire ou ramifié présentant 1 à 30 atomes de carbone, un groupe aryle monocyclique ou polycyclique présentant 6 à 30 atomes de carbone, et des combinaisons de ceux-ci.

8. Composé selon la revendication 1, dans lequel des groupes adjacents parmi R1 à R4 se lient entre eux pour former un cycle hydrocarboné monocyclique ou polycyclique substitué ou non substitué présentant 3 à 30 atomes de carbone ; ou un hétérocycle monocyclique ou polycyclique substitué ou non substitué présentant 2 à 30 atomes de carbone, et
dans lequel "substitué ou non substitué" signifie étant substitué par un ou plusieurs substituants sélectionnés dans le groupe consistant en du deutérium ; un groupe halogène ; un groupe cyano ; un groupe alkyle ; un groupe cycloalkyle ; un groupe alcoxy ; un groupe aryloxy ; un groupe alkylthioxy ; un groupe arylthioxy ; un groupe alcényle ; un groupe halogénoalkyle ; un groupe halogénoalcoxy ; un groupe arylalkyle ; un groupe silyle ; un groupe bore ; un groupe amine ; un groupe aryle ; un groupe cycle fusionné d'hydrocarbures aromatiques et d'hydrocarbures aliphatiques ; et un groupe hétérocyclique, ou étant substitué par un substituant liant deux substituants ou plus parmi les substituants illustrés ci-dessus, ou ne présentant aucun substituant.

9. Composé selon la revendication 1, dans lequel R' et R" sont identiques ou différents l'un de l'autre, et chacun indépendamment un groupe alkyle linéaire ou ramifié présentant 1 à 30 atomes de carbone ; un groupe arylalkyle présentant 6 à 30 atomes de carbone ; un cycle hydrocarboné monocyclique ou polycyclique présentant 3 à 30 atomes de carbone ; ou un groupe hétérocyclique monocyclique ou polycyclique présentant 2 à 30 atomes de carbone, et le substituant est non substitué ou substitué par un ou plusieurs éléments sélectionnés dans le groupe consistant en du deutérium, un groupe halogène, un groupe cyano, un groupe alkyle linéaire ou ramifié présentant 1 à 30 atomes de carbone, un groupe cycloalkyle monocyclique ou polycyclique présentant 3 à 30 atomes de carbone, un groupe alkylsilyle linéaire ou ramifié présentant 1 à 30 atomes de carbone, un groupe arylsilyle présentant 3 à 30 atomes de carbone, un groupe alcoxy linéaire ou ramifié présentant 1 à 30 atomes de carbone, un groupe halogénoalkyle linéaire ou ramifié présentant 1 à 30 atomes de carbone, un groupe halogénoalcoxy linéaire ou ramifié présentant 1 à 30 atomes de carbone, un groupe aryloxy présentant 6 à 30 atomes de carbone, un groupe arylalkyle monocyclique ou polycyclique présentant 6 à 30 atomes de carbone, un groupe diarylamine monocyclique ou polycyclique présentant 6 à 30 atomes de carbone, un groupe aryle monocyclique ou polycyclique présentant 6 à 30 atomes de carbone, un groupe hétérocyclique monocyclique ou polycyclique présentant 2 à 30 atomes de carbone, et des combinaisons de ceux-ci.

10. Composé selon la revendication 1, dans lequel la formule chimique 1 est l'un quelconque sélectionné parmi les composés suivants : 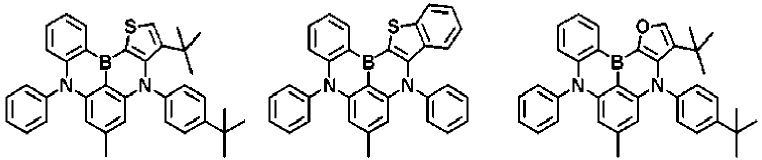 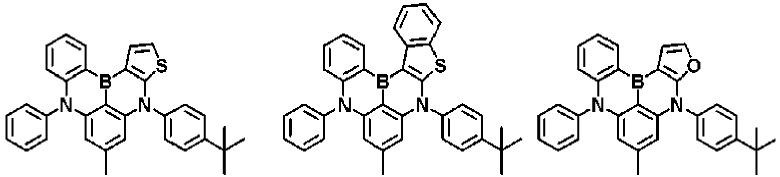 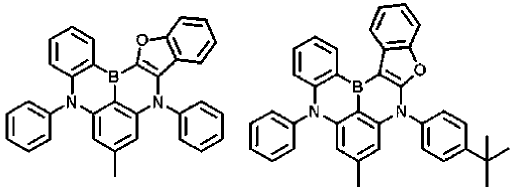 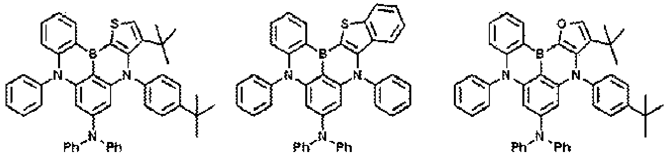 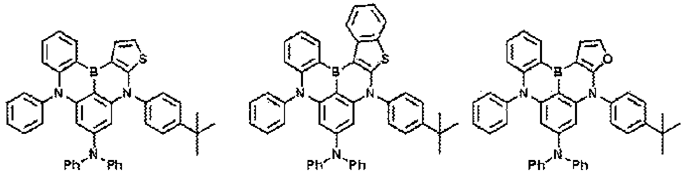 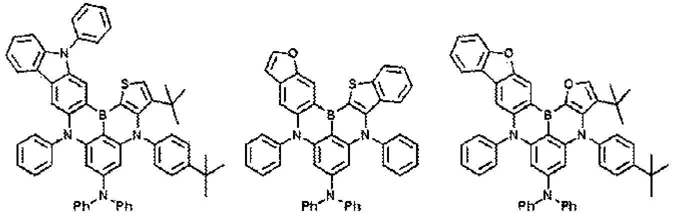 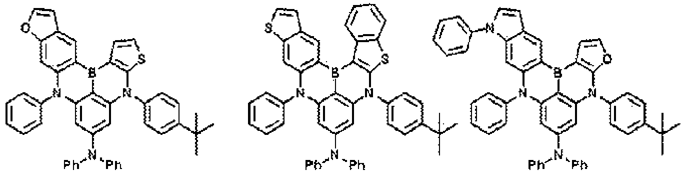 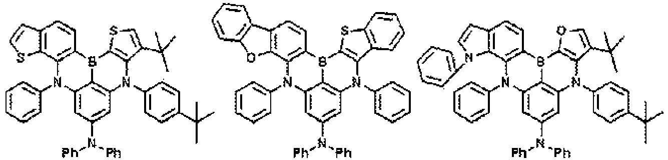 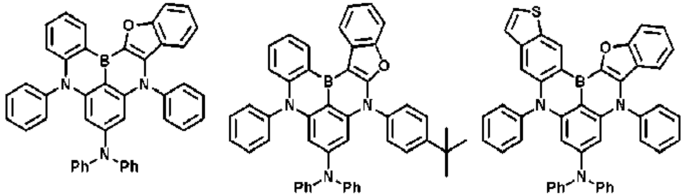 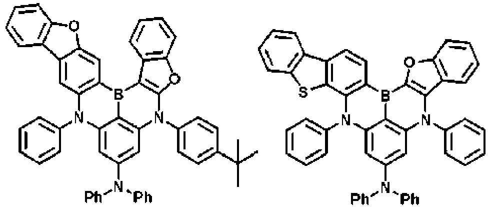 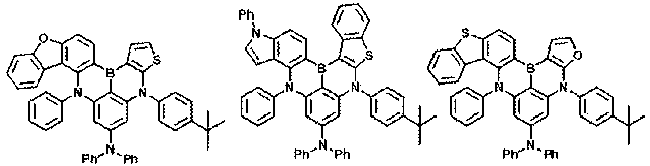 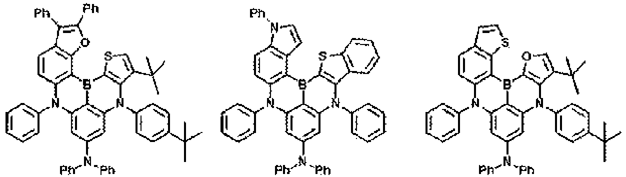 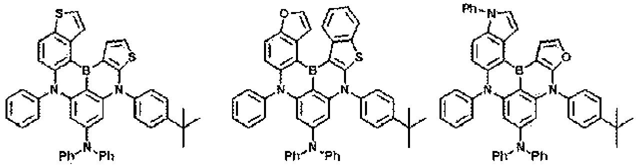 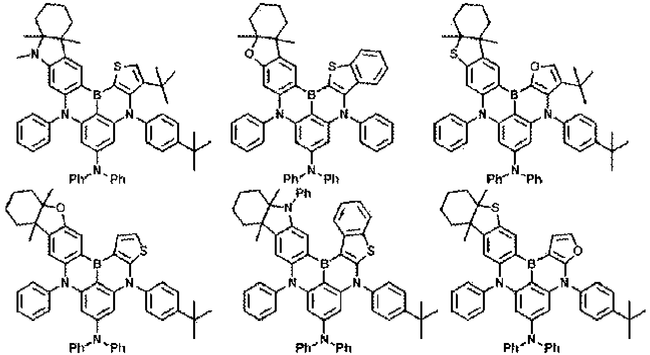 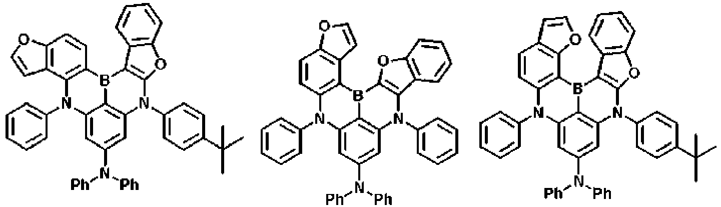 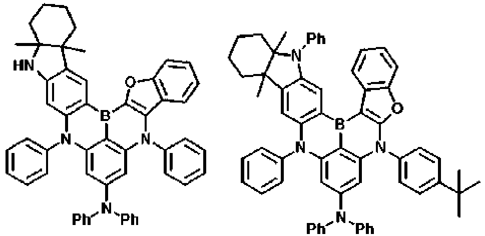 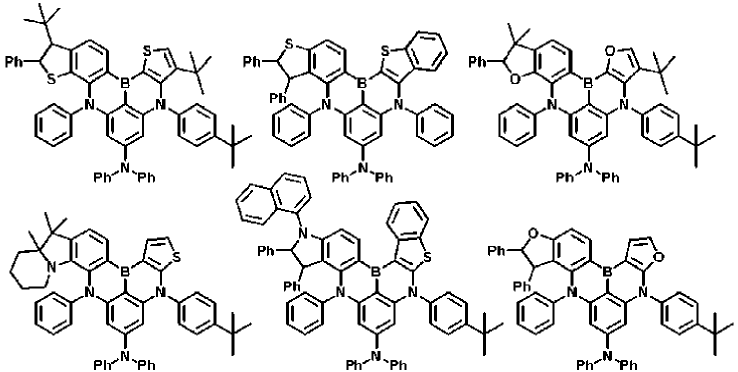 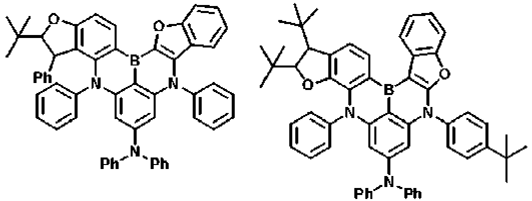 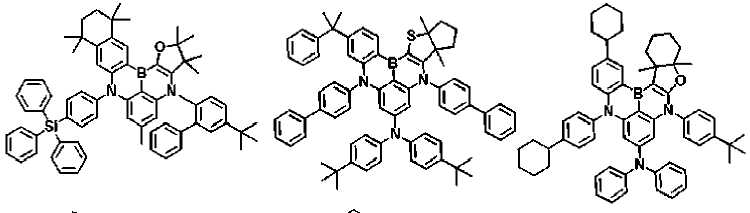 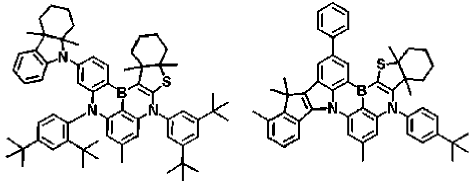 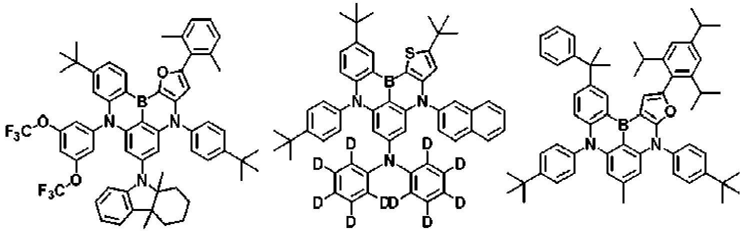 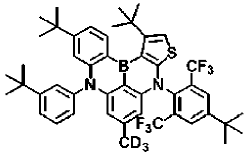 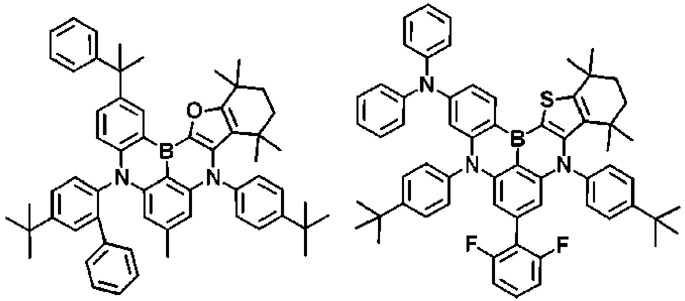 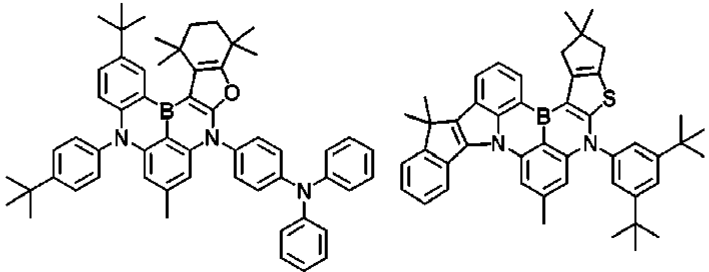 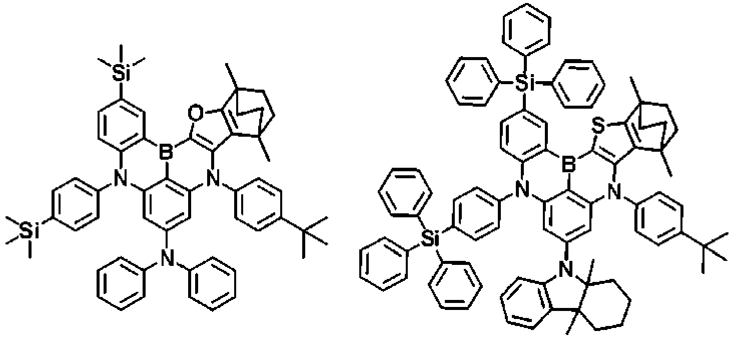 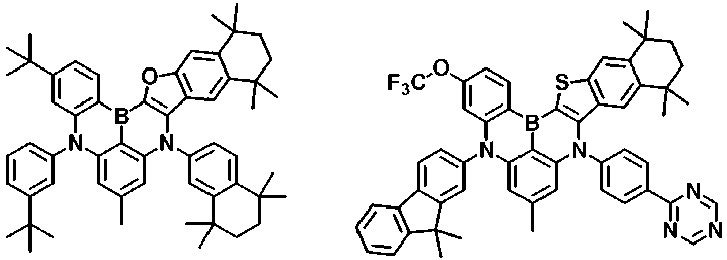 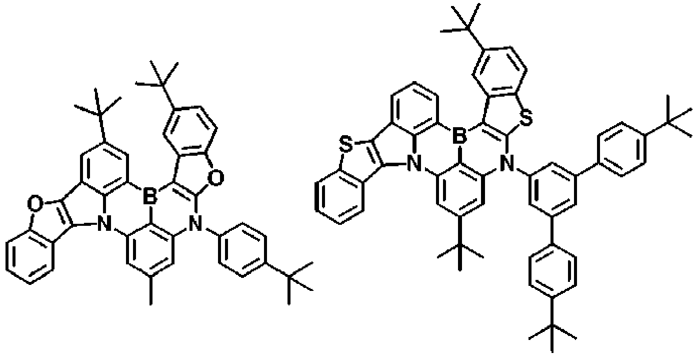 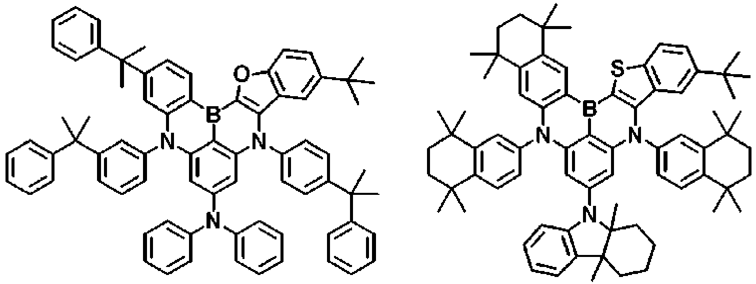 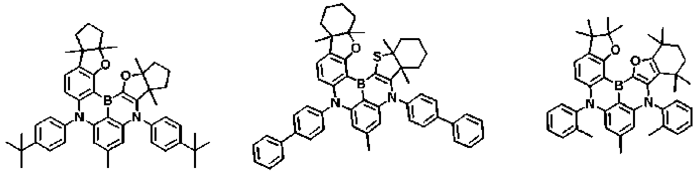 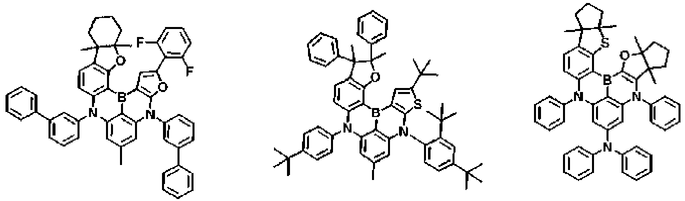 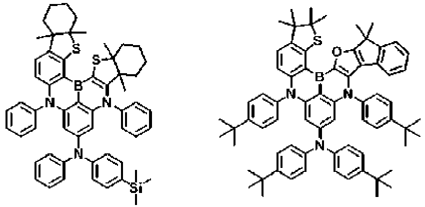 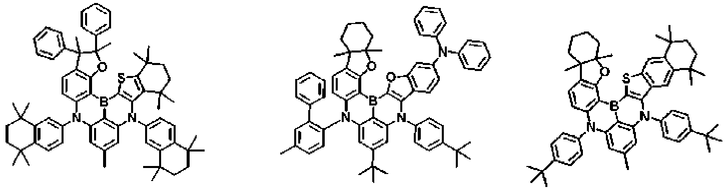 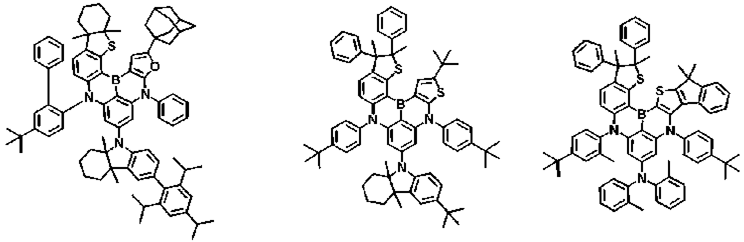 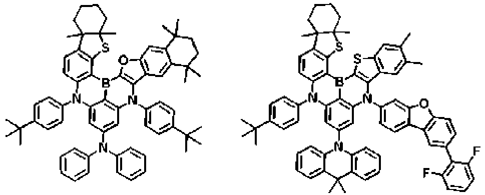 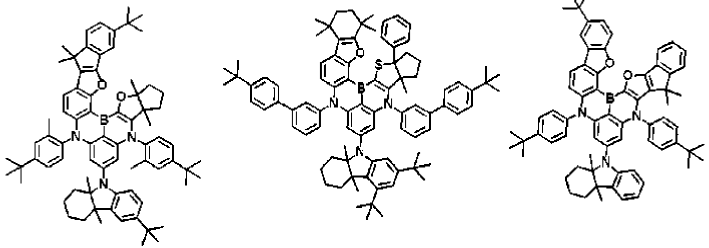 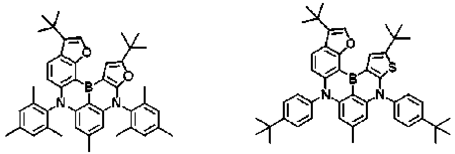 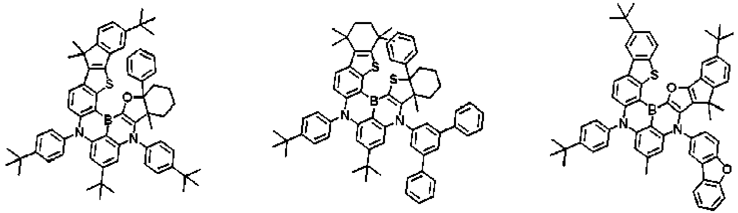 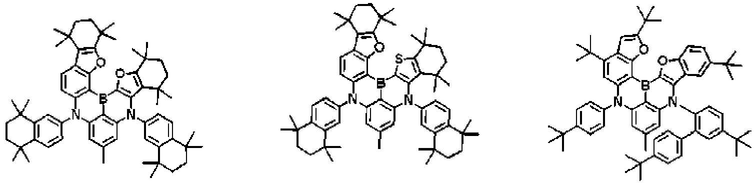 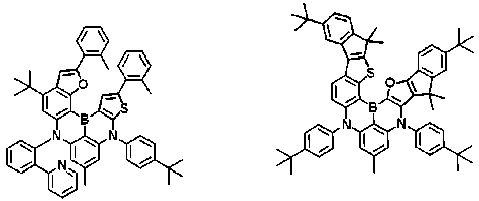 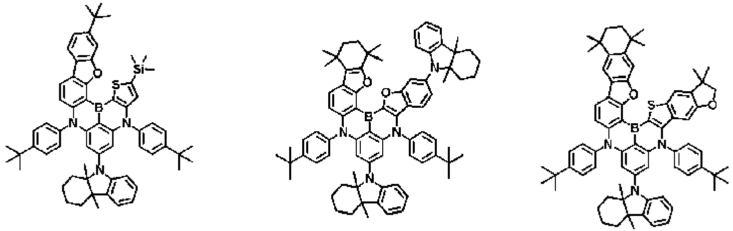 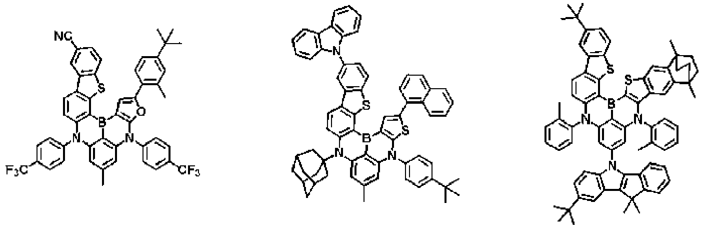 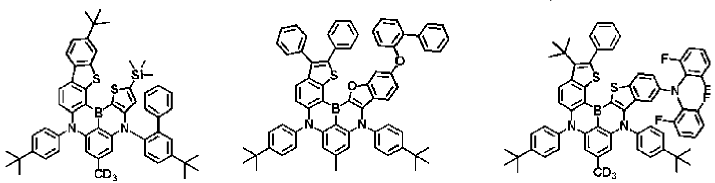 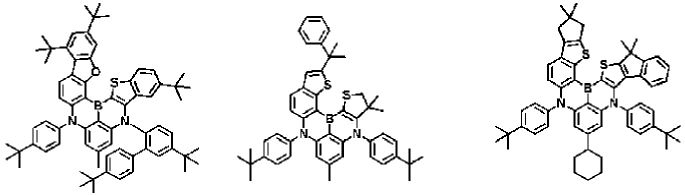 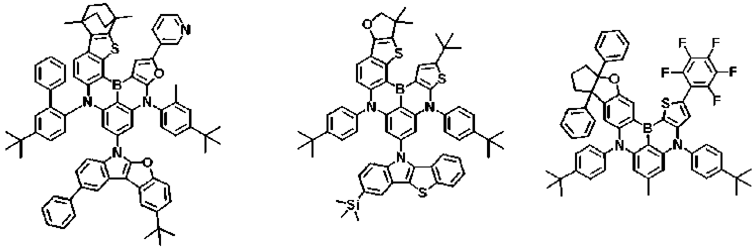 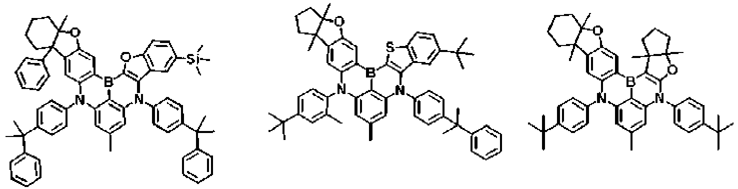 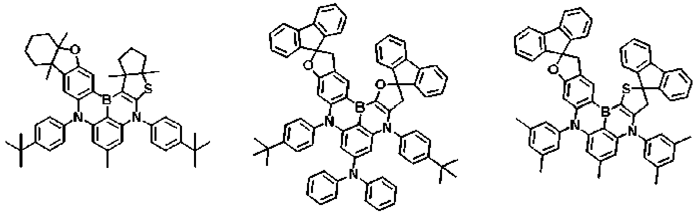 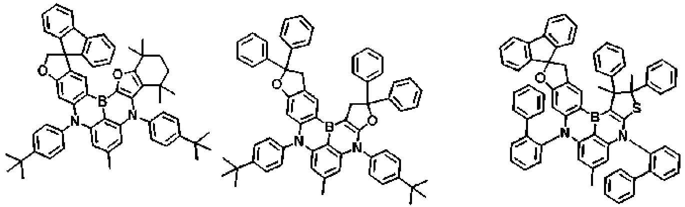 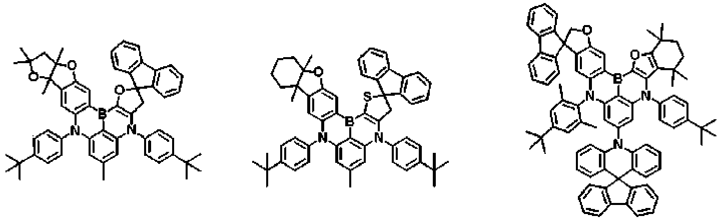 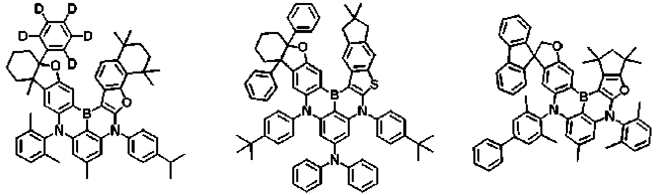 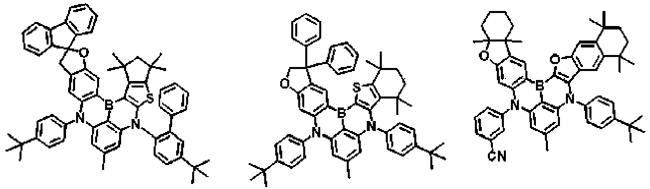 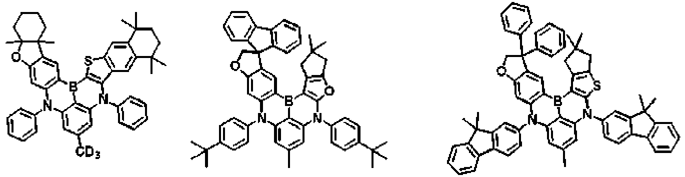 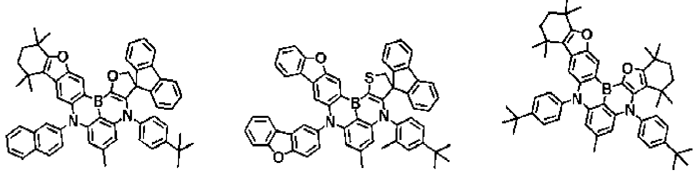 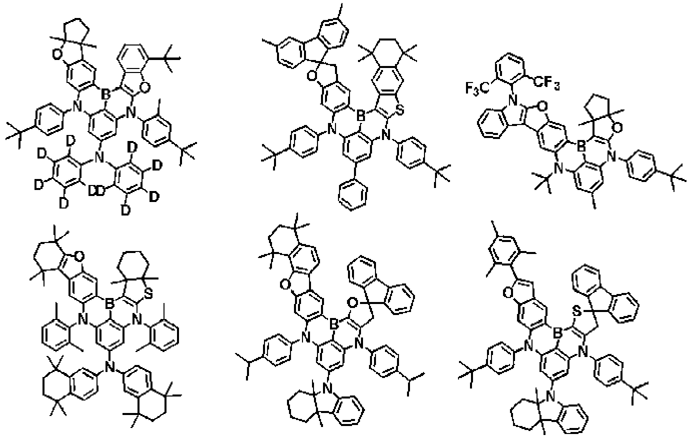 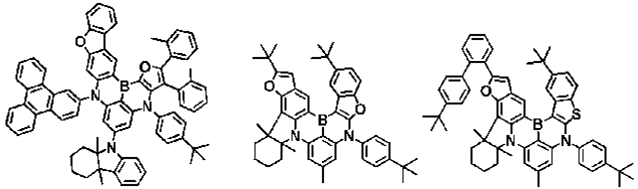 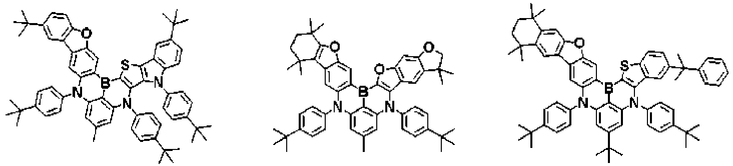 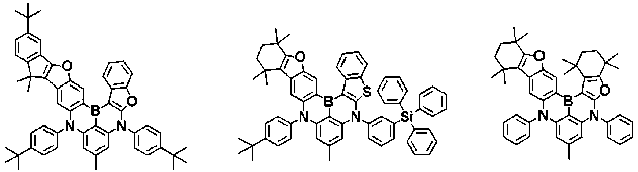 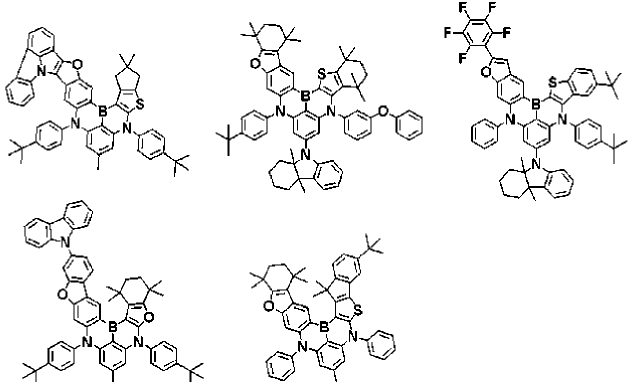 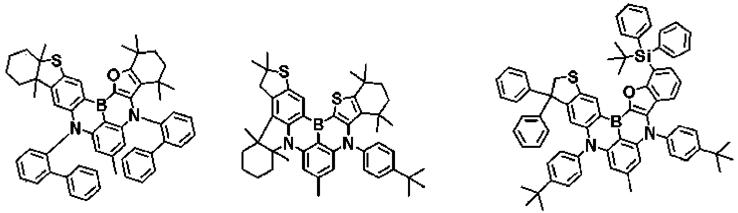 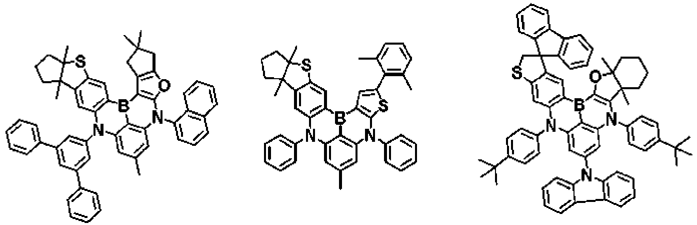 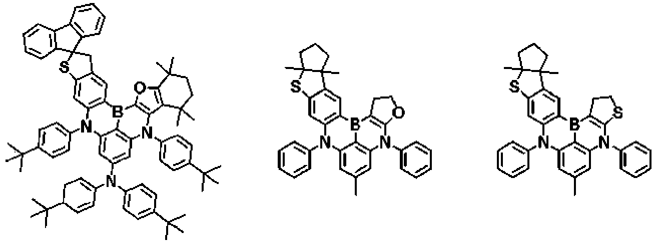 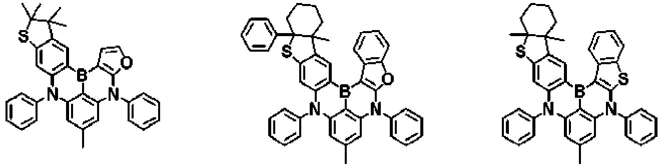 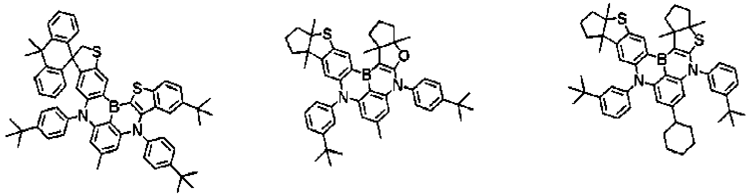 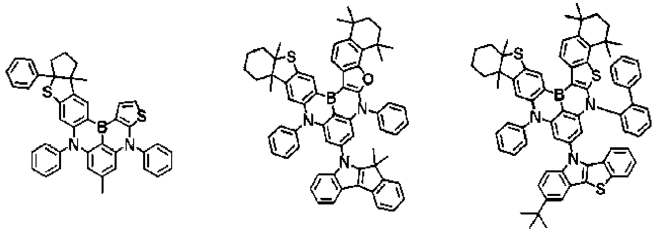 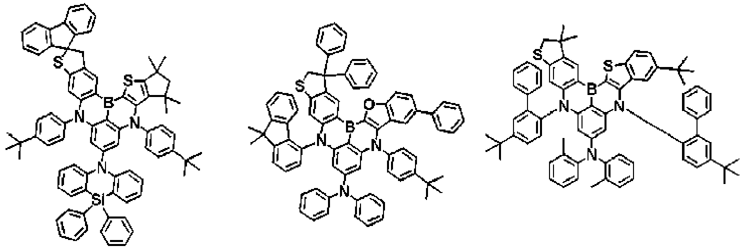 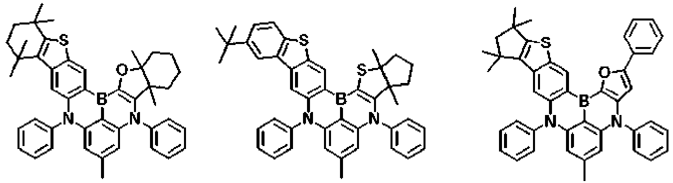 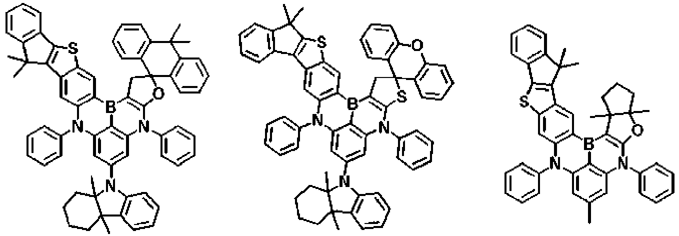 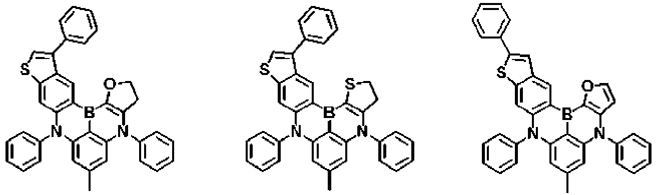 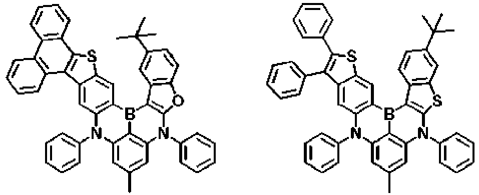 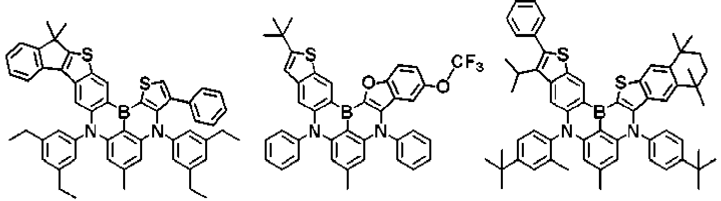 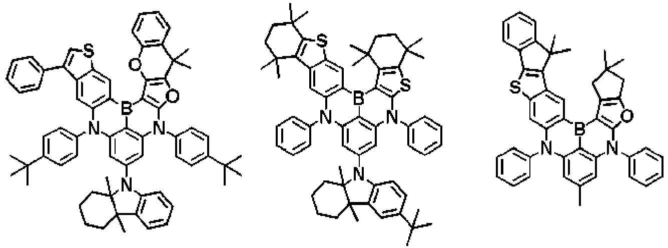 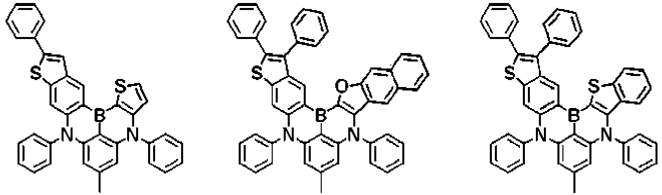 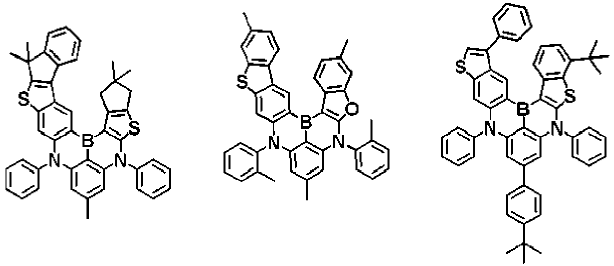 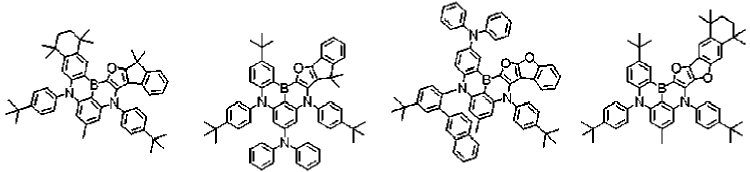 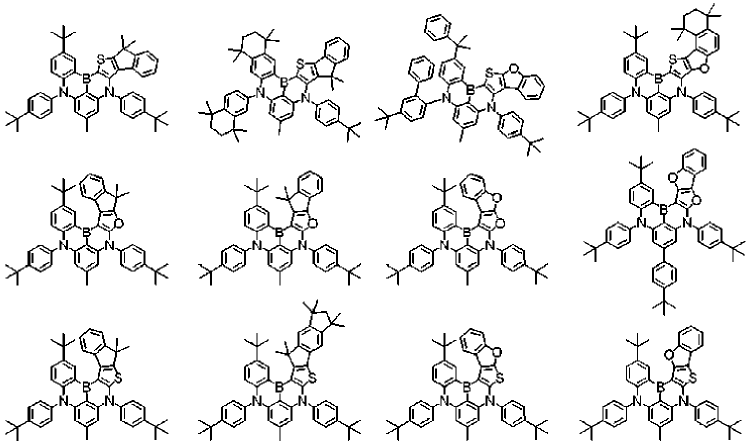 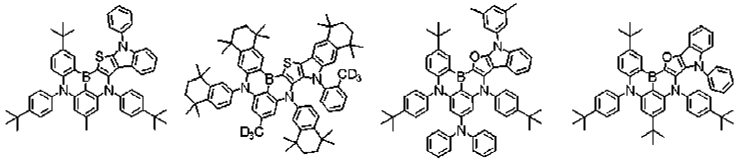 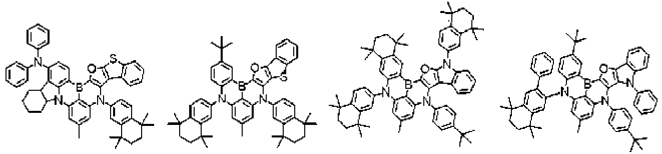 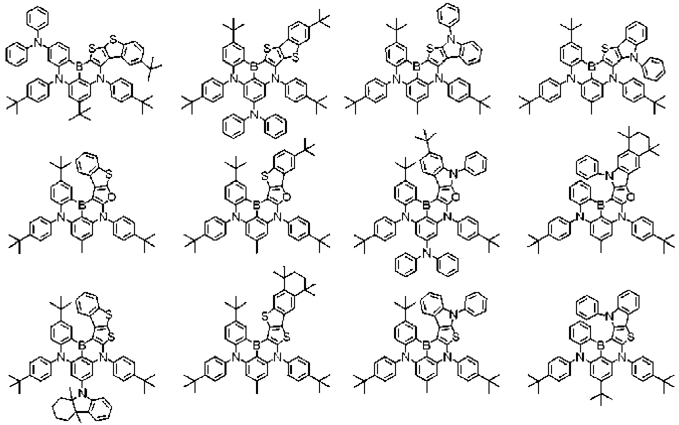 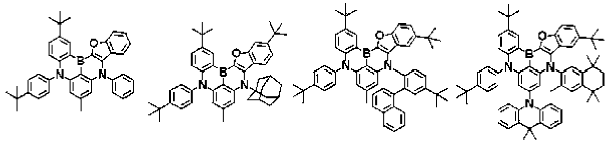 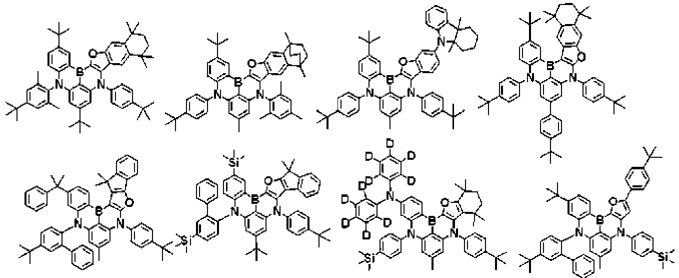 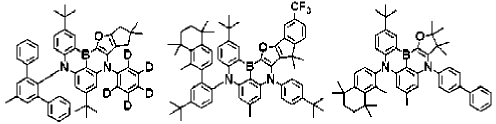 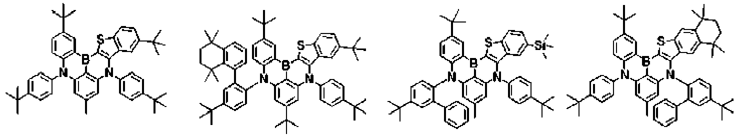 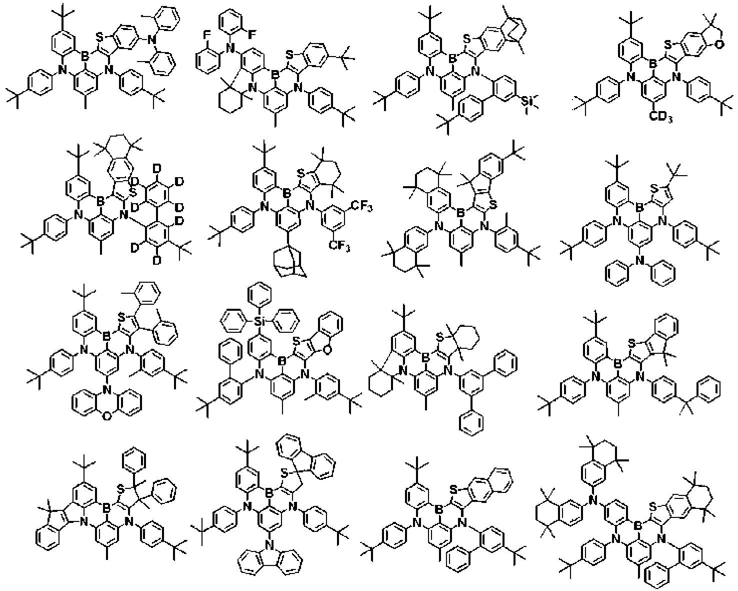 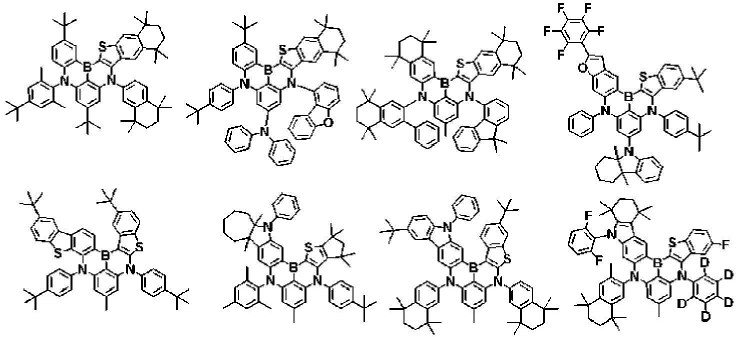 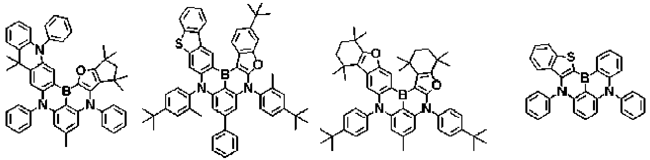 dans les composés,
Ph désigne un groupe phényle.

11. Dispositif électroluminescent organique comprenant :
une première électrode ;
une seconde électrode ; et
une ou plusieurs couches de matériau organique disposées entre la première électrode et la seconde électrode,
dans lequel une ou plusieurs couches des couches de matériau organique incluent le composé selon l'une quelconque des revendications 1 à 10.

12. Dispositif électroluminescent organique selon la revendication 11, dans lequel la couche de matériau organique inclut une couche électroluminescente, et la couche électroluminescente inclut le composé.

13. Dispositif électroluminescent organique selon la revendication 11, dans lequel la couche de matériau organique inclut une couche électroluminescente, la couche électroluminescente inclut un matériau dopant, et le matériau dopant inclut le composé.

14. Dispositif électroluminescent organique selon la revendication 11, dans lequel la couche de matériau organique inclut une couche électroluminescente, et la couche électroluminescente inclut en outre un composé représenté par la formule chimique H suivante : 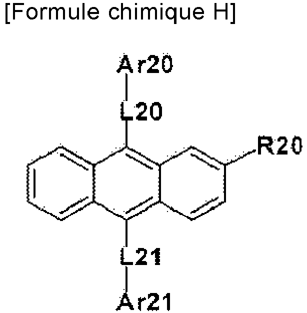 dans la formule chimique H,
L20 et L21 sont identiques ou différents l'un de l'autre, et chacun indépendamment une liaison directe ; un groupe arylène substitué ou non substitué ; ou un groupe hétéroarylène substitué ou non substitué ;
Ar20 et Ar21 sont identiques ou différents l'un de l'autre, et chacun indépendamment de l'hydrogène ; du deutérium ; un groupe aryle substitué ou non substitué ; ou un groupe hétérocyclique substitué ou non substitué ;
R20 est de l'hydrogène ; du deutérium ; un groupe halogène ; un groupe alkyle substitué ou non substitué ; un groupe cycloalkyle substitué ou non substitué ; un groupe aryle substitué ou non substitué ; ou un groupe hétérocyclique substitué ou non substitué, et
dans lequel "substitué ou non substitué" signifie étant substitué par un ou plusieurs substituants sélectionnés dans le groupe consistant en du deutérium ; un groupe halogène ; un groupe cyano ; un groupe alkyle ; un groupe cycloalkyle ; un groupe alcoxy ; un groupe aryloxy ; un groupe alkylthioxy ; un groupe arylthioxy ; un groupe alcényle ; un groupe halogénoalkyle ; un groupe halogénoalcoxy ; un groupe arylalkyle ; un groupe silyle ; un groupe bore ; un groupe amine ; un groupe aryle ; un groupe cycle fusionné d'hydrocarbures aromatiques et d'hydrocarbures aliphatiques ; et un groupe hétérocyclique, ou étant substitué par un substituant liant deux substituants ou plus parmi les substituants illustrés ci-dessus, ou ne présentant aucun substituant.

15. Dispositif électroluminescent organique selon la revendication 11, dans lequel la couche de matériau organique inclut une couche électroluminescente, et la couche électroluminescente inclut un ou plusieurs types d'hôtes et un dopant.
